(19) Europäisches Patentamt
European Patent Office
Office européen des brevets

(11) EP 3 959 200 B1

(12) **EUROPEAN PATENT SPECIFICATION**

(45) Date of publication and mention of the grant of the patent:
**20.03.2024 Bulletin 2024/12**

(21) Application number: **21806123.2**

(22) Date of filing: **11.01.2021**

(51) International Patent Classification (IPC):
***C07D 249/16*** *(2006.01)* ***C07D 249/04*** *(2006.01)*
***A61P 35/00*** *(2006.01)* ***A61K 31/4196*** *(2006.01)*
***C07D 417/14*** *(2006.01)* ***C07D 513/04*** *(2006.01)*

(52) Cooperative Patent Classification (CPC):
**A61P 35/00; C07D 417/14; C07D 513/04**

(86) International application number:
**PCT/TR2021/050013**

(87) International publication number:
**WO 2022/005414 (06.01.2022 Gazette 2022/01)**

(54) **NEW TRIAZOLE AND TRIAZOLOTHIADIAZINE DERIVATIVES EXERTING CYTOTOXIC AND APOPTOTIC EFFECTS ON A549 CELLS THROUGH AKT INHIBITION**

NEUE TRIAZOL- UND TRIAZOLOTHIADIAZINDERIVATE MIT CYTOTOXISCHEN UND APOPTOTISCHEN WIRKUNGEN AUF A549-ZELLEN DURCH AKT-HEMMUNG

NOUVEAUX DÉRIVÉS DE TRIAZOLE ET DE TRIAZOLOTHIADIAZINE EXERÇANT DES EFFETS CYTOTOXIQUES ET APOPTOTIQUES SUR DES CELLULES A549 PAR INHIBITION D’AKT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2020 TR 202010296**

(43) Date of publication of application:
**02.03.2022 Bulletin 2022/09**

(73) Proprietor: **Anadolu Universitesi**
**Tepebasi/Eskisehir (TR)**

(72) Inventors:
- **YILMAZ, Nalan**
  **Tepebasi/Eskisehir (TR)**
- **ALTINTOP, Mehlika Dilek**
  **Tepebasi/Eskisehir (TR)**
- **AKALIN CIFTCI, Gulsen**
  **Tepebasi/Eskisehir (TR)**
- **SEVER, Belgin**
  **Tepebasi/Eskisehir (TR)**

(74) Representative: **Sevinç, Cenk**
**Grup Ofis Marka Patent A.S.**
**Ataturk Bulvari 211/11**
**Kavaklidere**
**06680 Ankara (TR)**

(56) References cited:
**WO-A1-2008/036272**

- **Khan Imtiaz; Zaib Sumera; Ibrar Aliya; Rama Nasim Hasan; Simpson Jim; Iqbal Jamshed: "Synthesis, crystal structure and biological evaluation of some novel 1,2,4-triazolo[3,4-b]-1,3,4-thiadiazoles and 1,2,4-triazolo[3,4-b]-1,3,4-thiadiazines", EUROPEAN JOURNAL OF MEDICINAL CHEMISTRY, ELSEVIER, AMSTERDAM, NL, vol. 78, 19 March 2014 (2014-03-19), pages 167-177, XP028847854, AMSTERDAM, NL ISSN: 0223-5234, DOI: 10.1016/j.ejmech.2014.03.046**
- **Rashid Mohd; Husain Asif; Mishra Ravinesh; Karim Shahid; Khan Shamshir; Ahmad Makhmur; Al-wabel Naser; Husain Amjad; Ahmad Aftab;: "Design and synthesis of benzimidazoles containing substituted oxadiazole, thiadiazole and triazolo-thiadiazines as a source of new anticancer agents", ARABIAN JOURNAL OF CHEMISTRY, ELSEVIER, AMSTERDAM, NL, vol. 12, no. 8, 28 August 2015 (2015-08-28), pages 3202-3224, XP085972133, AMSTERDAM, NL ISSN: 1878-5352, DOI: 10.1016/j.arabjc.2015.08.019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 959 200 B1

• Alt?ntop Mehlika Dilek, Kaplanc?kl? Zafer As?m, Turan-Zitouni Gülhan, Özdemir Ahmet, Demirci Fatih, I??can Gökalp, Revial Gilbert: "Synthesis of Some Novel Triazole Derivatives and Investigation of Their Antimicrobial Activities", SYNTHETIC COMMUNICATIONS, Taylor & Francis Inc., US, vol. 41, no. 15, 1 August 2011 (2011-08-01), pages 2234-2250, XP055895685, US ISSN: 0039-7911, DOI: 10.1080/00397911.2010.501475

## Description

### Technical Field of the Invention

The invention is related to 1,2,4-triazole and 1,2,4-triazolo[3,4-*b*]-1,3,4-thiadiazine derivatives showing selective and potent cytotoxic effects and apoptosis-inducing effects in A549 cells by inhibiting the Akt enzyme, which is one of the target enzymes for the treatment of lung cancer and synthesis, *in vitro* anticancer activity studies, molecular docking and *in silico* Absorption, Distribution, Metabolism and Excretion (ADME) studies of these compounds.

The compounds of the invention are used in the preparation of medicaments for the treatment of lung cancer.

### Known State of the Invention (Prior Art)

Lung cancer is a very invasive, rapidly metastasizing type of cancer with a high prevalence. Lung cancer is the leading cause of cancer-related deaths worldwide. Despite improvements in combinatorial therapy, including chemotherapy and radiotherapy, the recovery rate of the patients has not reached the desired level.

The Akt enzyme is overexpressed or activated in various types of human cancers, including lung, breast, ovarian, gastric and pancreatic carcinomas. Therefore, Akt inhibitors have gained great importance for anti-cancer drug design and development. However, although many Akt inhibitors have been identified so far, no Akt inhibitor has yet passed the clinical trial stages and has not been presented to treatment as a drug. Accordingly, in recent years, researchers in the field of medicinal chemistry have turned to the discovery of Akt inhibitors as new anticancer agents.

In recent years, 1,2,4-triazoles and 1,2,4-triazolo[3,4-*b*]-1,3,4-thiadiazines have attracted attention in anticancer drug design and development studies. However, due to the limited number of targeted anticancer studies conducted on these ring systems, it has become important to design and develop new triazole and triazolothiadiazine derivatives with high potential to be anti-cancer drug acting on different target enzymes.

Many triazole derivatives effective on various types of cancer known in the art, synthesis and anticancer activity studies of these compounds are listed below:
(Duran, A. et al., 2002, Il Farmaco, 57, 559-564) synthesized 1,4-dihydro-3-(3-hydroxy-2-naphthyl)-4-substituted-5*H*-1,2,4-triazoline-5-thione derivatives and investigated the anticancer activities of these compounds. The cytotoxic activity of 1,4-dihydro-3-(3-benzoyloxy-2-naphthyl)-4-ethyl-*5*H-1,2,4-triazoline-5-thione against 52 human cancer cell lines was found promising for further studies.

(Shivarama Holla, B. et al., 2003, Eur. J. Med. Chem., 38, 759-767) synthesized 3-substituted-4-[5-(4-methoxy-2-nitrophenyl)-2-furfurylidene]amino-5-mercapto-1,2,4-triazole derivatives and examined the anticancer activities of these compounds against 60 cell lines derived from 7 cancer types (lung, colon, skin, kidney, ovarian, central nervous system and blood cancer). Although some compounds were found to be more active than others, the compounds generally showed low activity in the range of 10-100 $\mu$M.

(Sunil, D. et al., 2011, Med. Chem. Res., 20, 1024-1032) synthesized 5-substituted-4-amino-3-mercapto-1,2,4-triazole derivatives. They investigated the cytotoxic effects of some of these compounds against human hepatocellular carcinoma (HCC) cell line (HepG2) by MTT method. Compared to the standard drug doxorubicin, the most active compounds were found to be 4-((3-(4-chlorophenyl)-1*H*-pyrazole-4-methylene)amino)-5-(benzyloxy)-2,4-dihydro-1,2,4-triazole-3-thione ($IC_{50}$= 0.018 $\mu$M) and 4-((3-(4-fluorophenyl)-1H-pyrazole-4-methylene)amino)-2-(morpholinomethyl)-5-(4-methylbenzyloxy)-2,4-dihydro-1,2,4-triazole-3-thione ($IC_{50}$= 0.034 $\mu$M) .

(El-Sayed, W.A. et al., 2012, Der PharmaChemica, 4/1, 23-32) synthesized 1,2,4-triazole derivatives carrying pyrazole ring and investigated the cytotoxic effects of these derivatives on human breast cancer (MCF-7) and human cervical carcinoma (HeLa) cell lines. 3-((5-(1-(4-Methoxyphenyl)-5-phenyl-1*H*-pyrazole-3-yl)-4-phenyl-4*H*-1,2,4-triazole-3-yl)thio)propane-1-ol showed better anticancer activity against MCF-7 ($IC_{50}$= 2.72 $\mu$M) and HeLa ($IC_{50}$= 2.98 $\mu$M) cells compared to the standard drug doxorubicin ($IC_{50}$= 6.71 $\mu$M and 8.72 $\mu$M, respectively).

(Singha, T. et al., 2012, Ind. J. Pharm. Educ. Res., 46, 346-351) synthesized 1,2,4-triazole derivatives and compared these compounds (25 mg/kg, body weight) against Ehrlich acid tumor (EAC) with the standard drug 5-fluorouracil (20 mg/kg, body weight). The tumor cell inhibition (TCI) of 4-amino-5-mercapto-3-(2-chlorophenyl)-1,2,4-triazole, which showed the highest cytotoxic activity among the synthesized compounds, was determined as 63.59%.

(Hassan, G.S. et al., 2013, Eur. J. Med. Chem., 66, 135-145) synthesized compounds containing 5-(2-aminothiazole-4-yl)-4-phenyl-4*H*-1,2,4-triazole-3-thiol and investigated the antitumor effects of these compounds on human HCC (HepG2), breast adenocarcinoma (MCF-7), colon carcinoma (HCT116) and lung carcinoma (A549) cell lines by MTT method. 1-[4-(5-Mercapto-4-(4-phenoxyphenyl-4*H*-1,2,4-triazole-3-yl)thiazole-2-yl]-3-(4-phenoxyphenyl)thiourea, showed antitumor activity against HCT116 ($LC_{50}$= 23.5 $\mu$M) and MCF-7 ($LC_{50}$= 17.4 $\mu$M) cell lines comparable to doxorubicin ($LC_{50}$= 37.7 $\mu$M and 26.1 $\mu$M, respectively).

(Sumangala, V. et al., 2013, Med. Chem. Res., 22, 2921-2928) synthesized 5-[4-(methylsulfonyl)benzyl]-4-((arylmethylene)amino)-4*H*-1,2,4-triazole-3-thione derivatives and investigated the cytotoxicity of these derivatives

against human epithelial cervical carcinoma (HeLa) cells by MTT method. When compared to the standard drug doxorubicin ($EC_{50}$= 2.5 μM), 4-{ [(2-chloro-6-fluorophenyl)methylene]amino}-5-[4-(methylsulfonyl)benzyl]-2,4-dihydro-3*H*-1,2,4-triazole-3-thione ($EC_{50}$= 25 μM) showed better activity than other compounds.

(Cihan-Üstündağ, G. et al., 2014, Lett. Drug Des. Discov., 11, 290-296) synthesized 4,5-disubstituted-1,2,4-triazole-3-thione derivatives. 4-Benzyl-3-(benzylthio)-5-[hydroxy(phenyl)methyl]-4*H*-1,2,4-triazole showed antiproliferative activity in the range of 25-58% against leukemia (CCRF-CEM, RPMI-8226 and SR), non-small cell lung cancer (HOP-92), melanoma (UACC-62) and kidney cancer (UO-31) cells.

(Çıkla-Süzgün, P. et al., 2015, J. Enzyme Inhib. Med. Chem., 30 (5), 778-785) synthesized etodolac derivatives carrying 1,2,4-triazole ring and investigated the anticancer activities of these derivatives against human liver cancer cell lines (Huh7, Mahlavu, HepG2, FOCUS). 5-[(1,8-Diethyl-1,3,4,9-tetrahydropyrano[3,4-*b*]indole-1-yl)methyl]-4-methyl-2,4-dihydro-3*H*-1,2,4-triazole-3-thione showed significant anti-cancer activity against Huh7 cell line with an $IC_{50}$ value of 4.29 μM.

(Zhao, P.L. et al., 2016, Bioorg. Med. Chem. Lett., 26, 3679-3683) synthesized 3-alkylsulfanyl-4-amino-1,2,4-triazole derivatives and evaluated the antiproliferative effects of these compounds against human liver (HepG2), colon (HCT116), prostate (PC-3) and cervical (HeLa) cancer cells. When compared to 5-fluorouracil ($IC_{50}$= 68.71 μM for HCT116 cell line, $IC_{50}$= 57.17 μM for HeLa cell line and $IC_{50}$= 57.04 μM for PC-3 cell line), 2-(4-amino-5-(3,4,5-trimethoxyphenyl)-4*H*-1,2,4-triazole-3-ylthio)-1-(*p*-tolyl)ethanone showed strong and selective antiproliferative activity against HCT116, HeLa and PC-3 cells with $IC_{50}$ values of 0.37 μM, 2.94 μM and 31.31 μM, respectively. This compound induced apoptosis and blocked the cell cycle in the G2/M phase in a dose dependent manner in HeLa cells.

(Kulabas, N. et al., 2016, Eur. J. Med. Chem., 121, 58-70) synthesized thiosemicarbazide, 1,2,4-triazole-3-thione and 2-(4H-1,2,4-triazole-3-ylthio)acetamide derivatives. Among these compounds, three compounds were selected by the National Cancer Institute and their antiproliferative activities against 60 human cancer cell lines were investigated. 2-{ [3-[[5-Methyl-2-(propane-2-yl)phenoxy]methyl)-4-benzyl-4,5-dihydro-1*H*-1,2,4-triazole-5-yl]sulfanyl}-*N*-(4-sulfamoilphenyl)acetamide showed anticancer activity against PC-3 cells with an $IC_{50}$ value of 5.96 μM, 2-{[3-[[5-methyl-2-(propane-2-yl)phenoxy]methyl]-4-benzyl-4,5-dihydro-1*H*-1,2,4-triazole-5-yl]sulfanyl}-*N*-(4-chlorophenyl)acetamide showed anticancer activity against A549 with an $IC_{50}$ value of 7.90 μM, 2-[(3-{[4-(acetylamino)phenoxy]methyl}-4-benzyl-4,5-dihydro-1H-1,2,4-triazole-5-yl)sulfanyl]-*N*-(4-bromophenyl)acetamide showed anticancer activity against K562 cells with an $IC_{50}$ value of 7.71 μM. These compounds caused a significant increase in dose-dependent caspase-3 activity. 2-{ [3-[[5-Methyl-2-(propane-2-yl)phenoxy]methyl]-4-benzyl-4,5-dihydro-1*H*-1,2,4-triazole-5-yl]sulfanyl}-*N*-(4-chlorophenyl)acetamide provided a significant increase in caspase-8 activity at a dose of 50 μM. These compounds significantly reduced mitochondrial membrane potential and Bcl-2 expression.

(Milošev, M.Z. et al., 2017, Chem. Biol. Drug Des., 89, 943-952) synthesized Mannich bases from 5-(adamantan-1-yl)-2,4-dihydro-3*H*-1,2,4-triazole-3-thione and evaluated the cytotoxic effects of these compounds on human cervical adenocarcinoma (HeLa), human chronic myeloid leukemia (K562), human acute promyelocytic leukemia (HL-60), human lung adenocarcinoma (A549) and normal human lung fibroblast (MRC-5) cells. When compared to cisplatin ($IC_{50}$= 5.45±0.18 μM), 5-(adamantan-1-yl)-2-[(*o*-tolylamino)methyl]-2,4-dihydro-3*H*-1,2,4-triazole-3-thione, 5-(adamantan-1-yl)-2-[((2-fluorophenyl)amino)methyl]-2,4-dihydro-3*H*-1,2,4-triazole-3-thione and 5-(adamantan-1-yl)-2-[((2-chlorophenyl)amino)methyl]-2,4-dihydro-3*H*-1,2,4-triazole-3-thione, showed cytotoxic activity against K562 cell line with $IC_{50}$ values of 25.42±0.89 μM, 27.71±2.46 μM and 27.05±5.35 μM, respectively. These compounds caused cell cycle arrest in subG1 and G1 phases in K562 cells. It was found that these compounds exhibited their cytotoxic effects on K562 cells by inducing caspase-dependent apoptosis and increasing Bax expression. These compounds showed anti-angiogenic effects on EA.hy926 human endothelial cells.

Many triazolothiadiazine derivatives effective against various types of cancer known in the art and the synthesis and anticancer activity studies of these compounds are listed below:
(Shivarama Holla, B. et al., 2001, Il Farmaco, 56, 565-570) synthesized 7-arylidene-6-(2,4-dichloro-5-fluorophenyl)-3-substituted-1,2,4-triazolo[3,4-*b*]-1,3,4-thiadiazine derivatives and investigated the anticancer effects of these derivatives. It was determined that some compounds are effective against lung cancer (NCI-H460), breast cancer (MCF-7) and central nervous system cancer (SF 268) cells.

(Shivarama Holla, B. et al., 2006, Eur. J. Med. Chem., 41 (5), 657-663) synthesized 7-arylidene-6-(2,4-dichloro-5-fluorophenyl)-3-aryloxymethyl/anilinomethyl-1,2,4-triazolo[3,4-*b*]-1,3,4-thiadiazine derivatives and investigated the anticancer effects of these compounds against 60 cell lines derived from lung, colon, skin, kidney, ovarian, central nervous system and blood cancers. Among the synthesized compounds, 7-(4-chlorobenzylidene)-6-(2,4-dichloro-5-fluorophenyl)-3-((2-chlorophenoxy)methyl)-1,2,4-triazolo[3,4-*b*]-1,3,4-thiadiazine ($GI_{50}$<10 μM) showed the highest activity against all cells tested.

The patent document numbered WO2007/064797 is related to many compounds in the structure of triazolothiadiazole and triazolothiadiazine that can inhibit c-MET receptor tyrosine kinase, which is found to be overexpressed in many types of cancer.

(Bhat, K.S. et al., 2009, Eur. J. Med. Chem., 44, 5066-5070) synthesized 3-(2,4-dichloro-5-fluorophenyl)-6-(substitutedphenyl)-1,2,4-triazolo[3,4-*b*]-1,3,4-thiadiazine derivatives and investigated the *in vitro* antitumor effects of these derivatives against 60 cell lines derived from leukemia, non-small cell lung cancer, skin, ovarian, prostate and breast cancers. 3-(2,4-Dichloro-5-fluorophenyl)-6-(4-chlorophenyl)-1,2,4-triazolo[3,4-*b*]-1,3,4-thiadiazine ($GI_{50}$= 1.06-25.4 μM) showed significant antiproliferative activity against all cancer types tested.

(Puthiyapurayil, P. et al., 2012, Eur. J. Med. Chem., 57, 407-416) synthesized 6-aryl-3-[2-(4-substitutedphenyl)propane-2-yl]-7*H*-[1,2,4]triazolo[3,4-*b*][1,3,4]thiadiazine derivatives and investigated *in vitro* cytotoxic effects of these compounds by trypan blue test and MTT method. When compared with the standard drug doxorubicin ($IC_{50}$= 4.52 μM for A549 cell line, $IC_{50}$= 3.44 μM for MCF-7 cell line), among the synthesized compounds, 3-(2-(4-chlorophenyl)propane-2-yl)-6-(4-(trifluoromethoxy)phenyl)-7*H*-[1,2,4]triazolo[3,4-*b*] [1,3,4]thiadiazine showed the best activity against human lung cancer (A549) ($IC_{50}$= 18.20 μM) and human breast cancer (MCF-7) ($IC_{50}$= 10.54 μM) cells.

(Sumangala, V. et al., 2012, Eur. J. Med. Chem., 54, 59-64) synthesized 6-substituted-3-[4-(methylsulfonyl)benzyl]-7*H*-[1,2,4]triazolo[3,4-*b*][1,3,4]thiadiazine derivatives and investigated the cytotoxicities of these compounds. Among the synthesized compounds, 6-(biphenyl-4-yl)-3-(4-(methylsulfonyl)benzyl)-7*H*-[1,2,4]triazolo[3,4-*b*][1,3,4]thiadiazine ($IC_{50}$= 12.5 μM) and 3-(4-(methylsulfonyl)benzyl)-6-phenyl-7*H*-[1,2,4]triazolo[3,4-b][1,3,4]thiadiazine ($IC_{50}$= 25 μM) showed the best cytotoxic activity on human colon epithelial cells (HT-29).

(Kamel, M.M. and Megally Abdo, N.Y., 2014, Eur. J. Med. Chem., 86, 75-80) synthesized triazolothiadiazine derivatives and studied *in vitro* cytotoxic effects of these compounds against human gastric cancer (NUGC), human colon cancer (DLD1), human liver cancer (HA22T and HepG2), nasopharyngeal cancer (HONE1), human breast cancer (MCF) and normal fibroblast (WI38) cells. It was observed that 6-phenyl-3-(pyridine-4-yl)-7*H*-[1,2,4]triazolo[3,4-*b*][1,3,4]thiadiazine showed significant cytotoxic activity ($IC_{50}$<800 nM) against all other cancer cells tested except DLD1 cells and much less effect on WI38 cells ($IC_{50}$>10,000 nM).

(Khan, I. et al., 2014, Bioorg. Med. Chem., 22, 6163-6173) synthesized some triazolothiadiazine derivatives and investigated the cytotoxic activities of these compounds against lung cancer (H157) cells. When compared to the standard drug vincristine ($IC_{50}$= 1.03±0.04 μM), it was determined that 6-(3,4-dichlorophenyl)-3-(pyridine-3-yl)-7*H*-[1,2,4]triazolo[3,4-*b*][1,3,4]thiadiazine ($IC_{50}$= 0.96±0.43 μM) exhibited the highest antiproliferative activity.

(Khan I. et al., 2014, Eur. J. Med. Chem., 78, 167-177) synthesized 3,6-disubstituted-1,2,4-triazolo[3,4-*b*]-1,3,4-thiadiazine derivatives and studied the cytotoxic effects of these compounds on kidney fibroblast (BHK-21) cells. When compared to the standard drug vincristine (74.5% inhibition), 6-(3-methoxyphenyl)-3-(pyridine-4-yl)-7*H*-[1,2,4]triazolo[3,4-b][1,3,4]thiadiazine (73.3% inhibition) and 3-(pyridine-4-yl)-6-(*p*-tolyl)-7*H*-[1,2,4]triazolo[3,4-*b*][1,3,4]thiadiazine (72.6% inhibition) have proven to be potential inhibitors for cancer treatment by showing the best inhibition against BHK-21 cells.

(Sever, B. et al., 2016, Eur. J. Med. Chem., 113, 179-186) synthesized 3-[5-methoxy-2-methyl-1-(4-chlorobenzoyl)-1*H*-indole-3-yl)methyl]-6-aryl-7*H*-1,2,4-triazolo[3,4-*b*]-1,3,4-thiadiazine derivatives from indomethacin and investigated the antiproliferative effects of these compounds on T98 human glioma cell line. The effects of 3-[5-methoxy-2-methyl-1-(4-chlorobenzoyl)-1*H*-indole-3-yl)methyl]-6-(4-methylphenyl)-7*H*-1,2,4-triazolo[3,4-*b*]-1,3,4-thiadiazine found to be effective as an anticancer agent by MTT assay on apoptosis and relative quantification of COX-2, caspase 3, 8 and 9, cytochrome c messenger ribonucleic acid (mRNA) were investigated by flow cytometry and Real Time Polymerase Chain Reaction (RT-PCR), respectively. It was determined that this compound significantly decreased COX-2 mRNA levels compared to the control group and did not cause any significant change in other parameters (caspase 3, 8, 9, cytochrome c).

(Aytac, S.P. et al., 2016, Bioorg. Med. Chem., 24 (4), 858-872) synthesized 1,2,4-triazolo[3,4-b]-1,3,4-thiadiazine derivatives. The cytotoxic activities of these compounds against human liver (Huh7), breast (MCF-7) and colon (HCT116) cancer cell lines were evaluated. Compounds with an $IC_{50}$ value lower than ~5 μM were investigated for their cytotoxic effects on a panel of liver cancer cell lines (Huh7, HepG2, Hep3B, Mahlavu, FOCUS, SNU475) and effects on apoptosis and cell cycle in Huh7 cells. Some compounds induced apoptosis and SubG1 cell cycle arrest in Huh7 cells. Among these compounds, 3-[1-[4-(2-methylpropyl)phenyl]ethyl]-6-(4-methylphenyl)-7*H*-1,2,4-triazolo[3,4-*b*]-1,3,4-thiadiazine and 3-[1-[4-(2-methylpropyl)phenyl]ethyl]-6-(4-methoxyphenyl)-7*H*-1,2,4-triazolo[3,4-*b*]-1,3,4-thiadiazine were examined for their mechanism of action. These compounds induced apoptosis in Huh7 and Mahlavu cells through Apoptosis Signal-regulating Kinase-1 (ASK-1) activation and Akt inhibition.

(Ibrar, A. et al., 2016, Arch. Pharm. Chem. Life Sci., 349, 553-565) synthesized new hybrid compounds derived from coumarin-triazolothiadiazine and evaluated the antiproliferative effects of these compounds on renal fibroblast (BHK-21) and lung cancer (H-157) cell lines. 3-(6-(Biphenyl-4-yl)-7*H*-[1,2,4]triazolo[3,4-*b*][1,3,4]-thiadiazine-3-yl)-2*H*-chromen-2-one was found to be more effective on H-157 cell line with an $IC_{50}$ value of 1.01±0.12 μM than vincristine ($IC_{50}$= 1.03±0.04 μM) and cisplatin ($IC_{50}$= 1.31±0.03 μM). 3,3'-(7/7-[1,2,4]Triazolo[3,4-*b*][1,3,4]thiadiazine-3,6-diyl)-bis(2*H*-chromen-2-one) inhibited alkaline phosphatase (ALP) enzyme with an $IC_{50}$ value of 1.15± 1.0 μM.

(Radwan, R.R. et al., 2017, Chem. Biol. Interact., 274, 68-79) synthesized triazole, triazolothiadiazole and triazolothiadiazine derivatives and evaluated the cytotoxic effects of these compounds on human HCC (HepG2) cell

line. When compared to doxorubicin ($IC_{50}$= 36.3 μM), it was found that 1-phenyl-2-(2-(7-phenyl-5*H*-[1,2,4]triazolo[3,4-*b*][1,3,4]thiadiazine-3-yl)hydrazinyl)ethanone ($IC_{50}$= 19.6 μM) was effective on HepG2 cell line. This compound was found to reduce the level of vascular endothelial growth factor (VEGF) (30%) in HCC rats, thus inhibiting the formation new blood vessel and tumor growth. Also, this compound inhibited hepatic tyrosine kinase (HTK) expression (45%). Administration of this compound to HCC rats inhibited oxidative stress and improved liver levels of lipid peroxides (at a ratio of 62%) and glutathione (GSH) (at a ratio of 99%) compared to the HCC group.

(Ansari, M. et al., 2019, Bioorg. Chem., 93, 103300) synthesized 4-amino-5-aryl-4*H*-1,2,4-triazole-3-thione and 3,6-diaryl-7*H*-[1,2,4]triazolo[3,4-*b*][1,3,4]thiadiazine derivatives carrying 3,4,5-trimethoxyphenyl ring. The cytotoxic effects of these compounds on human lung adenocarcinoma (A549), breast adenocarcinoma (MCF-7) and ovarian carcinoma (SKOV3) cell lines were evaluated. When compared to etoposide ($IC_{50}$= 2.99 ± 0.22 μM for A549 cell line, $IC_{50}$= 1.89 ± 0.16 μM for MCF-7 cell line and $IC_{50}$= 2.30 ± 0.12 μM for SKOV3 cell line), 3-phenyl-6-(3,4,5-trimethoxyphenyl)-7*H*-[1,2,4]triazolo[3,4-*b*][1,3,4]thiadiazine ($IC_{50}$= 3.71 ± 0.13 μM for A549 cell line, $IC_{50}$= 0.70 ± 0.04 μM for MCF-7 cell line and $IC_{50}$= 4.63 ± 0.09 μM for SKOV3 cell line), 6-(4-bromophenyl)-3-(3,4,5-trimethoxyphenyl)-7H-[1,2,4]triazolo[3,4-*b*][1,3,4]thiadiazine ($IC_{50}$= 3.15 ± 0.01 μM for A549 cell line, $IC_{50}$= 3.34 ± 0.02 μM for MCF-7 cell line and $IC_{50}$= 0.30 ± 0.65 μM for SKOV3 cell line) and 6-(p-tolyl)-3-(3,4,5-trimethoxyphenyl)-7*H*-[1,2,4]triazolo[3,4-*b*][1,3,4]thiadiazine ($IC_{50}$= 0.60 ± 0.02 μM for A549 cell line, $IC_{50}$= 2.75 ± 0.05 μM for MCF-7 cell line and $IC_{50}$= 3.43 ± 0.33 μM for SKOV3 cell line) showed strong anti-cancer effects. These compounds showed no cytotoxic effects on healthy (L929) cell line at the tested concentrations, suggesting that their anticancer effects were selective. When compared to colchicine ($IC_{50}$= 0.40 μM), 6-(4-bromophenyl)-3-(3,4,5-trimethoxyphenyl)-7*H*-[1,2,4]triazolo[3,4-*b*][1,3,4]thiadiazine ($IC_{50}$= 1.03 μM) and 3-phenyl-6-(3,4,5-trimethoxyphenyl)-7*H*-[1,2,4]triazolo[3,4-b][1,3,4]thiadiazine ($IC_{50}$= 1.61 μM) inhibited tubulin polymerization in a dose-dependent manner. Fluorescence experiments showed that the compound carrying 4-bromophenyl ring at the position 6 of the triazolothiadiazine ring binds to the colchicine binding site in its interaction with tubulin. Molecular docking studies supported that these compounds inhibit tubulin polymerization by binding to the colchicine binding site.

(Arandkar, V. and Vedula, 2019, Phosphorus Sulfur Silicon Relat. Elem., 194/4-6, 533-539) synthesized new triazolothiadiazine derivatives from 4-amino-4*H*-1,2,4-triazole-3,5-dithiol and various phenacyl bromides and investigated the *in vitro* anticancer activities of these compounds against 60 cell lines. Among the synthesized compounds, 1-(4-fluorophenyl)-2-((6-(4-fluorophenyl)-7*H*-[1,2,4]triazolo[3,4-*b*][1,3,4]thiadiazine-3-yl)thio)ethanone (47.42% growth, 52.58% inhibition) and 1-(4-bromophenyl)-2-((6-(4-bromophenyl)-7*H*-[1,2,4]triazolo[3,4-b][1,3,4]thiadiazine-3-yl)thio)ethanone (46.76% growth, 53.24% inhibition) showed significant activity against kidney cancer (OU-31) cell line in terms of growth percentages with 47.42% and 46.76%, respectively. 2-((6-(4-Chlorophenyl)-7*H*-[1,2,4]triazolo[3,4-*b*][1,3,4]thiadiazine-3-yl)thio-1-(4-fluorophenyl)ethanone showed significant activity against OU-31 cell line with 48.14% and leukemia (MOLT-4) cell line with 49.82% in terms of growth percentages.

Considering the state of the art related to triazole and triazolothiadiazine rings, although the cytotoxic effects of the compounds carrying these ring systems on different cancer cell lines have been determined, specifically for the targeted therapy of lung cancer, there are no 1,2,4-triazole and 1,2,4-triazolo[3,4-*b*]-1,3,4-thiadiazine derivatives specific for targeted therapy of lung cancer showing selective and potent cytotoxic effects and apoptosis-inducing effects through Akt inhibition. With the invention, new 1,2,4-triazole and 1,2,4-triazolo[3,4-*b*]-1,3,4-thiadiazine derivatives specific for the targeted therapy of lung cancer showing selective and potent cytotoxic effects and apoptosis-inducing effects through Akt inhibition were identified.

## Brief Description of the Invention and its Aims

Within the scope of the invention, 10 new triazolothiadiazine and 7 new triazole derivatives were synthesized and the cytotoxic and apoptotic effects and Akt inhibitory effects of these compounds on A549 cells were investigated.

With the invention, new 1,2,4-triazolo[3,4-*b*]-1,3,4-thiadiazine **(2a-j)** and 1,2,4-triazole **(3a-g)** derivatives were synthesized and their structures were illuminated by spectroscopic methods. The cytotoxic effects of these derivatives on A549 human lung adenocarcinoma and NIH/3T3 mouse embryonic fibroblast cells were determined. The apoptosis-inducing and Akt inhibitory effects of the compounds showing selective anticancer effects on A549 cell line were evaluated. Molecular docking studies were performed in the Akt substrate binding site for active Akt inhibitors. In addition, *in silico* ADME studies were also conducted.

Among the inventive compounds, compounds **2a, 2b, 2c, 2d, 2j, 3a, 3b, 3c, 3d, 3f** and **3g** were found to be more effective on A549 cell line than cisplatin.

It was determined that among the inventive compounds, 6-(4-fluorophenyl)-3-(2-(pyridine-3-yl)ethyl)-7*H*-[1,2,4]triazolo[3,4-*b*][1,3,4]thiadiazine **(2c),** 6-(4-chlorophenyl)-3-(2-(pyridine-3-yl)ethyl)-7*H*-[1,2,4]triazolo[3,4-*b*][1,3,4]thiadiazine **(2d),** 2-((4-amino-5-(2-(pyridine-3-yl)ethyl)-4*H*-1,2,4-triazole-3-yl)thio)-N-(benzothiazole-2-yl)acetamide **(3b),** 2-((4-amino-5-(2-(pyridine-3-yl)ethyl)-4*H*-1,2,4-triazole-3-yl)thio)-*N*-(6-chlorobenzothiazole-2-yl)acetamide **(3d)** and 2-((4-amino-5-(2-(pyridine-3-yl)ethyl)-4H-1,2,4-triazole-3-yl)thio)-N-(6-methoxybenzothiazole -2-yl)acetamide

**(3g)** exhibited their cytotoxic and apoptotic effects on A549 cell line through Akt inhibition.

Compound **2c** showed Akt inhibitory activity similar to GSK690693, while compounds **2d, 3b, 3d** and **3g** showed better Akt inhibitory effects than Akt inhibitor GSK690693.

In compounds carrying the triazolothiadiazine ring system **(2a-j),** it was determined that the fluorine and the chlorine substituents at the position 4 of the phenyl ring attached to the position 6 of the triazolothiadiazine ring increased the Akt inhibitory activity.

In compounds carrying the triazole ring system **(3a-g),** it was determined that particularly the benzothiazole ring and the chlorine and the methoxy substituents bound to the position 6 of the benzothiazole ring play an important role in increasing Akt inhibitory activity.

According to molecular docking studies, these compounds showed strong interactions with important amino acids by showing high affinity to the Akt substrate binding site. In addition, according to Lipinski's rule of five and Jorgensen's rule of three, the oral bioavailability of these compounds is also predicted to be good. With the invention, these compounds were found to be potential targeted anticancer agents.

With the invention, new anticancer agents specific for the targeted therapy of lung cancer, which can be obtained readily and cheaply, have oral bioavailability, and show selective anticancer effects on A549 human lung adenocarcinoma cells were identified.

The pharmaceutical composition containing the inventive compounds is used in the preparation of the drug to be used in the treatment of lung cancer.

## Definitions of Drawings Illustrating the Invention

**Figure 1:** IR spectrum of Compound **2a**
**Figure 2:** $^1$H NMR spectrum of Compound **2a**
**Figure 3:** $^{13}$C NMR spectrum of Compound **2a**
**Figure 4:** HRMS spectrum of Compound **2a**
**Figure 5:** IR spectrum of Compound **2b**
**Figure 6:** $^1$H NMR spectrum of Compound **2b**
**Figure 7:** $^{13}$C NMR spectrum of Compound **2b**
**Figure 8:** HRMS spectrum of Compound **2b**
**Figure 9:** IR spectrum of Compound **2c**
**Figure 10:** $^1$H NMR spectrum of Compound **2c**
**Figure 11:** $^{13}$C NMR spectrum of Compound **2c**
**Figure 12:** HRMS spectrum of Compound **2c**
**Figure 13:** IR spectrum of Compound **2d**
**Figure 14:** $^1$H NMR spectrum of Compound **2d**
**Figure 15:** $^{13}$C NMR spectrum of Compound **2d**
**Figure 16:** HRMS spectrum of Compound **2d**
**Figure 17:** IR spectrum of Compound **2e**
**Figure 18:** $^1$H NMR spectrum of Compound **2e**
**Figure 19:** $^{13}$C NMR spectrum of Compound **2e**
**Figure 20:** HRMS spectrum of Compound **2e**
**Figure 21:** IR spectrum of Compound **2f**
**Figure 22:** $^1$H NMR spectrum of Compound **2f**
**Figure 23:** $^{13}$C NMR spectrum of Compound **2f**
**Figure 24:** HRMS spectrum of Compound **2f**
**Figure 25**:IR spectrum of Compound **2g**
**Figure 26:** $^1$H NMR spectrum of Compound **2g**
**Figure 27**:$^{13}$C NMR spectrum of Compound **2g**
**Figure 28:** HRMS spectrum of Compound **2g**
**Figure 29:** IR spectrum of Compound **2h**
**Figure 30:** $^1$H NMR spectrum of Compound **2h**
**Figure 31:** $^{13}$C NMR spectrum of Compound **2h**
**Figure 32:** HRMS spectrum of Compound **2h**
**Figure 33:** IR spectrum of Compound **2i**
**Figure 34:** $^1$H NMR spectrum of Compound **2i**
**Figure 35:** $^{13}$C NMR spectrum of Compound **2i**

**Figure 36:** HRMS spectrum of Compound **2i**
**Figure 37:** IR spectrum of Compound **2j**
**Figure 38:** $^{1}$H NMR spectrum of Compound **2j**
**Figure 39:** $^{13}$C NMR spectrum of Compound **2j**
**Figure 40:** HRMS spectrum of Compound **2j**
**Figure 41:** IR spectrum of Compound **3a**
**Figure 42:** $^{1}$H NMR spectrum of Compound **3a**
**Figure 43:** $^{13}$C NMR spectrum of Compound **3a**
**Figure 44:** HRMS spectrum of Compound **3a**
**Figure 45:** IR spectrum of Compound **3b**
**Figure 46:** $^{1}$H NMR spectrum of Compound **3b**
**Figure 47:** $^{13}$C NMR spectrum of Compound **3b**
**Figure 48:** HRMS spectrum of Compound **3b**
**Figure 49:** IR spectrum of Compound **3c**
**Figure 50:** $^{1}$H NMR spectrum of Compound **3c**
**Figure 51:** $^{13}$C NMR spectrum of Compound **3c**
**Figure 52:** HRMS spectrum of Compound **3c**
**Figure 53:** IR spectrum of Compound **3d**
**Figure 54:** $^{1}$H NMR spectrum of Compound **3d**
**Figure 55:** $^{13}$C NMR spectrum of Compound **3d**
**Figure 56:** HRMS spectrum of Compound **3d**
**Figure 57:** IR spectrum of Compound **3e**
**Figure 58:** $^{1}$H NMR spectrum of Compound **3e**
**Figure 59:** $^{13}$C NMR spectrum of Compound **3e**
**Figure 60:** HRMS spectrum of Compound **3e**
**Figure 61:** IR spectrum of Compound **3f**
**Figure 62:** $^{1}$H NMR spectrum of Compound **3f**
**Figure 63:** $^{13}$C NMR spectrum of Compound **3f**
**Figure 64:** HRMS spectrum of Compound **3f**
**Figure 65:** IR spectrum of Compound **3g**
**Figure 66:** $^{1}$H NMR spectrum of Compound **3g**
**Figure 67:** $^{13}$C NMR spectrum of Compound **3g**
**Figure 68:** HRMS spectrum of Compound **3g**
**Figure 69:** Flow cytometric analysis on A549 cells at $IC_{50}$ concentrations of compounds **2a, 2b, 2c, 2d, 2j, 3a, 3b, 3c, 3d, 3f, 3g** and cisplatin (7.23, 8.13, 11.83, 15.75, 4.00, 0.48, 0.68, 0.27, 0.34, 6.50, 0.48 and 11.50 μg/mL, respectively) (Q1: Necrotic cells, Q2: Late apoptotic cells, Q3: Viable cells, Q4: Early apoptotic cells). Quadrant analysis of at least 10.000 cells was performed for each sample. **A)** Control **B)** Control **C)** Compound **2a D)** Compound **2b E)** Compound **2c F)** Compound **2d G)** Compound **2j H)** Compound **3a I)** Compound **3b J)** Compound **3c K)** Compound **3d L)** Compound **3f M)** Compound **3g N)** Cisplatin
**Figure 70:** Molecular docking poses of compounds **2c, 2d, 3b, 3d** and **3g** in the Akt (PDB code: 3OW4) substrate binding site compared to GSK690693
**Figure 71:** RMSD values of compounds **2c, 2d, 3b, 3d** and **3g** compared to GSK690693
**Figure 72:** Docking interactions of compounds **2c** and **2d** in the substrate binding site of Akt enzyme
**Figure 73:** Docking interactions of compounds **3b, 3d** and **3g** and GSK690693 in the substrate binding site of Akt enzyme

**Detailed Description of the Invention**

In the invention, a total of 17 novel compounds, including 10 novel 1,2,4-triazolo[3,4-b]-1,3,4-thiadiazine **(2a-j)** and 7 novel 1,2,4-triazole derivative compound **(3a-g)** were synthesized. After characterization of the synthesized compounds by Infrared (IR), $^{1}$H Nuclear Magnetic Resonance (NMR), $^{13}$C NMR and High Resolution Mass Spectrometry (HRMS), their cytotoxic effects on A549 human lung adenocarcinoma and NIH/3T3 mouse embryonic fibroblast cells were determined. The apoptosis-inducing and Akt inhibitory effects of the compounds showing selective anti-cancer effects on the A549 cell line were evaluated. Molecular docking studies were performed in the Akt substrate binding site for active Akt inhibitors. In addition, *in silico* ADME studies were also conducted.

## 1. General Synthesis Methods of Starting and Final Compounds

### 1.1. Preparation of 4-amino-5-(2-(pyridine-3-yl)ethyl)-2,4-dihydro-3*H*-1,2,4-triazole-3-thione (Method 1)

The equivalent amounts of 3-(pyridine-3-yl)propionic acid and thiocarbohydrazide was stirred in an oil bath at 170-175°C for 2 hours. The product was washed with hot water and after drying, crystallized from ethanol (Scheme 1).

**Scheme 1:** Reaction equation (a) and mechanism (b) for preparation of 4-amino-5-(2-(pyridine-3-yl)ethyl)-2,4-dihydro-3*H*-1,2,4-triazole-3-thione

(a)

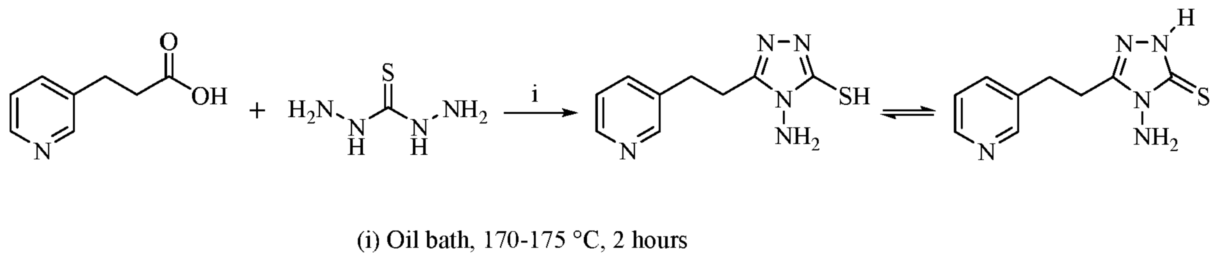


(b)

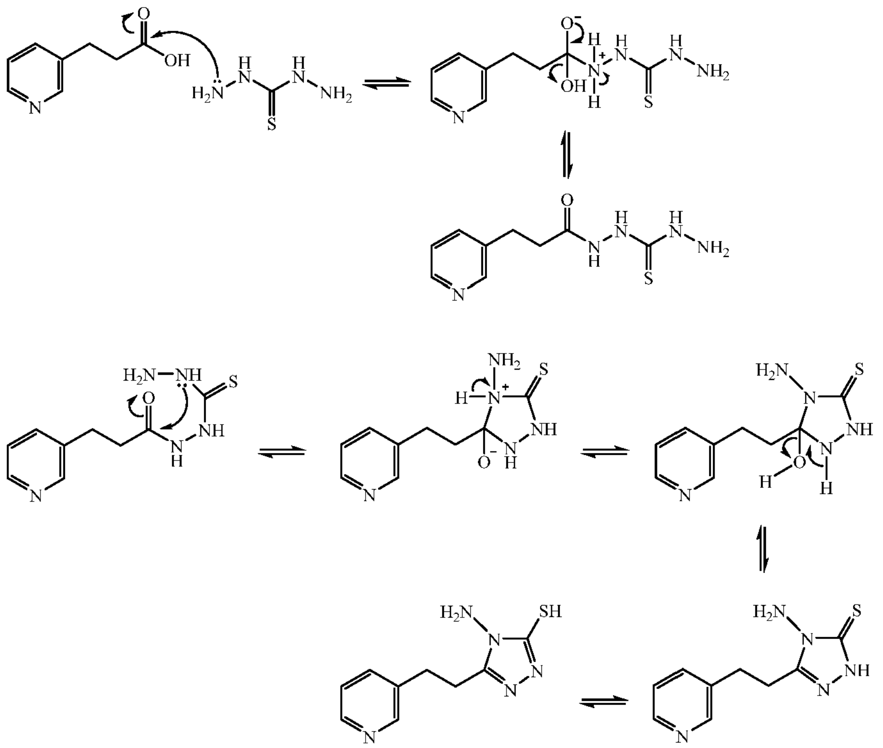

### 1.2. Preparation of 6-aryl-3-(2-(pyridine-3-yl)ethyl)-7*H*-[1,2,4]triazolo[3,4-*b*][1,3,4]thiadiazine derivatives (2a-j) (Method 2)

The equivalent amounts of 4-amino-5-(2-(pyridine-3-yl)ethyl)-2,4-dihydro-3*H*-1,2,4-triazole-3-thione and 2-bromo-1-arylethanone derivative was heated under reflux in ethanol for 8 hours. The precipitate was collected by filtration.

After drying, it was crystallized from ethanol (Scheme 2).

**Scheme 2.** Reaction equation (a) and mechanism (b) for preparation of 6-aryl-3-(2-(pyridine-3-yl)ethyl)-7*H*-[1,2,4]triazolo[3,4-*b*][1,3,4]thiadiazine derivatives

(a)

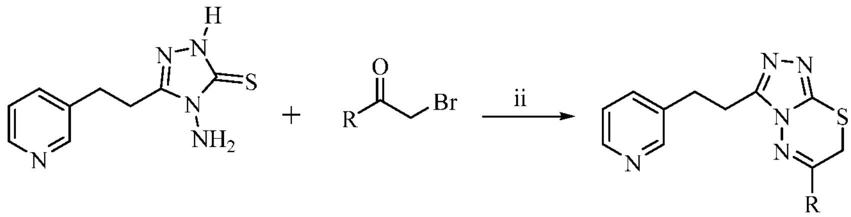


(ii) Ethanol, heating under reflux, 8 hours

(b)

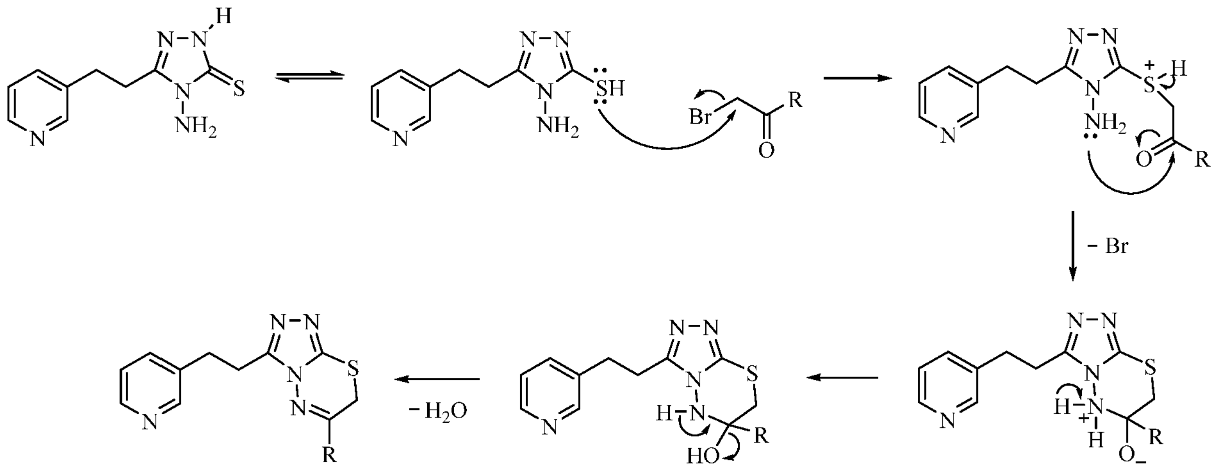


### 1.3. Preparation of 2-chloro-*N*-(aryl)acetamide derivatives (Method 3)

Aryl amine was dissolved in anhydrous toluene. To this solution was added an equivalent amount of triethylamine with aryl amine. An equivalent amount of chloroacetyl chloride with aryl amine was also dissolved in anhydrous toluene in the dropping funnel. The flask containing the aryl amine solution was placed in the ice bath. The chloroacetyl chloride solution in the dropping funnel was added dropwise onto the aryl amine solution. After this process, the solvent of the resulting mixture was evaporated in the rotavapor. The product was washed with water and after drying, crystallized with ethanol (Scheme 3).

**Scheme 3.** Reaction equation (a) and mechanism (b) for preparation of 2-chloro-*N*-(aryl)acetamide derivatives

(a)

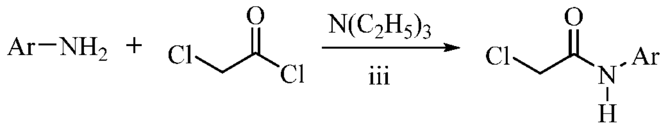


(iii) Toluene, room temperature

(b)

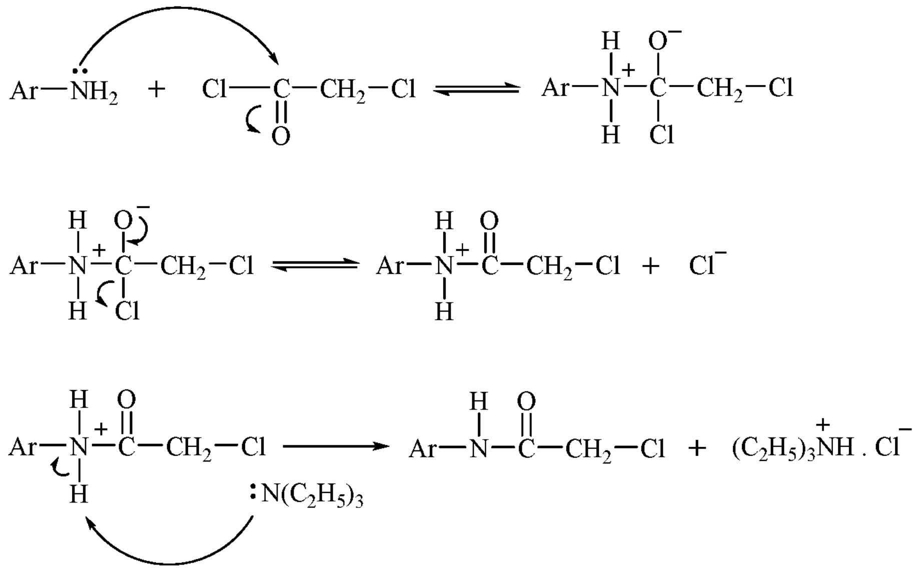

**1.4. Preparation of 2-[(4-amino-5-(2-(pyridine-3-yl)ethyl)-4*H*-1,2,4-triazole-3-yl)thio]-*N*-(aryl)acetamide (3a-g) derivatives (Method 4)**

The equivalent amounts of 4-amino-5-(2-(pyridine-3-yl)ethyl)-2,4-dihydro-3*H*-1,2,4-triazole-3-thione and 2-chloro-*N*-(aryl)acetamide in the presence of potassium carbonate as a catalyst was stirred in acetone at room temperature for 12 hours. The solvent of the reaction medium was evaporated in the rotavapor. The precipitate was collected by filtration, washed with water and dried. The product was crystallized with ethanol (Scheme 4).

**Scheme 4.** Reaction equation (a) and mechanism (b) for preparation of 2-((4-amino-5-(2-(pyridine-3-yl)ethyl)-4*H*-1,2,4-triazole-3-yl)thio)-*N*-(aryl)acetamide derivatives

(a)

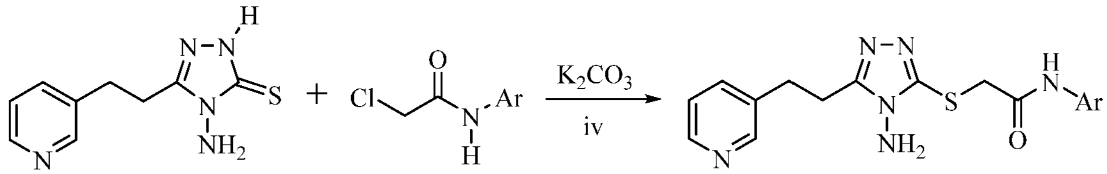


(iv) Acetone, room temperature, 12 hours

(b)

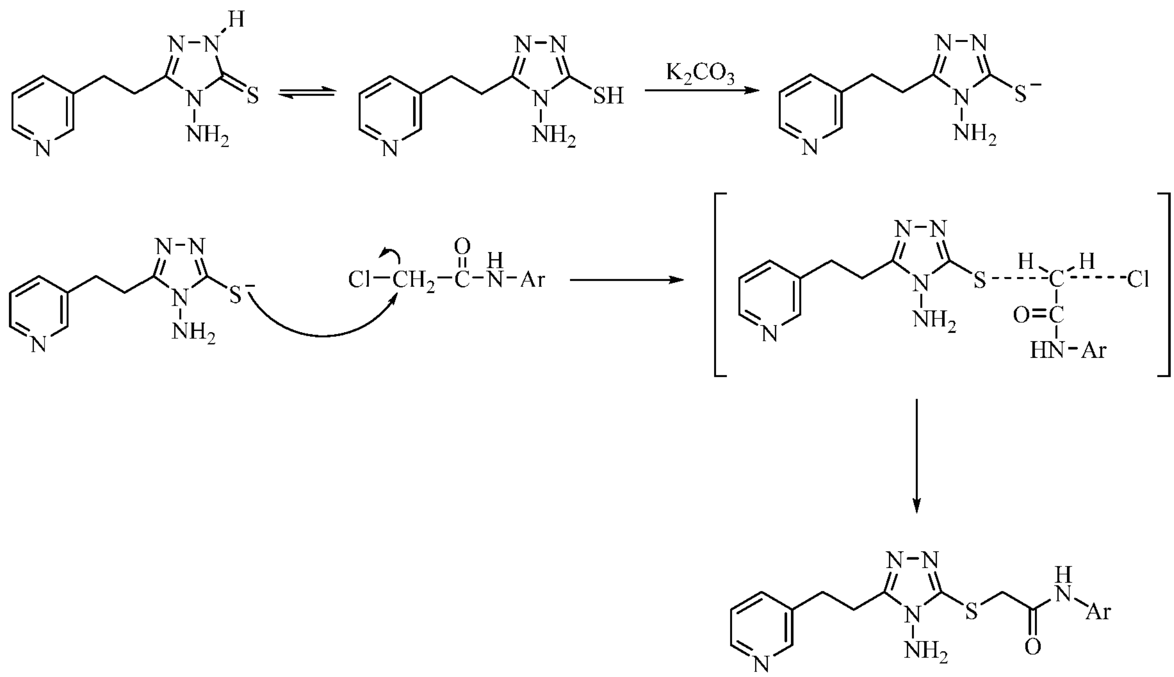


This invention is related to new targeted triazolothiadiazine and triazole derivatives in the treatment of lung cancer and their production methods and *in vitro* anticancer activity studies, molecular docking and *in silico* ADME studies; the said compounds of the invention are listed below:

**6-Phenyl-3-(2-(pyridine-3-yl)ethyl)-7*H*-[1,2,4]triazolo[3,4-*b*][1,3,4]thiadiazine (2a)**

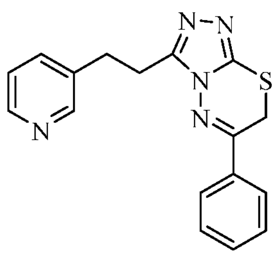

It was synthesized by using 4-amino-5-(2-(pyridine-3-yl)ethyl)-2,4-dihydro-3*H*-1,2,4-triazole-3-thione (1.5 mmol, 0.3319 g) and 2-bromoacetophenone (1.5 mmol, 0.2986 g) according to Method 2.
Yield: 81%, M.p.: 122.6°C

IR $\nu_{max}$ ($cm^{-1}$): 3057.17 (Aromatic C-H stretching band), 2993.52, 2926.01 (Aliphatic C-H stretching bands), 1633.71, 1597.06, 1570.06, 1535.34, 1475.54, 1448.54 (C=C and C=N stretching bands), 1406.11, 1359.82, 1336.67,

1309.67, 1276.88, 1261.45, 1222.87, 1186.22, 1163.08, 1120.64, 1083.99, 1064.71, 1004.91 (C-H bending, C-N stretching and aromatic C-H in-plane bending bands), 931.62, 835.18, 813.96, 792.74, 769.60, 731.02, 700.16, 682.80, 626.87 (Aromatic C-H out-of-plane bending bands and C-S stretching band) (Figure 1).

$^{1}H$ NMR (300 MHz, DMSO-$d_6$): 3.33 (brs, 4H), 4.41 (s, 2H), 7.54-7.63 (m, 3H), 7.99-8.02 (m, 3H), 8.54-8.57 (m, 1H), 8.78 (d, *J*= 4.80 Hz, 1H), 8.93 (s, 1H) (Figure 2).

$^{13}C$ NMR (75 MHz, DMSO-$d_6$): 23.50 ($CH_2$), 25.03 ($CH_2$), 28.93 ($CH_2$), 127.09 (CH), 127.96 (2CH), 129.52 (2CH), 132.45 (CH), 133.86 (C), 140.67 (CH), 141.09 (C), 142.57 (CH), 146.52 (CH), 146.69 (C), 152.57 (C), 155.47 (C) (Figure 3).

HRMS (ESI) (*m*/*z*): $[M+H]^+$ calculated for $C_{17}H_{15}N_5S$: 322.1121, found: 322.1119 (Figure 4).

**G-(4-Nitrophenyl)-3-(2-(pyridine-3-yl)ethyl)-7*H*-[1,2,4]triazolo[3,4-*b*] [1,3,4]thiadiazine (2b)**

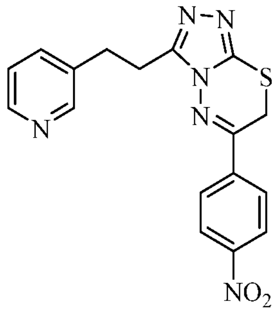

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-nitroacetophenone (1.5 mmol, 0.3661 g) according to Method 2.

Yield: 90%, M.p.: 227.6°C

IR $v_{max}$ ($cm^{-1}$): 3111.18, 3053.32 (Aromatic C-H stretching bands), 2981.95, 2910.58 (Aliphatic C-H stretching bands), 1633.71, 1598.99, 1512.19, 1471.69 ($NO_2$ stretching, C=C and C=N stretching bands), 1402.25, 1386.82, 1344.38, 1300.02, 1232.51, 1192.01, 1172.72, 1155.36, 1116.78, 1101.35, 1051.20, 1002.98 (C-H bending, $NO_2$, C-N stretching and aromatic C-H in-plane bending bands), 871.82, 854.47, 810.10, 783.10, 754.17, 690.52, 661.58 (Aromatic C-H out-of-plane bending bands and C-S stretching band) (Figure 5).

$^{1}H$ NMR (300 MHz, DMSO-$d_6$): 3.34 (t, *J*= 3.84 Hz, 3.96 Hz, 4H), 4.48 (s, 2H), 7.97-8.02 (m, 1H), 8.26 (d, *J*= 8.97 Hz, 2H), 8.40 (d, *J*= 9.03 Hz, 2H), 8.56 (d, *J*= 8.07 Hz, 1H), 8.77 (d, *J*= 5.52 Hz, 1H), 8.95 (s, 1H) (Figure 6).

$^{13}C$ NMR (75 MHz, DMSO-$d_6$): 23.60 ($CH_2$), 25.04 ($CH_2$), 28.97 ($CH_2$), 124.52 (2CH), 127.11 (CH), 129.36 (2CH), 139.78 (CH), 140.65 (C), 140.94 (C), 142.55 (CH), 146.63 (CH), 149.58 (2C), 152.80 (C), 153.71 (C) (Figure 7).

HRMS (ESI) (*m*/*z*): $[M+H]^+$ calculated for $C_{17}H_{14}N_6O_2S$: 367.0972, found: 367.0966 (Figure 8).

**G-(4-Fluorophenyl)-3-(2-(pyridine-3-yl)ethyl)-7*H*-[1,2,4]triazolo[3,4-*b*] [1,3,4]thiadiazine (2c)**

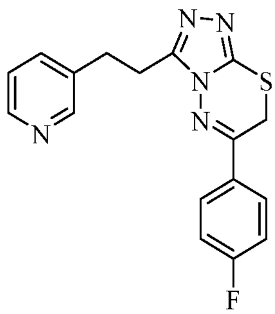

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-fluoroacetophenone (1.5 mmol, 0.3256 g) according to Method 2.

Yield: 79%, M.p.: 119.1°C

IR $v_{max}$ ($cm^{-1}$): 3043.67 (Aromatic C-H stretching band), 2916.37 (Aliphatic C-H stretching band), 1633.71,

1595.13, 1533.41, 1506.41, 1467.83 (C=C and C=N stretching bands), 1411.89, 1392.61, 1344.38, 1309.67, 1265.30, 1220.94, 1178.51, 1157.29, 1105.21, 1049.28, 1006.84 (C-H bending, C-N stretching and aromatic C-H in-plane bending bands), 952.84, 910.40, 856.39, 808.17, 775.38, 756.10, 732.95, 713.66, 678.94, 626.87 (Aromatic C-H out-of-plane bending bands and C-S stretching band) (Figure 9).

$^{1}$H NMR (300 MHz, DMSO-$d_6$): 3.32 (brs, 4H), 4.39 (s, 2H), 7.43 (t, *J*= 8.82 Hz, 8.85 Hz, 2H), 7.95-8.00 (m, 1H), 8.05-8.10 (m, 2H), 8.53 (d, *J*= 8.10 Hz, 1H), 8.76 (d, *J*= 5.40 Hz, 1H), 8.91 (s, 1H) (Figure 10).

$^{13}$C NMR (75 MHz, DMSO-$d_6$): 23.46 ($CH_2$), 25.02 ($CH_2$), 28.96 ($CH_2$), 116.64 (d, *J*= 21.75 Hz, 2CH), 127.03 (CH), 130.42 (C), 130.63 (d, *J*= 9.00 Hz, 2CH), 140.66 (CH), 140.76 (C), 142.67 (CH), 146.41 (CH), 152.56 (C), 154.46 (C), 163.06 (C), 166.38 (C) (Figure 11).

HRMS (ESI) (*m/z*): $[M+H]^+$ calculated for $C_{17}H_{14}FN_5S$: 340.1027, found: 340.1023 (Figure 12).

**G-(4-Chlorophenyl)-3-(2-(pyridine-3-yl)ethyl)-7*H*-[1,2,4]triazolo[3,4-*b*] [1,3,4]thiadiazine (2d)**

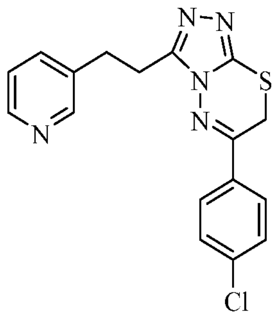

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-chloroacetophenone (1.5 mmol, 0.3502 g) according to Method 2.
Yield: 85%, M.p.: 112.1°C

IR $v_{max}$ ($cm^{-1}$): 3082.25, 3057.17, 3018.60 (Aromatic C-H stretching bands), 2916.37 (Aliphatic C-H stretching band), 1651.07, 1589.34, 1562.34, 1531.48, 1473.62 (C=C and C=N stretching bands), 1396.46, 1346.31, 1311.59, 1274.95, 1222.87, 1176.58, 1118.71, 1089.78, 1053.13, 1004.91 (C-H bending, C-N stretching and aromatic C-H in-plane bending bands), 921.97, 825.53, 804.32, 750.31, 682.80, 673.16, 632.65 (Aromatic C-H out-of-plane bending bands and C-S stretching band) (Figure 13).

$^{1}$H NMR (300 MHz, DMSO-$d_6$): 3.32 (brs, 4H), 4.39 (s, 2H), 7.66 (d, *J*= 8.70 Hz, 2H), 7.96-7.99 (m, 1H), 8.03 (d, *J*= 8.76 Hz, 2H), 8.54 (d, *J*= 8.13 Hz, 1H), 8.77 (d, *J*= 5.46 Hz, 1H), 8.92 (s, 1H) (Figure 14).

$^{13}$C NMR (75 MHz, DMSO-$d_6$): 23.38 ($CH_2$), 25.02 ($CH_2$), 28.95 ($CH_2$), 127.09 (CH), 129.61 (2CH), 129.78 (2CH), 132.69 (C), 137.26 (CH), 140.64 (C), 140.71 (C), 140.99 (CH), 142.54 (CH), 146.58 (C), 152.59 (C), 154.41 (C) (Figure 15).

HRMS (ESI) (*m/z*): $[M+H]^+$ calculated for $C_{17}H_{14}ClN_5S$: 356.0731, found: 356.0719 (Figure 16).

**G-(4-Bromophenyl)-3-(2-(pyridine-3-yl)ethyl)-7*H*-[1,2,4]triazolo[3,4-*b*][1,3,4]thiadiazine (2e)**

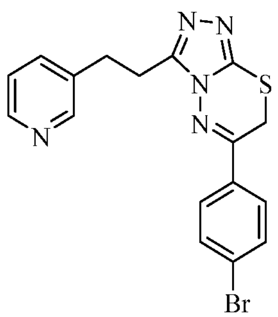

It was synthesized by using 4-amino-5-(2-(pyridine-3-yl)ethyl)-2,4-dihydro-3*H*-1,2,4-triazole-3-thione (1.5 mmol, 0.3319 g) and 2,4'-dibromoacetophenone (1.5 mmol, 0.4169 g) according to Method 2.

Yield: 87%, M.p.: 102.3°C

IR $v_{max}$ ($cm^{-1}$): 3078.39, 3043.67 (Aromatic C-H stretching bands), 2918.30, 2848.86 (Aliphatic C-H stretching bands), 1633.71, 1585.49, 1556.55, 1537.27, 1477.47, 1440.83 (C=C and C=N stretching bands), 1402.25, 1350.17, 1315.45, 1274.95, 1182.36, 1120.64, 1072.42, 1051.20, 1002.98 (C-H bending, C-N stretching and aromatic C-H in-plane bending bands), 914.26, 840.96, 806.25, 736.81, 680.87, 624.94 (Aromatic C-H out-of-plane bending bands and C-S stretching band) (Figure 17).

$^1H$ NMR (300 MHz, DMSO-$d_6$): 3.32 (brs, 4H), 4.39 (s, 2H), 7.79 (d, *J*= 8.67 Hz, 2H), 7.95 (d, *J*= 8.64 Hz, 2H), 7.98-8.01 (m, 1H), 8.54 (d, *J*= 5.16 Hz, 1H), 8.77 (d, *J*= 5.46 Hz, 1H), 8.93 (s, 1H) (Figure 18).

$^{13}C$ NMR (75 MHz, DMSO-$d_6$): 23.33 ($CH_2$), 25.03 ($CH_2$), 28.95 ($CH_2$), 126.26 (CH), 127.08 (C), 129.92 (2CH), 132.53 (2CH), 133.05 (C), 140.68 (CH), 140.99 (C), 142.58 (2CH), 146.54 (C), 152.59 (C), 154.54 (C) (Figure 19).

HRMS (ESI) (*m/z)*: $[M+H]^+$ calculated for $C_{17}H_{14}BrN_5S$: 400.0226, found: 400.0220 (Figure 20).

**G-(4-Cyanophenyl)-3-(2-(pyridine-3-yl)ethyl)-7*H*-[1,2,4]triazolo[3,4-*b*][1,3,4]thiadiazine (2f)**

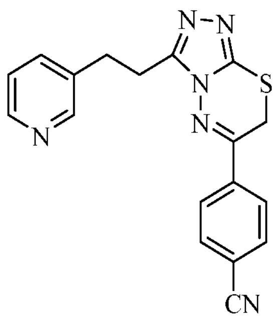

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-cyanoacetophenone (1.5 mmol, 0.3361 g) according to Method 2.

Yield: 89%, M.p.: 239.2°C

IR $v_{max}$ ($cm^{-1}$): 3099.61, 3059.10 (Aromatic C-H stretching bands), 2983.88, 2933.73 (Aliphatic C-H stretching bands), 2231.64 (C=N stretching band), 1604.77, 1546.91, 1537.27, 1469.76 (C=C and C=N stretching bands), 1433.11, 1404.18, 1371.39, 1319.31, 1309.67, 1284.59, 1257.59, 1222.87, 1174.65, 1112.93, 1045.42, 1004.91 (C-H bending, C-N stretching and aromatic C-H in-plane bending bands), 921.97, 864.11, 831.32, 808.17, 727.16, 684.73 (Aromatic C-H out-of-plane bending bands and C-S stretching band) (Figure 21).

$^1H$ NMR (300 MHz, DMSO-$d_6$): 3.33 (t, *J*= 3.87 Hz, 3.90 Hz, 4H), 4.44 (s, 2H), 7.99 (dd, *J*= 5.70 Hz, 7.95 Hz, 1H), 8.06 (d, *J*= 8.58 Hz, 2H), 8.18 (d, *J*= 8.58 Hz, 2H), 8.55 (d, *J* = 8.13 Hz, 1H), 8.77 (d, *J* = 5.46 Hz, 1H), 8.93 (s, 1H) (Figure 22).

$^{13}C$ NMR (75 MHz, DMSO-$d_6$): 23.42 ($CH_2$), 24.98 ($CH_2$), 28.93 ($CH_2$), 114.40 (C), 118.75 (C), 127.10 (CH), 128.71 (2CH), 133.40 (2CH), 138.09 (C), 140.62 (CH), 140.70 (C), 140.95 (CH), 142.52 (CH), 146.63 (C), 152.77 (C), 153.97 (C) (Figure 23).

HRMS (ESI) (*m/z*): $[M+H]^+$ calculated for $C_{18}H_{14}N_6S$: 347.1073, found: 347.1061 (Figure 24).

**3-(2-(Pyridine-3-yl)ethyl)-6-(4-(trifluoromethyl)phenyl)-7*H*-[1,2,4]triazolo[3,4-*b*][1,3,4]thiadiazine (2g)**

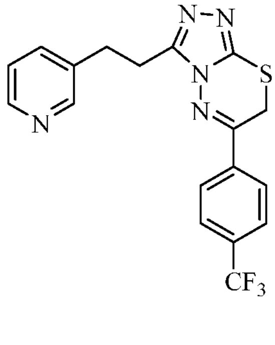

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-trifluoromethylacetophenone (1.5 mmol, 0.4006 g) according to Method 2.
Yield: 81%, M.p.: 190.9°C

IR $v_{max}$ ($cm^{-1}$): 3062.96, 3001.24 (Aromatic C-H stretching bands), 2918.30 (Aliphatic C-H stretching band), 1618.28, 1579.70, 1531.48, 1469.76 (C=C and C=N stretching bands), 1417.68, 1404.18, 1388.75, 1327.03, 1307.74, 1190.08, 1159.22, 1107.14, 1068.56, 1049.28, 1010.70 (C-H bending, C-N stretching and aromatic C-H in-plane bending bands), 862.18, 825.53, 796.60, 754.17, 738.74, 678.94, 648.08 (Aromatic C-H out-of-plane bending bands and C-S stretching band) (Figure 25).

$^1H$ NMR (300 MHz, DMSO-$d_6$): 3.25-3.31 (m, 4H), 4.44 (s, 2H), 7.76-7.81 (m, 1H), 7.94 (d, *J*= 8.40 Hz, 2H), 8.20 (d, *J*= 8.25 Hz, 2H), 8.25-8.31 (m, 1H), 8.66 (d, *J*= 5.34 Hz, 1H), 8.79 (s, 1H) (Figure 26).

$^{13}C$ NMR (75 MHz, DMSO-$d_6$): 23.57 ($CH_2$), 25.31 ($CH_2$), 29.19 ($CH_2$), 122.51 (CH), 126.12 (C), 126.39 (d, *J*= 3.75 Hz, 2CH), 128.82 (2CH), 131.86 (d, *J*= 31.5 Hz, C), 137.86 (CH), 139.44 (C), 140.89 (C), 142.76 (CH), 143.70 (CH), 144.74 (C), 152.86 (C), 154.17 (C) (Figure 27).

HRMS (ESI) (*m*/*z*): $[M+H]^+$ calculated for $C_{18}H_{14}F_3N_5S$: 390.0995, found: 390.0992 (Figure 28).

**3-(2-(Pyridine-3-yl)ethyl)-6-(*p*-tolyl)-7*H*-[1,2,4]triazolo[3,4-*b*][1,3,4]thiadiazine (2h)**

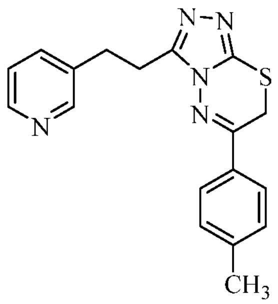

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-methylacetophenone (1.5 mmol, 0.3196 g) according to Method 2.
Yield: 75%, M.p.: 82.3°C

IR $v_{max}$ ($cm^{-1}$): 3084.18, 3057.17 (Aromatic C-H stretching bands), 2953.02, 2914.44 (Aliphatic C-H stretching bands), 1643.35, 1620.21, 1598.99, 1566.20, 1537.27, 1473.62, 1440.83 (C=C and C=N stretching bands), 1409.96, 1346.31, 1319.31, 1271.09, 1238.30, 1188.15, 1116.78, 1064.71, 1037.70, 1006.84 (C-H bending, C-N stretching and aromatic C-H in-plane bending bands), 921.97, 821.68, 806.25, 721.38, 680.87 (Aromatic C-H out-of-plane bending bands and C-S stretching band) (Figure 29).

$^1H$ NMR (300 MHz, DMSO-$d_6$): 2.38 (s, 3H), 3.32 (brs, 4H), 4.37 (s, 2H), 7.37 (d, *J*= 8.10 Hz, 2H), 7.90 (d, *J*= 8.22 Hz, 2H), 7.97-8.02 (m, 1H), 8.56 (d, *J*= 8.04 Hz, 1H), 8.78 (d, *J*= 5.16 Hz, 1H), 8.94 (s, 1H) (Figure 30).

$^{13}C$ NMR (75 MHz, DMSO-$d_6$): 21.55 ($CH_3$), 23.40 ($CH_2$), 25.03 ($CH_2$), 28.94 ($CH_2$), 127.15 (CH), 127.94 (2CH), 130.08 (2CH), 130.98 (C), 140.45 (CH), 140.81 (C), 141.17 (C), 142.33 (CH), 142.69 (CH), 146.76 (C), 152.49 (C), 155.39 (C) (Figure 31).

HRMS (ESI) (*m*/*z*): $[M+H]^+$ calculated for $C_{18}H_{17}N_5S$: 336.1277, found: 336.1271 (Figure 32).

**G-(4-Methoxyphenyl)-3-(2-(pyridine-3-yl)ethyl)-7*H*-[1,2,4]triazolo[3,4-*b*][1,3,4] thiadiazine (2i)**

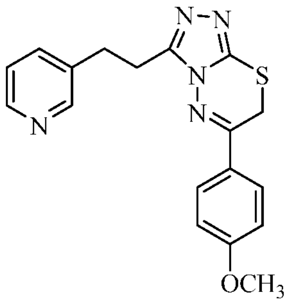

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-4'-methoxyacetophenone (1.5 mmol, 0.3436 g) according to Method 2.
Yield: 76%, M.p.: 91.9°C

IR $v_{max}$ ($cm^{-1}$): 3088.03, 3061.03, 3008.95 (Aromatic C-H stretching bands), 2927.94, 2841.15 (Aliphatic C-H stretching bands), 1651.07, 1602.85, 1595.13, 1558.48, 1539.20, 1514.12, 1469.76 (C=C and C=N stretching bands), 1423.47, 1390.68, 1315.45, 1257.59, 1234.44, 1188.15, 1112.93, 1055.06, 1024.20, 1008.77 (C-H bending, C-N, C-O stretching and aromatic C-H in-plane bending bands), 908.47, 848.68, 833.25, 804.32, 775.38, 754.17, 729.09, 677.01, 634.58 (Aromatic C-H out-of-plane bending bands and C-S stretching band) (Figure 33).

$^1$H NMR (300 MHz, DMSO-$d_6$): 3.32 (brs, 4H), 3.85 (s, 3H), 4.35 (s, 2H), 7.11 (d, *J* = 8.73 Hz, 2H), 7.98 (d, *J*= 8.76 Hz, 3H), 8.54 (d, *J*= 7.95 Hz, 1H), 8.77 (d, *J*= 5.40 Hz, 1H), 8.93 (s, 1H) (Figure 34).

$^{13}$C NMR (75 MHz, DMSO-$d_6$): 23.24 ($CH_2$), 25.06 ($CH_2$), 29.01 ($CH_2$), 56.08 ($CH_3$), 114.92 (2CH), 125.88 (CH), 127.06 (C), 129.85 (2CH), 140.75 (CH), 141.10 (C), 142.52 (2CH), 146.51 (C), 152.39 (C), 154.99 (C), 162.73 (C) (Figure 35).

HRMS (ESI) (*m/z*): $[M+H]^+$ calculated for $C_{18}H_{17}N_5OS$: 352.1227, found: 352.1222 (Figure 36).

**6-(Naphthalene-2-yl)-3-(2-(pyridine-3-yl)ethyl)-7*H*-[1,2,4]triazolo[3,4-*b*][1,3,4] thiadiazine (2j)**

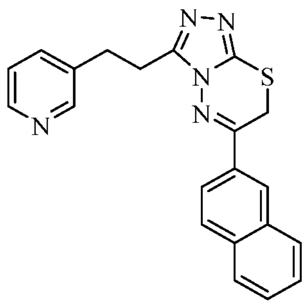

It was synthesized by using 4-amino-5-(2-(pyridine-3-yl)ethyl)-2,4-dihydro-3*H*-1,2,4-triazole-3-thione (1.5 mmol, 0.3319 g) and 2-bromo-2'-acetonaphthone (1.5 mmol, 0.3737 g) according to Method 2.
Yield: 84%, M.p.: 83.3°C

IR $v_{max}$ ($cm^{-1}$): 3061.03 (Aromatic C-H stretching band), 2918.30, 2848.86 (Aliphatic C-H stretching bands), 1627.92, 1597.06, 1568.13, 1537.27, 1475.54, 1438.90 (C=C and C=N stretching bands), 1402.25, 1344.38, 1311.59, 1278.81, 1199.72, 1157.29, 1128.36, 1062.78, 1035.77, 1016.49 (C-H bending, C-N stretching and aromatic C-H in-plane bending bands), 943.19, 900.76, 862.18, 825.53, 802.39, 775.38, 750.31, 711.73, 678.94, 628,79 (Aromatic C-H out-of-plane bending bands and C-S stretching band) (Figure 37).

$^1$H NMR (300 MHz, DMSO-$d_6$): 3.36-3.39 (m, 4H), 4.55 (s, 2H), 7.64-7.67 (m, 2H), 7.98-8.09 (m, 4H), 8.14-8.18 (m, 1H), 8.58 (d, *J*= 7.83 Hz, 1H), 8.64 (s, 1H), 8.77 (d, *J*= 5.19 Hz, 1H), 8.96 (s, 1H) (Figure 38).

$^{13}$C NMR (75 MHz, DMSO-$d_6$): 23.33 ($CH_2$), 25.07 ($CH_2$), 28.99 ($CH_2$), 123.69 (CH), 127.10 (CH), 127.68 (CH), 128.23 (CH), 128.82 (CH), 129.19 (CH), 129.46 (CH), 129.64 (CH), 131.06 (C), 132.87 (C), 134.73 (CH), 140.60 (C), 140.78 (C), 141.18 (CH), 142.51 (CH), 146.64 (C), 152.64 (C), 155.21 (C) (Figure 39).

HRMS (ESI) (*m/z*): $[M+H]^+$ calculated for $C_{21}H_{17}N_5S$: 372.1277, found: 372.1270 (Figure 40).

**2-((4-Amino-5-(2-(pyridine-3-yl)ethyl)-4*H*-1,2,4-triazole-3-yl)thio)-*N*-(thiazole-2-yl)acetamide (3a)**

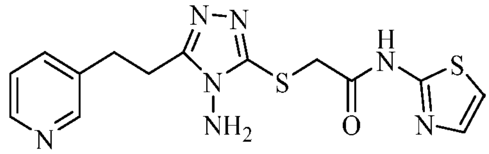

It was synthesized by using 4-amino-5-(2-(pyridine-3-yl)ethyl)-2,4-dihydro-3*H*-1,2,4-triazole-3-thione (1.5 mmol, 0.3319 g), 2-chloro-N-(thiazole-2-yl)acetamide (1.5 mmol, 0.2648 g) and potassium carbonate (1.5 mmol, 0.2073 g) according to Method 4.
Yield: 87%, M.p.: 222.9°C

IR $v_{max}$ ($cm^{-1}$): 3344.57 (N-H stretching band), 3076.46, 3026.31 (Aromatic C-H stretching bands), 2926.01 (Aliphatic C-H stretching band), 1662.64 (Amide C=O stretching band), 1631.78, 1579.70, 1525.69, 1479.40, 1442.75 (N-H bending, C=N and C=C stretching bands), 1421.54, 1379.10, 1348.24, 1319.31, 1265.30, 1240.23, 1184.29, 1165.00, 1103.28, 1066.64, 1026.13 (C-H bending, C-N stretching and aromatic C-H in-plane bending bands), 993.34, 970.19, 875.68, 806.25, 761.88, 740.67, 715.59, 669.30, 619.15 (Aromatic C-H out-of-plane bending bands and C-S stretching band) (Figure 41).

$^1H$ NMR (300 MHz, DMSO-$d_6$): 2.99 (brs, 4H), 4.18 (s, 2H), 5.97 (s, 2H), 7.23 (d, *J*= 3.54 Hz, 1H), 7.28-7.33 (m, 1H), 7.48 (d, *J*= 3.54 Hz, 1H), 7.68 (dt, *J*= 1.92 Hz, 7.86 Hz, 1H), 8.41 (dd, *J*= 1.59 Hz, 4.77 Hz, 1H), 8.49 (d, *J*= 1.92 Hz, 1H), 12.41 (s, 1H) (Figure 42).

$^{13}C$ NMR (75 MHz, DMSO-$d_6$): 25.58 ($CH_2$), 29.24 ($CH_2$), 34.71 ($CH_2$), 114.15 (CH), 123.85 (CH), 136.41 (CH), 136.71 (CH), 138.20 (C), 147.87 (CH), 150.16 (CH), 151.38 (C), 155.88 (C), 158.28 (C), 166.88 (C) (Figure 43).

HRMS (ESI) (*m/z*): $[M+H]^+$ calculated for $C_{14}H_{15}N_7OS_2$: 362.0852, found: 362.0849 (Figure 44).

**2-((4-Amino-5-(2-(pyridine-3-yl)ethyl)-4*H*-1,2,4-triazole-3-yl)thio)-*N*-(benzothiazole-2-yl)acetamide (3b)**

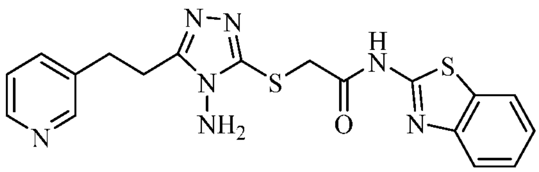

It was synthesized by using 4-amino-5-(2-(pyridine-3-yl)ethyl)-2,4-dihydro-3*H*-1,2,4-triazole-3-thione (1.5 mmol, 0.3319 g), 2-chloro-*N*-(benzothiazole-2-yl)acetamide (1.5 mmol, 0.3400 g) and potassium carbonate (1.5 mmol, 0.2073 g) according to Method 4.
Yield: 89%, M.p.: 220.4°C

IR $v_{max}$ ($cm^{-1}$): 3317.56, 3174.83 (N-H stretching bands), 3047.53 (Aromatic C-H stretching band), 2956.87, 2920.23 (Aliphatic C-H stretching bands), 1666.50 (Amide C=O stretching band), 1604.77, 1573.91, 1529.55, 1483.26, 1454.33, 1442.75 (N-H bending, C=N and C=C stretching bands), 1425.40, 1379.10, 1346.31, 1259.52, 1236.37, 1184.29, 1126.43, 1029.99, 1016.49, 1006.84 (C-H bending, C-N stretching and aromatic C-H in-plane bending bands), 981.77, 885.33, 871.82, 796.60, 779.24, 752.24, 727.16, 709.80, 669.30, 630.72 (Aromatic C-H out-of-plane bending bands and C-S stretching band) (Figure 45).

$^1H$ NMR (300 MHz, DMSO-$d_6$): 3.00 (brs, 4H), 4.25 (s, 2H), 5.99 (s, 2H), 7.29-7.34 (m,2H), 7.41-7.47 (m,1H), 7.68 (dt, *J*= 1.92 Hz, 7.89 Hz,1H), 7.76 (d, *J*= 7.92 Hz, 1H), 7.97 (d, *J*= 7.47 Hz, 1H), 8.40 (dd, *J*= 1.56 Hz, 4.76 Hz, 1H), 8.48 (d, *J*= 1.89 Hz, 1H), 12.67 (s, 1H) (Figure 46).

$^{13}C$ NMR (75 MHz, DMSO-$d_6$): 25.57 ($CH_2$), 29.23 ($CH_2$), 34.91 ($CH_2$), 121.11 (CH), 122.22 (CH), 123.85 (CH), 124.11 (CH), 126.65 (CH), 131.90 (C), 136.42 (CH), 136.72 (C), 147.86 (CH), 148.96 (CH), 150.16 (C), 151.39 (C), 155.94 (C), 158.22 (C), 168.02 (C) (Figure 47).

HRMS (ESI) (*m/z*): $[M+H]^+$ calculated for $C_{18}H_{17}N_7OS_2$: 412.1009, found: 412.1003 (Figure 48).

**2-((4-Amino-5-(2-(pyridine-3-yl)ethyl)-4*H*-1,2,4-triazole-3-yl)thio)-*N*-(6-fluorobenzothiazole-2-yl)acetamide (3c)**

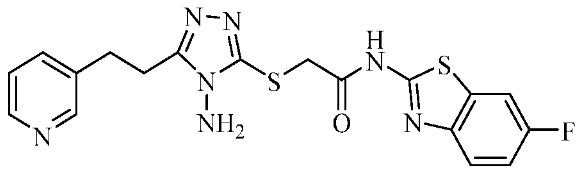

It was synthesized by using 4-amino-5-(2-(pyridine-3-yl)ethyl)-2,4-dihydro-3*H*-1,2,4-triazole-3-thione (1.5 mmol, 0.3319 g), 2-chloro-*N*-(6-fluorobenzothiazole-2-yl)acetamide (1.5 mmol, 0.3670 g) and potassium carbonate (1.5 mmol, 0.2073 g) according to Method 4.
Yield: 84%, M.p.: 230.7°C

IR $v_{max}$ ($cm^{-1}$): 3323.35, 3201.83 (N-H stretching bands), 3072.60 (Aromatic C-H stretching band), 2958.80, 2922.16 (Aliphatic C-H stretching bands), 1674.21 (Amide C=O stretching band), 1606.70, 1568.13, 1525.69, 1479.40, 1452.40 (N-H bending, C=N and C=C stretching bands), 1425.40, 1381.03, 1336.67, 1257.59, 1246.02, 1232.51, 1195.87, 1165.00, 1107.14, 1049.28, 1031.92 (C-H bending, C-N stretching and aromatic C-H in-plane bending bands), 979.84, 937.40, 910.40, 887.26, 869.90, 852.54, 825.53, 800.46, 773.46, 744.52, 713.66, 677.01, 659.66, 630.72 (Aromatic C-H out-of-plane bending bands and C-S stretching band) (Figure 49).

$^1H$ NMR (300 MHz, DMSO-$d_6$): 3.00 (brs, 4H), 4.25 (s, 2H), 5.99 (s, 2H), 7.26-7.33 (m,2H), 7.68 (dt, *J*= 1.92 Hz, 7.89 Hz, 1H), 7.77 (dd, *J*= 4.83 Hz, 8.89 Hz, 1H), 7.89 (dd, *J*= 2.67 Hz, 8.70 Hz, 1H), 8.40 (dd, *J*= 1.59 Hz, 4.77 Hz, 1H), 8.49 (d, *J*= 1.80 Hz, 1H), 12.70 (s, 1H) (Figure 50).

$^{13}C$ NMR (75 MHz, DMSO-$d_6$): 25.56 ($CH_2$), 29.22 ($CH_2$), 34.83 ($CH_2$), 108.68 (d, *J*= 27.00 Hz, CH), 114.77 (d, *J*= 24.00 Hz, CH), 122.23 (d, *J*= 9.00 Hz, CH), 123.86 (CH), 133.14 (d, *J*= 11.25 Hz, C), 136.43 (CH), 145.70 (C), 147.85 (CH), 150.15 (C), 151.38 (CH), 155.94 (C), 157.55 (C), 158.23 (d, *J*= 2.25 Hz, C), 160.73 (C), 168.12 (C) (Figure 51).

HRMS (ESI) (*m*/*z*): $[M+H]^+$ calculated for $C_{18}H_{16}FN_7OS_2$: 430.0915, found: 430.0908 (Figure 52).

**2-((4-Amino-5-(2-(pyridine-3-yl)ethyl)-4*H*-1,2,4-triazole-3-yl)thio)-*N*-(6-chlorobenzothiazole-2-yl)acetamide (3d)**

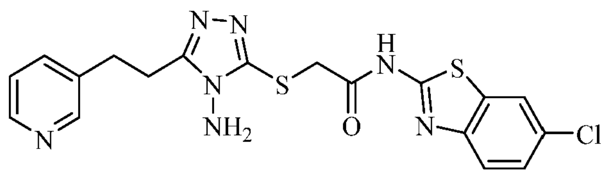

It was synthesized by using 4-amino-5-(2-(pyridine-3-yl)ethyl)-2,4-dihydro-3*H*-1,2,4-triazole-3-thione (1.5 mmol, 0.3319 g), 2-chloro-*N*-(6-chlorobenzothiazole-2-yl)acetamide (1.5 mmol, 0.3917 g) and potassium carbonate (1.5 mmol, 0.2073 g) according to Method 4.
Yield: 90%, M.p.: 223.7°C

IR $v_{max}$ ($cm^{-1}$): 3313.71, 3170.97 (N-H stretching bands), 3068.75 (Aromatic C-H stretching band), 2970.38, 2922.16, 2839.22 (Aliphatic C-H stretching bands), 1685.79 (Amide C=O stretching band), 1600.92, 1568.13, 1481.33, 1440.83 (N-H bending, C=N and C=C stretching bands), 1415.75, 1382.96, 1328.95, 1261.45, 1234.44, 1176.58, 1097.50, 1053.13, 1022.27, 1004.91 (C-H bending, C-N stretching and aromatic C-H in-plane bending bands), 983.70, 887.26, 848.68, 812.03, 802.39, 759.95, 709.80, 690.52 (Aromatic C-H out-of-plane bending bands and C-S stretching band) (Figure 53).

$^1H$ NMR (300 MHz, DMSO-$d_6$): 3.00 (brs, 4H), 4.25 (s, 2H), 5.99 (s, 2H), 7.30 (dd, J= 4.80 Hz, 7.68 Hz, 1H), 7.46 (dd, *J*= 2.13 Hz, 8.67 Hz, 1H), 7.68 (d, *J*= 7.80 Hz, 1H), 7.76 (d, *J*= 8.64 Hz, 1H), 8.13 (d, *J*= 2.07 Hz, 1H), 8.40 (dd, *J*= 1.35 Hz, 4.71 Hz, 1H), 8.49 (d, *J*= 1.68 Hz, 1H), 12.76 (s, 1H) (Figure 54).

$^{13}C$ NMR (75 MHz, DMSO-$d_6$): 25.56 ($CH_2$), 29.22 ($CH_2$), 34.85 ($CH_2$), 121.95 (CH), 122.33 (CH), 123.86 (CH), 127.00 (CH), 128.14 (C), 133.61 (C), 136.45 (CH), 136.72 (C), 147.84 (CH), 147.87 (CH), 150.13 (C), 151.37 (C), 155.95 (C), 159.08 (C), 168.25 (C) (Figure 55).

HRMS (ESI) (*m*/*z*): $[M+H]^+$ calculated for $C_{18}H_{16}ClN_7OS_2$: 446.0619, found: 446.0614 (Figure 56).

**2-((4-Amino-5-(2-(pyridine-3-yl)ethyl)-4*H*-1,2,4-triazole-3-yl)thio)-*N*-(6-bromobenzothiazole-2-yl)acetamide (3e)**

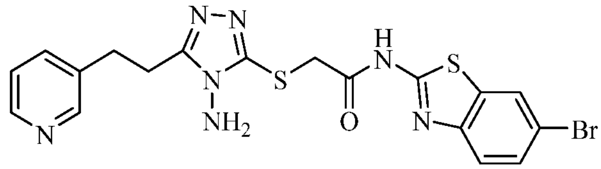

It was synthesized by using 4-amino-5-(2-(pyridine-3-yl)ethyl)-2,4-dihydro-3*H*-1,2,4-triazole-3-thione (1.5 mmol, 0.3319 g), 2-chloro-*N*-(6-bromobenzothiazole-2-yl)acetamide (1.5 mmol, 0.4584 g) and potassium carbonate (1.5 mmol, 0.2073 g) according to Method 4.
Yield: 88%, M.p.: 218.9°C

IR $v_{max}$ ($cm^{-1}$): 3307.92, 3163.26 (N-H stretching bands), 3055.24 (Aromatic C-H stretching band), 2956.87, 2918.30 (Aliphatic C-H stretching bands), 1668.43 (Amide C=O stretching band), 1600.92, 1568.13, 1529.55, 1489.05, 1440.83 (N-H bending, C=N and C=C stretching bands), 1419.61, 1379.10, 1346.31, 1257.59, 1234.44, 1188.15, 1083.99, 1041.56, 1028.06, 1006.84 (C-H bending, C-N stretching and aromatic C-H in-plane bending bands), 983.70, 960.55, 877.61, 810.10, 779.24, 746.45, 709.80, 682.80 (Aromatic C-H out-of-plane bending bands and C-S stretching band) (Figure 57).

$^1H$ NMR (300 MHz, DMSO-$d_6$): 2.99 (brs, 4H), 4.25 (s, 2H), 5.99 (s, 2H), 7.28-7.33 (m,1H), 7.57 (dd, *J*= 2.07 Hz, 8.63 Hz,1H), 7.67-7.72 (m, 2H), 8.26 (d, *J*= 1.98 Hz, 1H), 8.40 (dd, *J*= 1.62 Hz, 4.77 Hz, 1H), 8.49 (d, *J*= 1.92 Hz, 1H), 12.77 (s, 1H) (Figure 58).

$^{13}C$ NMR (75 MHz, DMSO-$d_6$): 25.56 ($CH_2$), 29.22 ($CH_2$), 34.85 ($CH_2$), 116.05 (C), 122.73 (CH), 123.85 (CH), 124.78 (CH), 129.68 (CH), 134.10 (C), 136.41 (CH), 136.71 (C), 147.86 (CH), 148.17 (CH), 150.16 (C), 151.37 (C), 155.95 (C), 159.05 (C), 168.27 (C) (Figure 59).

HRMS (ESI) (*m/z*): $[M+H]^+$ calculated for $C_{18}H_{16}BrN_7OS_2$: 490.0114, found: 490.0110 (Figure 60).

**2-((4-Amino-5-(2-(pyridine-3-yl)ethyl)-4*H*-1,2,4-triazole-3-yl)thio)-*N*-(6-methylbenzothiazole-2-yl)acetamide (3f)**

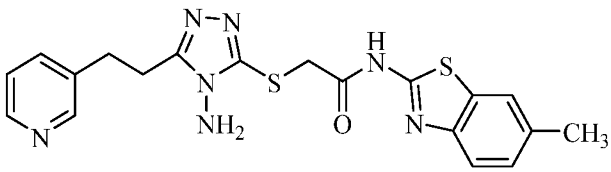

It was synthesized by using 4-amino-5-(2-(pyridine-3-yl)ethyl)-2,4-dihydro-3*H*-1,2,4-triazole-3-thione (1.5 mmol, 0.3319 g), 2-chloro-*N*-(6-methylbenzothiazole-2-yl)acetamide (1.5 mmol, 0.3611 g) and potassium carbonate (1.5 mmol, 0.2073 g) according to Method 4.
Yield: 79%, M.p.: 220.7°C

IR $v_{max}$ ($cm^{-1}$): 3350.35, 3321.42 (Amino group N-H stretching bands), 3169.04 (Amide N-H stretching band), 3057.17 (Aromatic C-H stretching band), 2922.16, 2856.58 (Aliphatic C-H stretching bands), 1668.43 (Amide C=O stretching band), 1610.56, 1577.77, 1529.55, 1460.11 (N-H bending, C=N and C=C stretching bands), 1421.54, 1377.17, 1340.53, 1257.59, 1230.58, 1209.37, 1176.58, 1128.36, 1109.07, 1026.13 (C-H bending, C-N stretching and aromatic C-H in-plane bending bands), 981.77, 898.83, 879.54, 810.10, 800.46, 777.31, 746.45, 709.80, 684.73, 659.66 (Aromatic C-H out-of-plane bending bands and C-S stretching band) (Figure 61).

$^1H$ NMR (300 MHz, DMSO-$d_6$): 2.41 (s, 3H), 3.00 (brs, 4H), 4.24 (s, 2H), 5.99 (s, 2H), 7.24-7.33 (m,2H), 7.63 (s,1H), 7.66-7.71 (m, 1H), 7.76 (s, 1H), 8.40 (dd, *J*= 1.59 Hz, 4.77 Hz, 1H), 8.49 (d, *J*= 1.83 Hz, 1H), 12.59 (s, 1H) (Figure 62).

$^{13}C$ NMR (75 MHz, DMSO-$d_6$): 21.47 ($CH_3$), 25.58 ($CH_2$), 29.23 ($CH_2$), 34.90 ($CH_2$), 120.74 (CH), 121.80 (CH), 123.85 (CH), 127.97 (CH), 132.04 (C), 133.58 (C), 136.41 (CH), 136.71 (C), 146.93 (CH), 147.86 (CH), 150.16 (C), 151.39 (C), 155.93 (C), 157.33 (C), 167.86 (C) (Figure 63).

HRMS (ESI) (*m/z*): $[M+H]^+$ calculated for $C_{19}H_{19}N_7OS_2$: 426.1165, found: 426.1156 (Figure 64).

**2-((4-Amino-5-(2-(pyridine-3-yl)ethyl)-4*H*-1,2,4-triazole-3-yl)thio)-*N*-(6-methoxybenzothiazole-2-yl)acetamide (3g)**

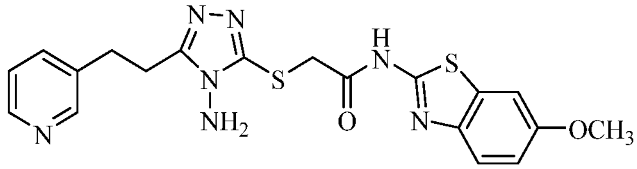

It was synthesized by using 4-amino-5-(2-(pyridine-3-yl)ethyl)-2,4-dihydro-3*H*-1,2,4-triazole-3-thione (1.5 mmol, 0.3319 g), 2-chloro-*N*-(6-methoxybenzothiazole-2-yl)acetamide (1.5 mmol, 0.3851 g) and potassium carbonate (1.5 mmol, 0.2073 g) according to Method 4.
Yield: 80%, M.p.: 215.9°C

IR $v_{max}$ ($cm^{-1}$): 3319.49 (N-H stretching band), 3074.53, 3005.10 (Aromatic C-H stretching bands), 2933.73, 2839.22 (Aliphatic C-H stretching bands), 1676.14 (Amide C=O stretching band), 1610.56, 1577.77, 1562.34, 1471.69 (N-H bending, C=N and C=C stretching bands), 1435.04, 1417.68, 1375.25, 1330.88, 1280.73, 1255.66, 1226.73, 1176.58, 1124.50, 1101.35, 1056.99, 1026.13 (C-H bending, C-N, C-O stretching and aromatic C-H in-plane bending bands), 981.77, 900.76, 833.25, 798.53, 744.52, 709.80, 661.58, 636.51 (Aromatic C-H out-of-plane bending bands and C-S stretching band) (Figure 65).

$^1$H NMR (300 MHz, DMSO-$d_6$): 3.00 (brs, 4H), 3.80 (s, 3H), 4.23 (s, 2H), 5.99 (s, 2H), 7.03 (dd, *J*= 2.61 Hz, 8.83 Hz, 1H), 7.30 (dd, *J*= 4.83 Hz, 7.66 Hz,1H), 7.56 (d, *J*= 2.55 Hz,1H), 7.64-7.71 (m, 2H), 8.40 (dd, *J*= 1.38 Hz, 4.73 Hz, 1H), 8.49 (d, *J*= 1.71 Hz, 1H), 12.54 (s, 1H) (Figure 66).

$^{13}$C NMR (75 MHz, DMSO-$d_6$): 25.58 ($CH_2$), 29.23 ($CH_2$), 34.86 ($CH_2$), 56.08 ($CH_3$), 105.16 (CH), 115.45 (CH), 121.72 (CH), 123.85 (CH), 133.22 (C), 136.42 (CH), 136.71 (C), 143.04 (C), 147.86 (CH), 150.16 (CH), 151.41 (C), 155.93 (C), 156.15 (C), 156.63 (C), 167.69 (C) (Figure 67).

HRMS (ESI) (*m/z*): $[M+H]^+$ calculated for $C_{19}H_{19}N_7O_2S_2$: 442.1114, found: 442.1107 (Figure 68).

## 2. Analysis of the Obtained Compounds and Evaluation of Results

### 2.1. Thin Layer Chromatography (TLC) studies

TLC was used in monitoring the reactions. In the TLC studies, 20x20 cm ready-made silica gel plates were used as adsorbent and petroleum ether-ethyl acetate (3:1, 1:1) was used as solvent system. Samples of the starting and the final compounds taken from the reaction medium were dissolved in ethyl alcohol and applied to the plate and dragged into the TLC tank. When the dragging was over, spots were detected under ultraviolet (UV) light at 254 and 366 nm wavelength.

### 2.2. Melting point (M.p.) detection

The melting points of the obtained compounds were determined with a melting point apparatus (Mettler Toledo, Ohio, USA) using one end closed capillary tubes.

### 2.3. Receiving IR spectra and Evaluation of Results

IR spectra of the obtained compounds were taken on IRPrestige-21 Fourier Transform (FT) IR spectrometer (Shimadzu, Japan).

In the spectra of the compounds carrying the triazolothiadiazine ring **(2a-j);** the absence of the N-H stretching band indicates that the triazolothiadiazine ring is closed. In the spectra of these compounds, aromatic and aliphatic C-H stretching bands were observed in the region 3111-3001 $cm^{-1}$ and 2993-2841 $cm^{-1}$, respectively. C=C and C=N stretching bands were observed at 1651-1438 $cm^{-1}$. C-H bending, C-N stretching and aromatic C-H in-plane bending bands were detected at 1433-1002 $cm^{-1}$. Aromatic C-H out-of-plane bending bands and C-S stretching band were observed at 953-624 $cm^{-1}$. In the spectrum of compound **2f** carrying the cyano substituent, the C≡N stretching band appeared at 2231.64 $cm^{-1}$.

In the spectra of the compounds carrying the triazole ring **(3a-g),** N-H stretching bands were observed at 3350-3163 $cm^{-1}$. Aromatic and aliphatic C-H stretching bands were detected at 3076-3005 $cm^{-1}$, and 2970-2839 $cm^{-1}$,

respectively. Amide C=O stretching band was observed at 1686-1662 $cm^{-1}$. N-H bending, C=N and C=C stretching bands appeared at 1632-1440 $cm^{-1}$. C-H bending, C-N stretching and aromatic C-H in-plane bending bands were detected at 1435-1004 $cm^{-1}$. Aromatic C-H out-of-plane bending bands and C-S stretching band were observed at 993-619 $cm^{-1}$.

### 2.4. Receiving $^1$H NMR spectra and Evaluation of Results

$^1$H NMR spectra of the obtained compounds were taken in NMR spectrometer (Bruker, Billerica, MA, USA) after dissolution in DMSO-$d_6$.

In the spectra of compounds carrying the triazolothiadiazine ring **(2a-j),** the absence of the protons belonging to the amino group indicates that the triazolothiadiazine ring is closed. The methylene protons bound to the position 7 of the triazolothiadiazine ring were observed as a singlet at 4.35-4.55 ppm. The ethyl protons bound to the third position of the triazolothiadiazine ring were detected at 3.25-3.39 ppm. Protons belonging to aromatic and heteroaromatic rings were observed at 7.37-8.96 ppm.

The protons belonging to the methyl substituent of compound **2h** were observed as a singlet at 2.38 ppm, and the protons belonging to the methoxy group of compound **2i** were observed as a singlet at 3.85 ppm.

The $CH_2$ protons of acetamide structure, which are common in the compounds carrying the triazole ring **(3a-g),** were recorded as a singlet at 4.18-4.25 ppm with the effect of the carbonyl group and the sulfur atom. The N-H proton belonging to the acetamide structure was observed as a singlet at 12.41-12.77 ppm. The protons belonging to the amino group bound to the fourth position of the triazole ring were determined as a singlet at 5.97-5.99 ppm. The protons belonging to the ethyl group bound to the fifth position of the triazole ring appeared at 2.99-3.00 ppm. The pyridine protons were observed at 7.23-8.49 ppm. The protons belonging to benzothiazole ring occurred in the region 7.03-8.49 ppm.

The thiazole ring protons belonging to compound **3a** appeared at 7.28-7.48 ppm. The protons belonging to the methyl group bound to the sixth position of the benzothiazole ring of compound **3f** were observed as a singlet at 2.41 ppm. The protons belonging to the methoxy group bound to the sixth position of the benzothiazole ring of compound **3g** were recorded as a singlet at 3.80 ppm.

### 2.5. Receiving $^{13}$C NMR spectra and Evaluation of Results

$^{13}$C NMR spectra of the obtained compounds were taken in NMR spectrometer (Bruker, Billerica, MA, USA) after dissolution in DMSO-$d_6$.

The $C_3$, $C_6$, $C_7$ and $C_{8a}$ carbons of the triazolothiadiazine ring, which are common in compounds **2a-j,** were observed in the region 144-153 ppm, 153-163 ppm, 24-25 ppm, 152-155 ppm, respectively. The methylene group carbon bound to the triazolothiadiazine ring occurred at 23-24 ppm and aromatic carbons occurred at 114-167 ppm. The pyridine carbons appeared 122-147 ppm, and the carbon of the methylene group bound to pyridine were observed in the region 28-29 ppm.

In compound **2f,** the cyano carbon was observed at 118.75 ppm, and in compound **2g,** the trifluoromethyl carbon was observed at 126.12 ppm. In compound **2h,** the methyl carbon was observed at 21.55 ppm, and in compound **2i,** the methoxy carbon was observed at 56.08 ppm. The naphthalene ring carbons bound to the sixth position of the triazolothiadiazine ring in compound **2j** were observed at 127-140 ppm.

The carbonyl carbon of the acetamide group, which is common in compounds carrying triazole ring **(3a-g),** occurred in the region 156-166 ppm. In the triazole ring, $C_3$ and $C_5$ carbons were observed at 150-156 ppm and 155-158 ppm, respectively. The carbon belonging to the methylene group bound to the triazole was observed at 25-26 ppm, whereas the carbon belonging to the methylene group between the carbonyl group and the sulfur atom was observed at 34-35 ppm. The pyridine carbons appeared at 123-151 ppm, and the carbon of the methylene group bound to pyridine occurred in the region 29-30 ppm. In the benzothiazole ring, which is common in compounds, $C_2$, $C_{3a}$, $C_{7a}$ and $C_6$ carbons were observed at 167-169 ppm, 143-152 ppm, 131-134 ppm, 116-158 ppm, respectively and other carbons were observed in the region 105-130 ppm.

In the thiazole ring of compound **3a,** $C_2$, $C_4$ and $C_5$ carbons were observed at 158.28 ppm, 136.41 ppm and 114.15 ppm, respectively. The carbon belonging to the methyl group at the sixth position of the benzothiazole ring of compound **3f** occurred at 21.47 ppm. The carbon belonging to the methoxy group at the sixth position of the benzothiazole ring of compound **3g** was recorded at 56.08 ppm.

### 2.6. Receiving mass spectra and Evaluation of Results

HRMS analyzes of the compounds were carried out in mass spectrometry (Shimadzu, Kyoto, Japan) using electrospray ionization (ESI) technique after they were dissolved in methanol, and the resulting charged atom or groups

were determined according to the mass/charge (m/z) ratio.

In the mass spectra of compounds **2a, 2b, 2c, 2d, 2f, 2g, 2h, 2i, 2j, 3a, 3b, 3c, 3d, 3f** and **3g,** $M^{+}$+1 peak was observed and in the spectra of compounds **2e** and **3e** carrying the bromine substituent, $M^{+}$ and $M^{+}$+2 peaks were observed.

### 3. *In vitro* Activity Studies

#### 3.1. Preparation of the compounds for testing

The stock solutions of triazolothiadiazine **(2a-j)** and triazole **(3a-g)** derivatives, positive control cisplatin (40 mg/mL) were prepared in DMSO. In order to be used from this stock, the concentrations ranging from 0.12 to 500 μg/mL (with the highest DMSO ratio of 0.1%) were prepared.

#### 3.2. The cells used in the experiments

A549 human lung adenocarcinoma cells (ATCC® CCL-185™) were grown in RPMI 1640 medium supplemented with 10% fetal bovine serum, 1% penicillin/streptomycin, in an incubator with carbon dioxide, in a culture medium at 37°C at 95% relative humidity.

NIH/3T3 mouse embryonic fibroblast cells (ATCC® CRL-1658™) were grown in DMEM medium supplemented with 10% fetal bovine serum, 1% penicillin/streptomycin, in an incubator with carbon dioxide, in a culture medium at 37°C at 95% relative humidity.

#### 3.3. Cytotoxicity assay

MTT protocol was applied to determine cell viability. Serial dilutions of compounds between 0.12-500 μg/mL were applied to plates with negative and positive control cisplatin and incubated for 24 hours at 37°C (5% $CO_2$, 95% humidity). At the end of the incubation period, 20 μL of MTT dye (5 mg/mL) was added into each well, and the cells were incubated for an additional 4 hours at 37°C. After this process, MTT dye was removed from the cells, 200 μL of DMSO was added to each well and left to incubate for 10 minutes. Color change was detected at 540 nm in the ELx808-IU Bio-Tek plate reader. Inhibition% values were determined for each concentration of compounds. Inhibitory concentration 50 ($IC_{50}$) values of the compounds were calculated by drawing a dose-response curve with their concentrations against these values. All experiments were carried out in triplicate. The results were given as mean±standard deviation.

$$\text{Inhibition\%} = 100-[(OD_{\text{test compound}} - OD_{\text{blank}}/OD_{\text{solvent control}} - OD_{\text{blank}}) \times 100]$$

Selective index (SI) values were determined using the following formula.

$$SI = IC_{50} \text{ for NIH/3T3 cell line} / IC_{50} \text{ for A549 cell line}$$

#### 3.4. Detection of apoptosis

The effects of the compounds determined to be active by the MTT method (at $IC_{50}$ concentrations) on apoptosis in A549 cells were determined by FITC Annexin V apoptosis detection kit in flow cytometry according to the manufacturer's instructions. The cells incubated with the compounds were centrifuged at 400 × g for 5 minutes in labeled tubes and washed twice with 1xPBS. Then, the cells were suspended in 1×Annexin-V binding solution at $1\times10^{6}$ cells/mL and 100 μL of this cell solution was taken and transferred to tubes. The cells were treated with 5 μL Annexin V FITC and 5 μL of propidium iodide (PI) dyes. Then, the cells were kept at room temperature in the dark for 15 minutes to allow the dye to bind. 400 μL of 1×Annexin-V binding cell solution was transferred to flow cytometry tubes and the amount of early and late apoptotic cells was determined relative to the positive control using the BD FACSAria flow cytometer FACSDiva version 6.1.1 software.

#### 3.5. Akt activity assay

After 10.000 cells/well were incubated with three different concentrations of compound 2a (1.81, 3.62, 7.23 μg/mL), **2b** (2.03, 4.07, 8.13 μg/mL), **2c** (2.95, 5.92, 11.83 μg/mL), **2d** (3.94, 7.88, 15.75 μg/mL), **2j** (1.0, 2.0, 4.0 μg/mL), **3a** (0.12, 0.24, 0.48 μg/mL), **3b** (0.98, 1.95, 3.90 μg/mL), **3c** (0.07, 0.14, 0.27 μg/mL), **3d** (0.98, 1.95, 3.90 μg/mL), **3f**

(1.63, 3.25, 6.50 μg/mL), **3g** (0.98, 1.95, 3.90 μg/mL), cisplatin (2.88, 5.75, 11.50 μg/mL) and Akt inhibitor GSK690693 (1.50, 3.075, 6.15 μg/mL) for 24 hours, Akt colorimetric in cell ELISA Kit protocol was applied in accordance with the manufacturer's recommendations. Briefly, the media was removed from the wells and 100 μL of 4% formaldehyde was added. The plate was incubated for 15 minutes in a fume hood. Formaldehyde was removed and the plate was washed twice with 1X tris-borate solution (TBS) to 100 μL/well. 1X TBS was removed and 100 μL of 1X permeabilization buffer was added to each well and left at room temperature for 15 minutes. After removing the 1X permeabilization buffer, the wells were washed once with 100 μL of 1X TBS. 1X TBS was removed and 100 μL of the quenching solution was added to the wells and left at room temperature for 20 minutes. Then, this solution was removed. The plate was washed once with 1X TBS, 100 μL of blocking buffer was added to the wells and left at room temperature for 30 minutes. At the end of this period, the blocking buffer was removed and 50 μL of primary antibody was added to the wells. The plate was closed with a sealer and incubated overnight at 4°C. The primary antibody was removed and plate wells were washed three times with 100 μL of wash buffer. After removing the wash buffer, 100 μL of diluted horseradish peroxidase (HRP) conjugate was added to the wells. It was incubated for 30 minutes at room temperature. After removing this conjugate, 100 μL of 3,3',5,5'-tetramethylbenzidine (TMB) stop solution was added and absorbance was measured at 450 nm for 30 minutes. The experiment was carried out in triplicate. The values of the blank wells were subtracted from the values of the untreated control and treated cells. The Akt activity% was determined as the relative absorbance of treated versus untreated control cells.

### *4. In silico* Studies

#### 4.1. Molecular Docking

Akt inhibitors were prepared by energy minimization at physiological pH through Optimized Potentials for Liquid Simulations (OPLS_2005) in the "ligand preparation" module of Schrödinger's Maestro molecular docking program. The complex X-ray crystal structure of the Akt enzyme was obtained from the protein data bank (PDB) (PDB code: 3OW4). The selected coded enzyme was optimized for docking analysis in the "protein preparation" module of the Schrödinger software. In molecular docking studies, Glide/XP docking protocols were applied in the substrate binding site of these enzymes to estimate the topology of ligands.

#### 4.2. *In Silico* ADME Studies

The QikProp module of Schrödinger's Maestro molecular modeling program was used to evaluate the pharmacokinetic properties of triazolothiadiazine **(2a-j)** and triazole **(3a-g)** derivatives and their compliance with Lipinski's rule of five and Jorgensen's rule of three.

### 5. Evaluation *of In Vitro* Activity Results

#### 5.1. Evaluation of MTT test results

According to MTT results, it is shown that compounds **2a, 2b, 2c, 2d, 2j, 3a, 3b, 3c, 3d, 3f** and **3g** were highly effective on A549 cell line with $IC_{50}$ values of 7.23±1.62, 8.13±0.81, 11.83±2.25, 15.75±0.35, 4.00, 0.48±0.04, 0.68±0.18, 0.27±0.02, 0.34±0.02, 6.50±0.50 and 0.48±0.03 μg/mL, respectively, compared to cisplatin ($IC_{50}$= 11.50±0.71 μg/mL). However, considering the cytotoxic effect of compound **2j** on the NIH/3T3 cell line, it was determined that its SI value was low compared to the other compounds. Although compound **2i** showed cytotoxic effect similar to cisplatin on A549 cell line ($IC_{50}$= 13.66±2.52 μg/mL), it was concluded that its anticancer effect was not selective due to its high cytotoxic effect ($IC_{50}$<3.90 μg/mL) on NIH/3T3 cell line. Among the triazolothiadiazine derivatives, compounds **2e, 2f** and **2g** carrying 4-bromophenyl, 4-cyanophenyl and 4-trifluoromethylphenyl moieties bound to the position 6 of the triazolothiadiazine ring, respectively did not show cytotoxic effects on A549 cell line in the tested concentration range. Among the benzothiazole derivatives **(3b-g),** compound **3e** carrying the bromine substituent at the position 6 of the benzothiazole ring showed very low cytotoxic effect ($IC_{50}$= 361.67±12.58 μg/mL) on A549 cells. This shows that the bromine substituent bound to the position 6 of the benzothiazole ring reduces the anti-cancer effect.

In particular, compound **3a** (SI= 430.54) carrying the thiazole ring system and **3c** (SI= 629.63) and compound **3g** (SI= 1041.66) carrying fluorine and methoxy substituents bound to the position 6 of the benzothiazole ring, respectively were determined to be the compounds with the most selective anticancer activity (Table 1).

**Table 1.** The cytotoxic effects of the compounds on A549 and NIH/3T3 cell lines

| Compound | $IC_{50}$ values (μg/mL) | | SI values* |
|---|---|---|---|
| | A549 cell line | NIH/3T3 cell line | |
| **2a** | 7.23±1.62 | >500 | >69.16 |
| **2b** | 8.13±0.81 | >500 | >61.50 |
| **2c** | 11.83±2.25 | >500 | >42.27 |
| **2d** | 15.75±0.35 | >500 | >31.75 |
| **2e** | >500 | >500 | - |
| **2f** | >500 | >500 | - |
| **2g** | >500 | >500 | - |
| **2h** | 41.33±0.58 | >500 | >12.10 |
| **2i** | 13.66±2.52 | <3.90 | - |
| **2j** | 4.00 | 6.66±1.04 | 1.67 |
| **3a** | 0.48±0.04 | 206.66±11.55 | 430.54 |
| **3b** | 0.68±0.18 | 23.33±6.11 | 34.31 |
| **3c** | 0.27±0.02 | 170±17.32 | 629.63 |
| **3d** | 0.34±0.02 | 4.90±0.36 | 14.41 |
| **3e** | 361.67±12.58 | >500 | >1.38 |
| **3f** | 6.50±0.50 | >500 | >76.92 |
| **3g** | 0.48±0.03 | >500 | >1041.66 |
| **Cisplatin** | 11.50±0.71 | - | - |
| **GSK690693** | 6.15±0.49 | - | - |

* SI= $IC_{50}$ for NIH/3T3 cell line / $IC_{50}$ for A549 cell line.

### 5.2. Evaluation of their effects on apoptosis

Flow cytometry analysis results show that particularly compounds **2c, 2d, 3d, 3f** and **3g** induce apoptosis. Compounds **2c, 3d** and **3f** (7.8%, 8.1% and 9.6%, respectively) caused higher apoptotic activity than cisplatin (7.6%) (Table 2, Figure 69). In compounds carrying triazolothiadiazine ring system **(2a-j),** it was determined that the fluorine substituents at the position 4 of the phenyl ring bound to the position 6 of the triazolothiadiazine ring increased the apoptotic effect. In compounds carrying the triazole ring system **(3a-g),** it was determined that particularly chlorine and methyl substituents bound to position 6 of the benzothiazole ring play an important role in apoptotic activity.

**Table 2.** Annexin V-FITC/PI flow cytometry quadrant analysis percentages of A549 cells treated with active compounds and cisplatin

| Compound | Early apoptotic cells % | Late apoptotic cells % | Viable cells% | Necrosis % |
|---|---|---|---|---|
| **Control** | 0.1 | 0.1 | 99.7 | 0.1 |
| **2a** | 1.7 | 2.7 | 95.0 | 0.7 |
| **2b** | 2.4 | 2.4 | 94.6 | 2.5 |
| **2c** | 1.8 | 6.0 | 87.6 | 4.6 |
| **2d** | 2.4 | 3.8 | 92.5 | 1.3 |
| **2j** | 1.0 | 3.6 | 90.3 | 5.0 |
| **3a** | 0.4 | 3.1 | 91.0 | 5.6 |
| **3b** | 0.7 | 3.0 | 93.1 | 3.2 |
| **3c** | 0.5 | 3.7 | 91.8 | 4.0 |
| **3d** | 1.8 | 6.3 | 87.2 | 4.8 |
| **3f** | 1.9 | 7.7 | 84.6 | 5.8 |
| **3g** | 0.9 | 5.5 | 84.5 | 9.1 |

(continued)

| Compound | Early apoptotic cells % | Late apoptotic cells % | Viable cells% | Necrosis % |
|---|---|---|---|---|
| **Cisplatin** | 1.8 | 5.8 | 85.4 | 7.0 |

**5.3. Evaluation of their Akt inhibitory effects**

Among the synthesized compounds, compounds **2d** ($IC_{50}$=0.80±0.10 µg/mL), **3b** ($IC_{50}$=2.23±0.12 µg/mL), **3d** ($IC_{50}$=0.19±0.05 µg/mL) and **3g** ($IC_{50}$=2.60±0.10 µg/mL) exhibited better Akt inhibitory activity than Akt inhibitor GSK690693 ($IC_{50}$=2.93±0.23 µg/mL). Compound **2c** ($IC_{50}$=2.95±0.05 µg/mL) exhibited Akt inhibitory activity similar to GSK690693. According to these results, it was determined that compounds **2c, 2d, 3b, 3d** and **3g** exhibited their cytotoxic and apoptotic effects on A549 cell line through Akt inhibition (Table 3).

**Table 3.** Akt inhibitory effects of the compounds

| Compound | Concentration | Inhibition % | $IC_{50}$ value |
|---|---|---|---|
| **2a** | **1.81 µg/mL** | 8.75±2.77 | ----- |
| | **3.62 µg/mL** | 7.69±2.91 | |
| | **7.23 µg/mL** | 35.45±5.82 | |
| **2b** | **2.03 µg/mL** | - | ----- |
| | **4.07 µg/mL** | 34.75±2.25 | |
| | **8.13 µg/mL** | 33.16±14.16 | |
| **2c** | **2.95 µg/mL** | 54.17±26.41 | 2.95±0.05 µg/mL |
| | **5.92 µg/mL** | 73.98±8.55 | |
| | **11.83 µg/mL** | 82.60±2.88 | |
| **2d** | **3.94 µg/mL** | 80.33±19.71 | 0.80±0.10 µg/mL |
| | **7.88 µg/mL** | 99.07±1.86 | |
| | **15.75 µg/mL** | 90.41±7.17 | |
| **2j** | **1.0 µg/mL** | - | ----- |
| | **2.0 µg/mL** | 17.80±2.08 | |
| | **4.0 µg/mL** | 48.23±7.29 | |
| **3a** | **0.12 µg/mL** | - | ----- |
| | **0.24 µg/mL** | - | |
| | **0.48 µg/mL** | 54.76±5.33 | |
| **3b** | **0.98 µg/mL** | 14.58±4.29 | 2.23±0.12 µg/mL |
| | **1.95 µg/mL** | - | |
| | **3.90 µg/mL** | 72.89±5.45 | |
| **3c** | **0.07 µg/mL** | 15.28±5.56 | ----- |
| | **0.14 µg/mL** | 36.64±13.39 | |
| | **0.27 µg/mL** | 39.84±6.49 | |
| **3d** | **0.98 µg/mL** | 80.23±7.70 | 0.19±0.05 µg/mL |
| | **1.95 µg/mL** | 76.94±10.75 | |
| | **3.90 µg/mL** | 100.00 | |

(continued)

| Compound | Concentration | Inhibition % | $IC_{50}$ value |
|---|---|---|---|
| 3f | **1.63 μg/mL** | - | ----- |
| | **3.25 μg/mL** | 37.59±9.46 | |
| | **6.50 μg/mL** | 40.29±11.27 | |
| 3g | **0.98 μg/mL** | - | 2.60±0.10 μg/mL |
| | **1.95 μg/mL** | 26.42±4.59 | |
| | **3.90 μg/mL** | 88.87±5.31 | |
| GSK690693 | **1.50 μg/mL** | 34.46±1.47 | 2.93±0.23 μg/mL |
| | **3.075 μg/mL** | 49.88±5.55 | |
| | **6.15 μg/mL** | 100 | |
| Cisplatin | **2.88 μg/mL** | - | 7.00±0.50 μg/mL |
| | **5.75 μg/mL** | 40.70±1.62 | |
| | **11.50 μg/mL** | 97.89±0.86 | |

Compounds **2a, 2b, 2j, 3a, 3c** and **3f** did not show significant Akt inhibitory effects. It is understood that these compounds exert their cytotoxic effects on A549 cell line through different pathways.

In compounds carrying the triazolothiadiazine ring system **(2a-j),** it was determined that fluorine and chlorine substituents at the position 4 of the phenyl ring attached to the position 6 of the triazolothiadiazine ring increased Akt inhibitory activity.

In compounds carrying the triazole ring system **(3a-g),** it was determined that particularly benzothiazole ring and chlorine and methoxy substituents bound to position 6 of the benzothiazole ring play an important role in increasing Akt inhibitory activity.

### 6. Evaluation *of In silico* Studies

### 6.1. Evaluation of *in silico* molecular docking studies

As a result of *in vitro* enzyme assays, molecular docking study was performed in Akt substrate binding site for compounds **2c, 2d, 3b, 3d** and **3g** showing the best Akt inhibition. For this purpose, Akt with PDB code 3OW4 was chosen. The docking poses of the compounds were compared to GSK690693 (Figure 70).

Glide uses the emodel (kcal/mol) results in selecting the best molecular docking pose for the ligand. For this reason, the glide emodel results given in Table 4 were taken into account in the selection of the conformations of the ligands. The conformations that give the best emodel results were compared with the glide gscore values. The gscore values (kcal/mol) of the compounds were compatible with that of GSK690693.

**Table 4.** Glide gscore (kcal/mol) and glide emodel (kcal/mol) results of the compounds for Akt (PDB code: 3OW4)

| Compound | Akt | |
|---|---|---|
| | Glide gscore | Glide emodel |
| **2c** | -6.21 | -70.14 |
| **2d** | -6.27 | -74.90 |
| **3b** | -7.46 | -95.37 |
| **3d** | -6.17 | -79.23 |
| **3g** | -7.81 | -98.75 |
| **GSK690693** | -6.72 | -87.38 |

Root mean square deviation (RMSD) values of compounds **2c, 2d, 3b, 3d** and **3g** compared to GSK690693 were determined (Figure 71). RMSD values for all compounds were found in the range of 0.00-0.04.

According to the molecular docking results, 4-fluorophenyl moiety bound to position 6 of the triazolothiadiazine

ring of compound **2c** formed a π-cation interaction with Arg4 and π-π interactions with Phe442, whereas 4-chlorophenyl moiety bound to position 6 of the triazolothiadiazine ring of compound **2d** showed a π-cation interaction with Arg4. The pyridine ring in the structure formed a hydrogen bond with Asp292 (Figure 72).

The 4/7-1,2,4-triazole ring of compound **3b** formed π-cation interactions with Arg4, while the amino group at the position 4 of the triazole ring formed a hydrogen bond with Glu278 and Asn279 amino acid residues. The amide group of this compound formed a hydrogen bond with Asp292. The amino group at the position 4 of the triazole ring of compound **3d** formed a hydrogen bond with Asp292 and water molecules, and the pyridine ring formed a hydrogen bond with Glu234. Finally, the 4*H*-1,2,4-triazole ring, the amide group and the pyridine ring of compound **3g** formed hydrogen bonds with Lys179, Lys158, and Glu191, respectively. The 6-methoxybenzothiazole ring of compound **3g** was also bound to the Akt substrate binding site via a π-cation interaction with Arg4. The docking interactions of compounds **3b, 3d** and **3g** and Akt inhibitor GSK690693 are shown in Figure 73.

The 4-amino-4*H*-1,2,4-triazole of compounds **3b, 3d** and **3g** played an important role for the interactions in the Akt enzyme. Compounds **2c** and **2d** showed less interaction than compounds **3b, 3d** and **3g.** This outcome indicates that the closure of the triazole ring in the form of the triazolothiadiazine ring system decreases the affinity in the substrate region of the enzyme.

### 6.2. Evaluation of *in silico* ADME study results

*In silico* ADME studies are important for lead discovery and optimization, preliminary evaluation of drug candidates.

The pharmacokinetic features such as predicted octanol/water partition coefficient (QPlogPo/w), conformation-independent predicted aqueous solubility (CIQPlogS), predicted human serum albumin binding rate (QPlogKhsa), total solvent accessible surface area (SASA), van der Waals surface area of polar nitrogen and oxygen atoms (PSA), oral absorption% of all compounds **(2a-j, 3a-g)** were predicted with the QikProp module of Schrödinger's Maestro molecular modeling program (Table 5).

**Table 5.** *In silico* ADME values of the compounds

| Compound | QPlogPo/w* | CIQPlogS* | QPlogKhsa* | SASA* | PSA* | Oral Absorption % ** |
|---|---|---|---|---|---|---|
| **2a** | 3.759 | -5.005 | 0.274 | 590.682 | 58.290 | 100.000 |
| **2b** | 3.083 | -5.497 | 0.196 | 635.480 | 102.739 | 84.431 |
| **2c** | 4.079 | -5.387 | 0.343 | 606.669 | 57.830 | 100.000 |
| **2d** | 4.345 | -5.737 | 0.425 | 621.725 | 57.830 | 100.000 |
| **2e** | 4.425 | -6.681 | 0.451 | 626.899 | 57.830 | 100.000 |
| **2f** | 2.973 | -5.840 | 0.039 | 634.824 | 84.098 | 87.746 |
| **2g** | 4.738 | -6.449 | 0.556 | 641.650 | 58.288 | 100.000 |
| **2h** | 4.163 | -5.303 | 0.470 | 629.611 | 57.833 | 100.000 |
| **2i** | 3.820 | -5.295 | 0.249 | 627.989 | 66.582 | 100.000 |
| **2j** | 4.799 | -6.308 | 0.694 | 666.246 | 58.309 | 100.000 |
| **3a** | 0.849 | -2.752 | -0.503 | 655.528 | 123.891 | 58.675 |
| **3b** | 1.759 | -3.919 | -0.197 | 729.459 | 122.761 | 64.653 |
| **3c** | 1.987 | -4.260 | -0.162 | 738.480 | 122.758 | 65.988 |
| **3d** | 2.233 | -4.575 | -0.100 | 753.502 | 122.758 | 67.428 |
| **3e** | 2.306 | -5.437 | -0.081 | 758.547 | 122.757 | 67.854 |
| **3f** | 2.043 | -4.185 | -0.065 | 761.674 | 122.759 | 66.313 |
| **3g** | 1.844 | -4.214 | -0.197 | 766.346 | 131.044 | 65.145 |

* Recommended value range for QPlogPo/w: -2-6.5. * Recommended value range for CIQPlogS: - 6.5-0.5. * Recommended value range for QPlogKhsa: -1.5-1.5. Recommended value range as $Å^2$ for SASA 300-1000. * Recommended value range for PSA: 7.0-200.0.

** Oral Absorption%: Human oral absorption between 0-100% (> 80% indicates high, <25% indicates low).

According to the *in silico* study, QPlogPo/w, QPlogKhsa, SASA and PSA values of all compounds were found in the recommended range. The CIQPlogS values of all compounds except for compound **2e** were also determined within the expected range. In terms of oral absorption%, it was determined that those with the triazolothiadiazine ring **(2a-2j)** were at high values, while those with the triazole ring **(3a-3g)** were at normal values.

According to Lipinski's rule of five, the molecular weight of a compound should be less than 500, the QPlogPo/w value should be less than 5, the number of hydrogen bond donor atoms should be 5 or less, and the number of hydrogen bond acceptor atoms should be 10 or less. Molecules that do not violate any of these rules, or only one, can be considered drug candidates.

According to Jorgensen's rule of three, the predicted aqueous solubility (QPlogS) value for oral use should be greater than -5.7, the predicted apparent Caco-2 cell permeability (QPPCaco) should be greater than 22 nm/s, the primary metabolites should be less than 7. Compounds that violate these rules less (preferably not at all) are considered more suitable agents for oral use.

According to the *in silico* ADME data, all compounds comply with Lipinski's rule of five and Jorgensen's rule of three and these derivatives are predicted to have good oral bioavailability (Table 6).

**Table 6.** The violation numbers of the compounds related to Lipinski's rule of five and Jorgensen's rule of three

| Compound | Violation numbers | |
|---|---|---|
| | **Lipinski's rule of five** | **Jorgensen's rule of three** |
| **2a** | 0 | 0 |
| **2b** | 0 | 1 |
| **2c** | 0 | 0 |
| **2d** | 0 | 0 |
| **2e** | 0 | 1 |
| **2f** | 0 | 0 |
| **2g** | 0 | 1 |
| **2h** | 0 | 1 |
| **2i** | 0 | 1 |
| **2j** | 0 | 1 |
| **3a** | 0 | 0 |
| **3b** | 0 | 0 |
| **3c** | 0 | 0 |
| **3d** | 0 | 0 |
| **3e** | 0 | 0 |
| **3f** | 0 | 1 |
| **3g** | 0 | 1 |

## Claims

1. The triazole or triazolothiadiazine derivative, **characterized in that** it is selected from the following group:

| | |
|---|---|
| 6-Phenyl-3-(2-(pyridine-3-yl)ethyl)-7*H*-[1,2,4] triazolo[3,4-*b*][1,3,4]thiadiazine | 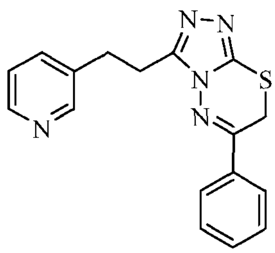 |

(continued)

| | |
|---|---|
| 6-(4-Nitrophenyl)-3-(2-(pyridine-3-yl)ethyl)-7*H*-[1,2,4]triazolo[3,4-*b*][1,3,4]thiadiazine | 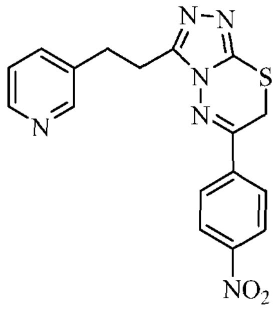 |
| 6-(4-Fluorophenyl)-3-(2-(pyridine-3-yl)ethyl)-7*H*-[1,2,4]triazolo[3,4-*b*][1,3,4]thiadiazine | 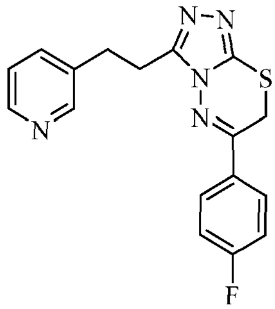 |
| 6-(4-Chlorophenyl)-3-(2-(pyridine-3-yl)ethyl)-7*H*-[1,2,4]triazolo[3,4-*b*][1,3,4]thiadiazine | 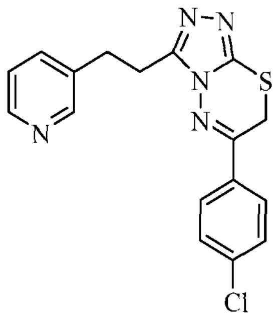 |
| 6-(4-Bromophenyl)-3-(2-(pyridine-3-yl)ethyl)-7*H*-[1,2,4]triazolo[3,4-*b*][1,3,4]thiadiazine | 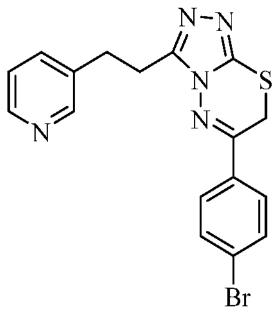 |
| 6-(4-Cyanophenyl)-3-(2-(pyridine-3-yl)ethyl)-7*H*-[1,2,4]triazolo[3,4-*b*][1,3,4]thiadiazine | 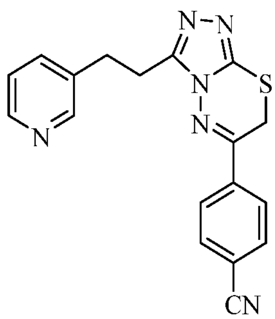 |

(continued)

| | |
|---|---|
| 3-(2-(Pyridine-3-yl)ethyl)-6-(4-(trifluoromethyl) phenyl)-7*H*-[1,2,4]triazolo[3,4-*b*][1,3,4]thiadiazine | 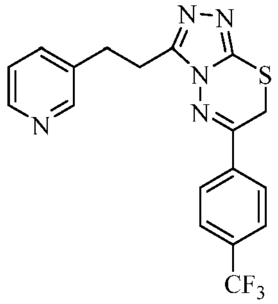 |
| 3-(2-(Pyridine-3-yl)ethyl)-6-(*p*-tolyl)-7*H*-[1,2,4] triazolo[3,4-*b*] [1,3,4]thiadiazine | 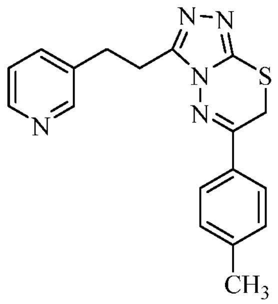 |
| 6-(4-Methoxyphenyl)-3-(2-(pyridine-3-yl)ethyl)-7*H*-[1,2,4]triazolo[3,4-*b*] [1,3,4]thiadiazine | 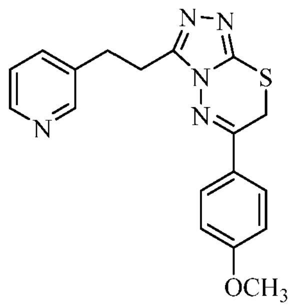 |
| 6-(Naphthalene-2-yl)-3-(2-(pyridine-3-yl)ethyl)-7*H*-[1,2,4]triazolo[3,4-*b*] [1,3,4]thiadiazine | 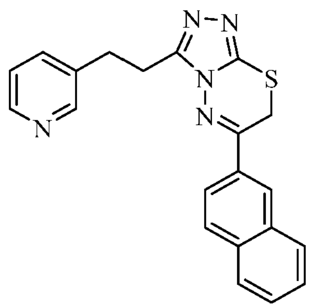 |
| 2-((4-Amino-5-(2-(pyridine-3-yl)ethyl)-4*H*-1,2,4-triazole-3-yl)thio)-*N*-(thiazole-2-yl)acetamide | 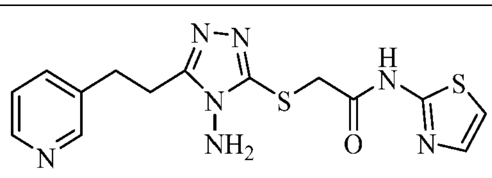 |
| 2-((4-Amino-5-(2-(pyridine-3-yl)ethyl)-4*H*-1,2,4-triazole-3-yl)thio)-*N*-(benzothiazole-2-yl) acetamide | 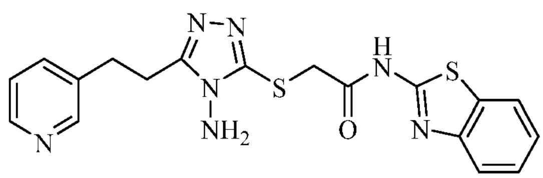 |

(continued)

| | |
|---|---|
| 2-((4-Amino-5-(2-(pyridine-3-yl)ethyl)-4*H*-1,2,4-triazole-3-yl)thio)-*N*-(6-fluorobenzothiazole-2-yl) acetamide | 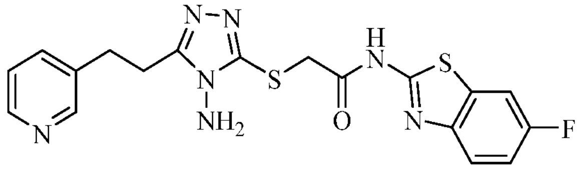 |
| 2-((4-Amino-5-(2-(pyridine-3-yl)ethyl)-4*H*-1,2,4-triazole-3-yl)thio)-*N*-(6-chlorobenzothiazole-2-yl) acetamide | 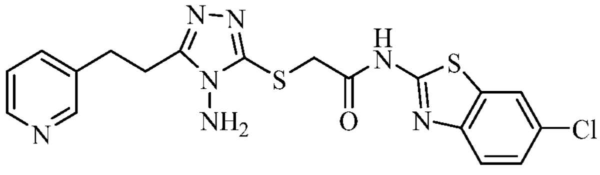 |
| 2-((4-Amino-5-(2-(pyridine-3-yl)ethyl)-4*H*-1,2,4-triazole-3-yl)thio)-*N*-(6-bromobenzothiazole-2-yl) acetamide | 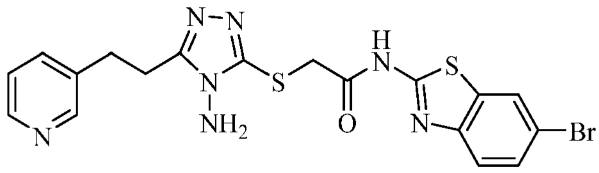 |
| 2-((4-Amino-5-(2-(pyridine-3-yl)ethyl)-4*H*-1,2,4-triazole-3-yl)thio)-*N*-(6-methylbenzothiazole-2-yl) acetamide | 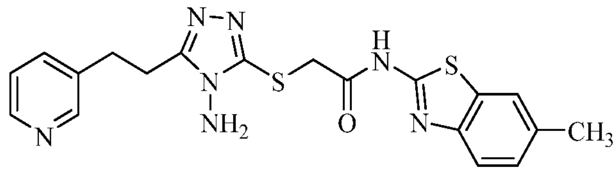 |
| 2-((4-Amino-5-(2-(pyridine-3-yl)ethyl)-4*H*-1,2,4-triazole-3-yl)thio)-*N*-(6-methoxybenzothiazole-2-yl)acetamide | 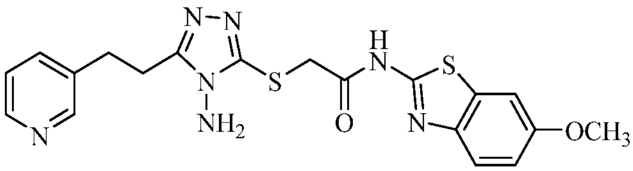 |

**2.** A compound according to claim 1, **characterized in that** at least one of the compounds, namely 6-Phenyl-3-(2-(pyridine-3-yl)ethyl)-7*H*-[1,2,4]triazolo[3,4-*b*][1,3,4]thiadiazine, 6-(4-Nitrophenyl)-3-(2-(pyridine-3-yl)ethyl)-7*H*-[1,2,4]triazolo[3,4-*b*][1,3,4]thiadiazine, 6-(4-Fluorophenyl)-3-(2-(pyridine-3-yl)ethyl)-7*H*-[1,2,4]triazolo[3,4-*b*][1,3,4]thiadiazine, 6-(4-Chlorophenyl)-3-(2-(pyridine-3-yl)ethyl)-7*H*-[1,2,4]triazolo[3,4-*b*][1,3,4]thiadiazine, 6-(Naphthalene-2-yl)-3-(2-(pyridine-3-yl)ethyl)-7*H*-[1,2,4]triazolo[3,4-*b*][1,3,4]thiadiazine, 2-((4-Amino-5-(2-(pyridine-3-yl)ethyl)-4H-1,2,4-triazole-3-yl)thio)-*N*-(thiazole-2-yl)acetamide, 2-((4-Amino-5-(2-(pyridine-3-yl)ethyl)-4H-1,2,4-triazole-3-yl)thio)-*N*-(benzothiazole-2-yl)acetamide, 2-((4-Amino-5-(2-(pyridine-3-yl)ethyl)-4*H*-1,2,4-triazole-3-yl)thio)-N-(6-fluorobenzothiazole-2-yl)acetamide, 2-((4-Amino-5-(2-(pyridine-3-yl)ethyl)-4*H*-1,2,4-triazole-3-yl)thio)-*N*-(6-chlorobenzothiazole-2-yl)acetamide, 2-((4-Amino-5-(2-(pyridine-3-yl)ethyl)-4*H*-1,2,4-triazole-3-yl)thio)-N-(6-methylbenzothiazole-2-yl)acetamide, 2-((4-Amino-5-(2-(pyridine-3-yl)ethyl)-4*H*-1,2,4-triazole-3-yl)thio)-*N*-(6-methoxybenzothiazole-2-yl)acetamide has selective anticancer activity against A549 human lung adenocarcinoma cells.

**3.** A compound according to claim 1, wherein the compound is selected between the group comprising of 6-Phenyl-3-(2-(pyridine-3-yl)ethyl)-7*H*-[1,2,4]triazolo[3,4-*b*][1,3,4]thiadiazine, 6-(4-Nitrophenyl)-3-(2-(pyridine-3-yl)ethyl)-7*H*-[1,2,4]triazolo[3,4-*b*][1,3,4]thiadiazine, 6-(4-Fluorophenyl)-3-(2-(pyridine-3-yl)ethyl)-7*H*-[1,2,4]triazolo[3,4-*b*][1,3,4]thiadiazine, 6-(4-Chlorophenyl)-3-(2-(pyridine-3-yl)ethyl)-7*H*-[1,2,4]triazolo[3,4-*b*][1,3,4]thiadiazine, 6-(Naphthalene-2-yl)-3-(2-(pyridine-3-yl)ethyl)-7*H*-[1,2,4]triazolo[3,4-*b*][1,3,4]thiadiazine, 2-((4-Amino-5-(2-(pyridine-3-yl)ethyl)-4*H*-1,2,4-triazole-3-yl)thio)-N-(thiazole-2-yl)acetamide, 2-((4-Amino-5-(2-(pyridine-3-yl)ethyl)-4*H*-1,2,4-triazole-3-yl)thio)-*N*-(benzothiazole-2-yl)acetamide, 2-((4-Amino-5-(2-(pyridine-3-yl)ethyl)-4*H*-1,2,4-triazole-3-yl)thio)-*N*-(6-fluorobenzothiazole-2-yl)acetamide, 2-((4-Amino-5-(2-(pyridine-3-yl)ethyl)-4*H*-1,2,4-triazole-3-yl)thio)-*N*-(6-chlorobenzothiazole-2-yl)acetamide, 2-((4-Amino-5-(2-(pyridine-3-yl)ethyl)-4*H*-1,2,4-triazole-3-yl)thio)-*N*-(6-methylbenzothiazole-2-yl)acetamide, 2-((4-Amino-5-(2-(pyridine-3-yl)ethyl)-4*H*-1,2,4-triazole-3-yl)thio)-*N*-(6-methoxybenzothiazole-2-yl)acetamide for use as an apoptosis-inducer in A549 human lung adenocarcinoma cells.

4. A compound according to claim 1, wherein the compound is selected between the group comprising of 6-(4-Fluorophenyl)-3-(2-(pyridine-3-yl)ethyl)-7*H*-[1,2,4]triazolo[3,4-*b*][1,3,4]thiadiazine, 6-(4-Chlorophenyl)-3-(2-(pyridine-3-yl)ethyl)-7*H*-[1,2,4]triazolo[3,4-*b*][1,3,4]thiadiazine, 2-((4-Amino-5-(2-(pyridine-3-yl)ethyl)-4*H*-1,2,4-triazole-3-yl)thio)-*N*-(benzothiazole-2-yl)acetamide, 2-((4-Amino-5-(2-(pyridine-3-yl)ethyl)-4*H*-1,2,4-triazole-3-yl)thio)-N-(6-chlorobenzothiazole-2-yl)acetamide and 2-((4-Amino-5-(2-(pyridine-3-yl)ethyl)-4*H*-1,2,4-triazole-3-yl)thio)-*N*-(6-methoxybenzothiazole-2-yl)acetamide for use as an Akt inhibitor in A549 human lung adenocarcinoma cells.

5. A compound for use according to claim 4, **characterized in that** the $IC_{50}$ value of 6-(4-Fluorophenyl)-3-(2-(pyridine-3-yl)ethyl)-7*H*-[1,2,4]triazolo[3,4-b][1,3,4]thiadiazine as an Akt inhibitor in A549 human lung adenocarcinoma cells is $2.95 \pm 0.05$ μg/mL.

6. A compound for use according to claim 4, **characterized in that** the $IC_{50}$ value of 6-(4-Chlorophenyl)-3-(2-(pyridine-3-yl)ethyl)-7*H*-[1,2,4]triazolo[3,4-*b*][1,3,4]thiadiazine as an Akt inhibitor in A549 human lung adenocarcinoma cell line is $0.80 \pm 0.10$ μg/mL.

7. A compound for use according to claim 4, **characterized in that** the $IC_{50}$ value of 2-((4-amino-5-(2-(pyridine-3-yl)ethyl)-4*H*-1,2,4-triazole-3-yl)thio)-*N*-(benzothiazole-2-yl)acetamide as an Akt inhibitor in A549 human lung adenocarcinoma cell line is $2.23 \pm 0.12$ μg/mL.

8. A compound for use according to claim 4, **characterized in that** the $IC_{50}$ value of 2-((4-amino-5-(2-(pyridine-3-yl)ethyl)-4*H*-1,2,4-triazole-3-yl)thio)-*N*-(6-chlorobenzothiazole-2-yl)acetamide as an Akt inhibitor in A549 human lung adenocarcinoma cell line is $0.19 \pm 0.05$ μg/mL.

9. A compound for use according to claim 4, **characterized in that** the $IC_{50}$ value of 2-((4-amino-5-(2-(pyridine-3-yl)ethyl)-4*H*-1,2,4-triazole-3-yl)thio)-*N*-(6-methoxybenzothiazole-2-yl)acetamide as an Akt inhibitor in A549 human lung adenocarcinoma cell line is $2.60 \pm 0.10$ μg/mL.

10. A pharmaceutical composition, comprising a compound according to any of the preceding claims.

11. The use of a pharmaceutical composition according to Claim 10 in the preparation of a drug to be used in the treatment of lung cancer.

12. A method for the synthesis of the compounds according to claim 1, namely 6-Phenyl-3-(2-(pyridine-3-yl)ethyl)-7*H*-[1,2,4]triazolo[3,4-*b*][1,3,4]thiadiazine, 6-(4-Nitrophenyl)-3-(2-(pyridine-3-yl)ethyl)-7H-[1,2,4]triazolo[3,4-*b*][1,3,4]thiadiazine, 6-(4-Fluorophenyl)-3-(2-(pyridine-3-yl)ethyl)-7*H*-[1,2,4]triazolo[3,4-*b*][1,3,4]thiadiazine, 6-(4-Chlorophenyl)-3-(2-(pyridine-3-yl)ethyl)-7*H*-[1,2,4]triazolo[3,4-*b*][1,3,4]thiadiazine, 6-(4-Bromophenyl)-3-(2-(pyridine-3-yl)ethyl)-7*H*-[1,2,4]triazolo[3,4-*b*][1,3,4]thiadiazine, 6-(4-Cyanophenyl)-3-(2-(pyridine-3-yl)ethyl)-7*H*-[1,2,4]triazolo[3,4-b][1,3,4]thiadiazine, 3-(2-(Pyridine-3-yl)ethyl)-6-(4-(trifluoromethyl)phenyl)-7*H*-[1,2,4]triazolo[3,4-*b*][1,3,4]thiadiazine,3-(2-(Pyridine-3-yl)ethyl)-6-(*p*-tolyl)-7*H*-[1,2,4]triazolo[3,4-*b*][1,3,4]thiadiazine, 6-(4-Methoxyphenyl)-3-(2-(pyridine-3-yl)ethyl)-7*H*-[1,2,4]triazolo[3,4-*b*][1,3,4]thiadiazine or 6-(Naphthalene-2-yl)-3-(2-(pyridine-3-yl)ethyl)-7*H*-[1,2,4]triazolo[3,4-*b*][1,3,4]thiadiazine, **characterized by** the following steps:

   • heating 4-amino-5-(2-(pyridine-3-yl)ethyl)-2,4-dihydro-3*H*-1,2,4-triazole-3-thione with 2-bromo-1-arylethanone derivative, namely 2-bromoacetophenone, 2-bromo-4'-nitroacetophenone, 2-bromo-4'-fluoroacetophenone, 2-bromo-4'-chloroacetophenone, 2,4'-dibromoacetophenone, 2-bromo-4'-cyanoacetophenone, 2-bromo-4'-trifluoromethylacetophenone, 2-bromo-4'-methylacetophenone, 2-bromo-4'-methoxyacetophenone or 2-bromo-2'-acetonaphthone at a 1:1 molar ratio in ethanol under reflux for 8 hours,
   • collecting the precipitate by filtration.

13. A method according to claim 12, **characterized in that** after the precipitate is collected by filtration and dried, it is subjected to crystallization process with ethanol for purification.

14. A method for the synthesis of the compounds according to claim 1, namely 2-((4-Amino-5-(2-(pyridine-3-yl)ethyl)-4*H*-1,2,4-triazole-3-yl)thio)-*N*-(thiazole-2-yl)acetamide, 2-((4-Amino-5-(2-(pyridine-3-yl)ethyl)-4*H*-1,2,4-triazole-3-yl)thio)-*N*-(benzothiazole-2-yl)acetamide, 2-((4-Amino-5-(2-(pyridine-3-yl)ethyl)-4*H*-1,2,4-triazole-3-yl)thio)-*N*-(6-fluorobenzothiazole-2-yl)acetamide, 2-((4-Amino-5-(2-(pyridine-3-yl)ethyl)-4*H*-1,2,4-triazole-3-yl)thio)-*N*-(6-chlorobenzothiazole-2-yl)acetamide, 2-((4-Amino-5-(2-(pyridine-3-yl)ethyl)-4*H*-1,2,4-triazole-3-yl)thio)-*N*-(6-bromobenzothiazole-2-yl)acetamide, 2-((4-Amino-5-(2-(pyridine-3-yl)ethyl)-4*H*-1,2,4-triazole-3-yl)thio)-*N*-(6-methylbenzothiazole-2-yl)acetamide or 2-((4-Amino-5-(2-(pyridine-3-yl)ethyl)-4*H*-1,2,4-triazole-3-yl)thio)-*N*-(6-methoxybenzothiazole-2-yl)acetamide **characterized by** the following steps:

- mixing 4-amino-5-(2-(pyridine-3-yl)ethyl)-2,4-dihydro-3*H*-1,2,4-triazole-3-thione with 2-chloro-*N*-(aryl)acetamide derivative, namely 2-chloro-*N*-(thiazole-2-yl)acetamide, 2-chloro-*N*-(benzothiazole-2-yl)acetamide, 2-chloro-*N*-(6-fluorobenzothiazole-2-yl)acetamide, 2-chloro-*N*-(6-chlorobenzothiazole-2-yl)acetamide, 2-chloro-*N*-(6-bromobenzothiazole-2-yl)acetamide, 2-chloro-*N*-(6-methylbenzothiazole-2-yl)acetamide or 2-chloro-*N*-(6-methoxybenzothiazole-2-yl)acetamide in the presence of potassium carbonate at a 1:1 molar ratio in acetone at room temperature for 12 hours,
- evaporating the solvent in the rotavapor,
- collecting the precipitate by filtration.

**15.** A method according to claim 14, **characterized in that** after the precipitate is collected by filtration, washed with water and dried, it is then subjected to crystallization process with ethanol for purification.

## Patentansprüche

**1.** Das Triazol- oder Triazolothiadiazin-Derivat, **dadurch gekennzeichnet, dass** es aus der folgenden Gruppe ausgewählt ist:

| | |
|---|---|
| 6-Phenyl-3 -(2-(pyridine-3-yl)ethyl)-7H-[1,2,4]triazolo [3,4-b][l,3,4]thiadiazin | 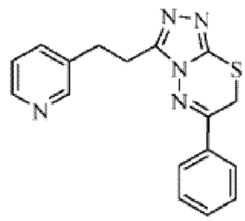 |
| 6-(4-Nitrophenyl)-3-(2-(pyridine-3-yl)ethyl)-7H-[1,2,4] triazolo[3,4-b][l,3,4]thiadiazin | 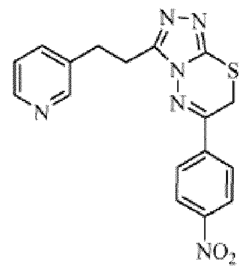 |
| 6-(4-Fluorophenyl)-3-(2-(pyridine-3-yl)ethyl)-7H-[1,2,4] triazolo[3,4-b][1,3,4]thiadiazin | 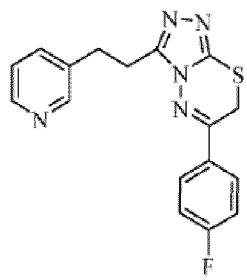 |

(fortgesetzt)

| | |
|---|---|
| 6-(4-Chlorophenyl)-3-(2-(pyridine-3-yl)ethyl)-7H-[1,2,4] triazolo[3,4-b][1,3,4]thiadiazin | 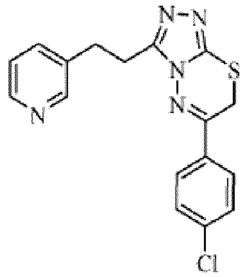 |
| 6-(4-Bromophenyl)-3-(2-(pyridine-3-yl)ethyl)-7H-[1,2,4] triazolo[3,4-b][1,3,4]thiadiazin | 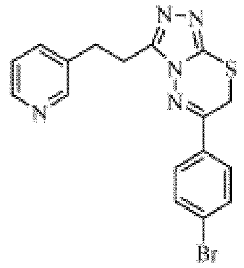 |
| 6-(4-Cyanophenyl)-3-(2-(pyridine-3-yl)ethyl)-7H-[1,2,4] triazolo[3,4-b][l,3,4]thiadiazin | 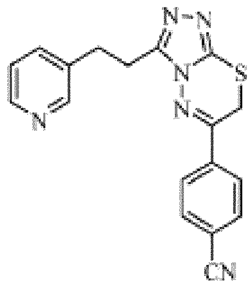 |
| 3-(2-(Pyridine-3-yl)ethyl)-6-(4-(trifluoromethyl)phenyl)-7H-[1,2,4]triazolo[3,4-b] [1,3,4]thiadiazin | 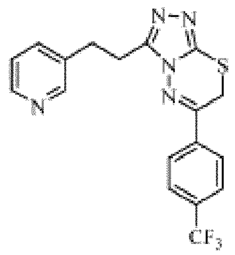 |
| 3-(2-(Pyridine-3-yl)ethyl)-6-(p-tolyl)-7H-[1,2,4]triazolo [3,4-b] [1,3,4]thiadiazin | 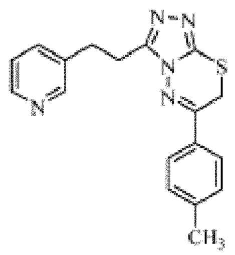 |
| 6-(4-Methoxyphenyl)-3-(2-(pyridine-3-yl)ethyl)-7H-[1,2,4]triazolo[3,4-b] [1,3,4]thiadiazin | 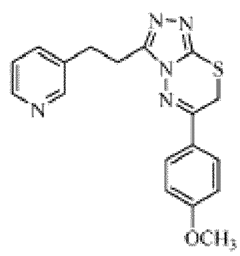 |

(fortgesetzt)

| | |
|---|---|
| 6-(Naphthalene-2-yl)-3-(2-(pyridine-3-yl)ethyl)-7H-[1,2,4]triazolo[3,4-b] [1,3,4]thiadiazin | 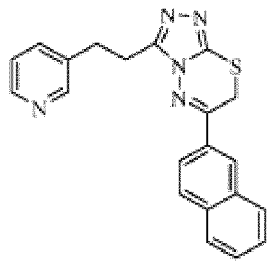 |
| 2-((4-Amino-5-(2-(pyridine-3-yl)ethyl)-4H-1,2,4-triazole-3-yl)thio)-N- (thiazole-2-yl)acetamid | 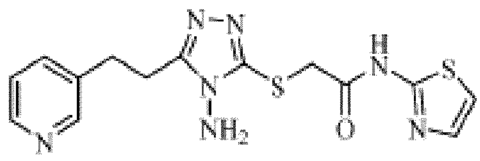 |
| 2-((4-Amino-5-(2-(pyridin-3-yl)ethyl)-4H-1,2,4-triazol-3-yl)thio)-*N*-(benzothiazol-2-yl)acetamid | 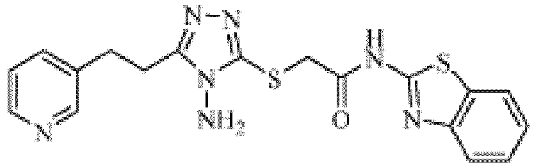 |
| 2-((4-Amino-5-(2-(pyridin-3-yl)ethyl)-4H-1,2,4-triazol-3-yl)thio)-N-(6-fluorbenzothiazol-2-yl)acetamid | 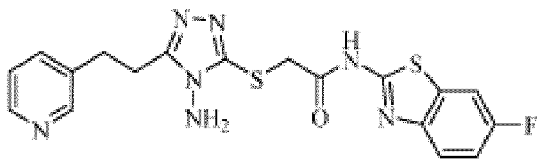 |
| 2-((4-Amino-5-(2-(pyridin-3-yl)ethyl)-4H-1,2,4-triazol-3-yl)thio)-N-(6-Chlorbenzothiazol-2-yl)acetamid | 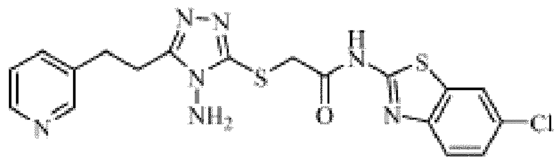 |
| 2-((4-Amino-5-(2-(pyridin-3-yl)ethyl)-4H-1,2,4-triazol-3-yl)thio)-N-(6-brombenzothiazol-2-yl)acetamid | 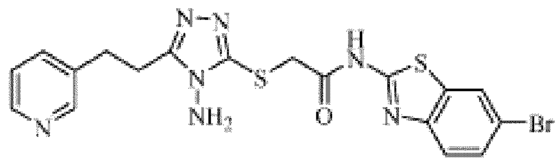 |
| 2-((4-Amino-5-(2-(pyridin-3-yl)ethyl)-4H-1,2,4-triazol-3-yl)thio)-N-(6-methylbenzothiazol-2-yl)acetamid | 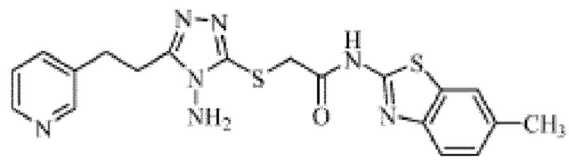 |
| 2-((4-Amino-5-(2-(pyridin-3-yl)ethyl)-4H-1,2,4-triazol-3-yl)thio)-N-(6-methoxybenzothiazol-2-yl)acetamid | 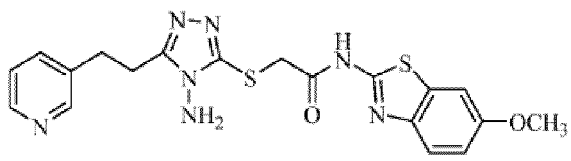 |

**2.** Eine Verbindung nach Anspruch 1,
**dadurch gekennzeichnet, dass** mindestens eine der Verbindungen, 6-Phenyl-3-(2-(pyridin-3-yl)ethyl)-7H-[1,2,4]triazolo[3,4-b][1,3,4]thiadiazin, 6-(4-Nitrophenyl)-3-(2-(pyridine-3-yl)ethyl)-7H-[1,2,4]triazolo[3,4-b][1,3,4]thiadiazine,6-(4-Fluorophenyl)-3-(2-(pyridine-3-yl)ethyl)-7H-[1,2, 4]triazolo[3,4-b][l,3,4]thiadiazin, 6-(4-Chlorophenyl)-3-(2-(pyridine-3-yl)ethyl)-7H-[1,2,4]triazolo[3,4-b][l,3,4]thiadiazine,

6-(Naphthalene-2-yl)-3-(2- (pyridine-3-yl)ethyl)-7H-[1, 2,4]triazolo[3,4-b][1,3,4]thiadiazin, 2-((4-Amino-5-(2-(pyridine-3-yl)ethyl)-4H-1,2,4-triazole-3-yl)thio)-N-(thiazole-2-yl)acetamide, 2-((4-Amino-5-(2-(pyridine-3-yl)ethyl)-4H-1,2,4-triazole-3-yl)thio)-N-(benzothiazole-2-yl)acetamide, 2-((4-Amino-5-(2-(pyridine-3-yl)ethyl)-4H-1,2, 4-triazol-3-yl)thio)-N-(6-fluorbenzothiazol-2-yl)acetamid, 2-((4-Amino-5-(2-(pyridin-3-yl)ethyl)-4H-1,2,4-triazol-3-yl)thio)-N-(6-chlorbenzothiazol-2-yl)acetamid, 2-((4-Amino-5-(2-(pyridine-3-yl)ethyl)-4H-1,2,4-triazole-3-yl)thio)-N-(6-methylbenzothiazole-2-yl)acetamide, 2-((4-Amino-5-(2-(pyridine-3-yl)ethyl)-4H-1,2,4-Triazol-3-yl)thio)-N-(6-Methoxybenzothiazol-2-yl)acetamid hat eine selektive krebshemmende Wirkung gegen A549-Lungen-Adenokarzinomzellen.

**3.** Eine Verbindung nach Anspruch 1,
wobei die Verbindung ausgewählt ist aus der Gruppe
bestehend aus 6-Phenyl-3-(2-(pyridin-3-yl)ethyl)-7H-[1,2,4]triazolo[3,4-b][1, 3,4]thiadiazin, 6-(4-Nitrophenyl)-3-(2-(pyridine-3-yl)ethyl)-7H-[1,2,4]triazolo[3,4-b][1,3,4]thiadiazine, 6-(4-Fluorophenyl)-3-(2-(pyridine-3-yl)ethyl)-7H-[1,2,4]triazolo[3,4-b][1,3,4]thiadiazin, 6-(4-Chlorophenyl)-3-(2-(pyridine-3-yl)ethyl)-7H-[1,2,4]triazolo[3,4-b][l,3,4]thiadiazine, 6-(Naphthalene-2-yl)-3-(2- (pyridine-3-yl)ethyl)-7H-[1, 2,4]triazolo[3,4-b][1,3,4]thiadiazin, 2-((4-Amino-5-(2-(pyridine-3-yl)ethyl)-4H-1,2,4-triazole-3-yl)thio)-N-(thiazole-2-yl)acetamide, 2-((4-Amino-5-(2-(pyridine-3-yl)ethyl)-4H-1,2,4-triazole-3-yl)thio)-N-(benzothiazole-2-yl)acetamide, 2-((4-Amino-5-(2-(pyridine-3-yl)ethyl)-4H-1,2, 4-triazol-3-yl)thio)-N-(6-fluorbenzothiazol-2-yl)acetamid, 2-((4-Amino-5-(2-(pyridin-3-yl)ethyl)-4H-1,2,4-triazol-3-yl)thio)-N-(6-chlorbenzothiazol-2-yl)acetamid, 2-((4-Amino-5-(2-(pyridine-3-yl)ethyl)-4H-1,2,4-triazole-3-yl)thio)-N-(6-methylbenzothiazole-2-yl)acetamide, 2-((4-Amino-5-(2-(pyridine-3-yl)ethyl )-4H-1, 2,4-Triazol-3-yl)thio)-N-(6-Methoxybenzothiazol-2-yl)acetamid zur Verwendung als Apoptose-Induzierer in A549 menschlichen Lungenadenokarzinomzellen.

**4.** Eine Verbindung nach Anspruch 1,
wobei die Verbindung ausgewählt ist aus der Gruppe
bestehend aus 6-(4-Fluorphenyl)-3-(2-(pyridin-3-yl)ethyl)-7H-[1,2,4]triazolo[3, 4-b][1,3,4]thiadiazin,6-(4-Chlorophenyl)-3-(2-(pyridine-3-yl)ethyl)-7H-[1,2,4]triazolo[3,4-b][1,3,4]thiadiazine, 2-((4-Amino-5-(2-(pyridine-3-yl)ethyl)-4H-1,2, 4-triazol-3-yl)thio)-N-(benzothiazol-2-yl)acetamid, 2-((4-Amino-5-(2-(pyridin-3-yl)ethyl)-4H-1,2,4-triazol-3-yl)thio)-N-(6-chlorbenzothiazol-2-yl)acetamid und 2-((4-Amino-5-(2-(pyridin-3-yl)ethyl)-4H-1, 2,4-Triazol-3-yl)thio)-N-(6-Methoxybenzothiazol-2-yl)acetamid zur Verwendung als Akt-Inhibitor in A549-Lungenadenokarzinomzellen.

**5.** Verbindung zur Verwendung nach Anspruch 4,
**dadurch gekennzeichnet, dass** der $IC_{50}$-Wert von 6-(4-Fluorphenyl)-3-(2-(pyridin-3-yl)ethyl)-7H-[1,2,4]triazolo[3,4-b][1,3,4]thiadiazin als Akt-Inhibitor in A549 menschlichen Lungen-Adenokarzinomzellen $2{,}95 \pm 0{,}05$ µg/mL beträgt.

**6.** Verbindung zur Verwendung nach Anspruch 4,
**dadurch gekennzeichnet, dass** der $IC_{50}$-Wert von 6-(4-Chlorphenyl)-3-(2-(pyridin-3-yl)ethyl)-7H-[1,2,4]triazolo[3,4-b][1,3,4]thiadiazin als Akt-Inhibitor in der A549-Zelllinie des menschlichen Lungenadenokarzinoms $0{,}80 \pm 0{,}10$ µg/mL beträgt.

**7.** Verbindung zur Verwendung nach Anspruch 4,
**dadurch gekennzeichnet, dass** der $IC_{50}$-Wert von 2-((4-Amino-5-(2-(pyridin-3-yl)ethyl)-4H-1,2,4-triazol-3-yl)thio)-N-(benzothiazol-2- yl)acetamid als Akt-Inhibitor in der A549-Zelllinie des menschlichen Lungenadenokarzinoms $2{,}23 \pm 0{,}12$ µg/mL beträgt.

**8.** Verbindung zur Verwendung nach Anspruch 4,
**dadurch gekennzeichnet, dass** der $IC_{50}$-Wert von 2-((4-Amino-5-(2-(pyridin-3-yl)ethyl)-4H-1,2,4-triazol-3-yl)thio)-N-(6-chlorbenzothiazol-2- yl)acetamid als Akt-Inhibitor in der A549-Zelllinie des menschlichen Lungenadenokarzinoms $0{,}19 \pm 0{,}05$ µg/mL beträgt.

**9.** Verbindung zur Verwendung nach Anspruch 4,
**dadurch gekennzeichnet, dass** der $IC_{50}$-Wert von 2-((4-Amino-5-(2-(pyridin-3-yl)ethyl)-4H-1,2,4-triazol-3-yl)thio)-N-(6-methoxybenzothiazol-2- yl)acetamid als Akt-Inhibitor in der A549-Zelllinie des menschlichen Lungenadenokarzinoms $2{,}60 \pm 0{,}10$ µg/mL beträgt.

**10.** Pharmazeutische Zusammensetzung, enthaltend eine Verbindung nach einem der vorangehenden Ansprüche.

**11.** Verwendung einer pharmazeutischen Zusammensetzung nach Anspruch 10 bei der Herstellung eines Arzneimittels zur Verwendung bei der Behandlung von Lungenkrebs.

**12.** Verfahren zur Synthese der Verbindungen nach Anspruch 1, nämlich 6-Phenyl-3-(2-(pyridin-3-yl)ethyl)-7H-[1,2,4]triazolo[3,4-b][1,3,4]thiadiazin, 6-(4-Nitrophenyl)-3-(2-(pyridin-3-yl)ethyl)-7H-[1,2,4]triazolo[3,4-b][1,3,4]thiadiazin, 6-(4-Fluorophenyl)-3-(2-(pyridine-3-yl)ethyl)-7H-[1,2,4]triazolo[3,4-b][1,3,4]thiadiazine, 6-(4-Chlorophenyl)-3-(2-(pyridine-3-yl)ethyl)-7H-[1, 2,4]triazolo[3,4-b][1,3,4]thiadiazin, 6-(4-Bromophenyl)-3-(2-(pyridine-3-yl)ethyl)-7H-[1,2,4]triazolo[3,4-b][l,3, 4]thiadiazin, 6-(4-Cyanophenyl)-3-(2-(pyridine-3-yl)ethyl)-7H-[1,2,4]triazolo[3,4-b][1,3,4]thiadiazine, 3-(2-(Pyridine-3-yl)ethyl)-6-(4-(trifluoromethyl)phenyl)-7H-[1,2,4]triazolo[3,4-b][1,3,4]thiadiazine,3-(2-(Pyridine-3-yl)ethyl)-6-(p-tolyl)-7H-[1,2, 4]triazolo[3,4-b][1,3,4]thiadiazin, 6-(4-Methoxyphenyl)-3-(2-(pyridine-3-yl)ethyl)-7H-[1,2,4]triazolo[3,4-b][1,3,4]thiadiazin oder 6-(Naphthalin-2-yl)-3-(2-(pyridin-3-yl)ethyl)-7H-[1,2,4]triazolo[3,4-b][1,3,4]thiadiazin, **gekennzeichnet durch** die folgenden Schritte:

- Erhitzen von 4-Amino-5-(2-(pyridin-3-yl)ethyl)-2,4-dihydro-3H-1,2,4-triazol-3-thion mit 2-Brom-1-arylethanon-Derivaten, nämlich 2-Bromacetophenon, 2-Brom-4'-nitroacetophenon, 2-Brom-4'-fluoracetophenon, 2-Brom-4'-Chloracetophenon, 2,4'-Dibromacetophenon, 2-Brom-4'-Cyanacetophenon, 2-Brom-4'-Trifluormethylacetophenon, 2-Brom-4'-Methylacetophenon, 2-Brom-4'-Methoxyacetophenon oder 2-Brom-2'-Acetonaphthon in einem 1: 1 molaren Verhältnis in Ethanol unter Rückfluss für 8 Stunden,
- Auffangen des Niederschlags durch Filtration.

**13.** Verfahren nach Anspruch 12,
**dadurch gekennzeichnet, dass** der Niederschlag, nachdem er durch Filtration gesammelt und getrocknet wurde, zur Reinigung einem Kristallisationsverfahren mit Ethanol unterzogen wird.

**14.** Verfahren zur Synthese der Verbindungen nach Anspruch 1, nämlich 2-((4-Amino-5-(2-(pyridin-3-yl)ethyl)-4H-1,2,4-triazol-3-yl)thio)-N-(thiazol-2-yl)acetamid, 2-((4-Amino-5-(2-(pyridin-3-yl)ethyl)-4H-1, 2,4-Triazol-3-yl)thio)-N-(benzothiazol-2-yl)acetamid, 2-((4-Amino-5-(2-(pyridin-3-yl)ethyl)-4H-1,2,4-triazol-3-yl)thio)-N-(6-fluorbenzothiazol-2-yl)acetamid, 2-((4-Amino-5-(2-(pyridin-3-yl)ethyl)-4H-1,2, 4-Triazol-3-yl)thio)-N-(6-Chlorbenzothiazol-2-yl)acetamid, 2-((4-Amino-5-(2-(Pyridin-3-yl)ethyl)-4H-1,2,4-triazol-3-yl)thio)-N-(6-Brombenzothiazol-2-yl)acetamid, 2-((4-Amino-5-(2-(Pyridin-3-yl)ethyl)-4H-1, 2,4-triazol-3-yl)thio)-N-(6-methylbenzothiazol-2-yl)acetamid oder 2-((4-Amino-5-(2-(pyridin-3-yl)ethyl)-4H-1,2,4-triazol-3-yl)thio)-N-(6-methoxybenzothiazol-2-yl)acetamid, **gekennzeichnet durch** die folgenden Schritte:

- Mischen von 4-Amino-5-(2-(pyridin-3-yl)ethyl)-2,4-dihydro-3H-1,2,4-triazol-3- thion mit 2-Chlor-N-(aryl)acetamid-Derivat, nämlich 2-Chlor-N-(thiazol-2-yl)acetamid, 2-Chlor-N-(benzothiazol-2-yl)acetamid, 2-Chlor-N-(6-fluorbenzothiazol-2-yl)acetamid, 2-Chlor-N-(6-chlorbenzothiazol-2-yl)acetamid, 2-Chlor-N-(6-brombenzothiazol-2-yl)acetamid, 2-Chlor-N-(6-methylbenzothiazol-2-yl)acetamid oder 2-Chlor-N-(6-methoxybenzothiazol-2-yl)-acetamid in Gegenwart von Kaliumcarbonat in einem 1: 1 molaren Verhältnis in Aceton bei Raumtemperatur 12 Stunden lang,
- Verdampfen des Lösungsmittels im Rotavapor,
- Auffangen des Niederschlags durch Filtration.

**15.** Verfahren nach Anspruch 4,

**dadurch gekennzeichnet, dass** der Niederschlag nach dem Sammeln durch Filtration,
Waschen mit Wasser und Trocknen einem Kristallisationsverfahren mit Ethanol zur Reinigung unterzogen wird.

## Revendications

**1.** Dérivé de triazole ou de triazolothiadiazine, **caractérisé en ce qu'**il est choisi dans le groupe suivant:

| | |
|---|---|
| 6-phényl-3-(2-(pyridine-3-yl)éthyl)-7H-[1,2,4]triazolo [3,4-b][l,3,4]thiadiazine | 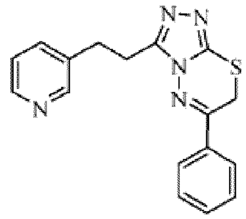 |
| 6-(4-Nitrophényl)-3-(2-(pyridine-3-yl)éthyl)-7H-[1,2,4] triazolo[3,4-b][l,3,4]thiadiazine | 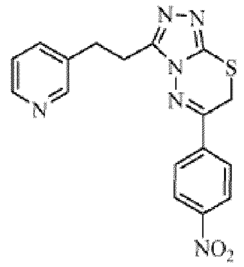 |
| 6-(4-Fluorophényl)-3-(2-(pyridine-3-yl)éthyl)-7H-[1,2,4] triazolo[3,4-b][1,3,4]thiadiazine | 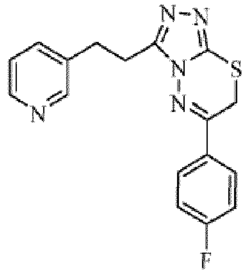 |
| 6-(4-Chlorophényl)-3-(2-(pyridine-3-yl)éthyl)-7H-[1,2,4] triazolo[3,4-b][1,3,4]thiadiazine | 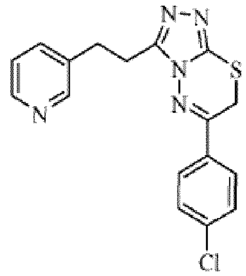 |
| 6-(4-Bromophényl)-3-(2-(pyridine-3-yl)éthyl)-7H-[1,2,4] triazolo[3,4-b][l,3,4]thiadiazine | 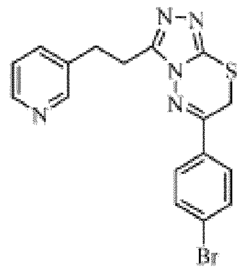 |
| 6-(4-Cyanophényl)-3-(2-(pyridine-3-yl)éthyl)-7H-[1,2,4] triazolo[3,4-b][l,3,4]thiadiazine | 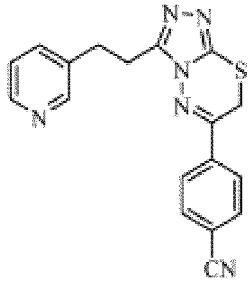 |

(suite)

| | |
|---|---|
| 3-(2-(Pyridine-3-yl)éthyl)-6-(4-(trifluorométhyl)phényl)-7H-[1,2,4]triazolo[3,4-b] [1,3,4]thiadiazine | 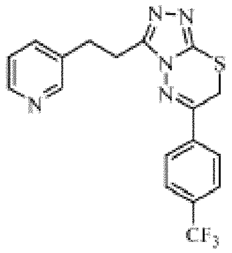 |
| 3-(2-(Pyridine-3-yl)éthyl)-6-(p-tolyl)-7H-[1,2,4]triazolo [3,4-b] [1,3,4]thiadiazine | 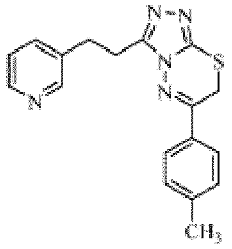 |
| 6-(4-Méthoxyphényl)-3-(2-(pyridine-3-yl)éthyl)-7H-[1,2,4]triazolo[3,4-b] [1,3,4]thiadiazine | 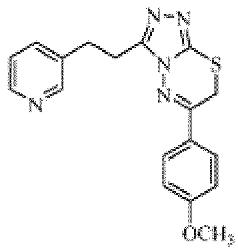 |
| 6-(Naphthalène-2-yl)-3-(2-(pyridine-3-yl)éthyl)-7H-[1,2,4]triazolo[3,4-b] [1,3,4]thiadiazine | 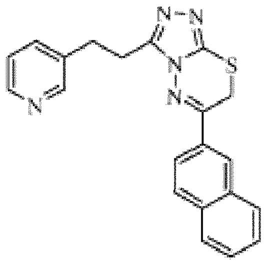 |
| 2-((4-Amino-5-(2-(pyridine-3-yl)éthyl)-4H-1,2,4-triazole-3-yl)thio)-N- (thiazole-2-yl)acétamide | 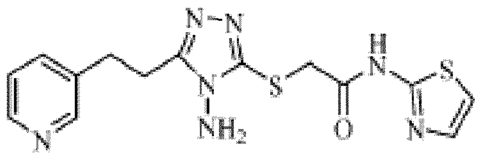 |
| 2-((4-Amino-5-(2-(pyridine-3-yl)éthyl)-4H-1,2,4-triazole-3-yl)thio)-*N*-(benzothiazole-2-yl)acétamide | 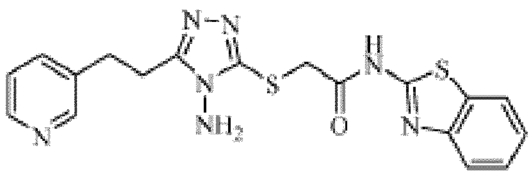 |
| 2-((4-Amino-5-(2-(pyridine-3-yl)éthyl)-4H-1,2,4-triazole-3-yl)thio)-N-(6- fluorobenzothiazole-2-yl)acétamide | 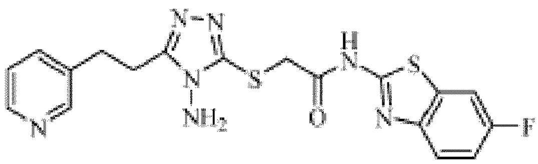 |

(suite)

| | |
|---|---|
| 2-((4-Amino-5-(2-(pyridine-3-yl)éthyl)-4H-1,2,4-triazole-3-yl)thio)-N-(6- chlorobenzothiazole-2-yl)acétamide | 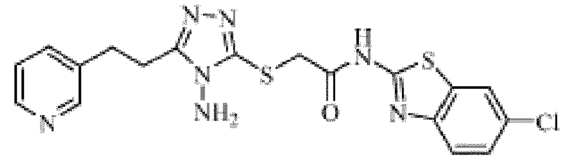 |
| 2-((4-Amino-5-(2-(pyridine-3-yl)éthyl)-4H-1,2,4-triazole-3-yl)thio)-N-(6- bromobenzothiazole-2-yl)acétamide | 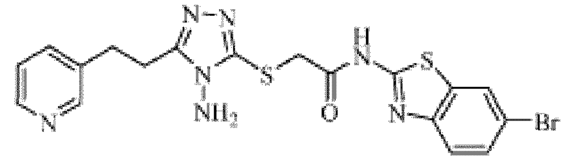 |
| 2-((4-Amino-5-(2-(pyridine-3-yl)éthyl)-4H-1,2,4-triazole-3-yl)thio)-N-(6- méthylbenzothiazole-2-yl)acétamide | 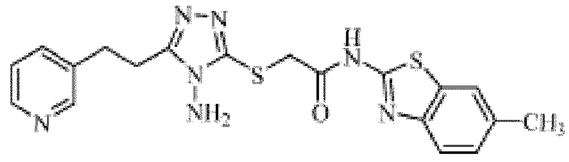 |
| 2-((4-Amino-5-(2-(pyridine-3-yl)éthyl)-4H-1,2,4-triazole-3-yl)thio)-N-(6-méthoxybenzothiazole-2-yl)acétamide | 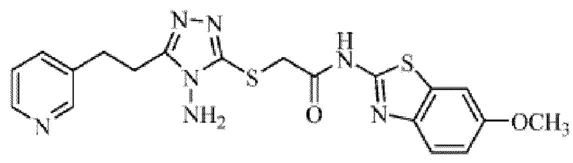 |

**2.** Composé selon la revendication 1, **caractérisé en ce qu'** au moins un des composés, à savoir le 6-Phényl-3-(2-(pyridine-3-yl)éthyl)-7H-[1,2,4]triazolo[3,4-b][1,3,4]thiadiazine, 6-(4-Nitrophényl)-3-(2-(pyridine-3-yl)éthyl)-7H-[1,2,4]triazolo[3,4-b][1,3,4]thiadiazine,6-(4-Fluorophényl)-3-(2-(pyridine-3-yl)éthyl)-7H-[1,2,4]triazolo[3,4-b][l,3,4]thiadiazine, 6-(4-Chlorophényl)-3-(2-(pyridine-3-yl)éthyl)-7H-[1,2,4]triazolo[3,4-b][l,3,4]thiadiazine, 6-(Naphthalène-2-yl)-3-(2- (pyridine-3-yl)éthyl)-7H-[1,2,4]triazolo[3,4-b][1,3,4]thiadiazine, 2-((4-Amino-5-(2-(pyridine-3-yl)éthyl)-4H-1,2,4-triazole-3-yl)thio)-N-(thiazole-2-yl)acétamide, 2-((4-Amino-5-(2-(pyridine-3-yl)éthyl)-4H-1,2,4-triazole-3-yl)thio)-N-(benzothiazole-2-yl)acétamide, 2-((4-Amino-5-(2-(pyridine-3-yl)éthyl)-4H-1,2,4-triazole-3-yl)thio)-N-(6-fluorobenzothiazole-2-yl)acétamide, 2-((4-Amino-5-(2-(pyridine-3-yl)éthyl)-4H-1,2,4-triazole-3-yl)thio)-N-(6-chlorobenzothiazole-2-yl)acétamide, 2-((4-Amino-5-(2- (pyridine-3-yl)éthyl)-4H-1,2,4-triazole-3-yl)thio)-N-(6-méthylbenzothiazole-2-yl)acétamide, 2-((4-Amino-5-(2-(pyridine-3-yl)éthyl)-4H-1,2,4-triazole-3-yl)thio)-N-(6-methoxybenzothiazole-2-yl)acétamide a une activité anticancéreuse sélective contre les cellules d'adénocarcinome du poumon humain A549.

**3.** Composé selon la revendication 1, dans lequel le composé est choisi parmi le groupe comprenant le 6-Phényl-3-(2-(pyridine-3-yl)éthyl)-7H-[1,2,4]triazolo[3,4-b][1,3,4]thiadiazine, 6-(4-Nitrophényl)-3-(2-(pyridine-3-yl)éthyl)-7H-[1,2,4]triazolo[3,4-b][1,3,4]thiadiazine, 6-(4-Fluorophényl)-3-(2-(pyridine-3-yl)éthyl)-7H-[1,2,4]triazolo[3,4-b][l,3,4]thiadiazine, 6-(4-Chlorophényl)-3-(2-(pyridine-3-yl)éthyl)-7H-[1,2,4]triazolo[3,4-b][l,3,4]thiadiazine, 6-(Naphthalène-2-yl)-3-(2- (pyridine-3-yl)éthyl)-7H-[1,2,4]triazolo[3,4-b][1,3,4]thiadiazine, 2-((4-Amino-5-(2-(pyridine-3-yl)éthyl)-4H-1,2,4-triazole-3-yl)thio)-N-(thiazole-2-yl)acétamide, 2-((4-Amino-5-(2-(pyridine-3-yl)éthyl)-4H-1,2,4-triazole-3-yl)thio)-N-(benzothiazole-2-yl)acétamide, 2-((4-Amino-5-(2-(pyridine-3-yl)éthyl)-4H-1,2,4-triazole-3-yl)thio)-N-(6-fluorobenzothiazole-2-yl)acétamide, 2-((4-Amino-5-(2-(pyridine-3-yl)éthyl)-4H-1,2,4-triazole-3-yl)thio)-N-(6-chlorobenzothiazole-2-yl)acétamide, 2-((4-Amino-5-(2-(pyridine-3-yl)éthyl)-4H-1,2,4-triazole-3-yl)thio)-N-(6-méthylbenzothiazole-2-yl)acétamide, 2-((4-Amino-5-(2-(pyridine-3-yl)éthyl)-4H-1,2,4-triazole-3-yl)thio)-N-(6-methoxybenzothiazole-2-yl)acétamide pour être utilisé comme inducteur d'apoptose dans les cellules d'adénocarcinome du poumon humain A549.

**4.** Composé selon la revendication 1, dans lequel le composé est choisi parmi le groupe comprenant le 6-(4-Fluorophényl)-3-(2-(pyridine-3-yl)éthyl)-7H-[1,2,4]triazolo[3,4-b][1,3,4]thiadiazine, 6-(4-Chlorophényl)-3-(2-(pyridine-3-yl)éthyl)-7H-[1,2,4]triazolo[3,4-b][1,3,4]thiadiazine, 2-((4-Amino-5-(2-(pyridine-3-yl)éthyl)-4H- 1,2,4-triazole-3-yl)thio)-N-(benzothiazole-2-yl)acétamide, 2-((4-Amino-5-(2-(pyridine-3-yl)éthyl)-4H-1,2,4-triazole-3-yl)thio)-N-(6-chlorobenzothiazole-2- yl)acétamide et 2-((4-Amino-5-(2-(pyridine-3-yl)éthyl)-4H-1,2,4-triazole-3-yl)thio)-N-(6-methoxybenzothiazole-2-yl)acétamide pour être utilisé comme inhibiteur de l'Akt dans les cellules d'adénocarcinome du poumon humain A549.

5. Composé selon la revendication 4, **caractérisé en ce que** la valeur ICso de 6-(4-Fluorophényl)-3-(2-(pyridine-3-yl)éthyl)-7H-[1,2,4]triazolo[3,4-b][1,3,4]thiadiazine en tant qu'inhibiteur de l'Akt dans les cellules d'adénocarcinome du poumon humain A549 est de 2.95 ± 0.05 μg/mL.

6. Composé selon la revendication 4, **caractérisé en ce que** la valeur ICso de 6-(4-Chlorophényl)-3-(2-(pyridine-3-yl)éthyl)-7H-[1,2,4]triazolo[3,4-b][1,3,4]thiadiazine en tant qu'inhibiteur de l'Akt dans les cellules d'adénocarcinome du poumon humain A549 est de 0.80 ± 0.10 μg/mL.

7. Composé selon la revendication 4, **caractérisé en ce que** la valeur ICso de 2-((4-amino-5-(2-(pyridine-3-yl)éthyl)-4H-1,2,4-triazole-3-yl)thio)-N-(benzothiazole-2- yl)acétamide en tant qu'inhibiteur de l'Akt dans les cellules d'adénocarcinome du poumon humain A549 est de 2.23 ± 0.12 μg/mL.

8. Composé selon la revendication 4, **caractérisé en ce que** la valeur ICso de 2-((4-amino-5-(2-(pyridine-3-yl)éthyl)-4H-1,2,4-triazole-3-yl)thio)-N-(6-chlorobenzothiazole-2-yl)acétamide en tant qu'inhibiteur de l'Akt dans les cellules d'adénocarcinome du poumon humain A549 est de 0.19 ± 0.05 μg/mL.

9. Composé selon la revendication 4, **caractérisé en ce que** la valeur ICso de 2-((4-amino-5-(2-(pyridine-3-yl)éthyl)-4H-1,2,4-triazole-3-yl)thio)-N-(6-méthoxybenzothiazole-2-yl)acétamide en tant qu'inhibiteur de l'Akt dans les cellules d'adénocarcinome du poumon humain A549 est de 2.60 ± 0.10 μg/mL.

10. Composition pharmaceutique comprenant un composé selon l'une quelconque des revendications précédentes.

11. Utilisation d'une composition pharmaceutique selon la Revendication 10 dans la préparation d'un médicament destiné à être utilisé dans le traitement du cancer du poumon.

12. Procédé de synthèse des composés selon la revendication 1, à savoir le 6-Phényl-3-(2-(pyridine-3-yl)éthyl)-7H-[1,2,4]triazolo[3,4-b][1,3,4]thiadiazine, 6-(4-Nitrophényl)-3-(2-(pyridine-3-yl)éthyl)-7H-[1,2,4]triazolo[3,4-b][l,3,4]thiadiazine, 6-(4-Fluorophényl)-3-(2-(pyridine-3-yl)éthyl)-7H-[1,2,4]triazolo[3,4-b][1,3,4]thiadiazine, 6-(4-Chlorophényl)-3-(2-(pyridine-3-yl)éthyl)-7H-[1,2,4]triazolo[3,4-b][1,3,4]thiadiazine, 6-(4-Bromophényl)-3-(2-(pyridine-3-yl)éthyl)-7H-[1,2,4]triazolo[3,4-b][l,3,4]thiadiazine, 6-(4-Cyanophényl)-3-(2-(pyridine-3-yl)éthyl)-7H-[1,2,4]triazolo[3,4-b][1,3,4]thiadiazine, 3-(2-(Pyridine-3-yl)éthyl)-6-(4-(trifluorométhyl)phényl)-7H-[1,2,4]triazolo[3,4-b][1,3,4]thiadiazine,3-(2-(Pyridine-3-yl)éthyl)-6-(p-tolyl)-7H-[1,2,4]triazolo[3,4-b][1,3,4]thiadiazine, 6-(4-Méthoxyphényl)-3-(2-(pyridine-3-yl)éthyl)-7H-[1,2,4]triazolo[3,4-b][1,3,4]thiadiazine ou le 6-(Naphthalène-2-yl)-3-(2-(pyridine-3-yl)éthyl)-7H-[1,2,4]triazolo[3,4-b][1,3,4]thiadiazine, **caractérisée par** les étapes suivantes :

- le chauffage de 4-amino-5-(2-(pyridine-3-yl)éthyl)-2,4-dihydro-3H-1,2,4-triazole-3-thione avec un dérivé de 2-bromo-1-aryléthanone dérivative, à savoir 2-bromoacétophénone, 2-bromo- 4'-nitroacétophénone, 2-bromo-4'-fluoroacétophénone, 2-bromo-4'-chloroacétophénone, 2,4'-dibromoacétophénone, 2-bromo-4'-cyanoacétophénone, 2-bromo-4'-trifluorométhylacétophénone, 2-bromo-4'-méthylacétophénone, 2-bromo-4'-méthoxyacétophénone ou 2-bromo-2'-acétonaphthone dans un rapport molaire 1:1 dans l'éthanol à reflux pendant 8 heures,
- la collecte du précipité par filtration.

13. Procédé selon la revendication 12, **caractérisé en ce qu'**après que le précipité a été collecté par filtration et séché, il est soumis à un processus de cristallisation avec de l'éthanol pour purification.

14. Procédé de synthèse des composés selon la revendication 1, à savoir 2-((4-Amino-5-(2-(pyridine-3-yl)éthyl)-4H-1,2,4-triazole-3-yl)thio)-N-(thiazole-2-yl)acétamide, 2-((4-Amino-5-(2-(pyridine-3-yl)éthyl)-4H-1,2,4-triazole-3-yl)thio)-N-(benzothiazole-2-yl)acétamide, 2-((4-Amino-5-(2-(pyridine-3-yl)éthyl)-4H-1,2,4- triazole-3-yl)thio)-N-(6-fluorobenzothiazole-2-yl)acétamide, 2-((4-Amino-5-(2-(pyridine-3-yl)éthyl)-4H-1,2,4-triazole-3-yl)thio)-N-(6-chlorobenzothiazole-2-yl)acétamide, 2-((4-Amino-5-(2-(pyridine-3-yl)éthyl)-4H-1,2,4-triazole-3-yl)thio)-N- (6-bromobenzothiazole-2-yl)acétamide, 2-((4-Amino-5-(2-(pyridine-3-yl)éthyl)-4H-1,2,4-triazole-3-yl)thio)-N-(6-méthylbenzothiazole-2-yl)acétamide ou 2-((4-Amino-5-(2-(pyridine-3-yl)éthyl)-4H-1,2,4-triazole-3-yl)thio)-N-(6-méthoxybenzothiazole-2-yl)acétamide, **caractérisé par** les étapes suivantes:

• mélange du 4-amino-5-(2-(pyridine-3-yl)éthyl)-2,4-dihydro-3H-1,2,4-triazole-3-thione avec du 2-chloro-N-(aryl)acétamide dérivé, à savoir 2-chloro-N-(thiazole-2-yl)acétamide, 2-chloro-N-(benzothiazole-2-yl)acétamide, 2-chloro-N-(6-fluorobenzothiazole-2-yl)acétamide, 2-chloro-N-(6-chlorobenzothiazole-2-yl)acétamide, 2-chloro-N-(6-bromobenzothiazole-2-yl)acétamide, 2-chloro-N-(6-méthylbenzothiazole-2-yl)acétamide ou 2-chloro-N-(6-méthoxybenzothiazole-2-yl)acétamide en présence de carbonate de potassium dans un rapport molaire 1:1 dans l'acétone à température ambiante pendant 12 heures,
• évaporation du solvant dans l'évaporateur rotatif,
• récupération du précipité par filtration.

**15.** Procédé selon la revendication 4, **caractérisé en ce qu'**après que le précipité a été collecté par filtration, lavé avec de l'eau et séché et est ensuite soumis à un processus de cristallisation avec de l'éthanol pour purification.

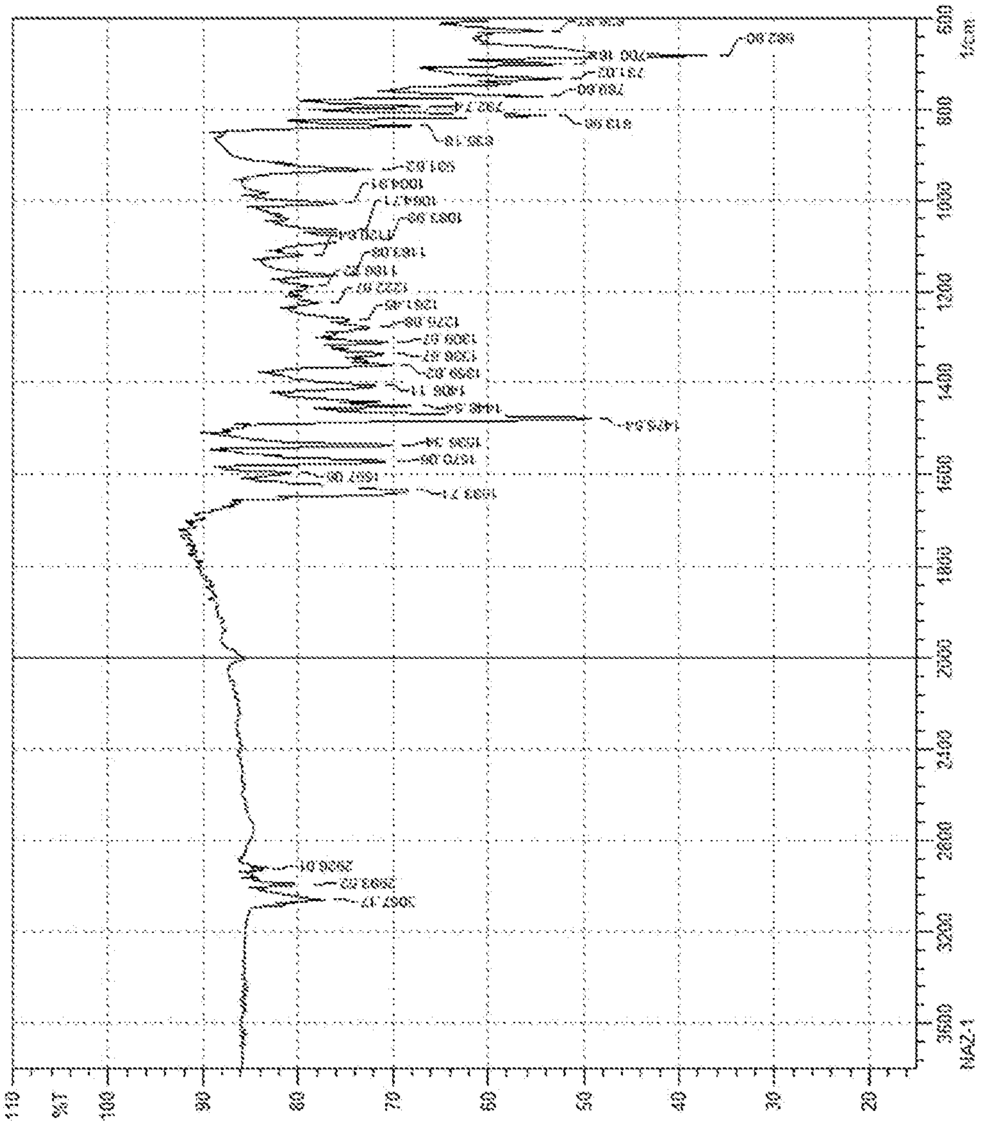

Figure 1

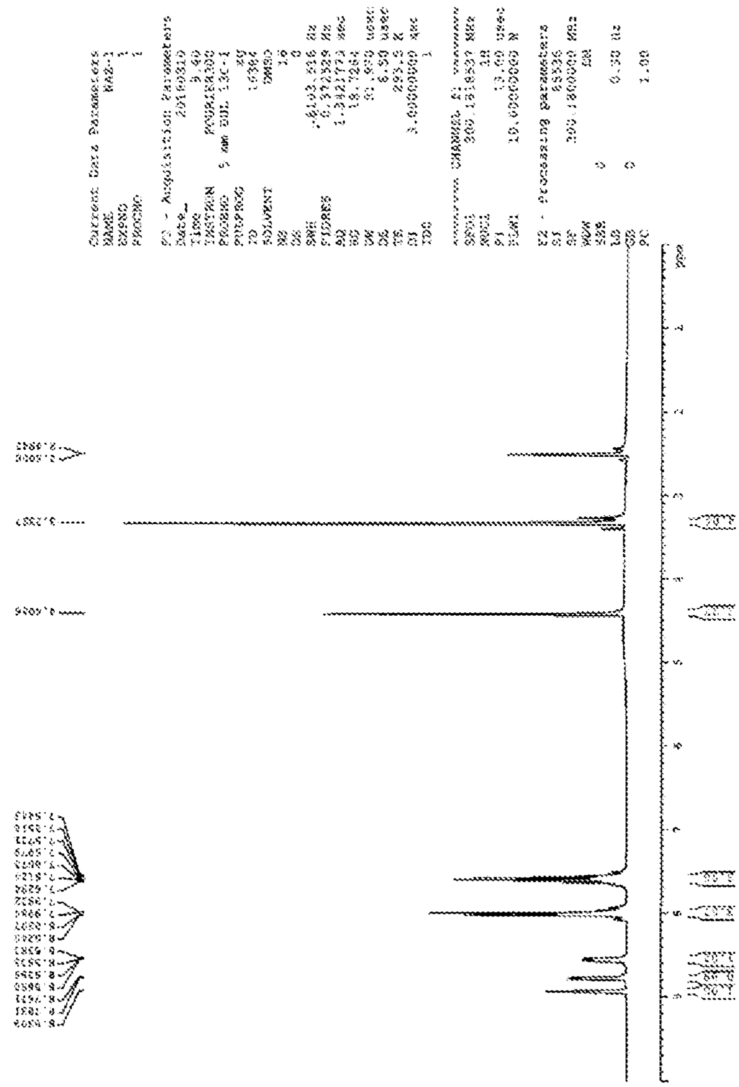

Figure 2

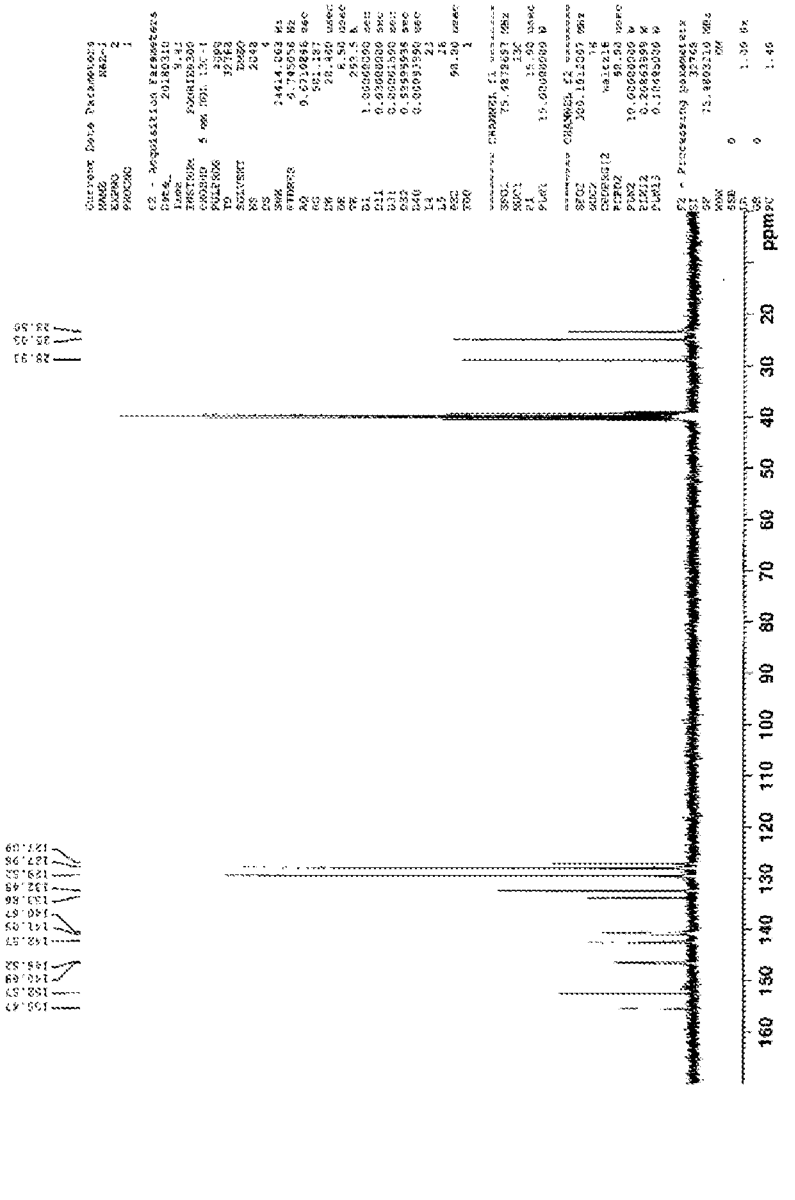

Figure 3

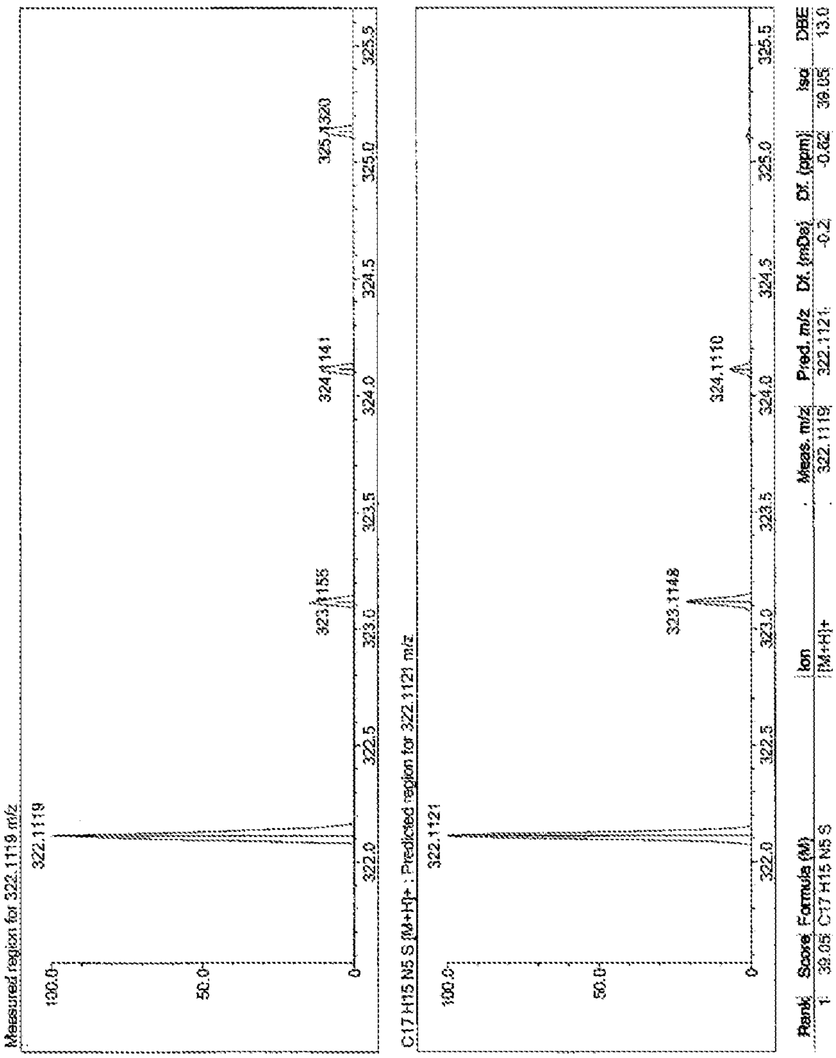
Measured region for 322.1119 m/z
322.1119
323.1155
324.1141
325.1320
C17 H15 N5 S [M+H]+ : Predicted region for 322.1121 m/z
322.1121
323.1148
324.1110
Rank Score Formula (M) Ion Meas. m/z Pred. m/z Df. (mDa) Df. (ppm) Iso DBE
1 39.05 C17 H15 N5 S [M+H]+ 322.1119 322.1121 -0.2 -0.62 39.05 13.0

Figure 4

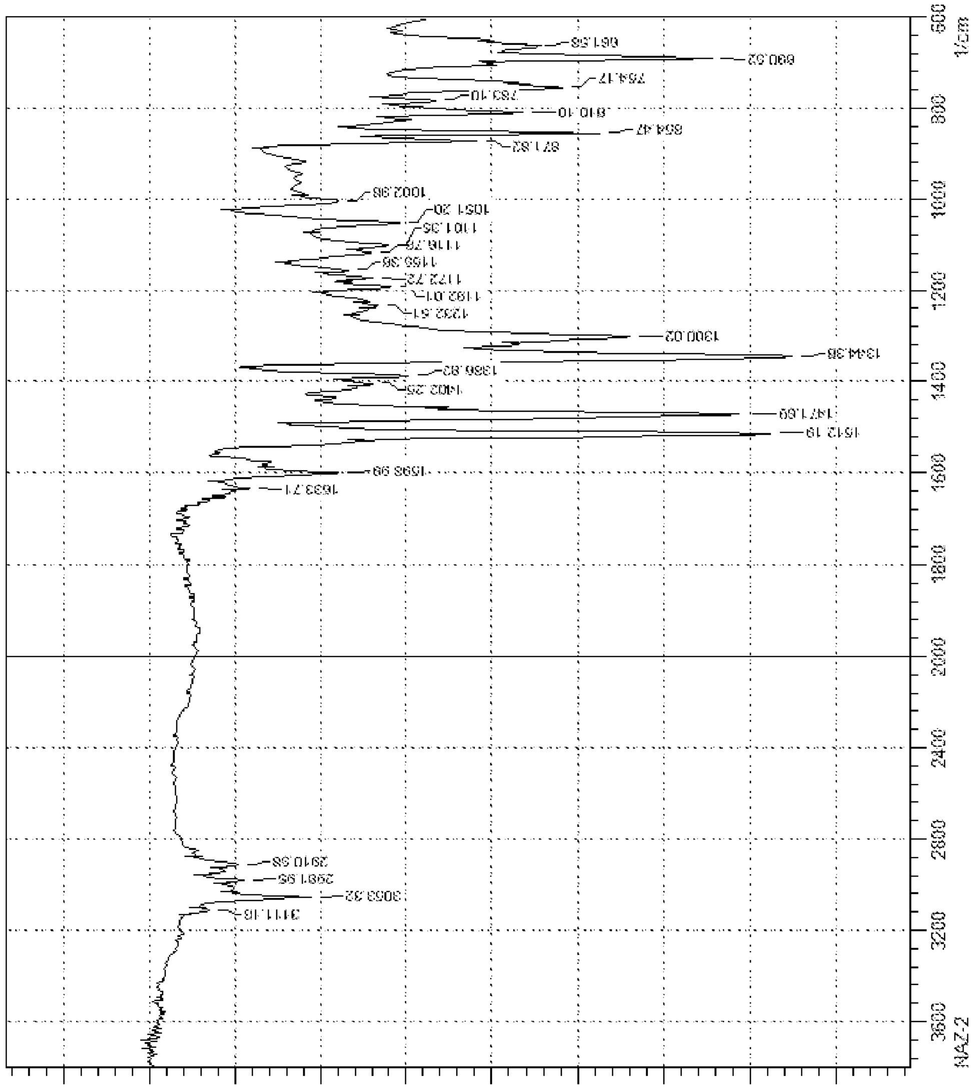
%T
105
97.5
90
82.5
75
67.5
60
52.5
45
37.5
1/cm
3600
3200
2800
2400
2000
1800
1600
1400
1200
1000
800
600
NAZ-2
661.58
690.52
754.17
783.10
810.10
854.47
871.82
1002.86
1051.20
1101.35
1116.78
1155.36
1172.72
1192.01
1232.51
1300.02
1344.38
1386.82
1402.25
1471.69
1512.19
1598.99
1633.71
2910.56
2981.95
3053.32
3111.18

Figure 5

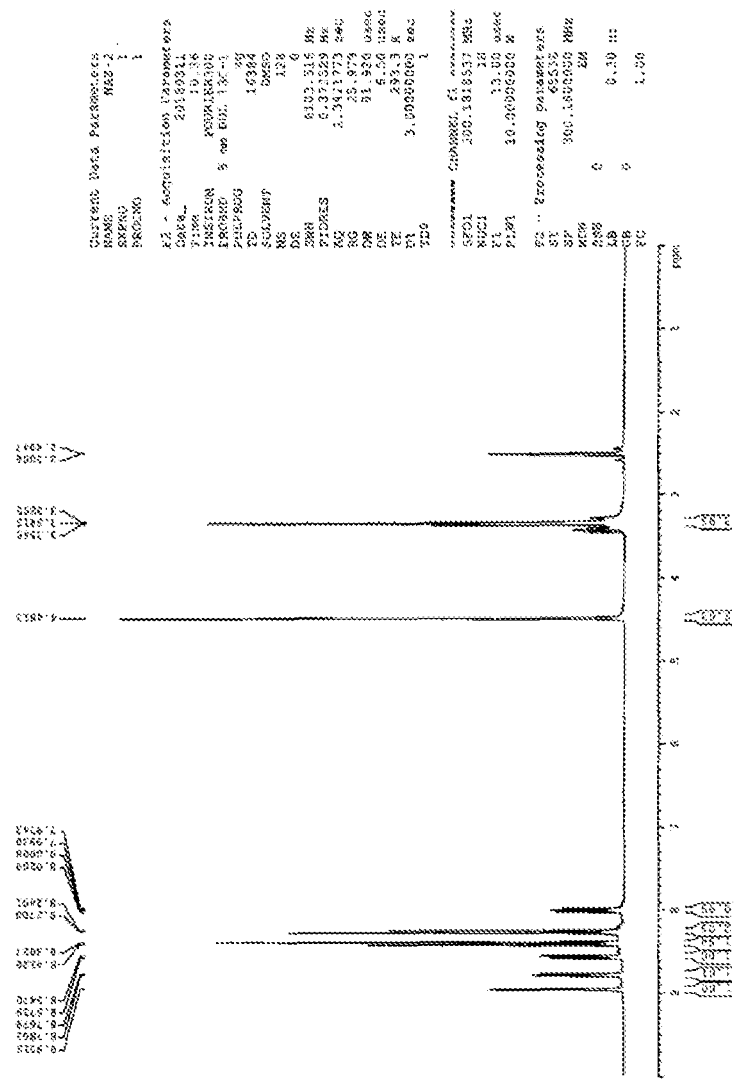

Figure 6

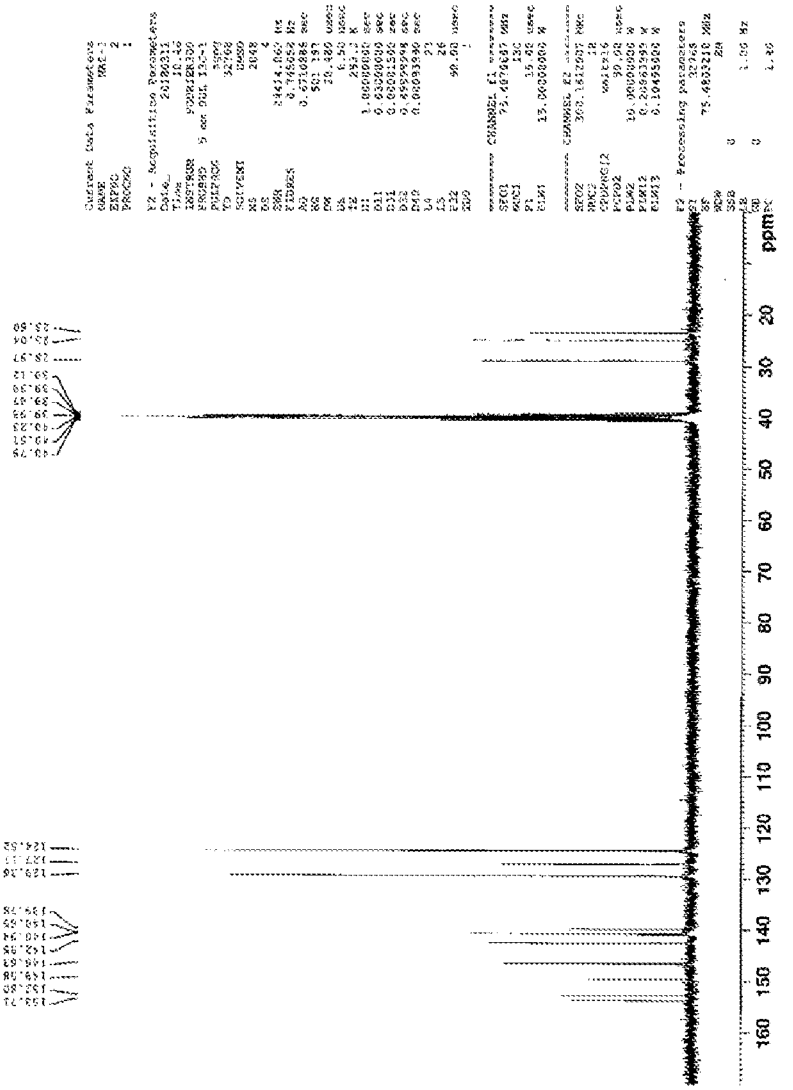

Figure 7

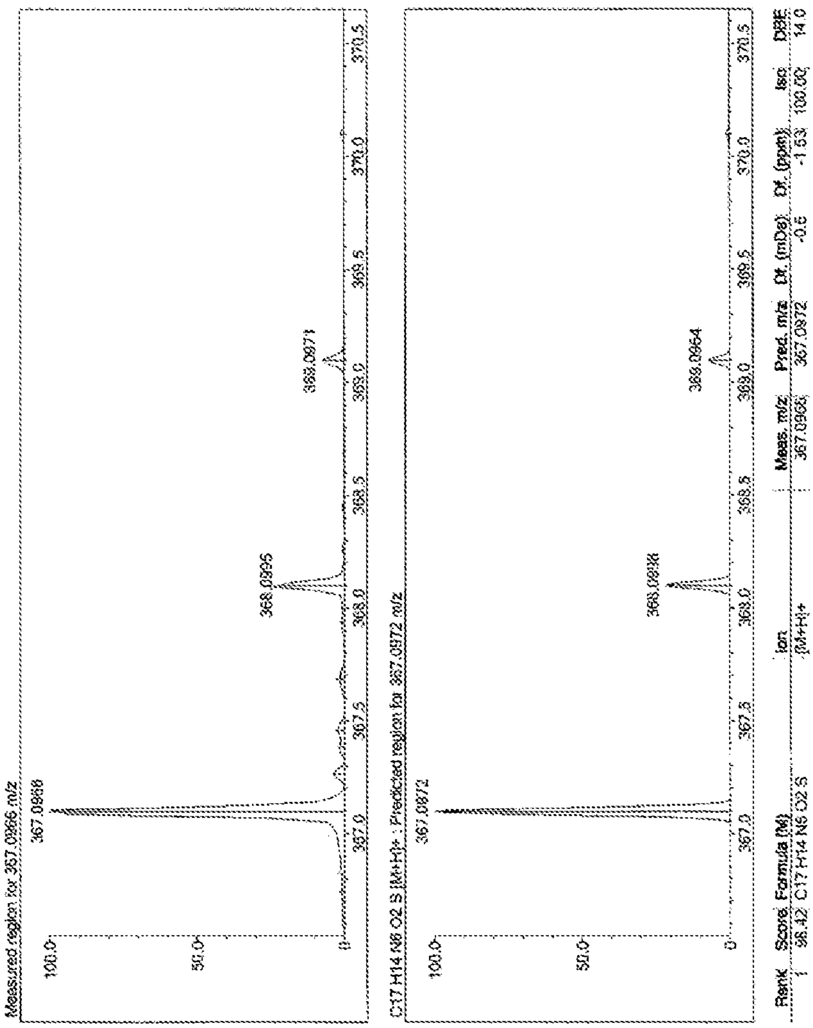
Measured region for 367.0966 m/z
367.0966
368.0995
369.0971
C17 H14 N6 O2 S [M+H]+ : Predicted region for 367.0972 m/z
367.0972
369.0964
Rank Score Formula (M) Ion Meas. m/z Pred. m/z Df. (mDa) Df. (ppm) Iso DBE
1 96.42 C17 H14 N6 O2 S [M+H]+ 367.0966 367.0972 -0.6 -1.63 100.00 14.0

Figure 8

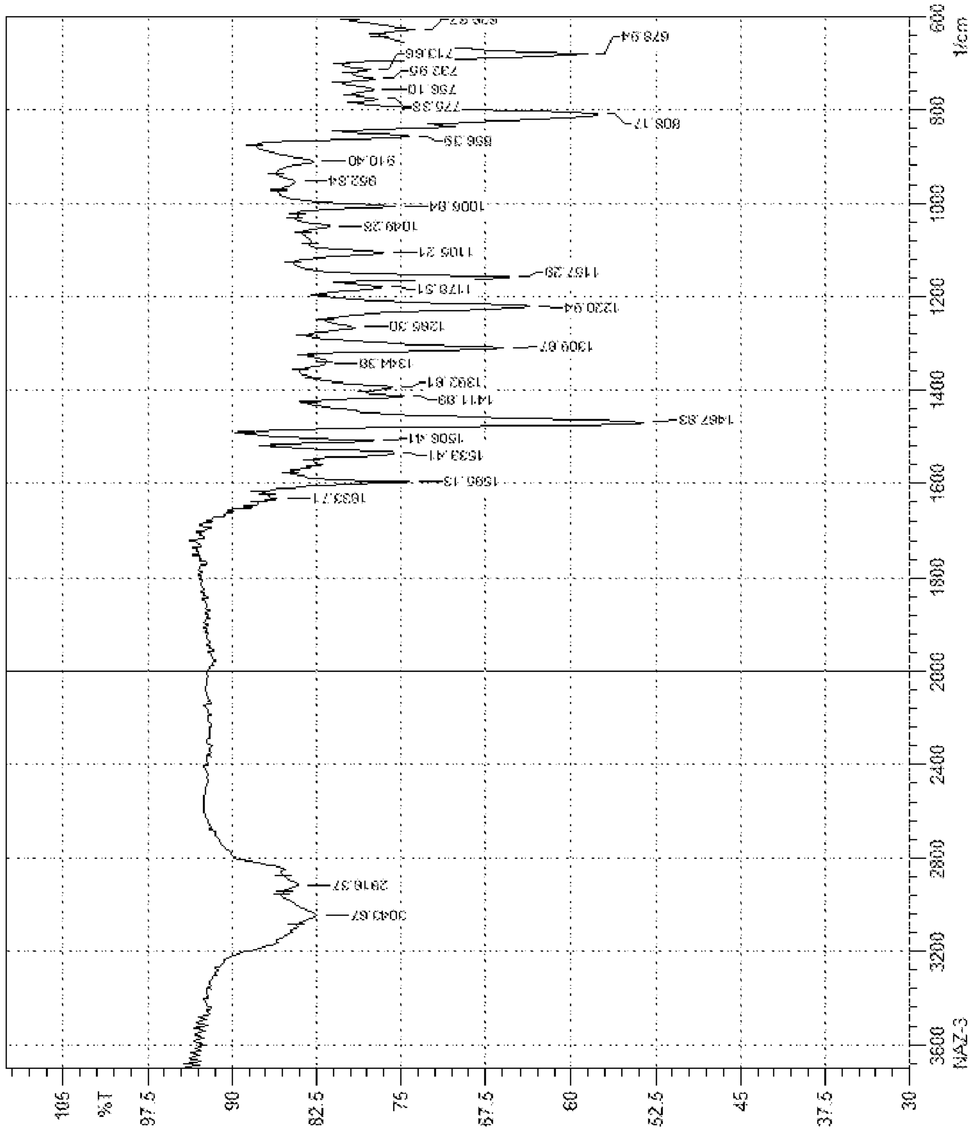
105
%T
97.5
90
82.5
75
67.5
60
52.5
45
37.5
30
3600
3200
2800
2400
2000
1800
1600
1400
1200
1000
800
600
1/cm
NAZ-3
3043.67
2916.57
1633.71
1595.13
1533.41
1506.41
1467.83
1411.89
1392.61
1344.38
1309.67
1265.30
1220.94
1178.51
1157.29
1105.21
1049.28
1006.84
952.84
910.40
856.39
808.17
775.38
756.10
732.95
713.66
678.94

**Figure 9**

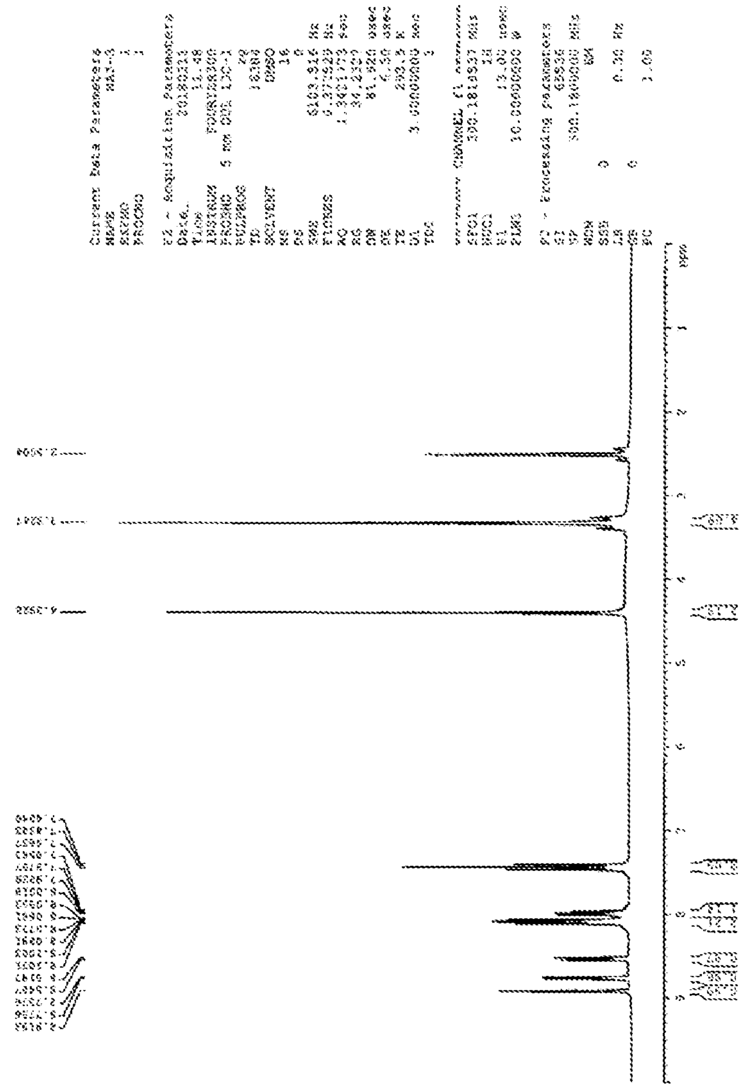

Figure 10

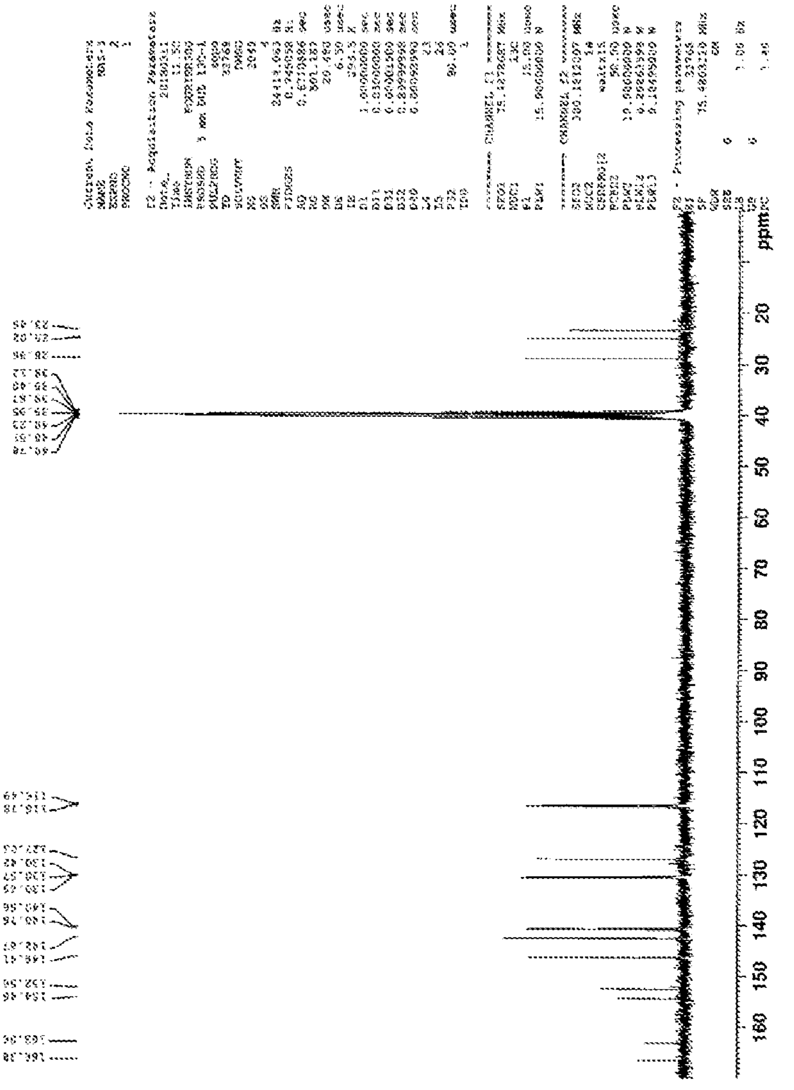

Figure 11

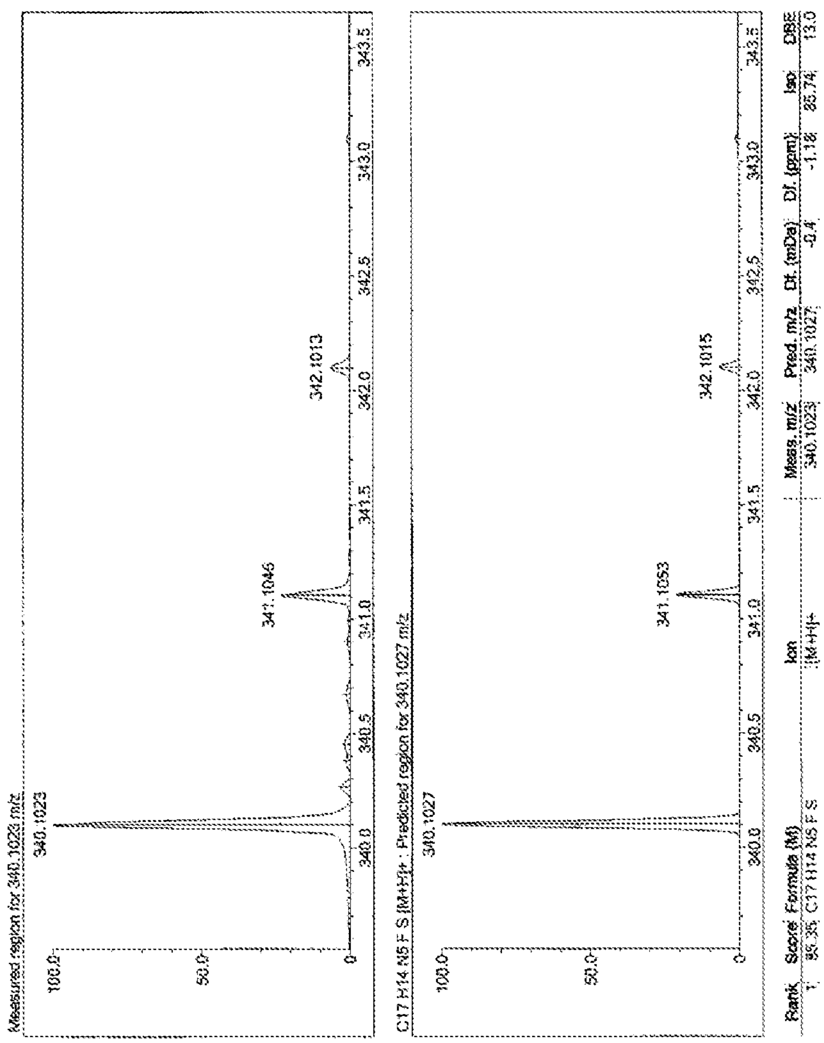
Measured region for 340.1023 m/z
100.0
50.0
0
340.1023
341.1046
342.1013
340.0
340.5
341.0
341.5
342.0
342.5
343.0
343.5
C17 H14 N5 F S [M+H]+ : Predicted region for 340.1027 m/z
100.0
50.0
0
340.1027
341.1053
342.1015
340.0
340.5
341.0
341.5
342.0
342.5
343.0
343.5
Rank Score Formula (M) Ion Meas. m/z Pred. m/z Df. (mDa) Df. (ppm) Iso DBE
1 85.35 C17 H14 N5 F S [M+H]+ 340.1023 340.1027 -0.4 -1.18 85.74 13.0

Figure 12

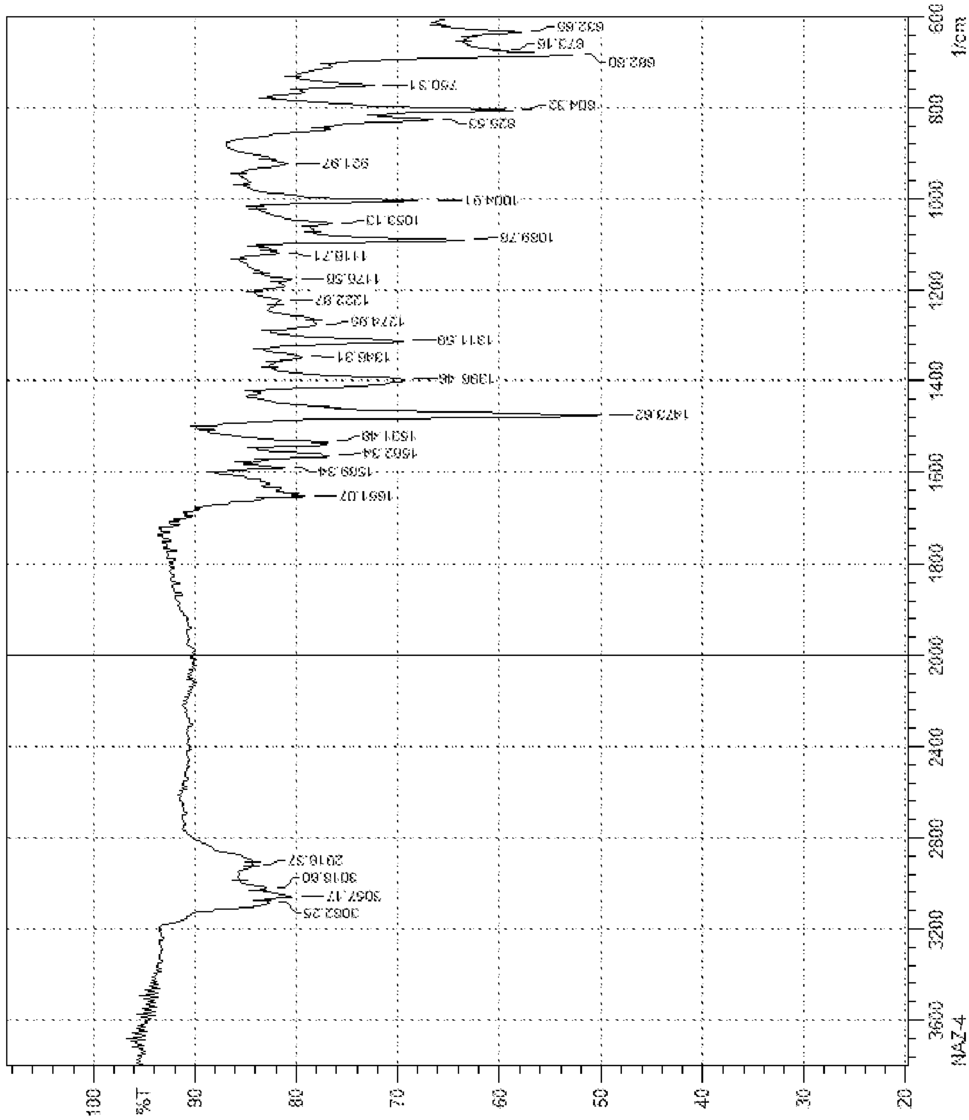
%T
1/cm
NAZ-4
632.65
673.16
692.80
750.31
804.32
825.53
921.97
1004.91
1053.13
1089.76
1118.71
1176.58
1222.87
1274.95
1311.59
1346.31
1396.46
1473.62
1531.48
1562.34
1589.34
1651.07
2916.37
3018.60
3057.17
3082.25

Figure 13

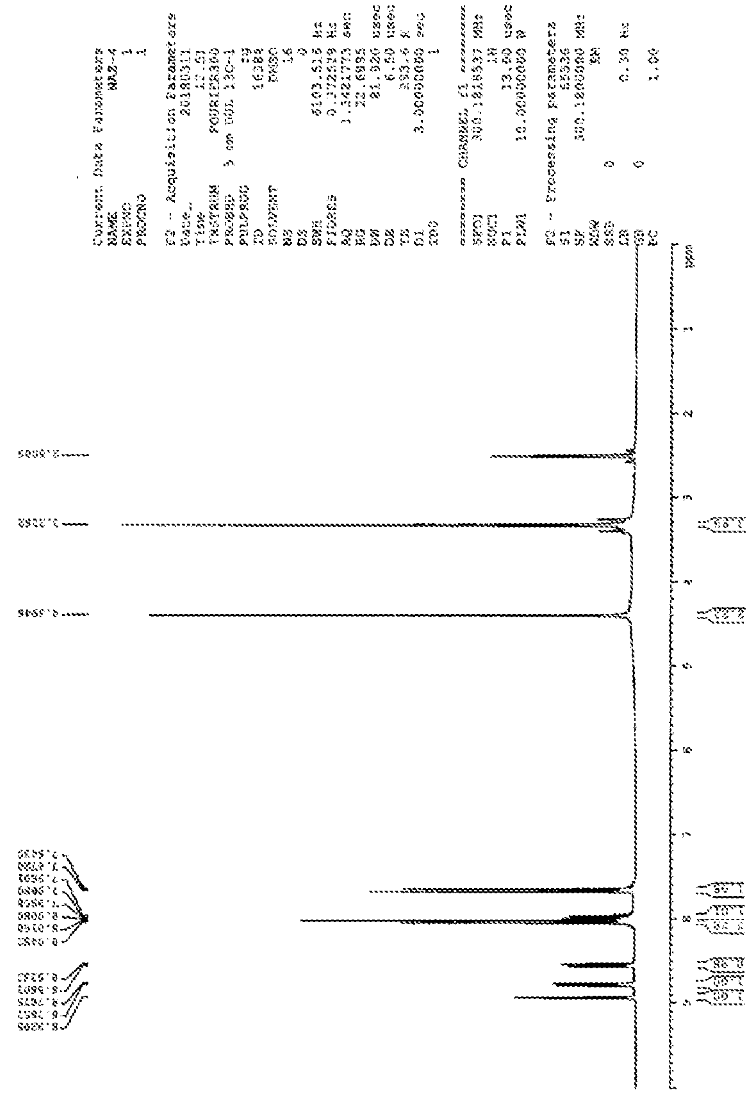

Figure 14

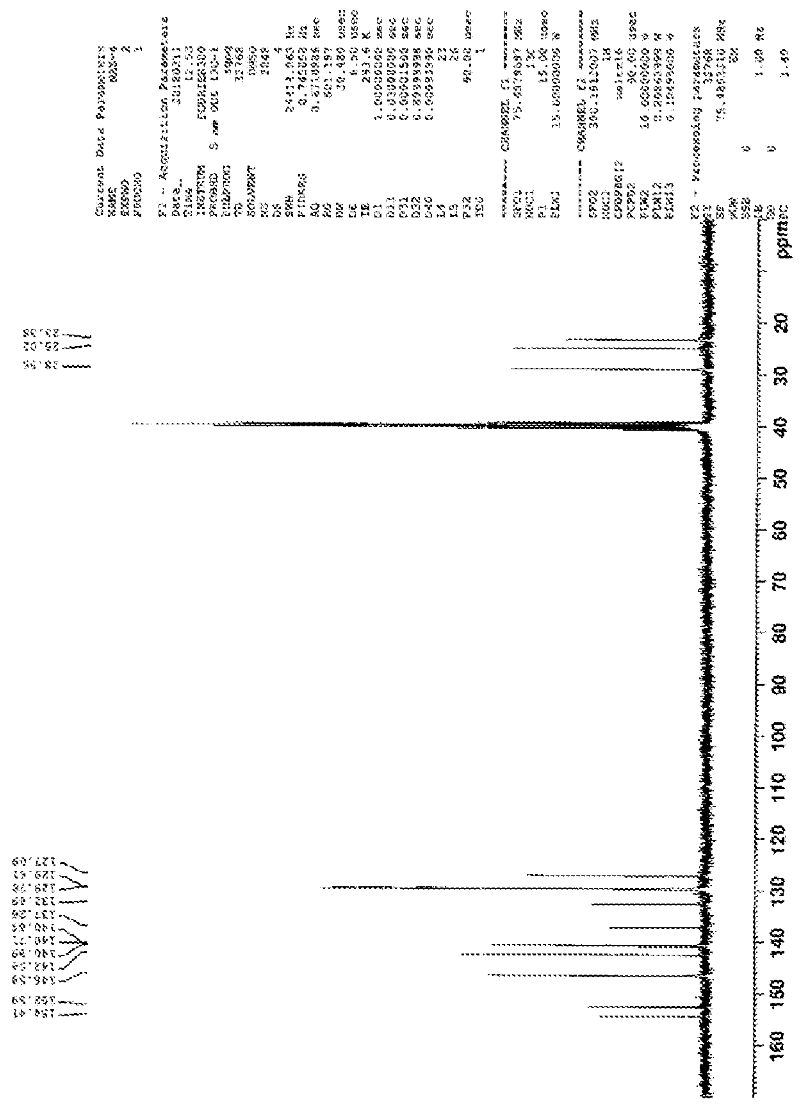

Figure 15

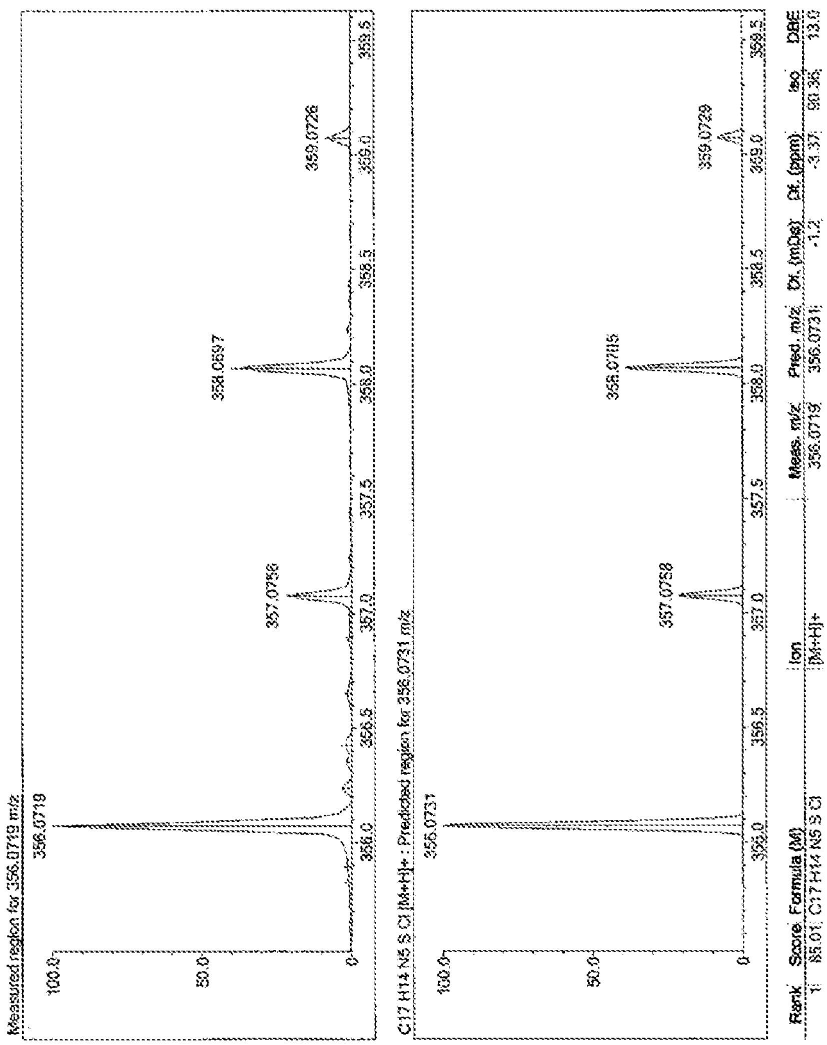
Measured region for 356.0719 m/z
C17 H14 N5 S Cl [M+H]+ : Predicted region for 356.0731 m/z
Rank Score Formula (M) Ion Meas. m/z Pred. m/z Df. (mDa) Df. (ppm) Iso DBE
1 85.01 C17 H14 N5 S Cl [M+H]+ 356.0719 356.0731 -1.2 -3.37 93.36 13.0

Figure 16

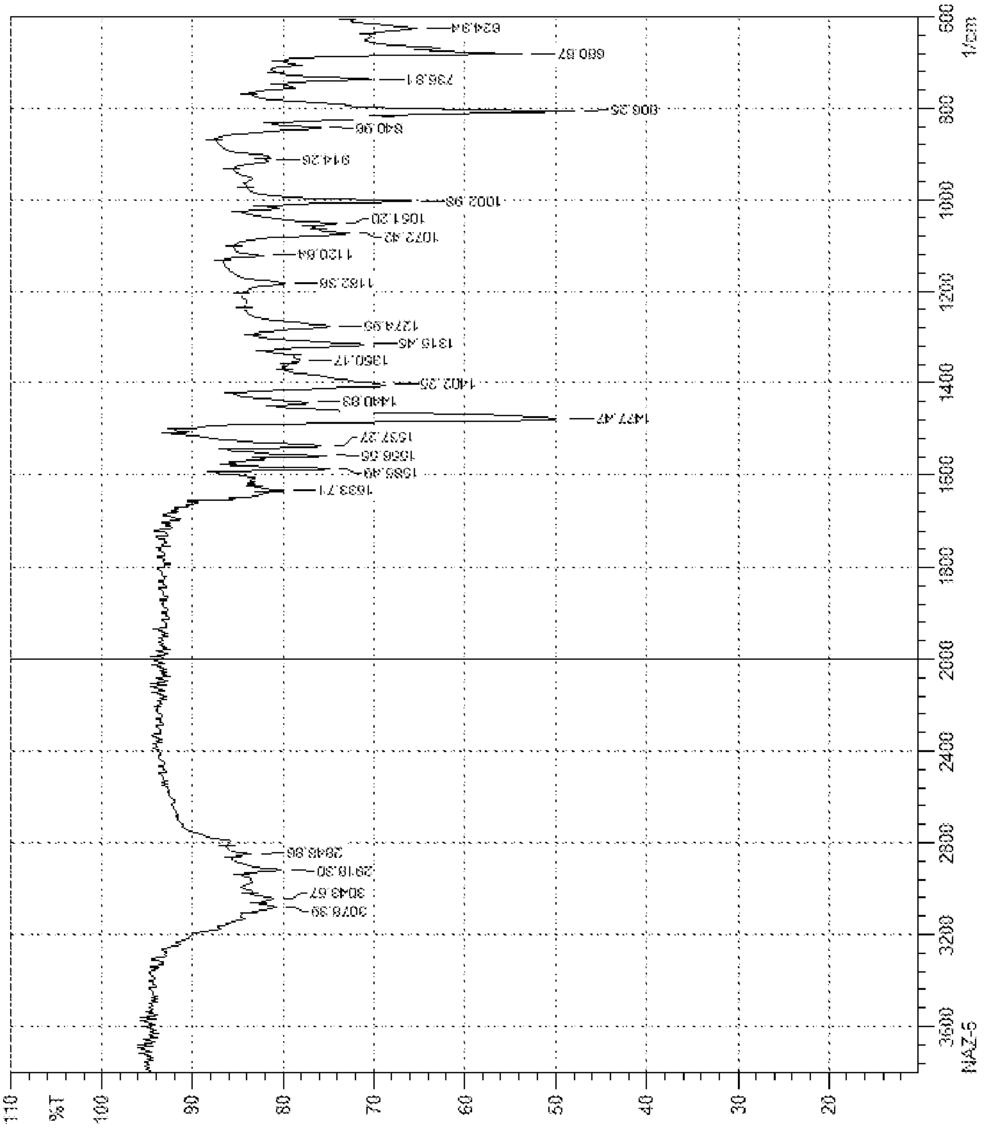
%T
110
100
90
80
70
60
50
40
30
20
1/cm
600
800
1000
1200
1400
1600
1800
2000
2400
2800
3200
3600
NAZ-5
624.34
680.57
736.81
806.25
840.96
914.26
1002.98
1051.20
1072.42
1120.64
1182.36
1274.95
1315.45
1350.17
1402.25
1440.83
1477.47
1537.27
1585.49
1633.71
2848.86
2918.30
3043.57
3078.39

Figure 17

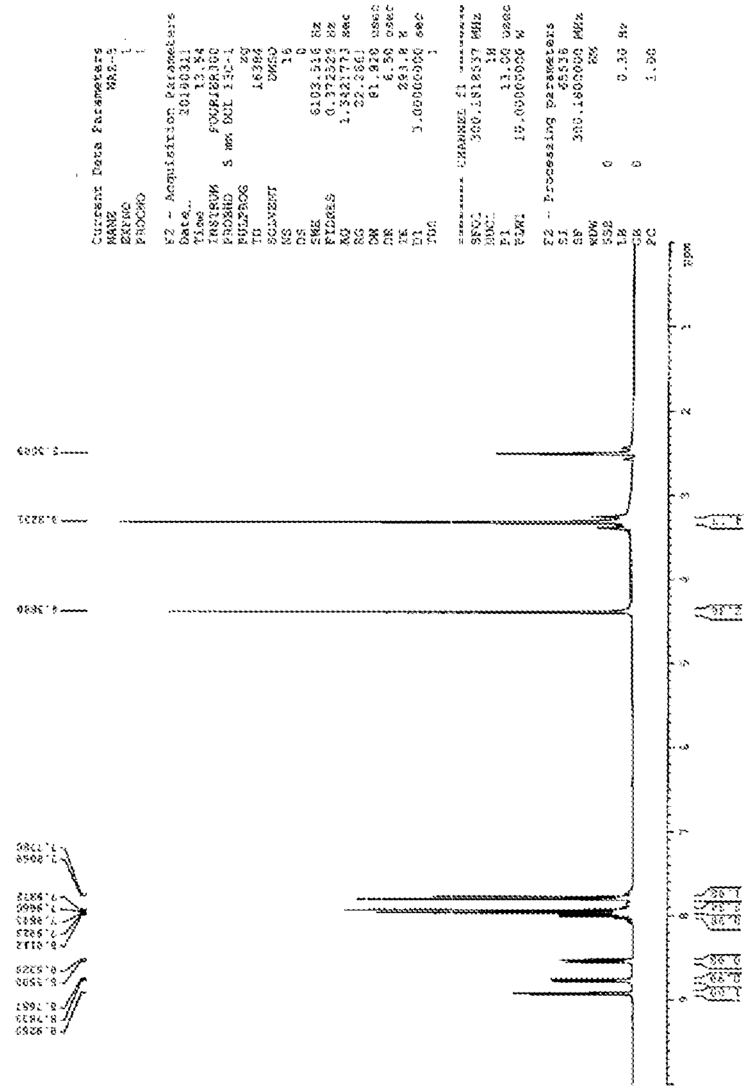

Figure 18

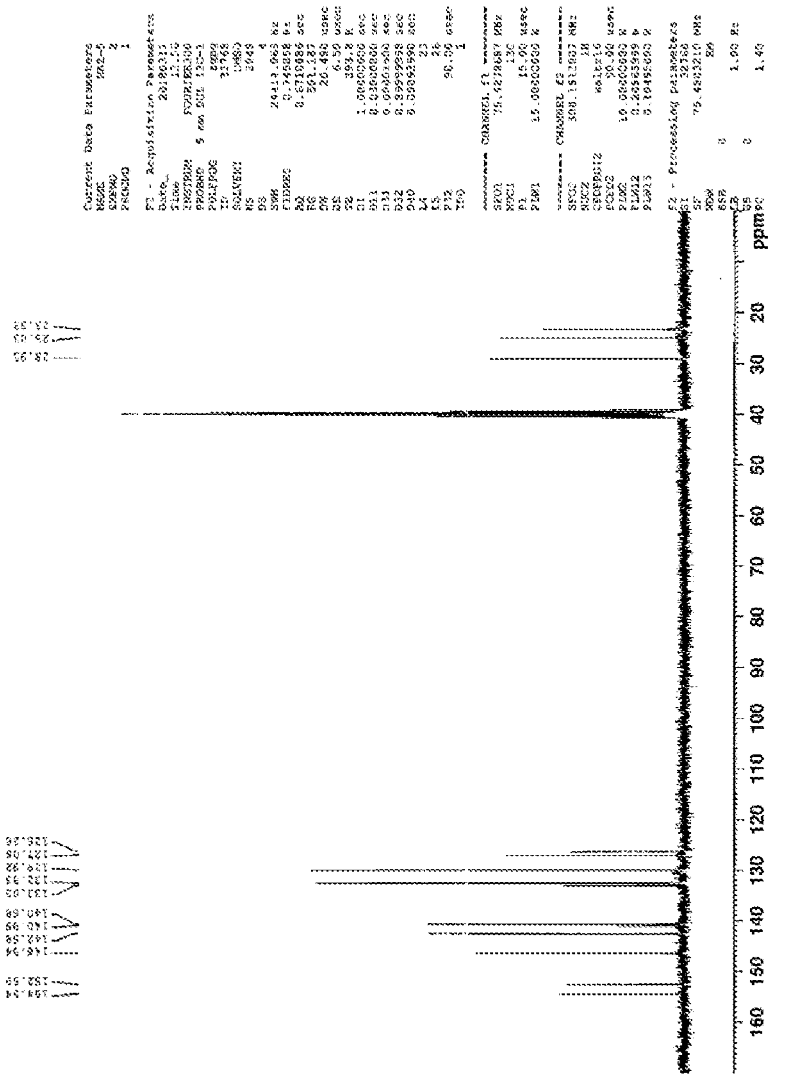

Figure 19

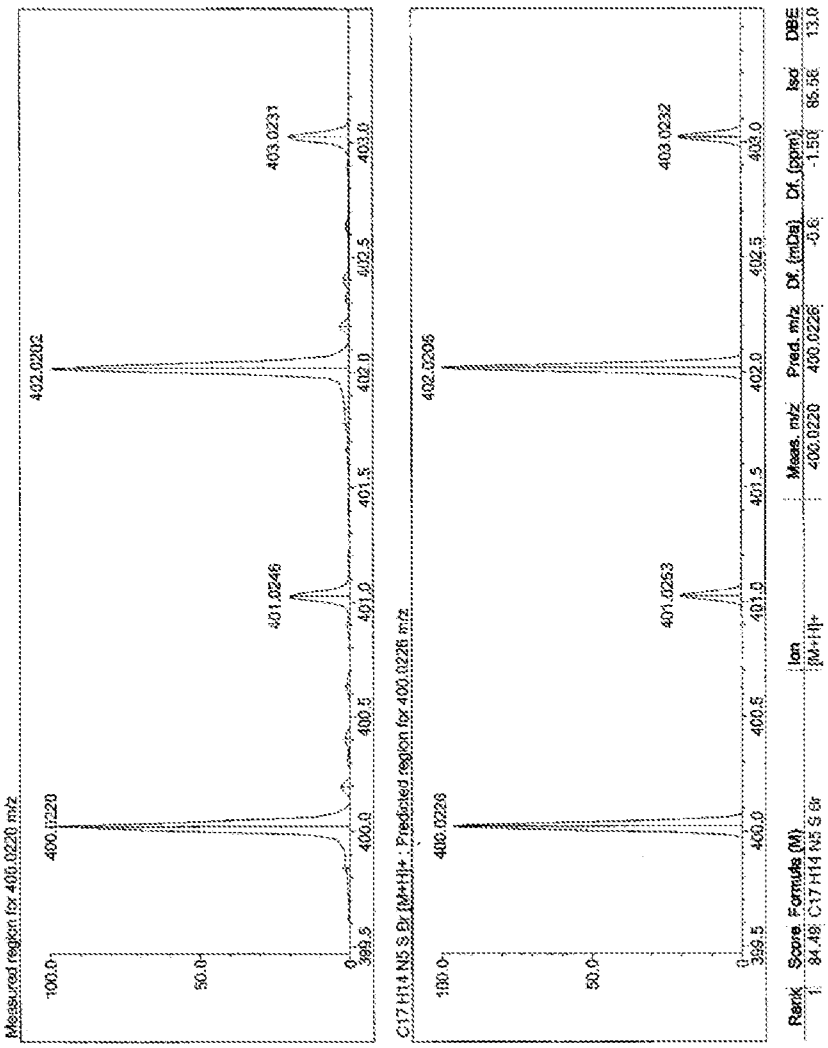
Measured region for 400.0220 m/z
100.0
50.0
0
399.5
400.0
400.5
401.0
401.5
402.0
402.5
403.0
400.0220
401.0246
402.0202
403.0231
C17 H14 N5 S Br [M+H]+ : Predicted region for 400.0226 m/z
400.0226
401.0253
402.0205
403.0232
Rank Score Formula (M) Ion Meas. m/z Pred. m/z Df. (mDa) Df. (ppm) Iso DBE
1 84.49 C17 H14 N5 S Br [M+H]+ 400.0220 400.0226 -0.6 -1.50 85.56 13.0

Figure 20

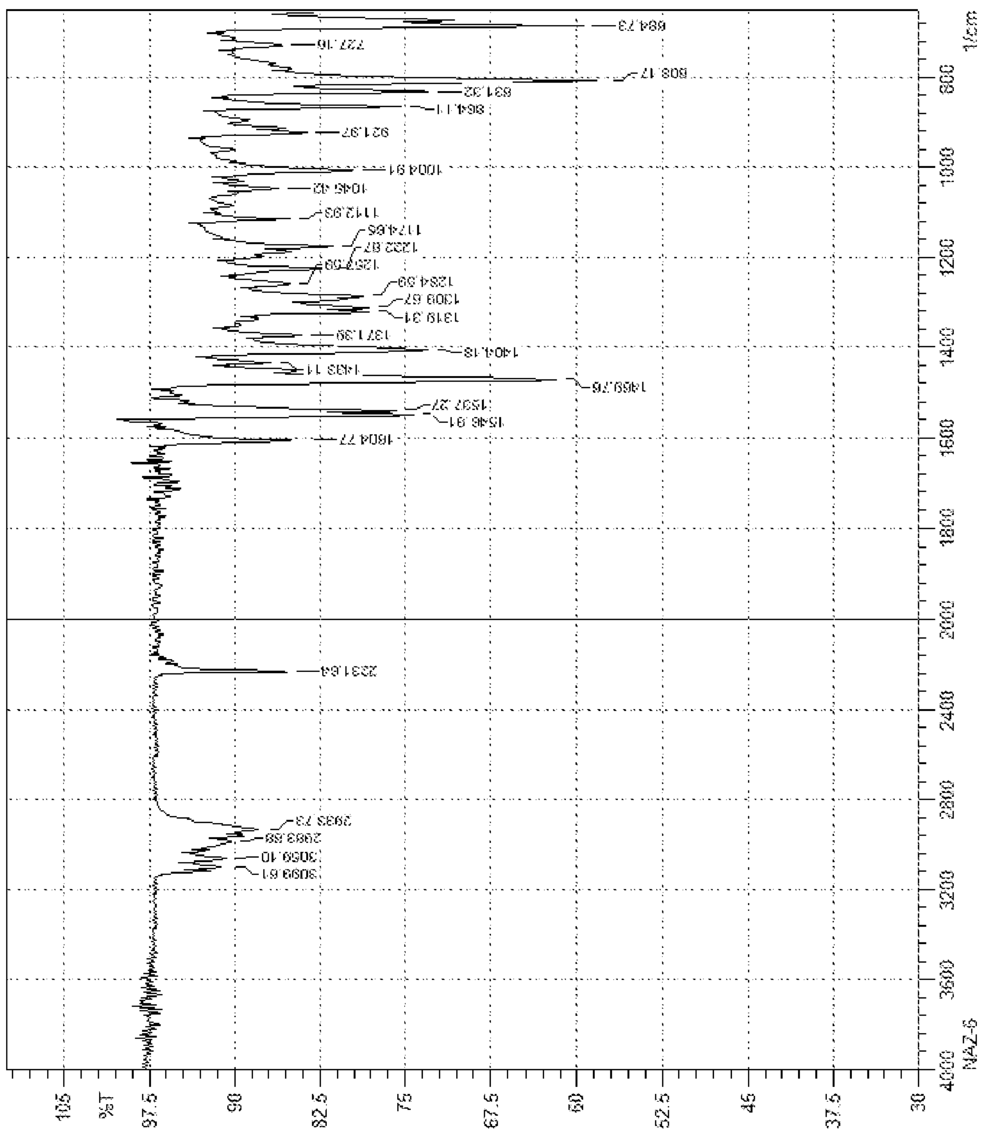
%T
1/cm
NAZ-6
684.73
727.16
808.17
831.32
864.11
921.97
1004.91
1045.42
1112.93
1174.85
1222.87
1257.59
1284.59
1309.67
1319.31
1371.39
1404.18
1438.11
1469.76
1537.27
1546.91
1604.77
2231.64
2933.73
2963.88
3059.10
3089.61

**Figure 21**

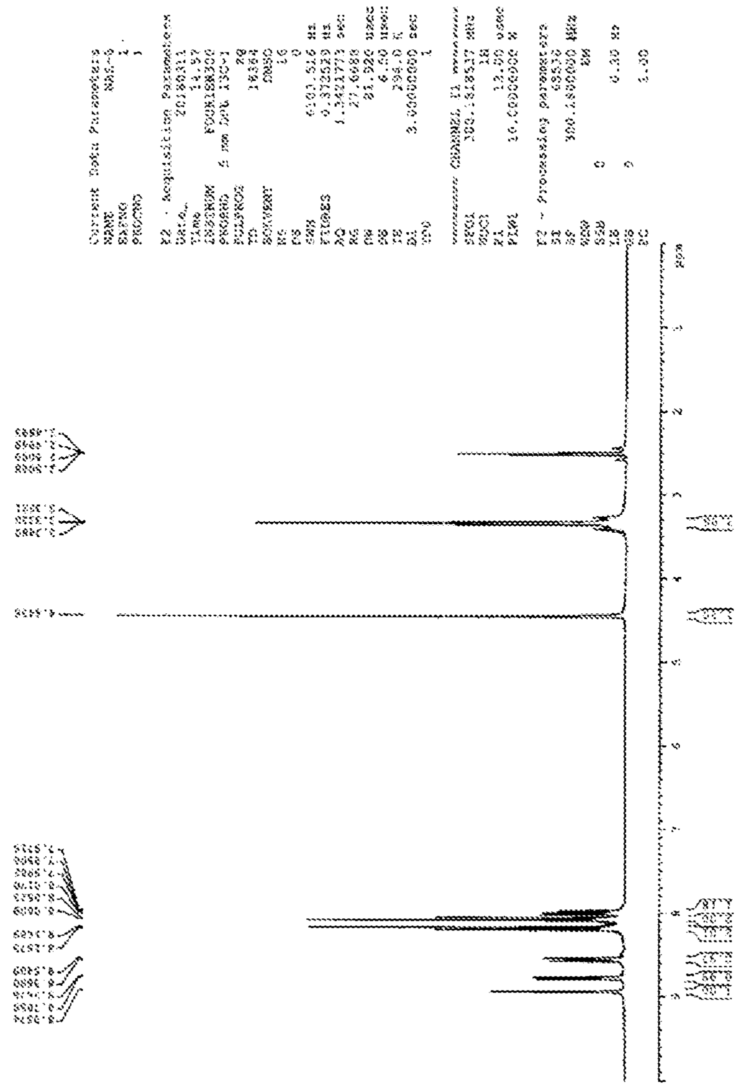

Figure 22

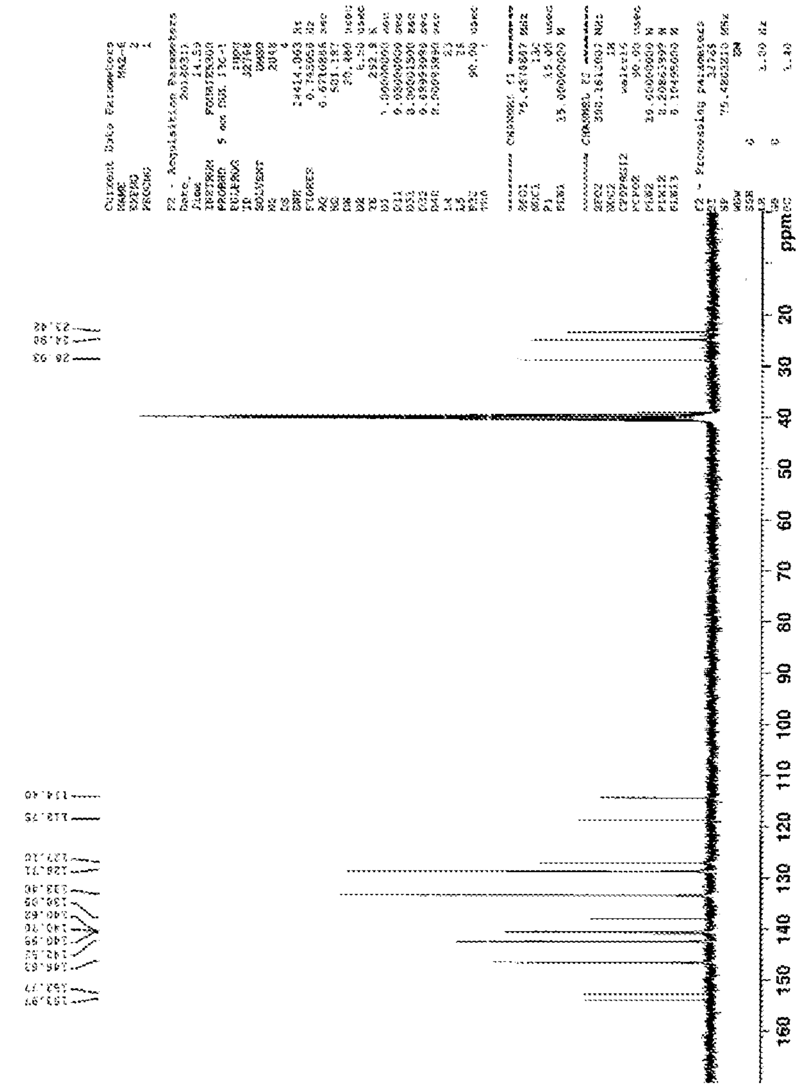

Figure 23

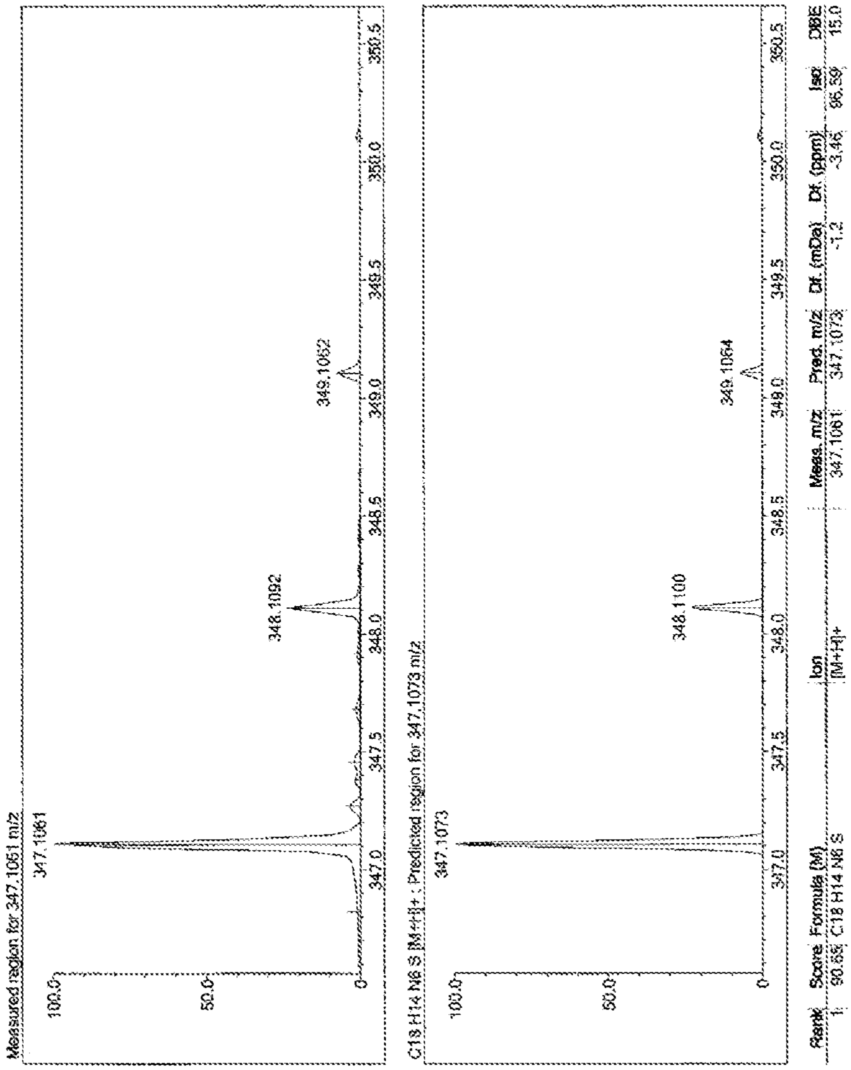
Measured region for 347.1061 m/z
347.1061
348.1092
349.1062
C18 H14 N6 S [M+H]+ : Predicted region for 347.1073 m/z
347.1073
348.1100
349.1064
Rank Score Formula (M) Ion Meas. m/z Pred. m/z Df. (mDa) Df. (ppm) Iso DBE
1 93.69 C18 H14 N6 S [M+H]+ 347.1061 347.1073 -1.2 -3.46 95.59 15.0

Figure 24

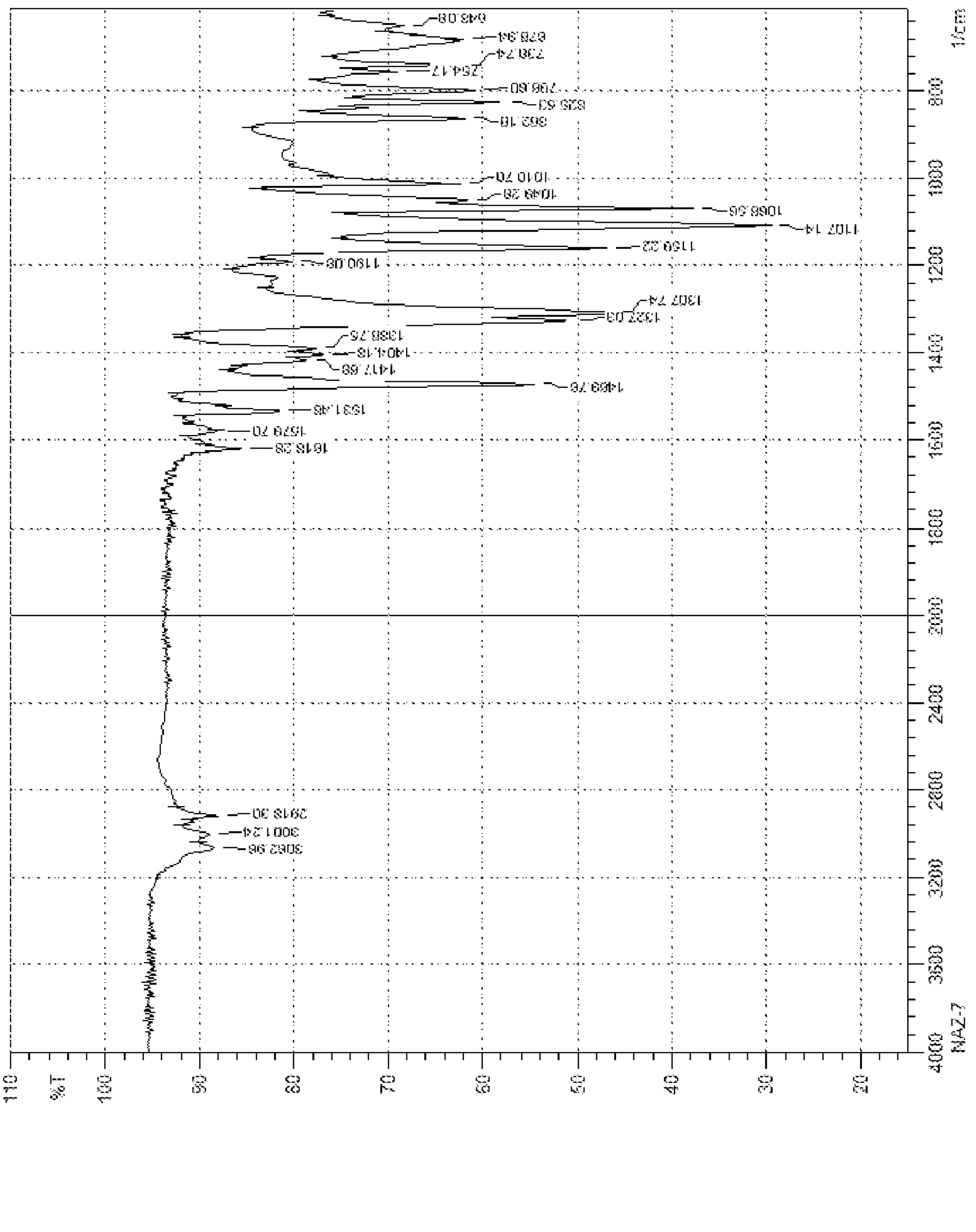
%T
1/cm
NAZ-7
648.08
678.94
738.74
754.17
796.60
825.53
862.18
1010.70
1049.28
1068.56
1107.14
1159.22
1190.08
1307.74
1327.03
1388.75
1404.18
1417.68
1469.76
1531.48
1579.70
1618.28
2918.30
3001.24
3062.96

**Figure 25**

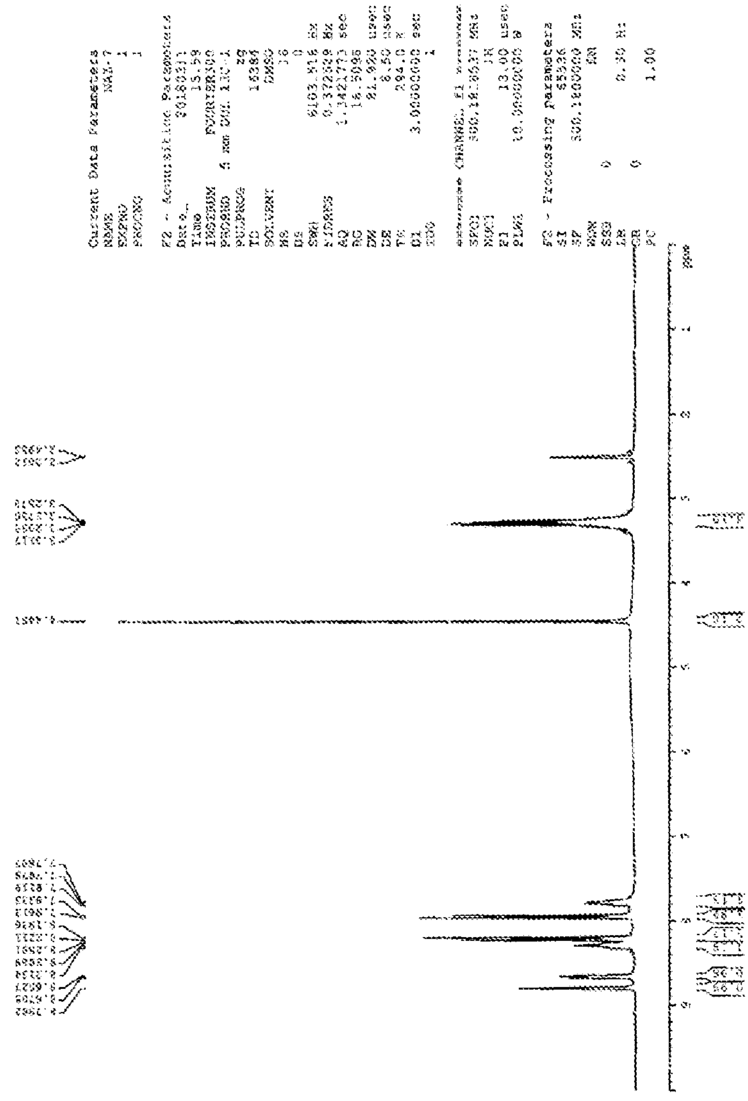

Figure 26

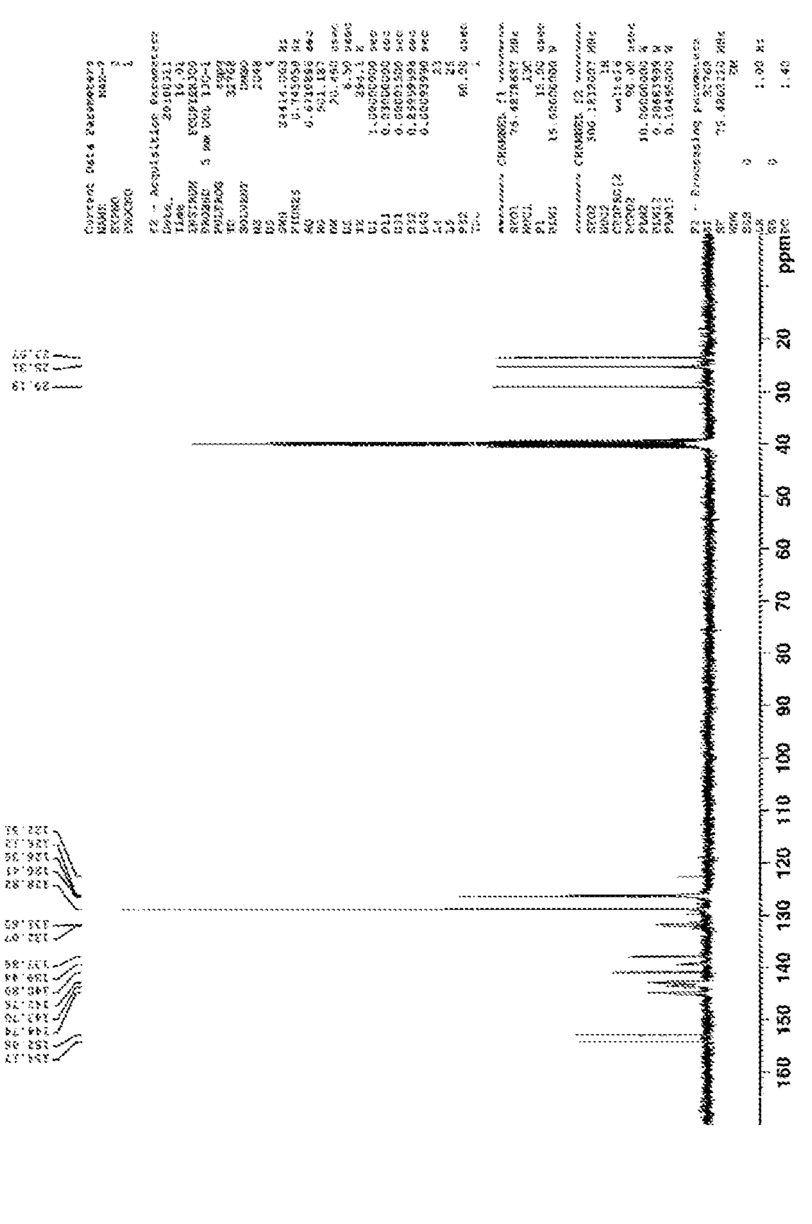

Figure 27

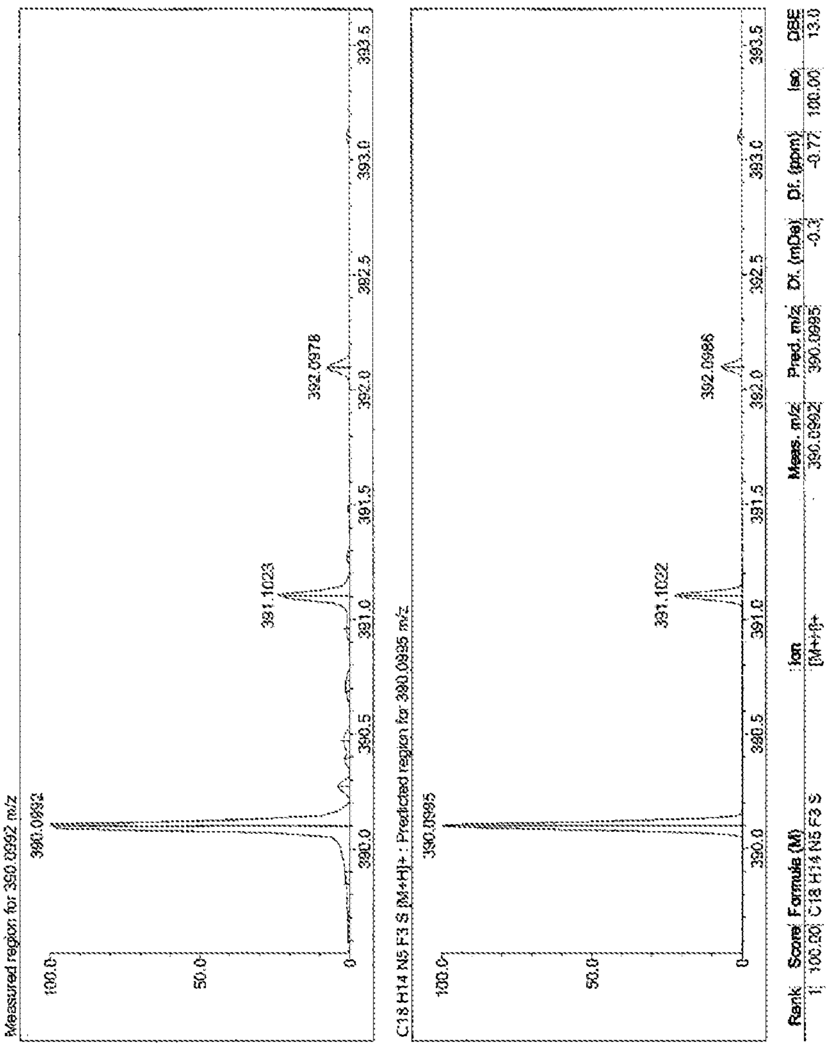
Measured region for 390.0992 m/z
390.0992
391.1023
392.0978
C18 H14 N5 F3 S [M+H]+ : Predicted region for 390.0995 m/z
390.0995
391.1032
392.0986
Rank Score Formula (M) Ion Meas. m/z Pred. m/z Df. (mDa) Df. (ppm) Iso DBE
1 100.00 C18 H14 N5 F3 S [M+H]+ 390.0992 390.0995 -0.3 -0.77 100.00 13.0

Figure 28

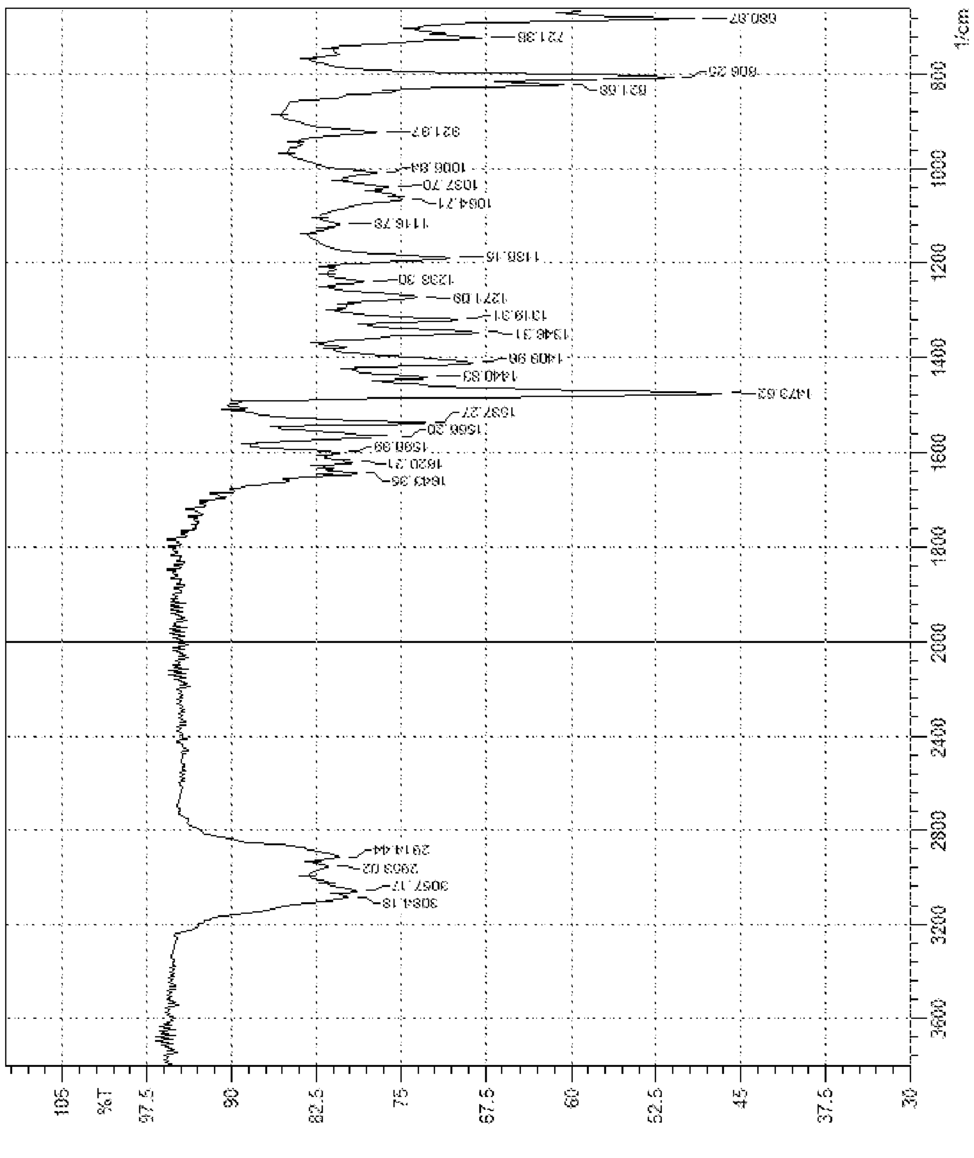


Figure 29

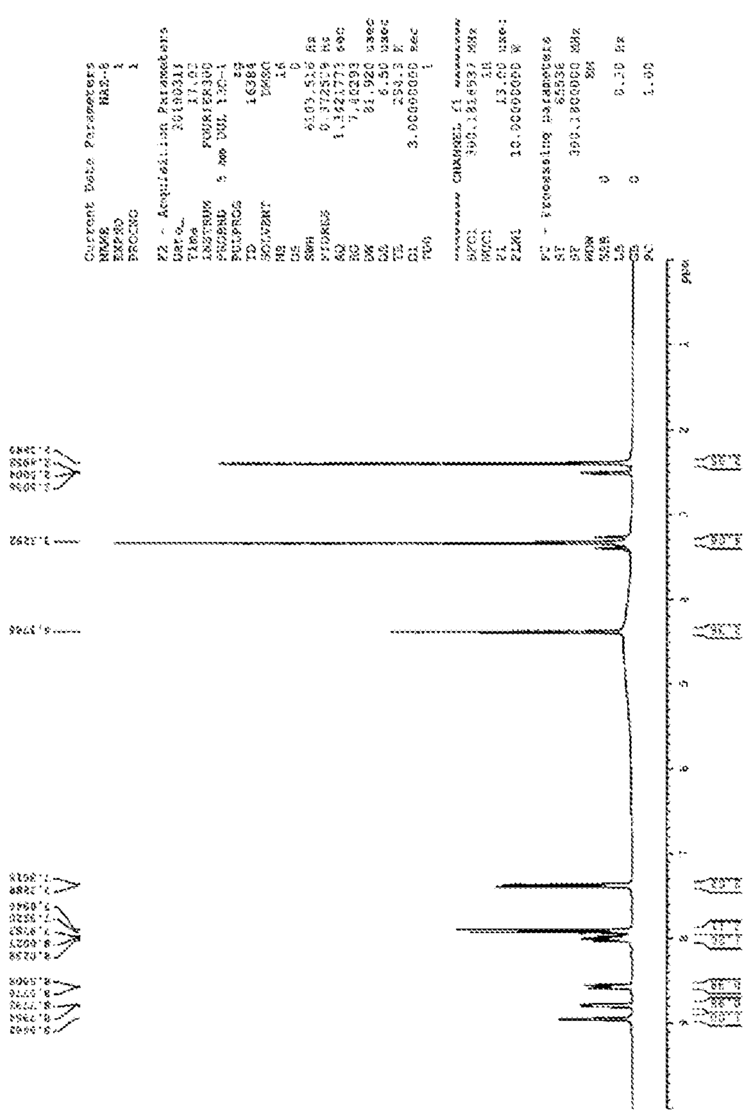

Figure 30

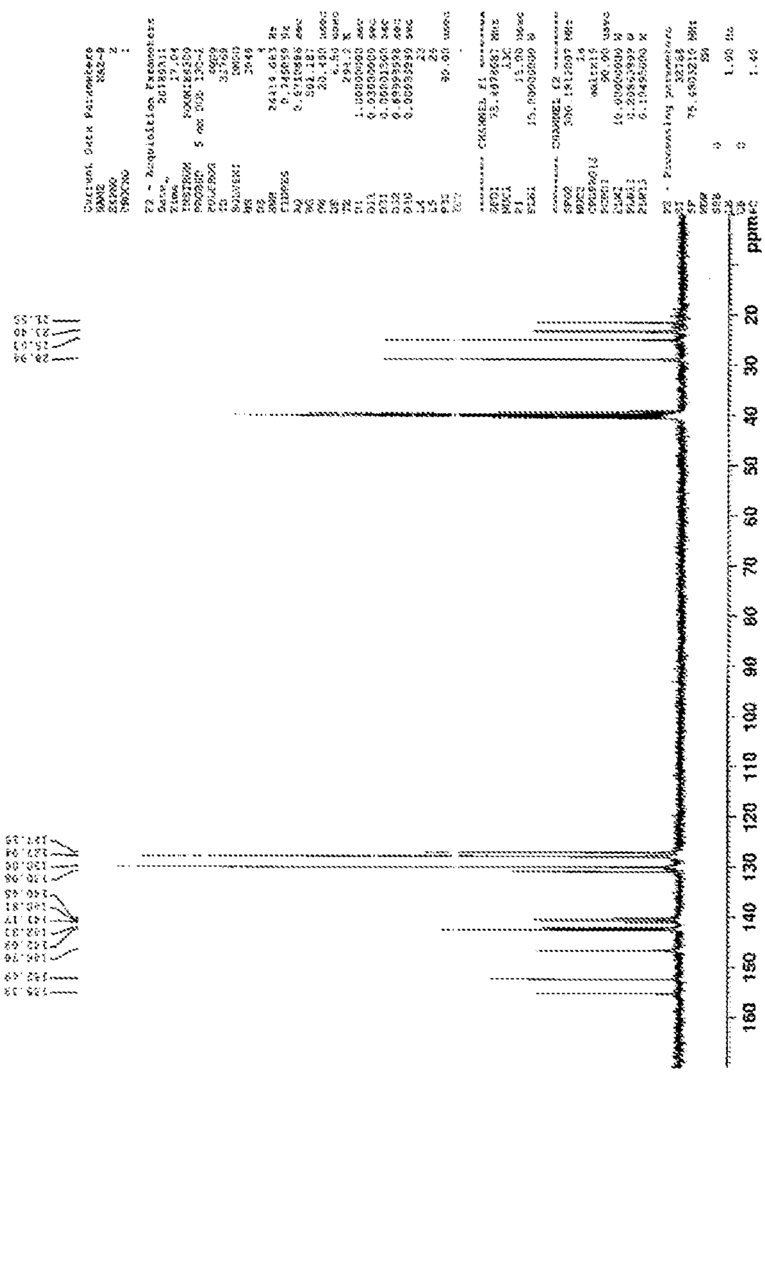

Figure 31

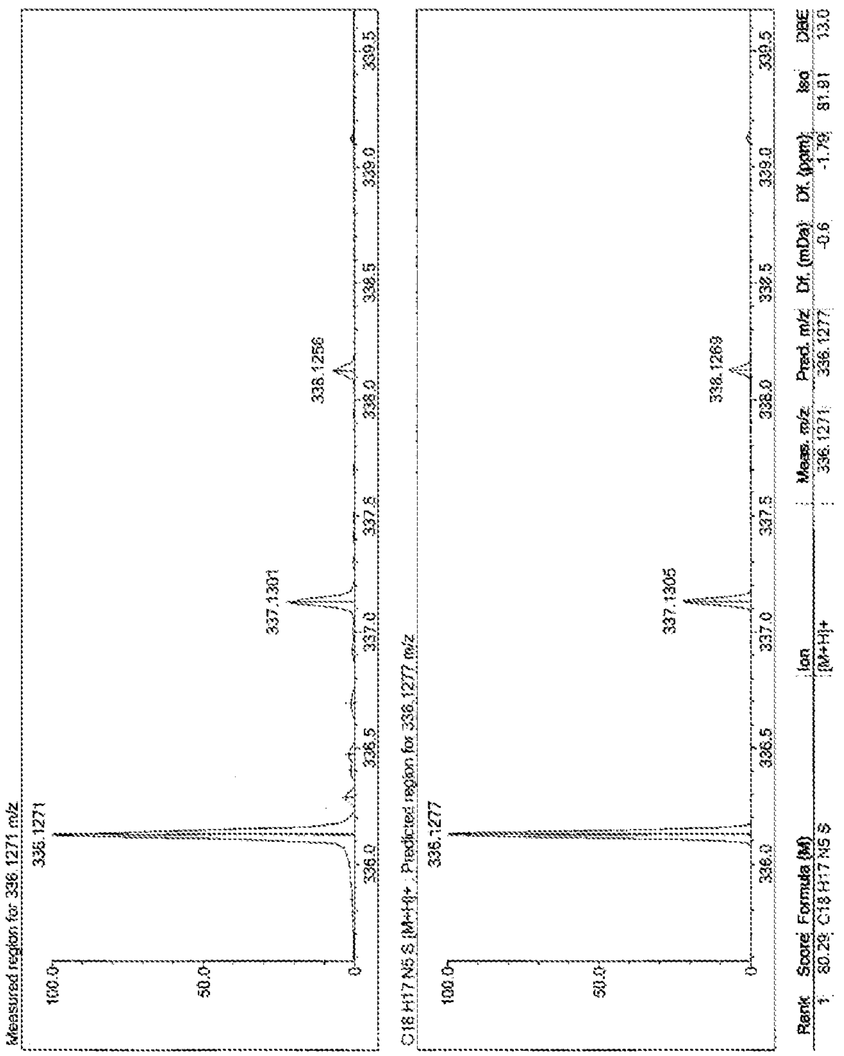
Measured region for 336.1271 m/z
336.1271
337.1301
338.1256
C18 H17 N5 S [M+H]+ , Predicted region for 336.1277 m/z
336.1277
337.1305
338.1269
Rank Score Formula (M) Ion Meas. m/z Pred. m/z Df. (mDa) Df. (ppm) Iso DBE
1 80.29 C18 H17 N5 S [M+H]+ 336.1271 336.1277 -0.6 -1.79 81.81 13.0

Figure 32

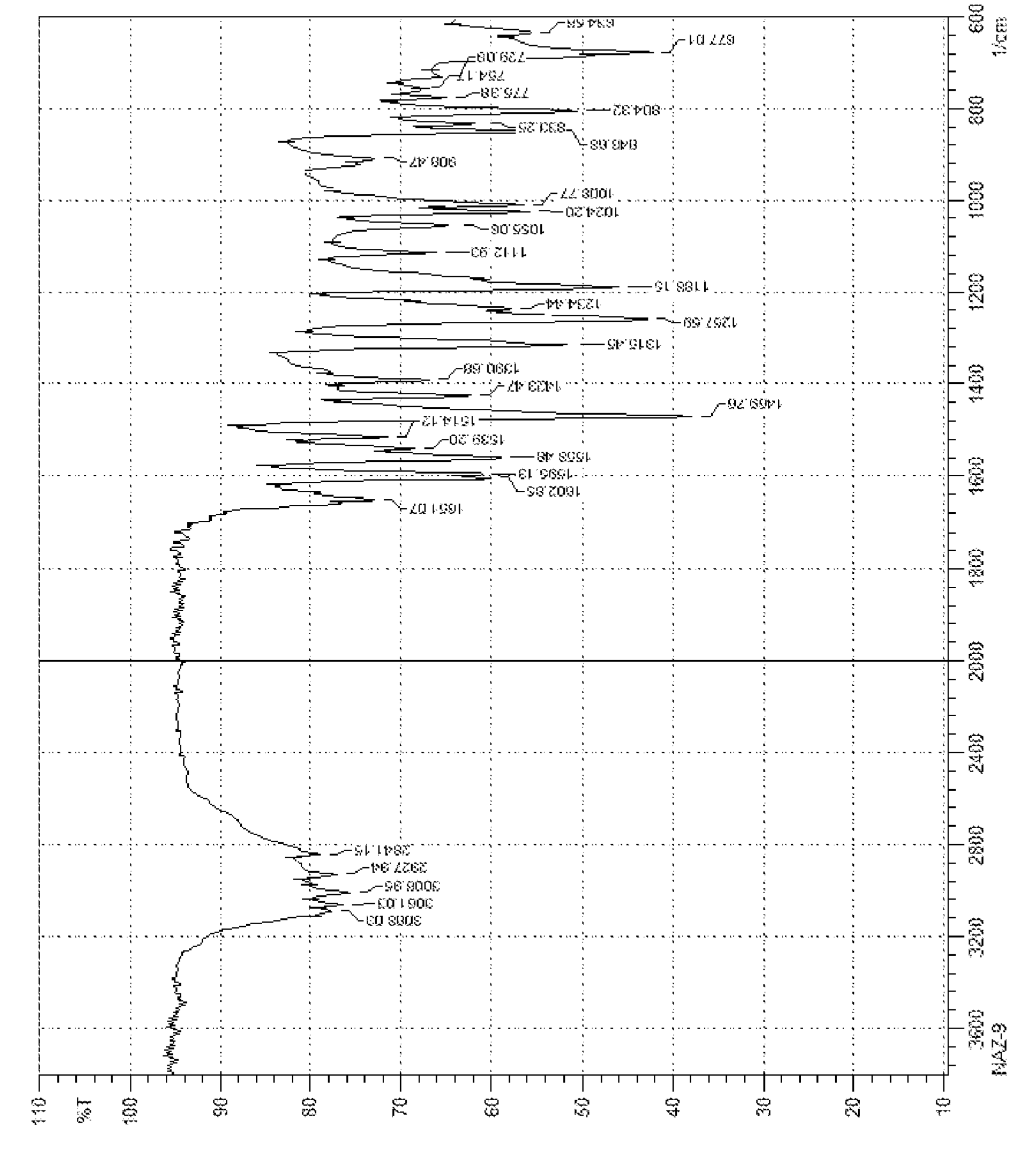
%T
1/cm
NAZ-9
694.58
677.01
729.09
754.17
776.38
804.32
833.25
846.68
906.47
1008.77
1024.20
1055.06
1112.93
1188.15
1234.44
1257.59
1315.45
1380.88
1423.47
1469.76
1514.12
1539.20
1568.48
1595.13
1602.85
1651.07
2841.15
2927.94
3008.95
3061.03
3088.03

**Figure 33**

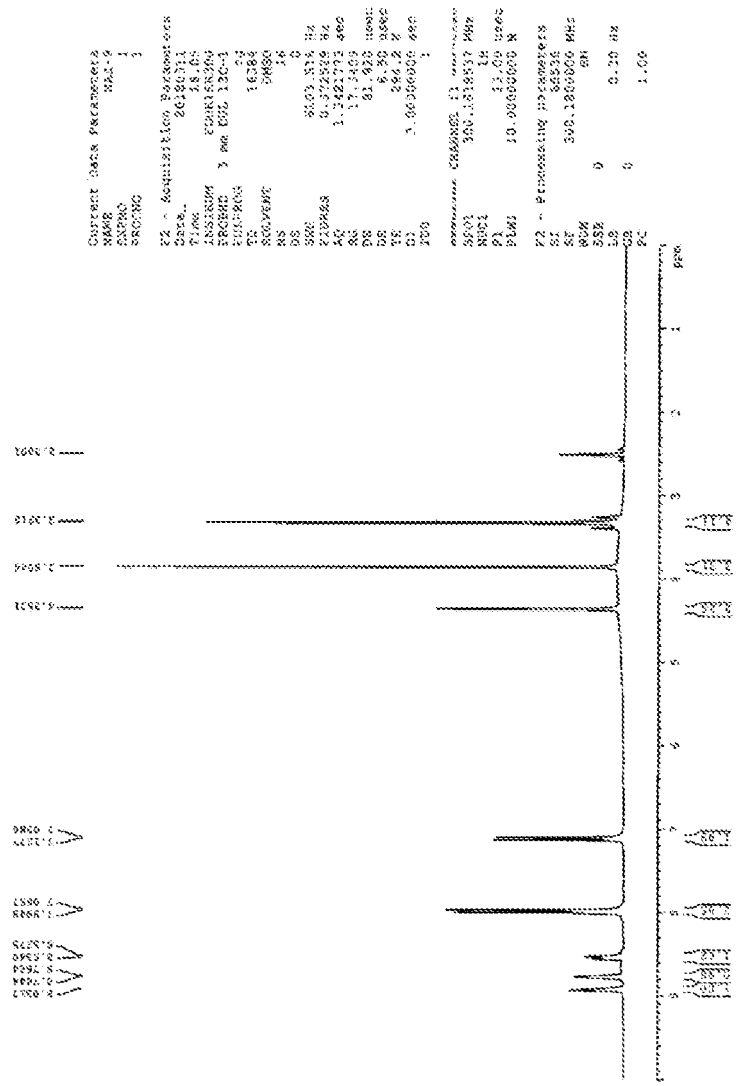

Figure 34

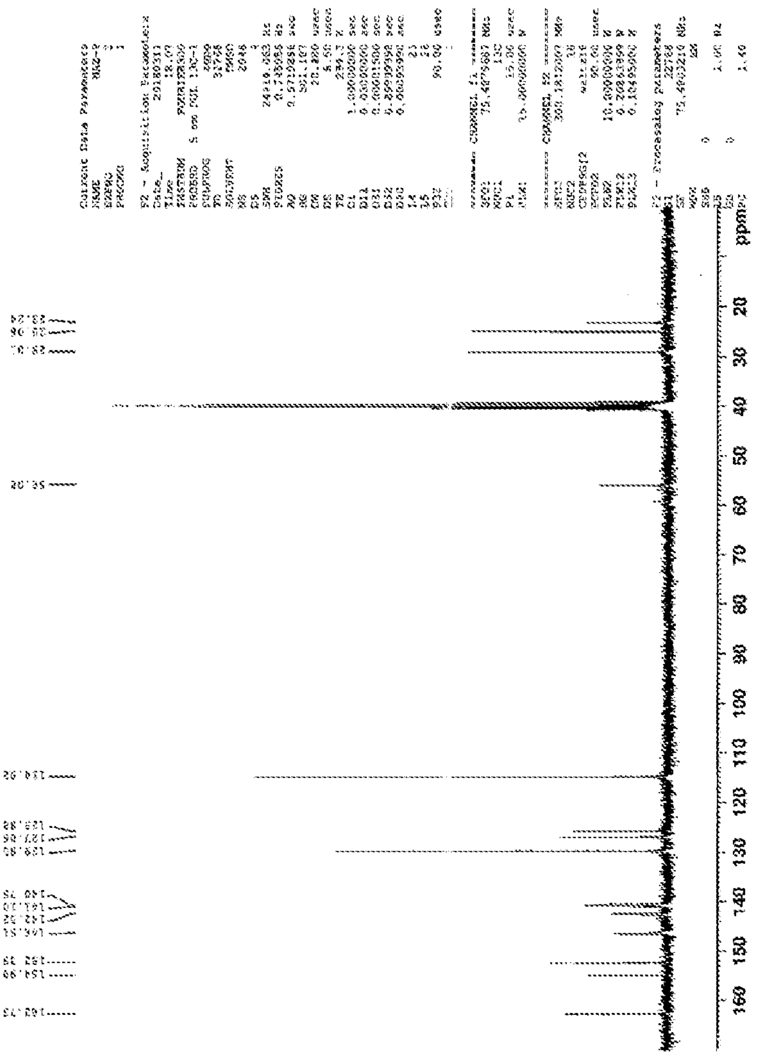

Figure 35

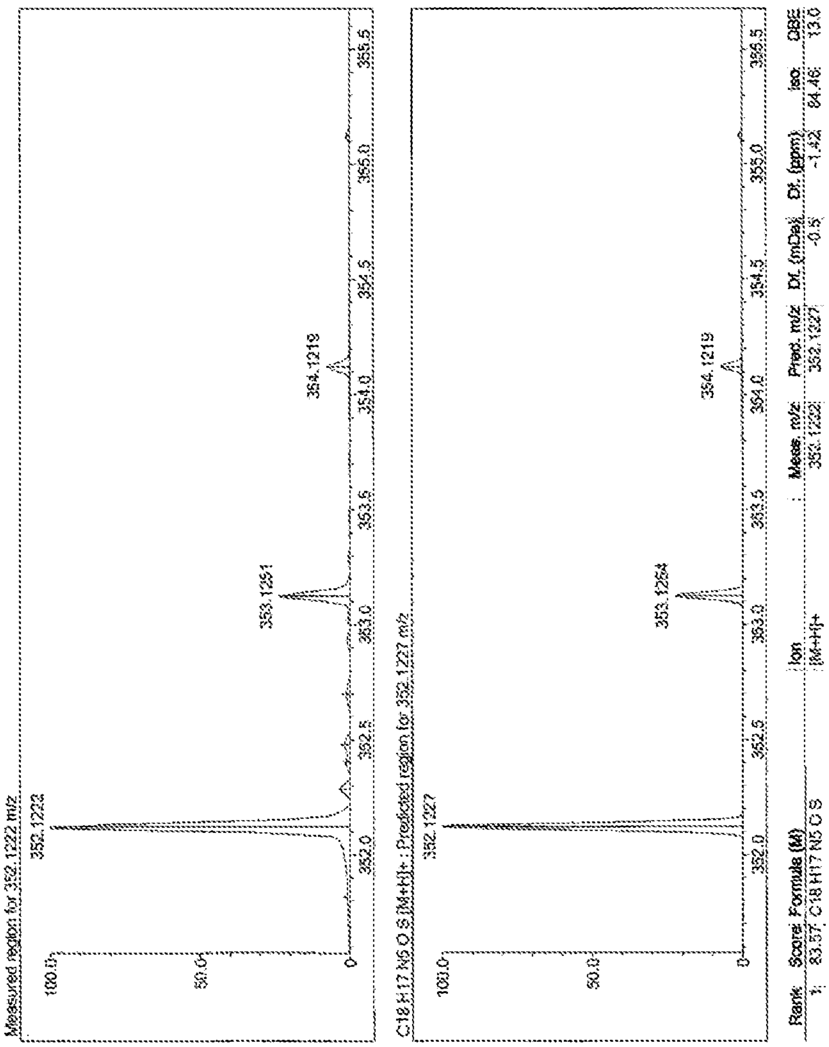
Measured region for 352.1222 m/z
352.1222
353.1251
354.1219
C18 H17 N5 O S [M+H]+ : Predicted region for 352.1227 m/z
352.1227
353.1254
354.1219
Rank Score Formula (M) Ion Meas. m/z Pred. m/z Df. (mDa) Df. (ppm) Iso DBE
1 83.57 C18 H17 N5 O S [M+H]+ 352.1222 352.1227 -0.5 -1.42 84.46 13.0

Figure 36

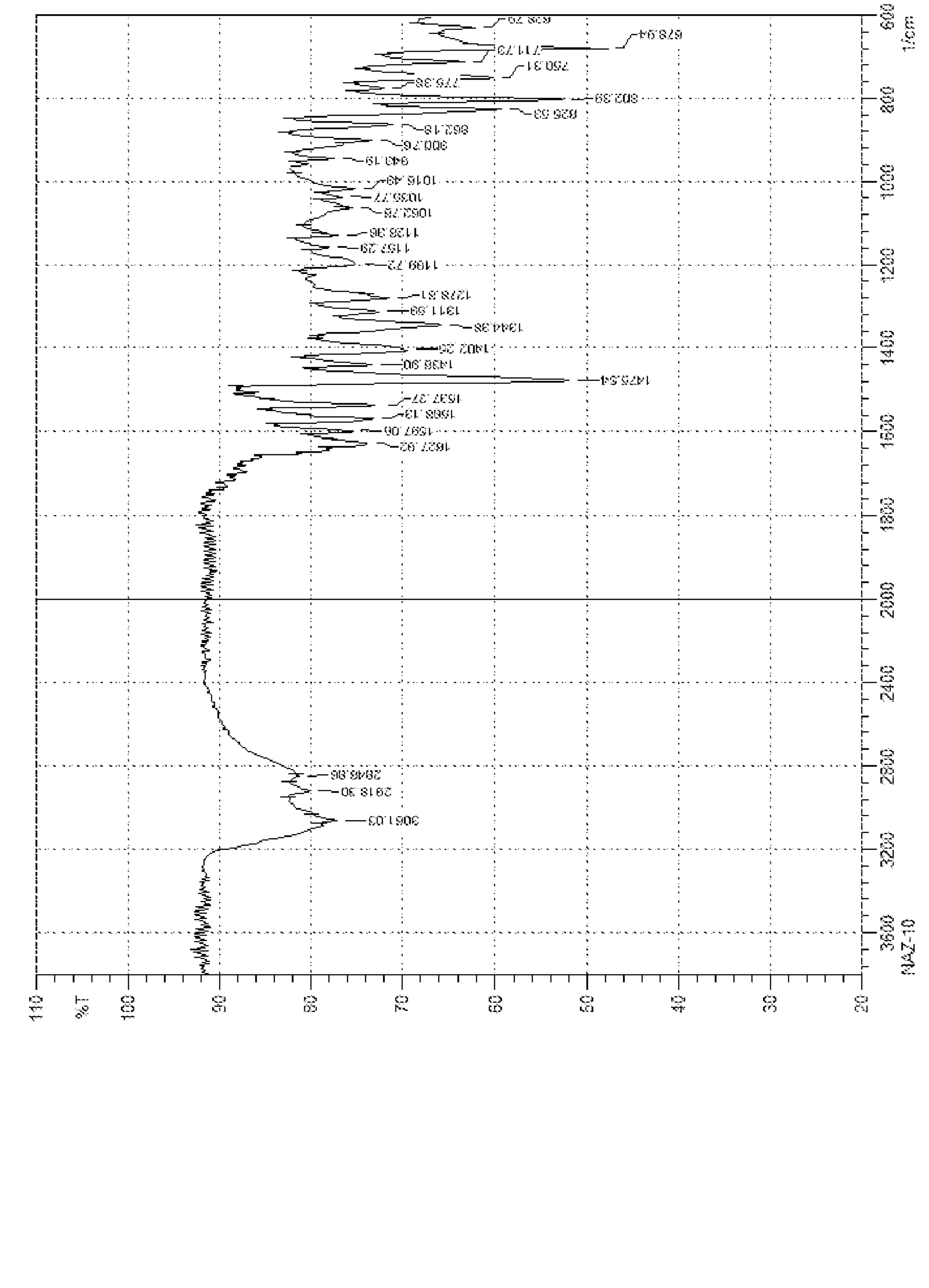

%T
110
100
90
80
70
60
50
40
30
20
1/cm
600
800
1000
1200
1400
1600
1800
2000
2400
2800
3200
3600
NAZ-10
678.94
711.73
750.31
775.38
802.39
825.53
862.18
900.76
948.19
1018.49
1035.77
1062.78
1128.36
1157.29
1199.72
1278.81
1311.59
1344.38
1402.25
1438.90
1475.54
1537.27
1568.13
1597.06
1627.92
2846.86
2918.30
3061.03


**Figure 37**

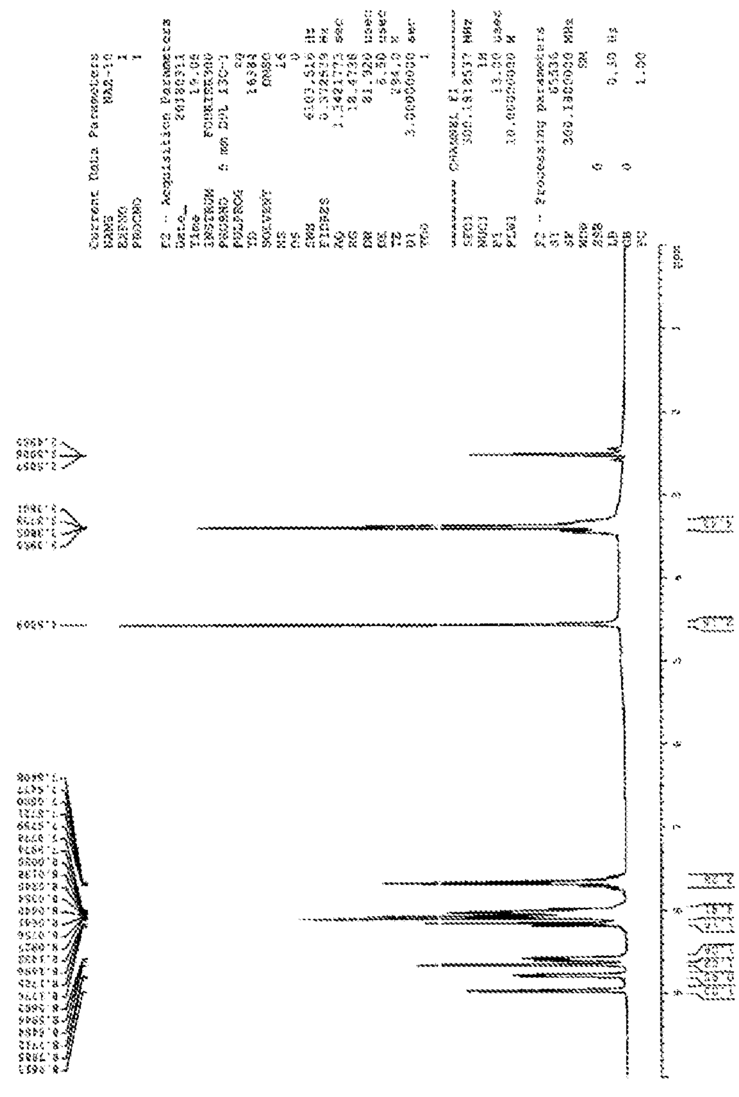

Figure 38

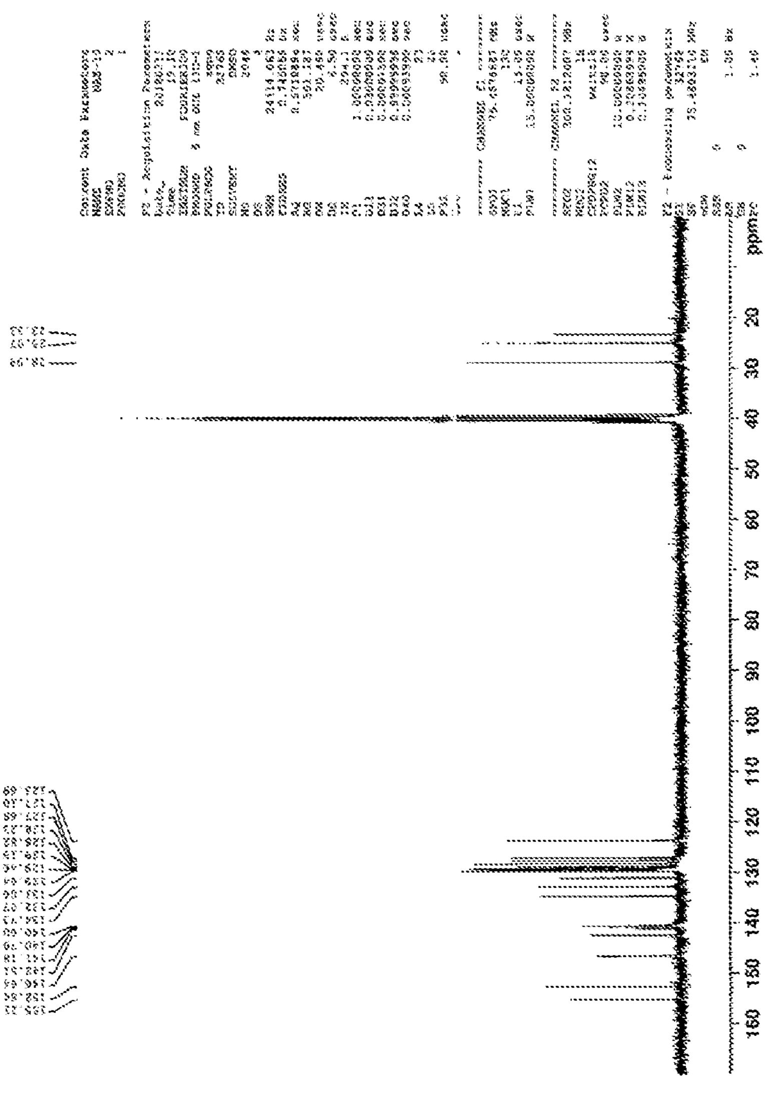

Figure 39

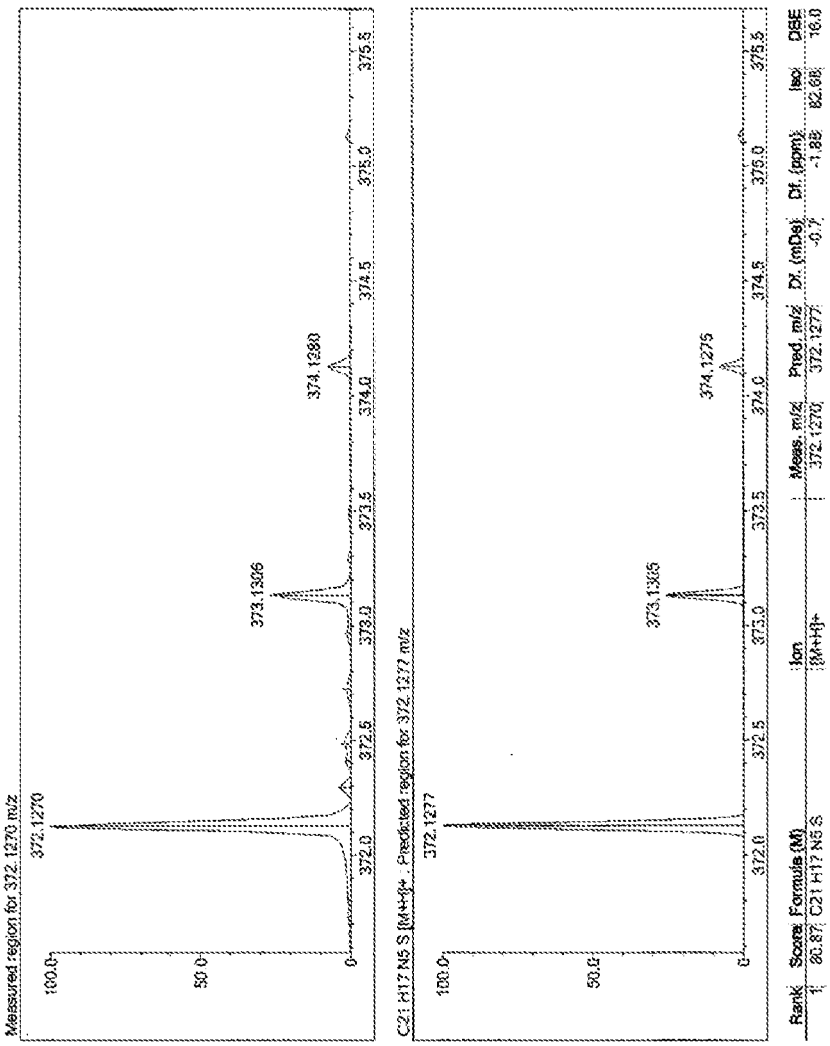
Measured region for 372.1270 m/z
372.1270
373.1306
374.1280
C21 H17 N5 S [M+H]+ : Predicted region for 372.1277 m/z
372.1277
373.1305
374.1275
Rank Score Formula (M) Ion Meas. m/z Pred. m/z Df. (mDa) Df. (ppm) Iso DBE
1 80.87 C21 H17 N5 S [M+H]+ 372.1270 372.1277 -0.7 -1.88 82.68 16.0

Figure 40

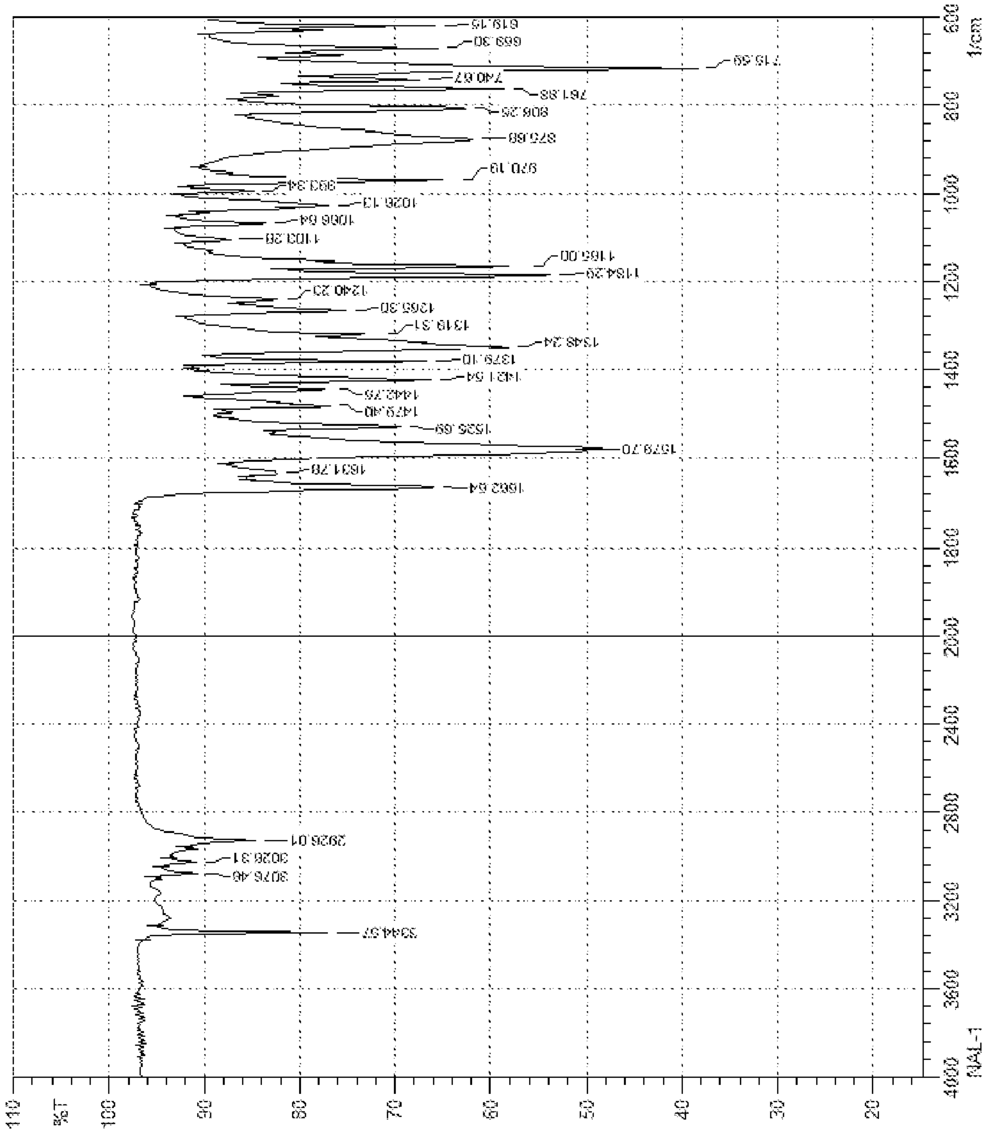
%T
1/cm
619.15
669.30
715.59
740.67
761.88
808.25
875.86
970.19
993.34
1026.13
1066.64
1103.28
1165.00
1184.29
1240.23
1265.30
1319.31
1348.24
1379.10
1421.54
1442.75
1479.40
1525.69
1579.70
1631.78
1662.64
2926.01
3028.31
3076.46
3344.57
NAL-1

**Figure 41**

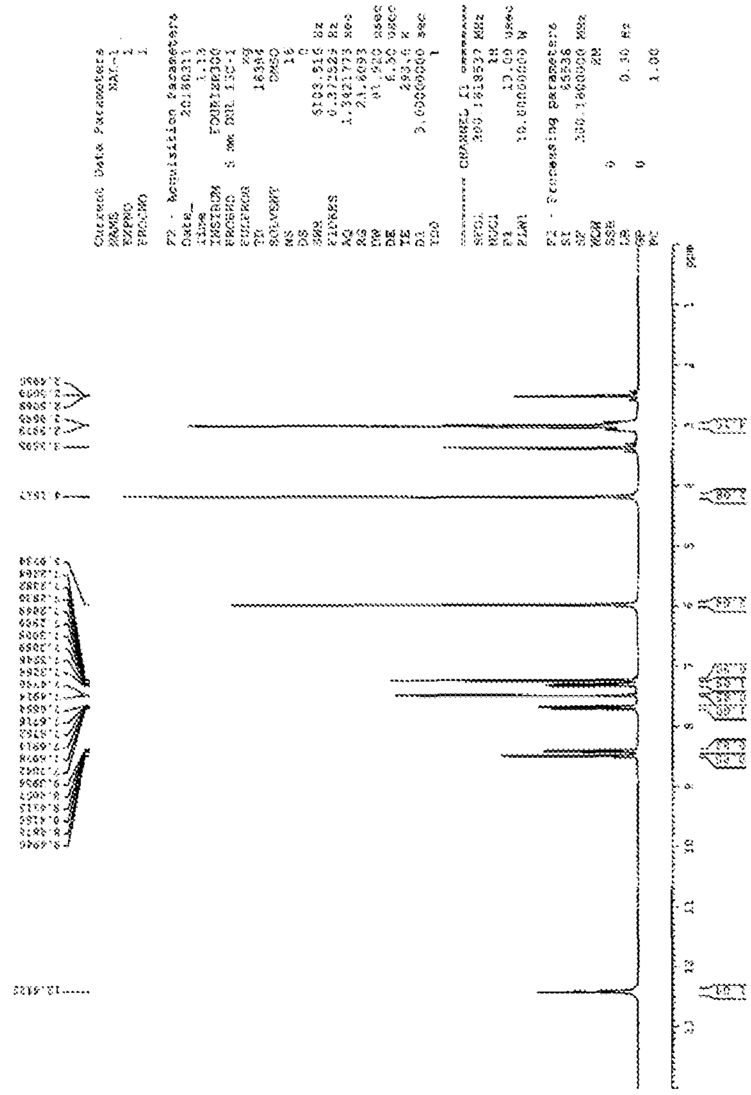

Figure 42

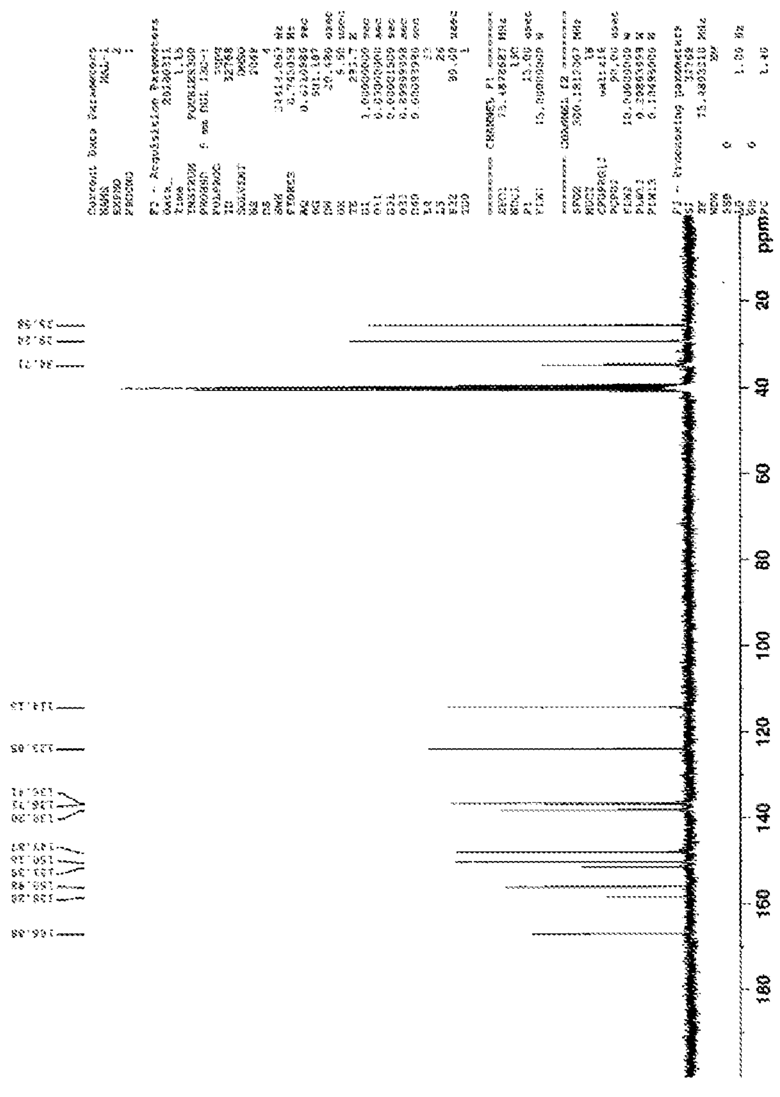

Figure 43

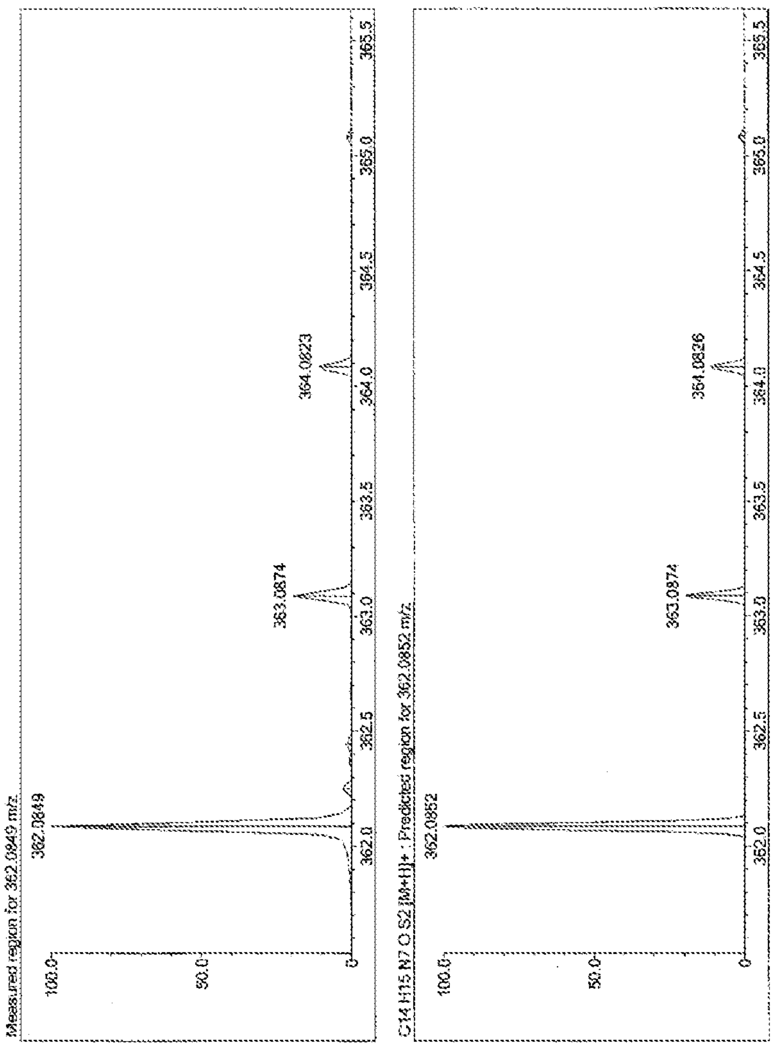


| Rank | Score | Formula (M) | Ion | Meas. m/z | Pred. m/z | Df. (mDa) | Df. (ppm) | Iso | DBE |
|---|---|---|---|---|---|---|---|---|---|
| 1 | 100.00 | C14 H15 N7 O S2 | [M+H]+ | 362.0849 | 362.0852 | -0.3 | -0.83 | 100.00 | 11.0 |

Figure 44

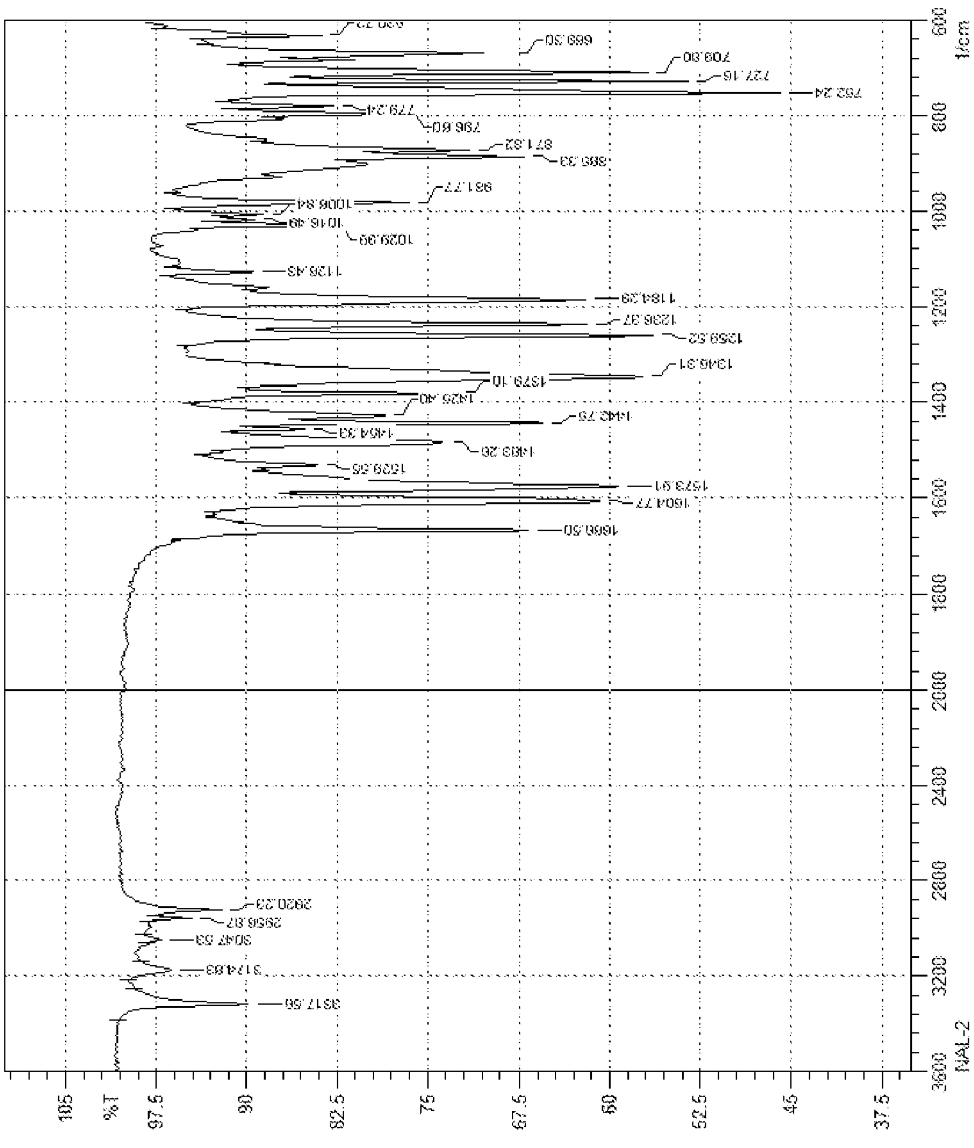
%T
105
97.5
90
82.5
75
67.5
60
52.5
45
37.5
3600
3200
2800
2400
2000
1800
1600
1400
1200
1000
800
600
1/cm
NAL-2
3317.56
3174.83
3047.53
2956.87
2920.23
1660.50
1604.77
1573.91
1529.55
1483.26
1454.33
1442.75
1425.40
1379.10
1346.31
1259.52
1236.37
1184.29
1126.43
1029.99
1016.49
1006.84
981.77
885.33
871.82
796.60
779.24
752.24
727.16
709.80
669.30

Figure 45

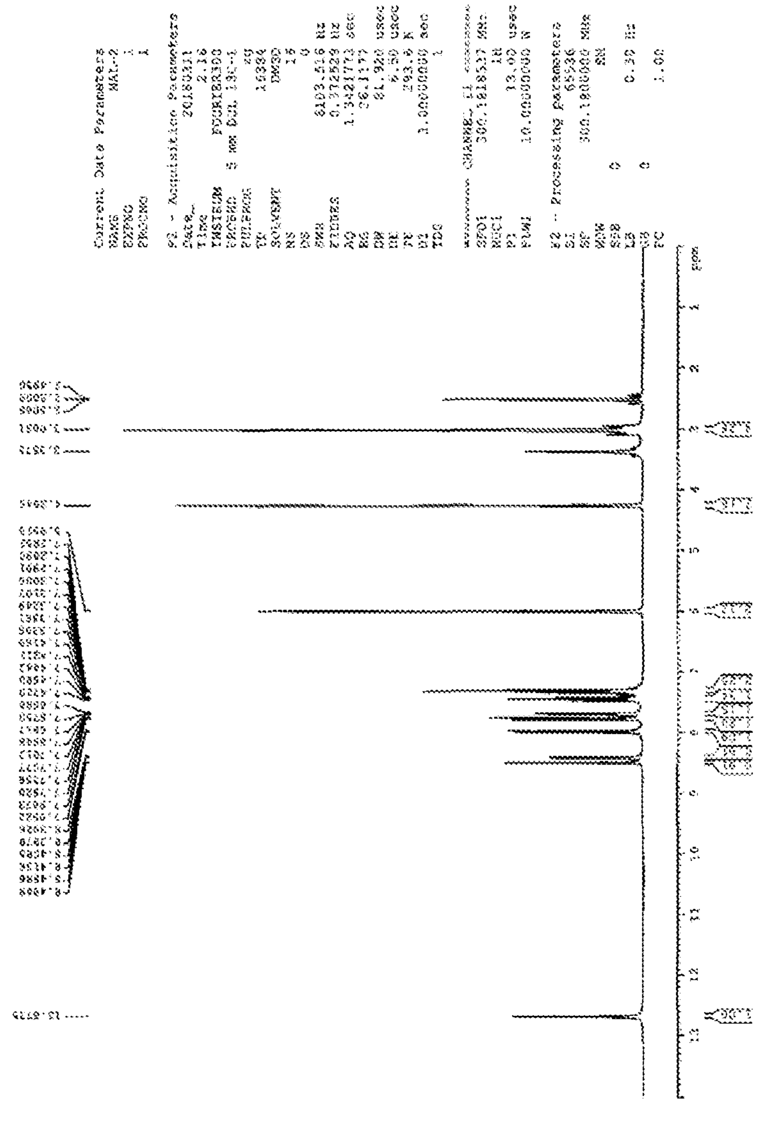

Figure 46

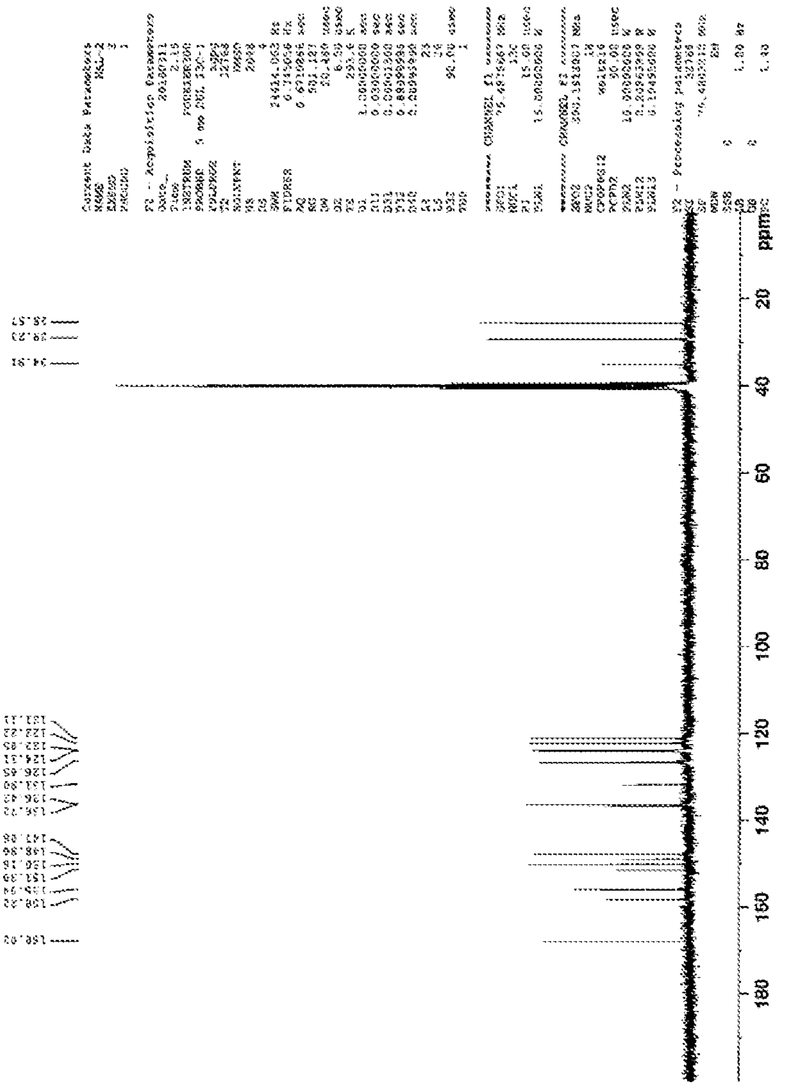

Figure 47

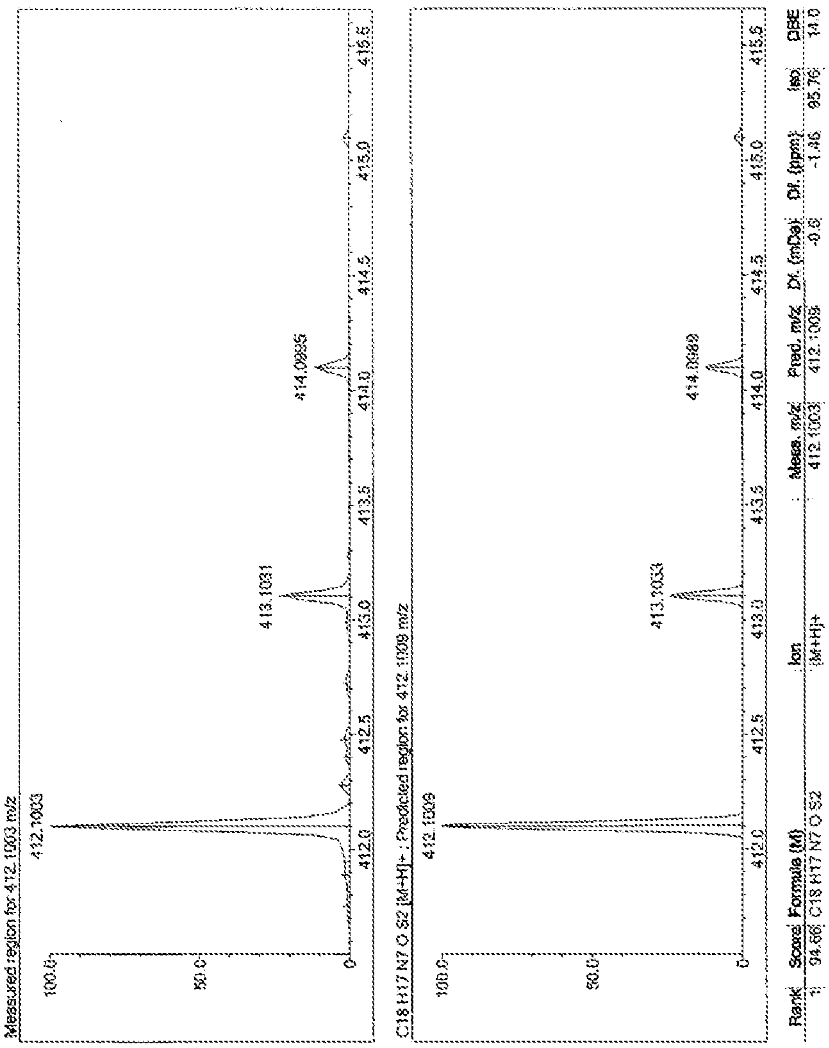
Measured region for 412.1003 m/z
412.1003
413.1031
414.0985
C18 H17 N7 O S2 [M+H]+ : Predicted region for 412.1009 m/z
412.1009
413.1034
414.0989
Rank Score Formula (M) Ion Meas. m/z Pred. m/z Df. (mDa) Df. (ppm) Iso DBE
1 94.66 C18 H17 N7 O S2 [M+H]+ 412.1003 412.1009 -0.6 -1.46 95.76 14.0

Figure 48

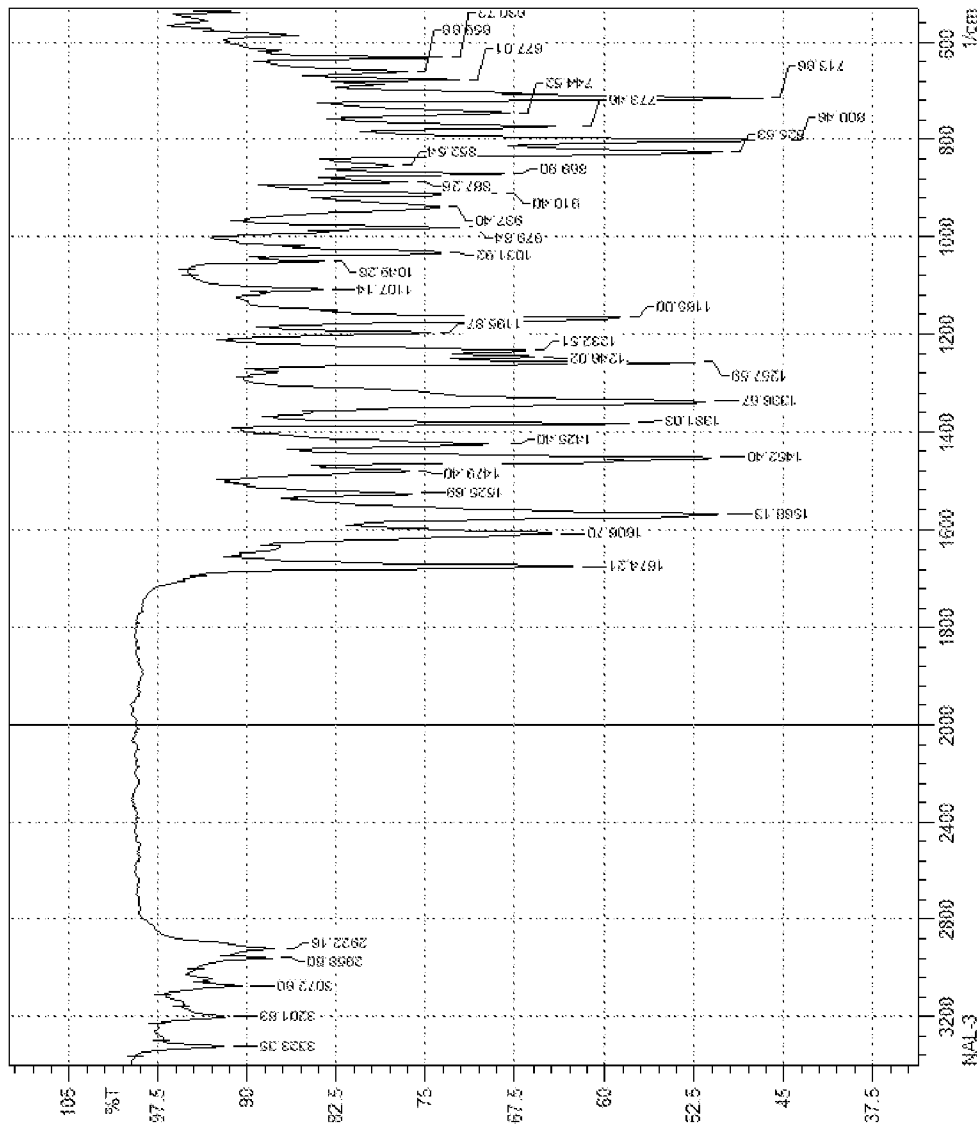
%T
105
97.5
90
82.5
75
67.5
60
52.5
45
37.5
1/cm
600
800
1000
1200
1400
1600
1800
2000
2400
2800
3200
NAL-3

Figure 49

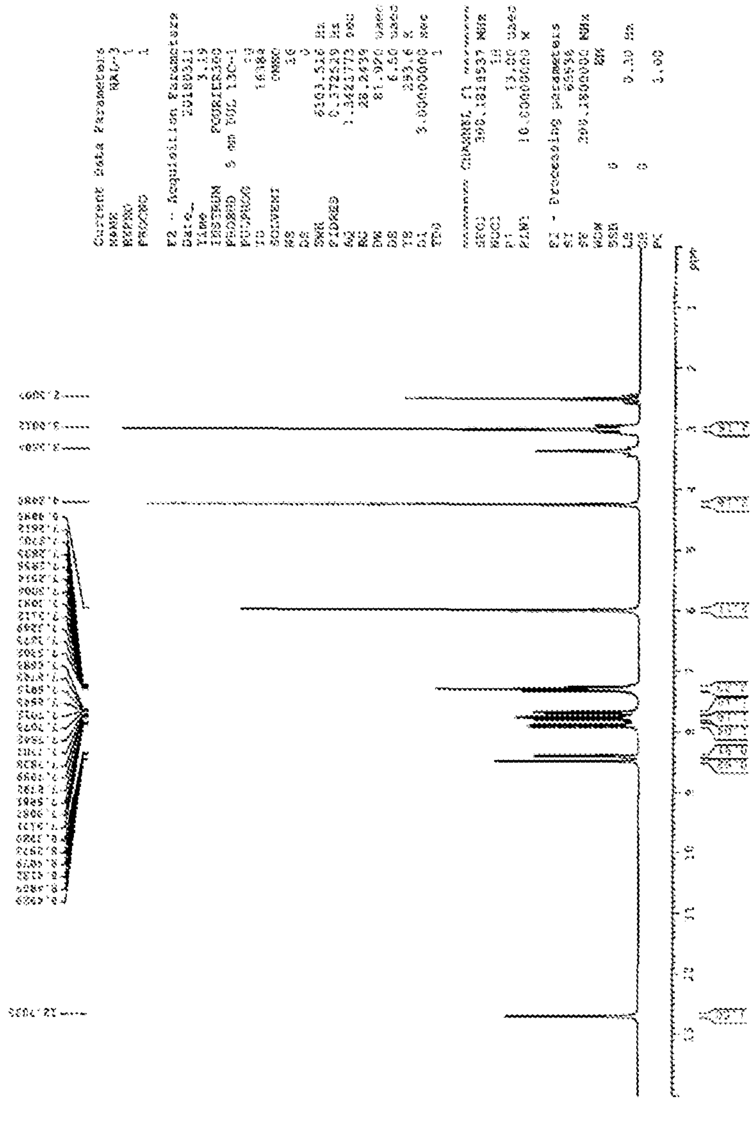

Figure 50

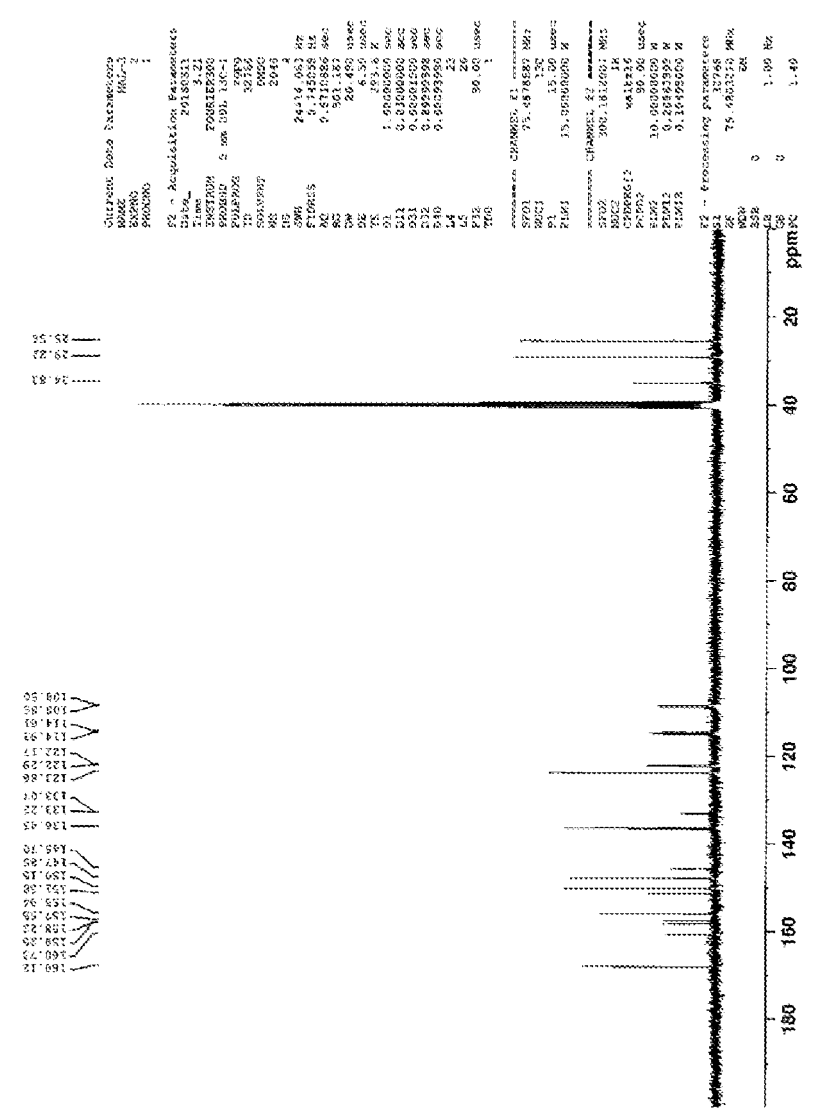

Figure 51

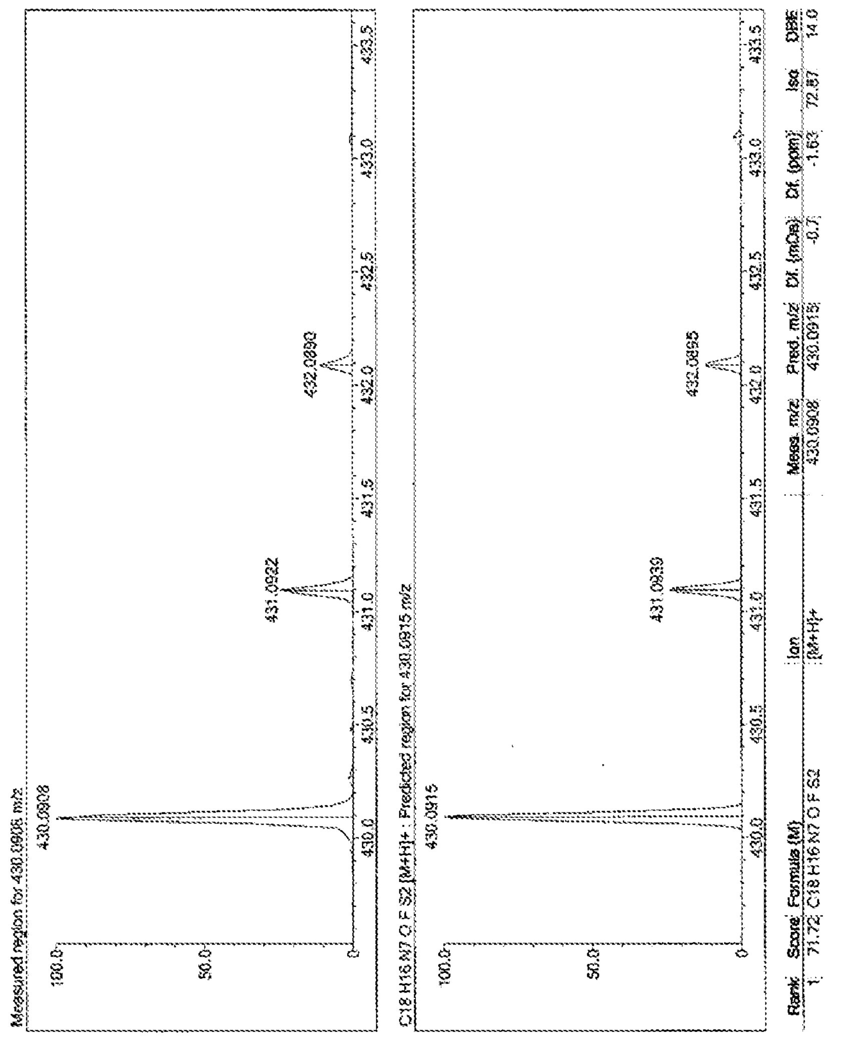
Measured region for 430.0908 m/z
430.0908
431.0922
432.0890
C18 H16 N7 O F S2 [M+H]+ : Predicted region for 430.0915 m/z
430.0915
431.0939
432.0895
Rank Score Formula (M) Ion Meas. m/z Pred. m/z Df. (mDa) Df. (ppm) Iso DBE
1 71.72 C18 H16 N7 O F S2 [M+H]+ 430.0908 430.0915 -0.7 -1.63 72.87 14.0

Figure 52

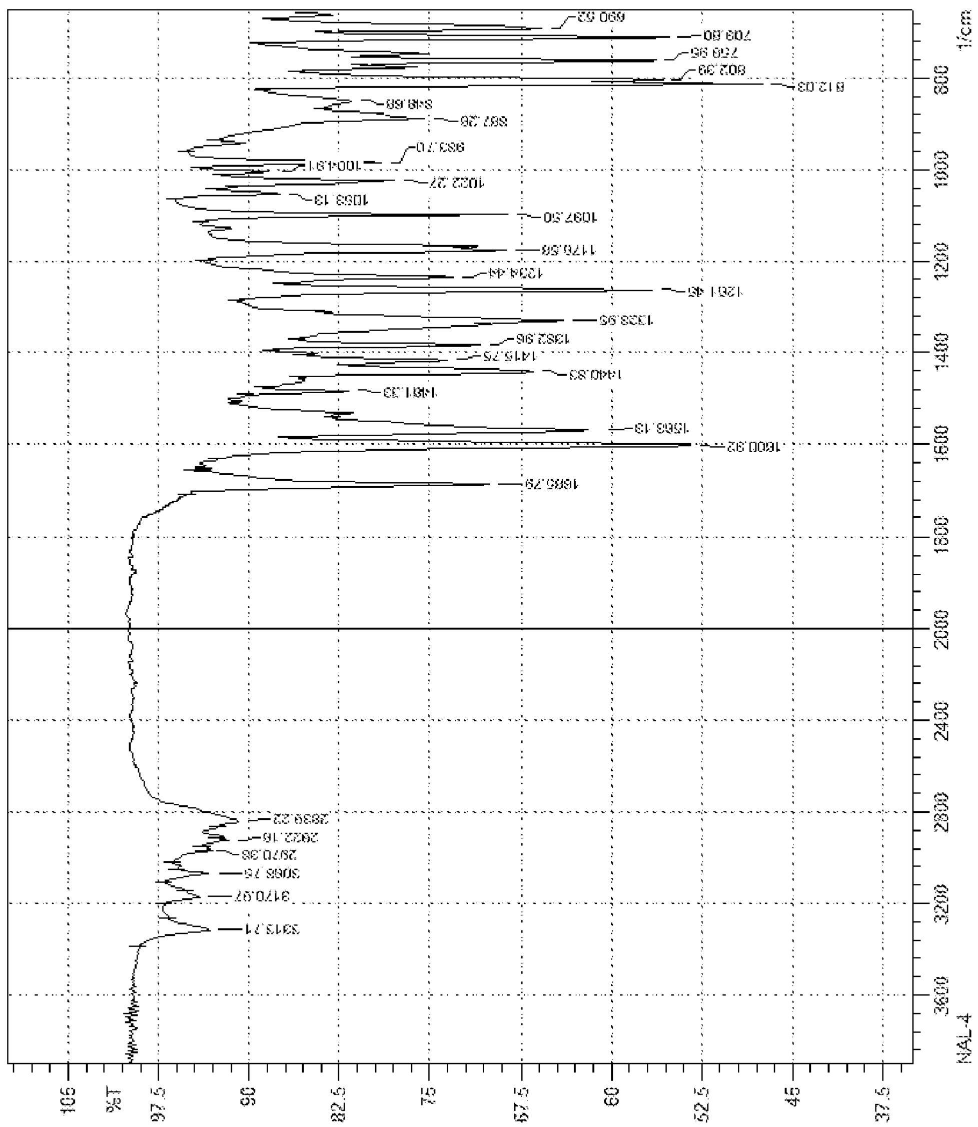

%T
105
97.5
90
82.5
75
67.5
60
52.5
45
37.5
1/cm
3600
3200
2800
2400
2000
1600
1200
1000
800
NAL-4
690.62
709.80
759.95
802.39
812.03
848.68
887.26
983.70
1004.81
1022.27
1053.13
1097.50
1170.58
1234.44
1261.45
1328.95
1382.96
1415.75
1440.83
1481.33
1568.13
1600.92
1685.79
2839.22
2922.16
2970.38
3068.75
3170.97
3313.71


Figure 53

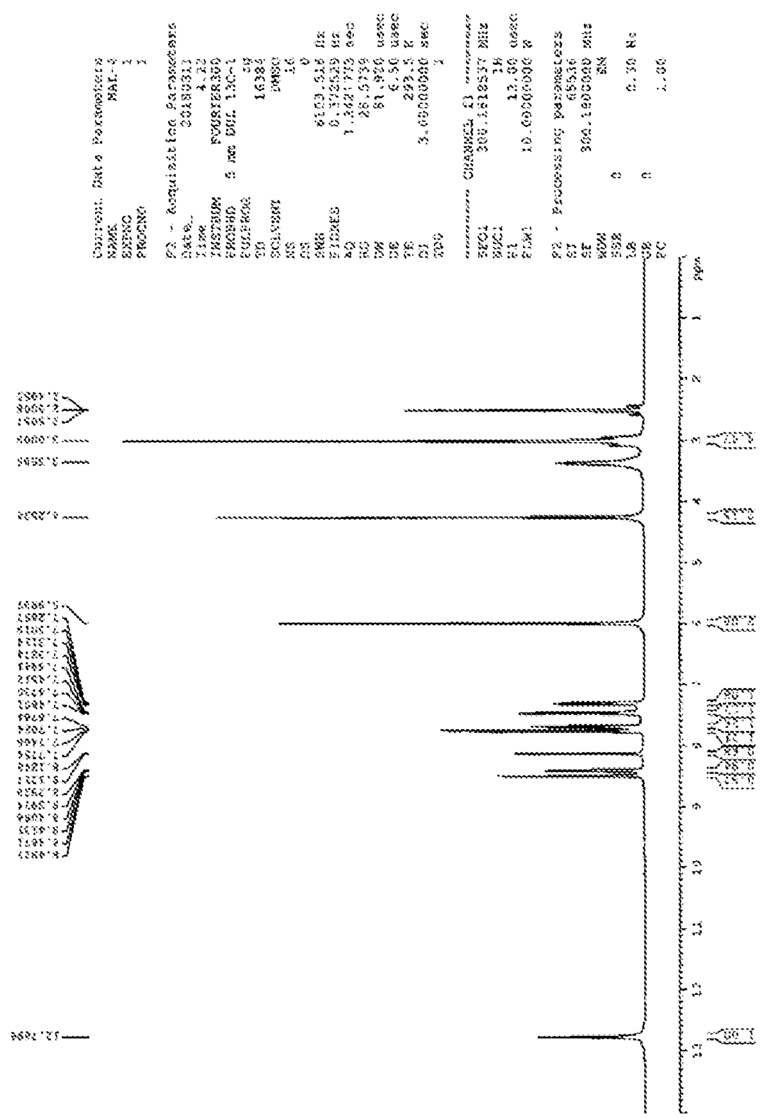

Figure 54

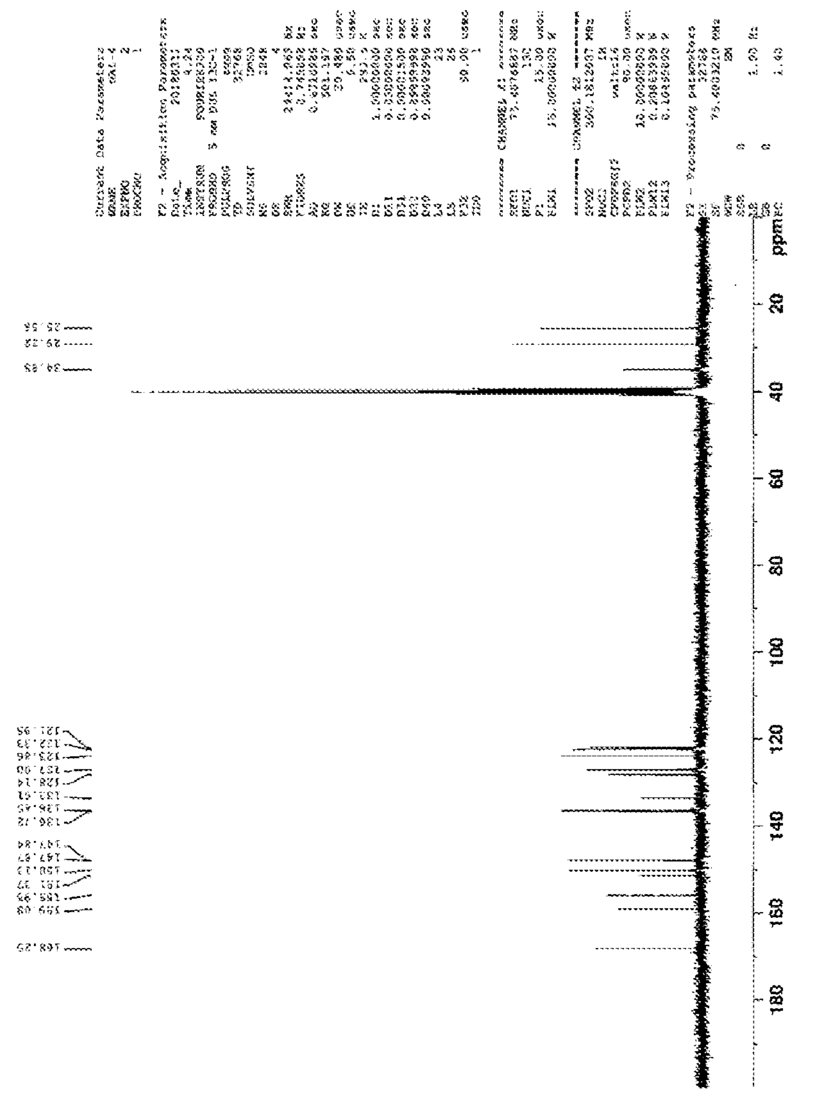

Figure 55

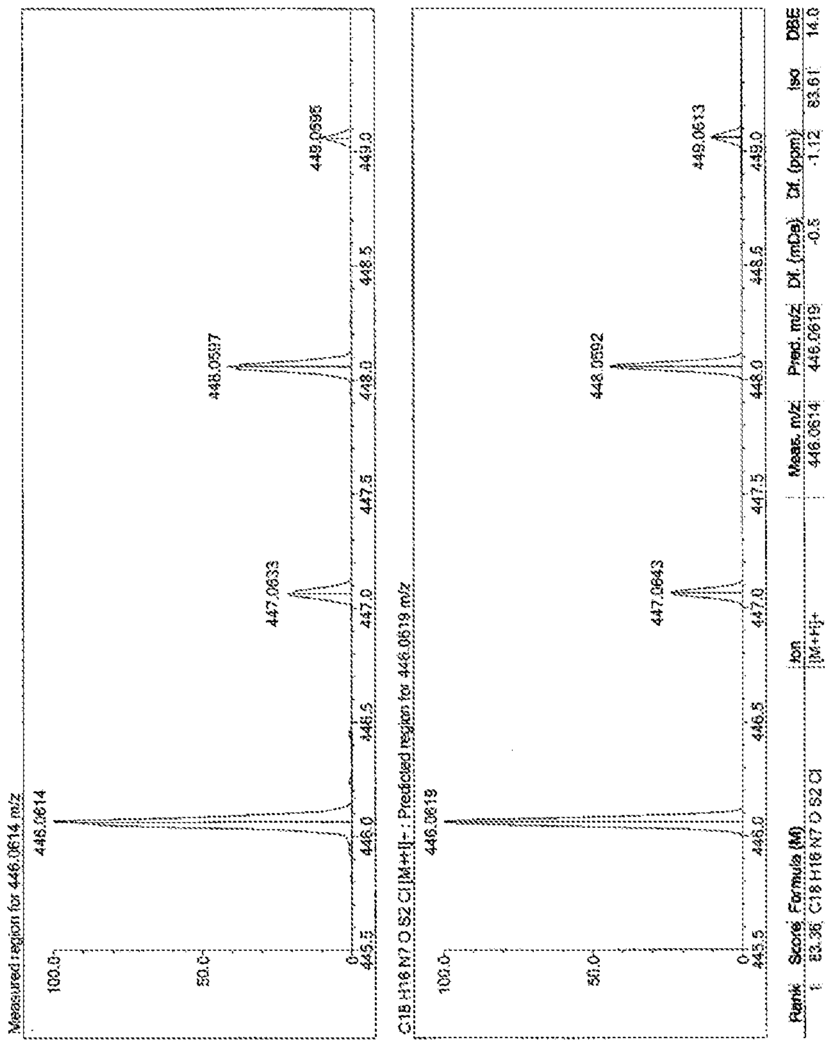
Measured region for 446.0614 m/z
446.0614
447.0633
448.0587
449.0595
C18 H18 N7 O S2 Cl [M+H]+ : Predicted region for 446.0619 m/z
446.0619
447.0643
448.0592
449.0613
Rank Score Formula (M) Ion Meas. m/z Pred. m/z Df. (mDa) Df. (ppm) Iso DBE
1 83.36 C18 H18 N7 O S2 Cl [M+H]+ 446.0614 446.0619 -0.5 -1.12 83.61 14.0

Figure 56

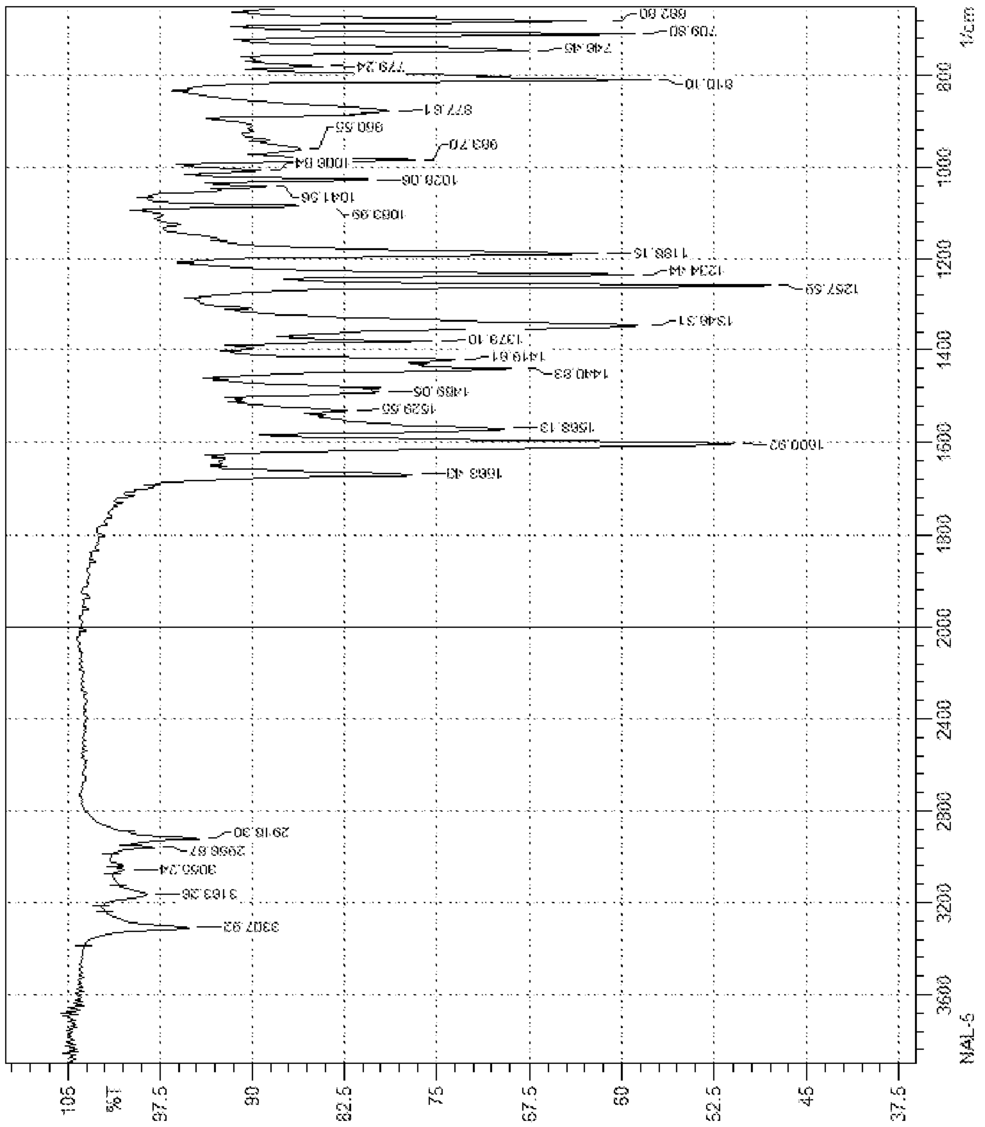
%T
105
97.5
90
82.5
75
67.5
60
52.5
45
37.5
1/cm
3600
3200
2800
2400
2000
1800
1600
1400
1200
1000
800
NAL-5
3307.92
3163.26
3055.24
2956.87
2916.30
1683.43
1600.92
1568.13
1529.55
1489.05
1440.83
1419.61
1379.10
1346.31
1257.59
1234.44
1188.15
1083.99
1041.56
1028.06
1006.84
983.70
960.55
877.61
810.10
779.24
748.45
709.80
682.60

Figure 57

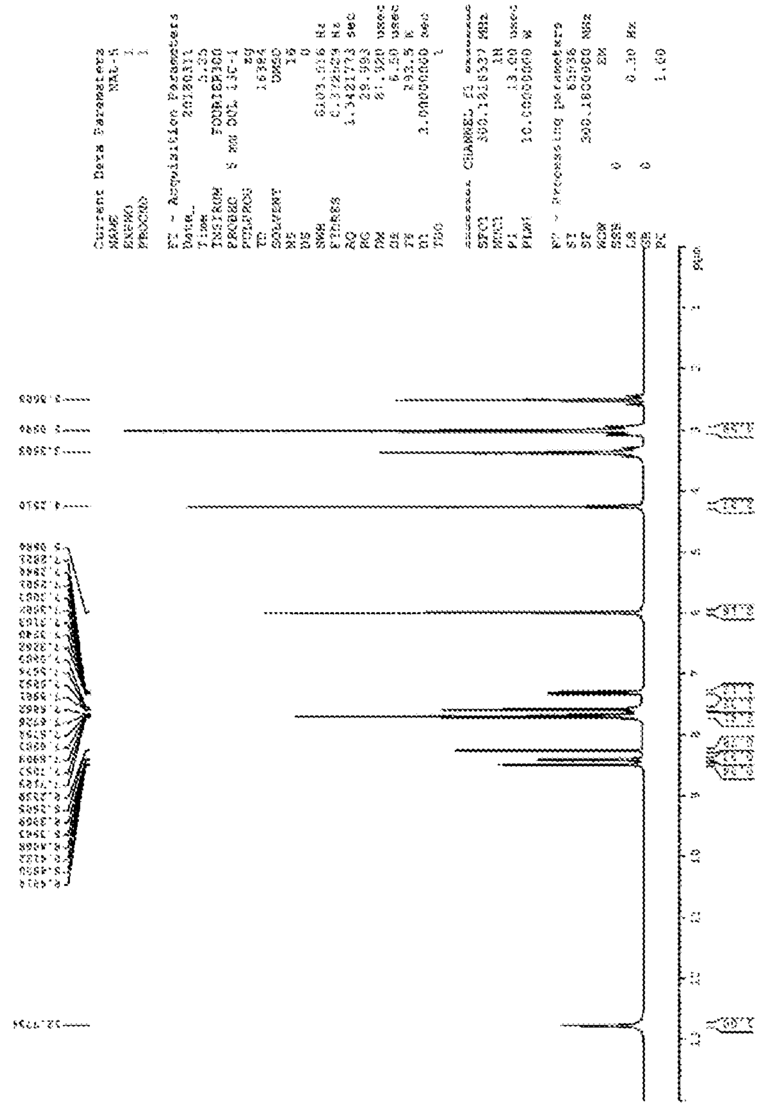

Figure 58

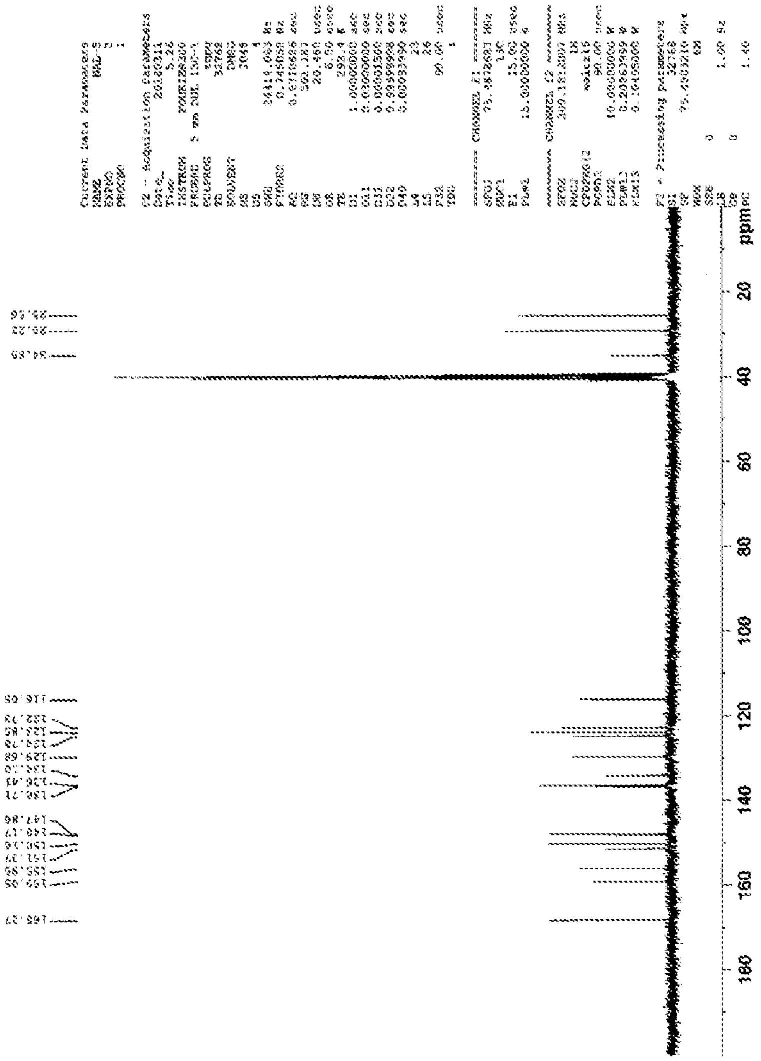

Figure 59

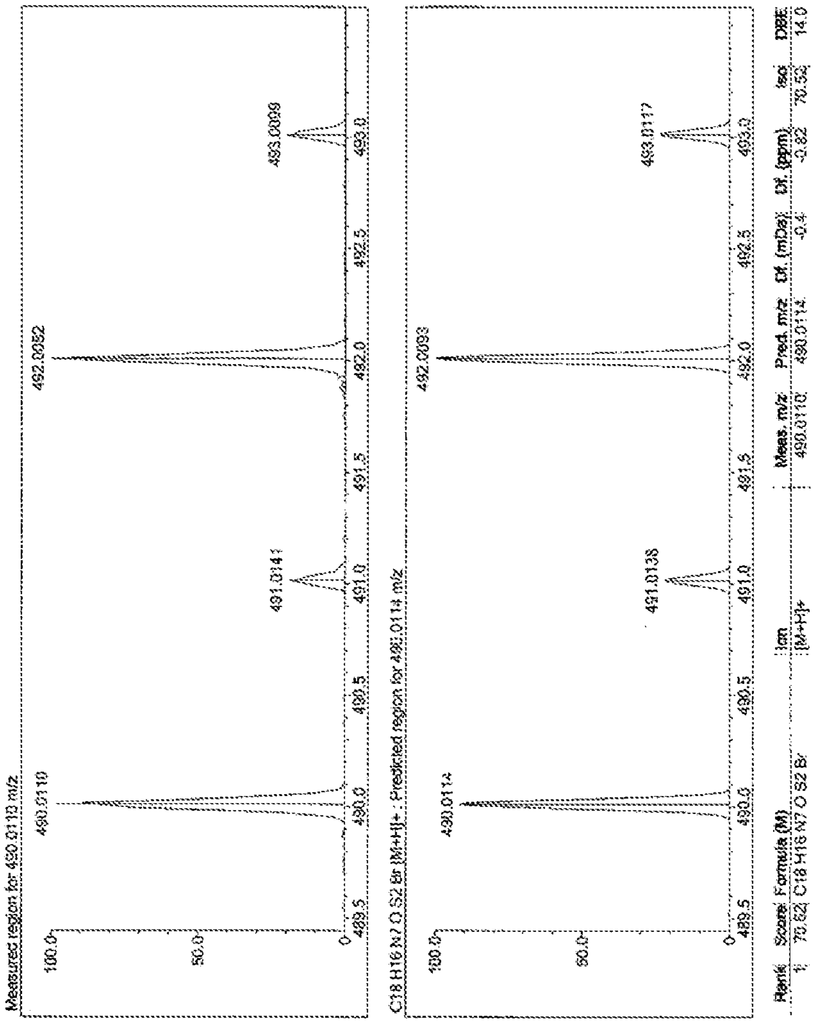
Measured region for 490.0113 m/z
100.0
50.0
0
490.0110
491.0141
492.0082
493.0099
489.5
490.0
490.5
491.0
491.5
492.0
492.5
493.0
C18 H16 N7 O S2 Br [M+H]+ : Predicted region for 490.0114 m/z
100.0
50.0
0
490.0114
491.0138
492.0093
493.0117
489.5
490.0
490.5
491.0
491.5
492.0
492.5
493.0
Rank Score Formula (M) Ion Meas. m/z Pred. m/z Df. (mDa) Df. (ppm) Iso DBE
1 70.52 C18 H16 N7 O S2 Br [M+H]+ 490.0110 490.0114 -0.4 -0.82 70.52 14.0

**Figure 60**

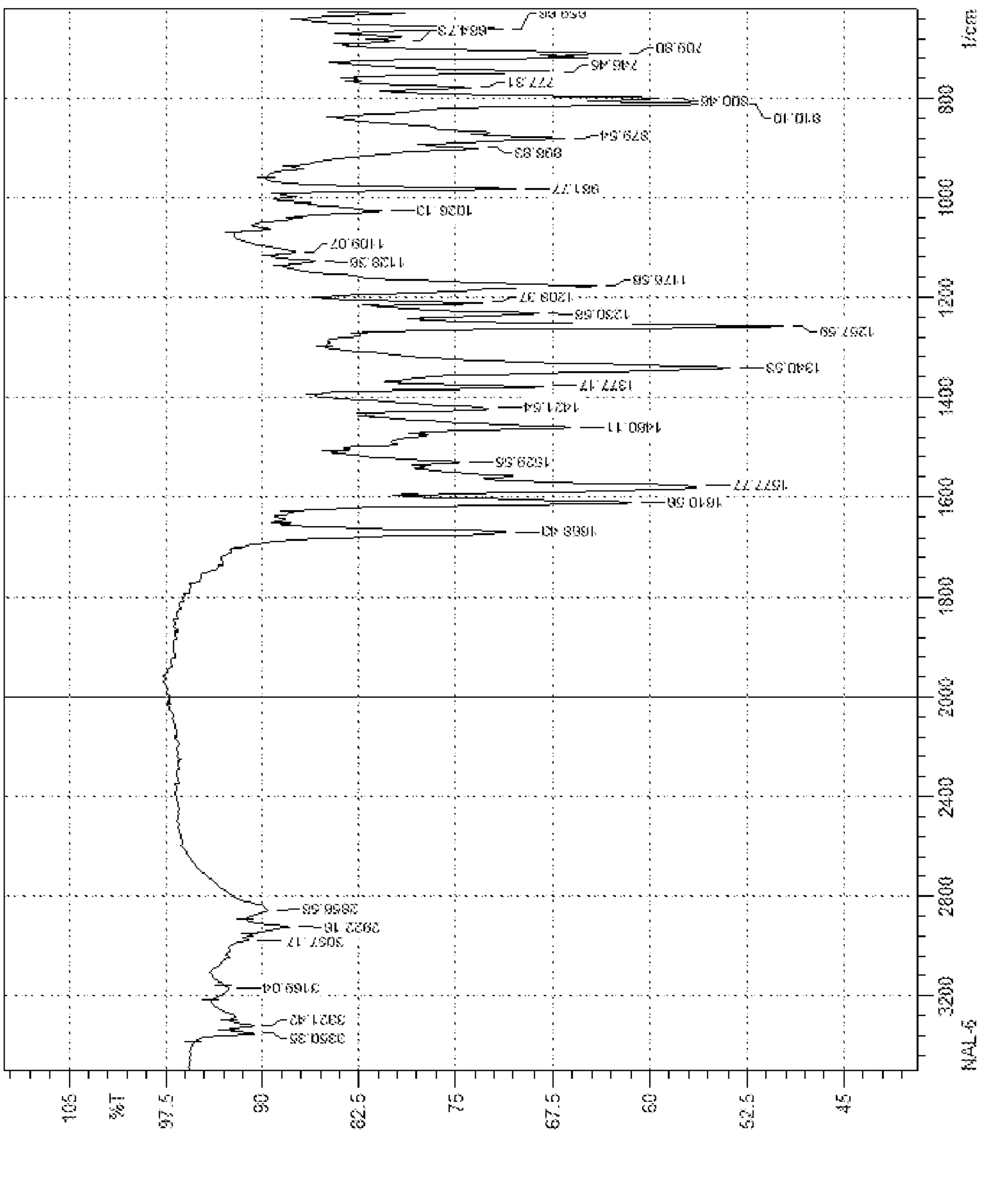
%T
1/cm
NAL-5
105
97.5
90
82.5
75
67.5
60
52.5
45
3200
2800
2400
2000
1800
1600
1400
1200
1000
800
3350.35
3321.42
3169.04
3057.17
2922.16
2856.56
1668.43
1610.56
1577.77
1529.56
1460.11
1421.54
1377.17
1340.53
1257.59
1230.58
1209.37
1176.58
1138.38
1109.07
1026.13
981.77
898.83
879.54
810.10
800.46
777.31
746.45
709.80
684.73

**Figure 61**

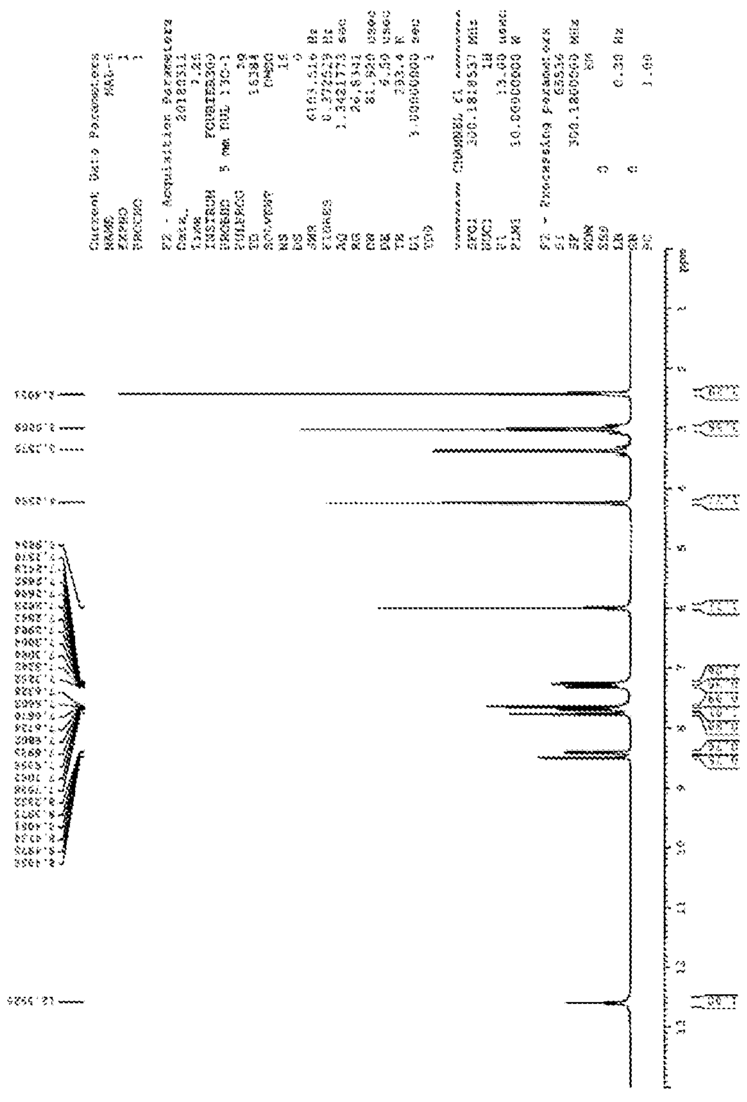

Figure 62

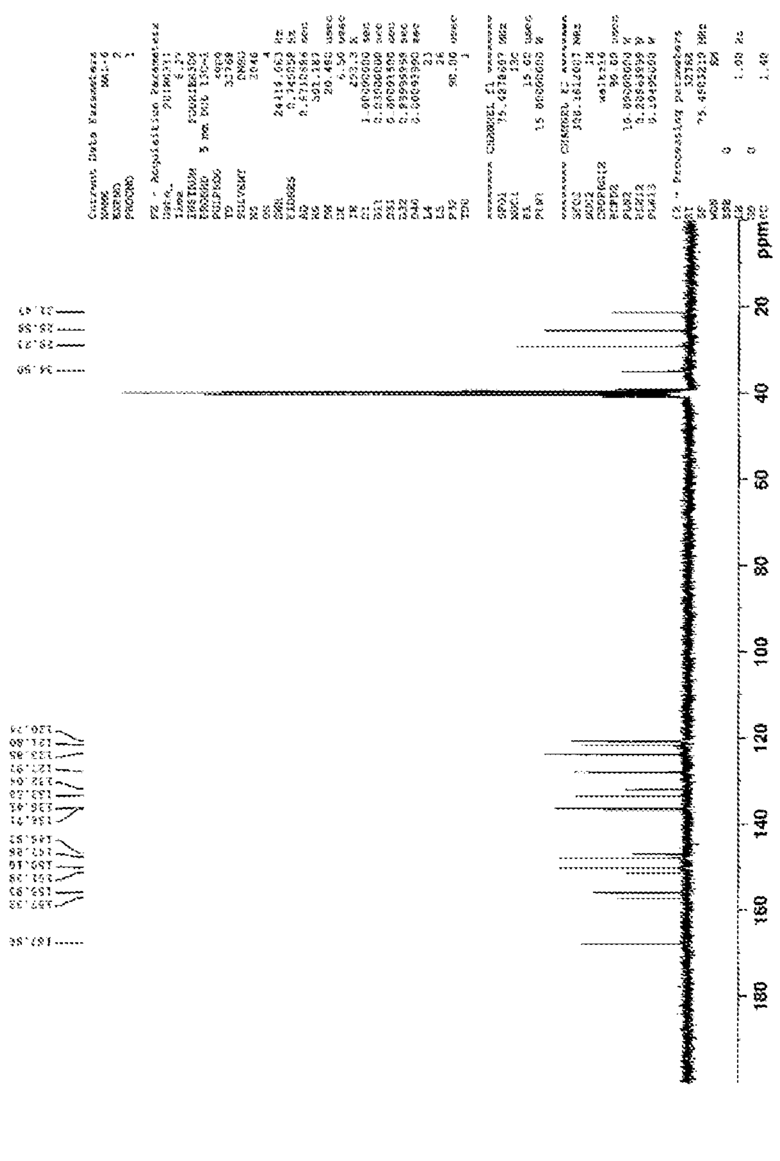

Figure 63

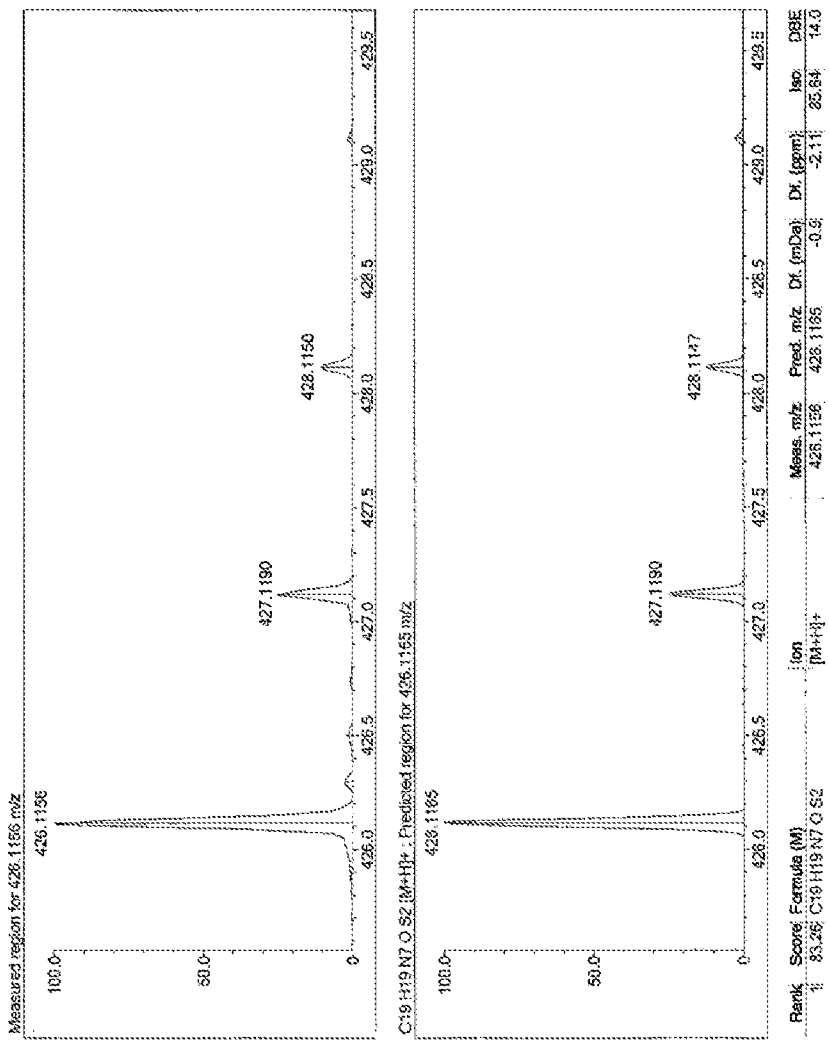
Measured region for 426.1156 m/z
426.1156
427.1190
428.1150
C19 H19 N7 O S2 [M+H]+ : Predicted region for 426.1165 m/z
426.1165
427.1190
428.1147
100.0
50.0
0
426.0
426.5
427.0
427.5
428.0
428.5
429.0
429.5
Rank Score Formula (M) Ion Meas. m/z Pred. m/z Df. (mDa) Df. (ppm) Iso DBE
1 83.26 C19 H19 N7 O S2 [M+H]+ 426.1156 426.1165 -0.9 -2.11 85.64 14.0

Figure 64

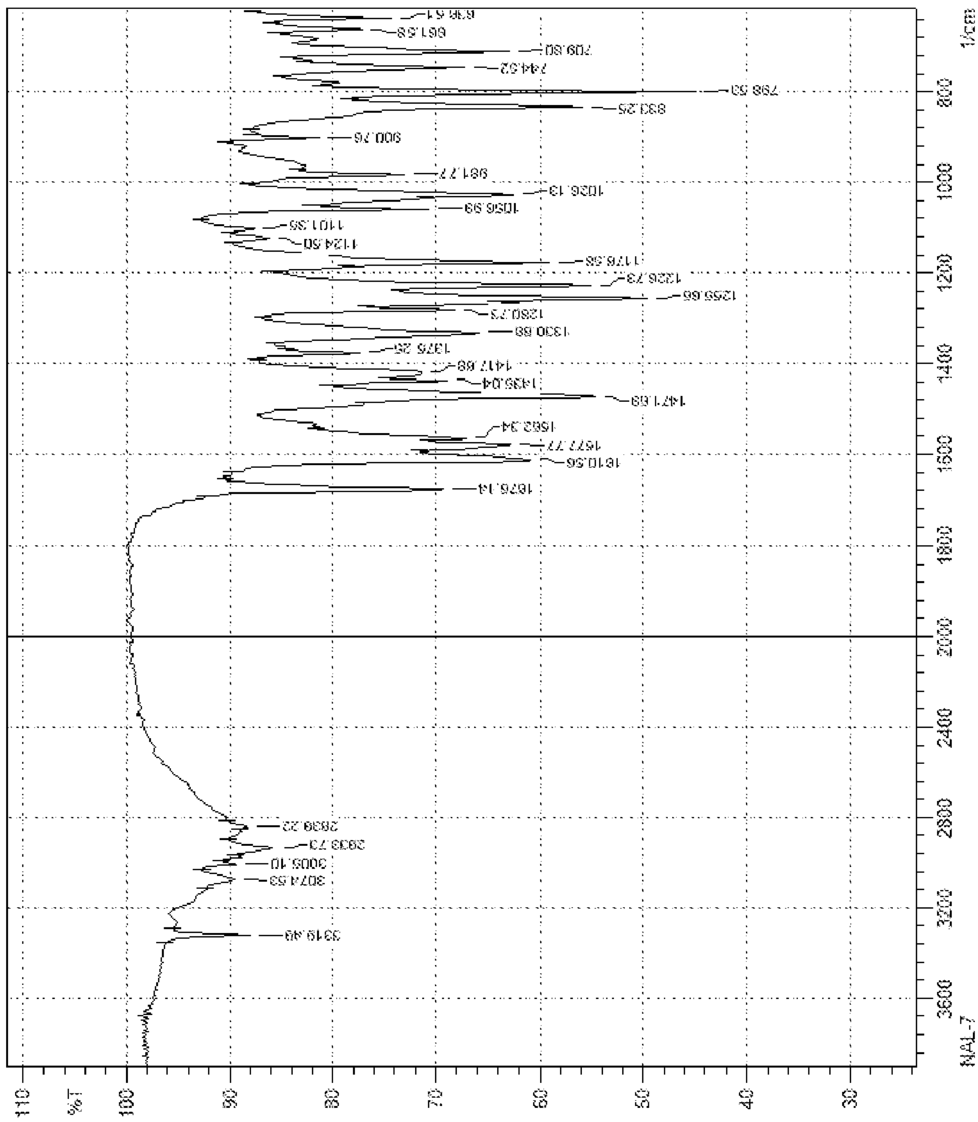
%T
1/cm
NAL_7
3319.48
3074.53
3005.10
2933.73
2839.22
1676.14
1610.56
1577.77
1562.34
1471.69
1435.04
1417.68
1375.25
1330.88
1280.73
1255.66
1226.73
1176.58
1124.50
1101.35
1056.99
1026.13
981.77
900.76
833.25
798.53
744.52
709.80
661.58

**Figure 65**

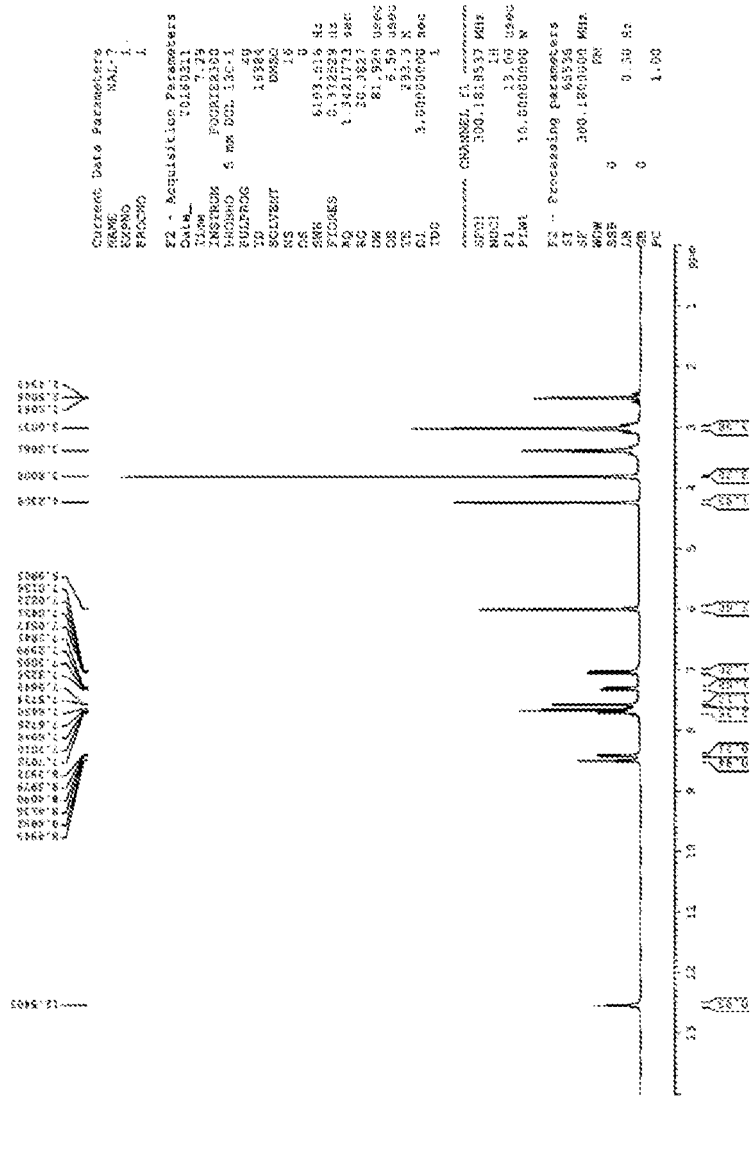

Figure 66

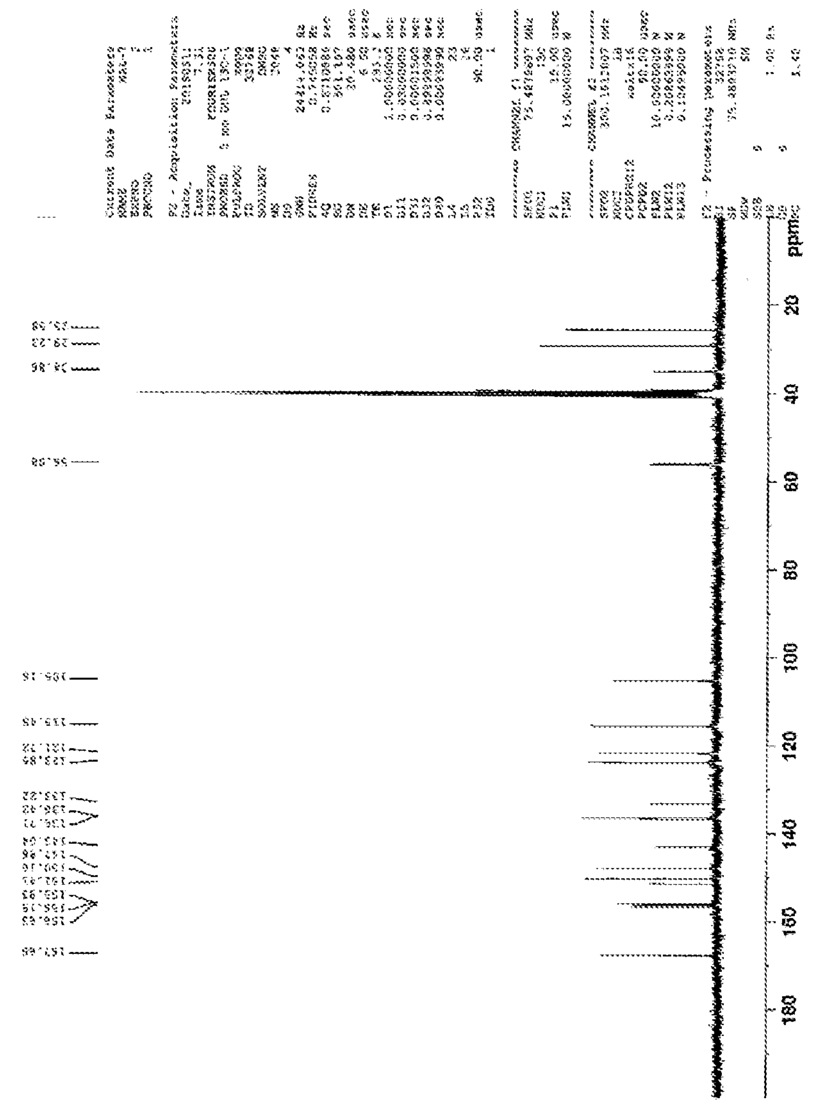


Figure 67

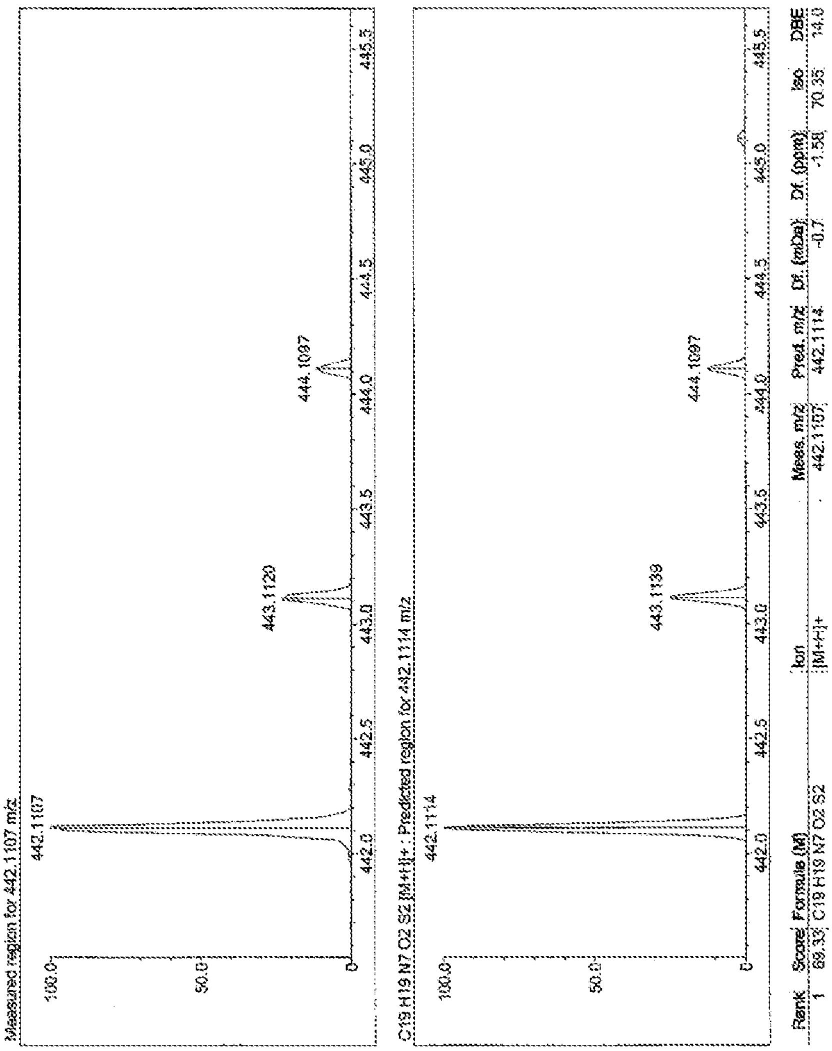
Measured region for 442.1107 m/z
100.0
50.0
0
442.1107
443.1129
444.1097
442.0
442.5
443.0
443.5
444.0
444.5
445.0
445.5
C19 H19 N7 O2 S2 [M+H]+ : Predicted region for 442.1114 m/z
100.0
50.0
0
442.1114
443.1139
444.1097
442.0
442.5
443.0
443.5
444.0
444.5
445.0
445.5
Rank Score Formula (M) Ion Meas. m/z Pred. m/z Df. (mDa) Df. (ppm) Iso DBE
1 69.33 C19 H19 N7 O2 S2 [M+H]+ 442.1107 442.1114 -0.7 -1.58 70.35 14.0

Figure 68

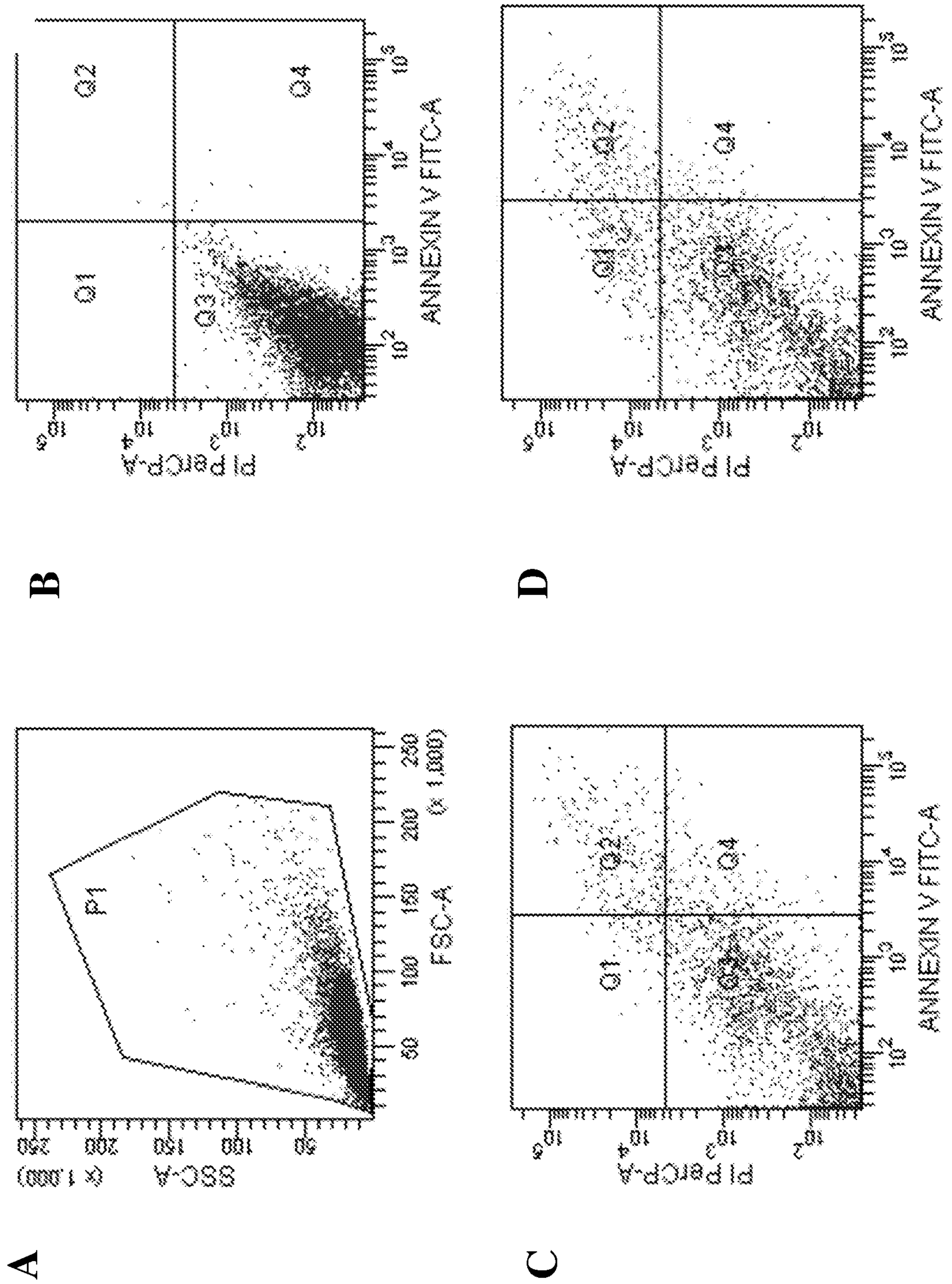
A
B
C
D
SSC-A (x 1,000)
FSC-A (x 1,000)
P1
Q1
Q2
Q3
Q4
PI PerCP-A
ANNEXIN V FITC-A

Figure 69

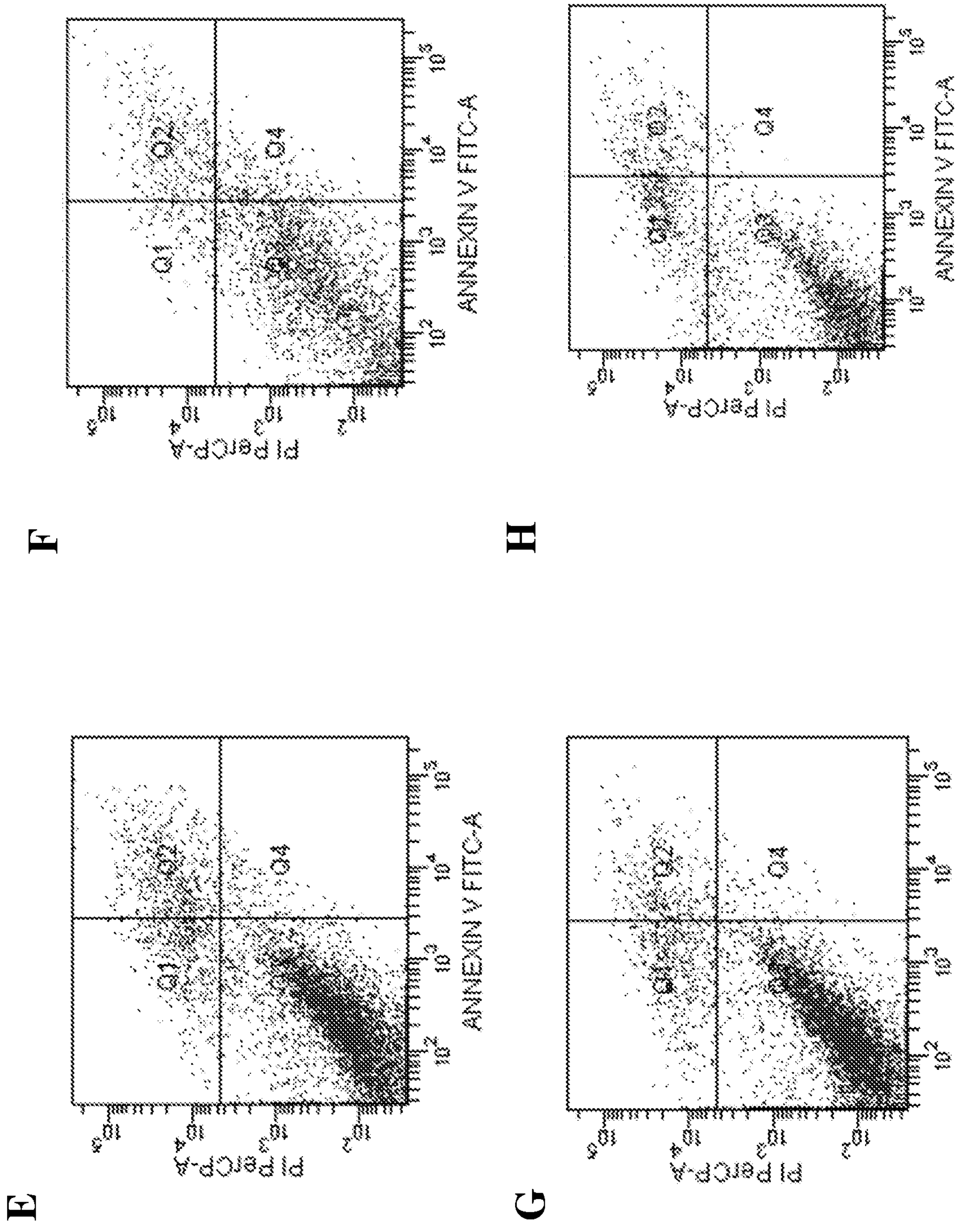


Figure 69 (Continued)

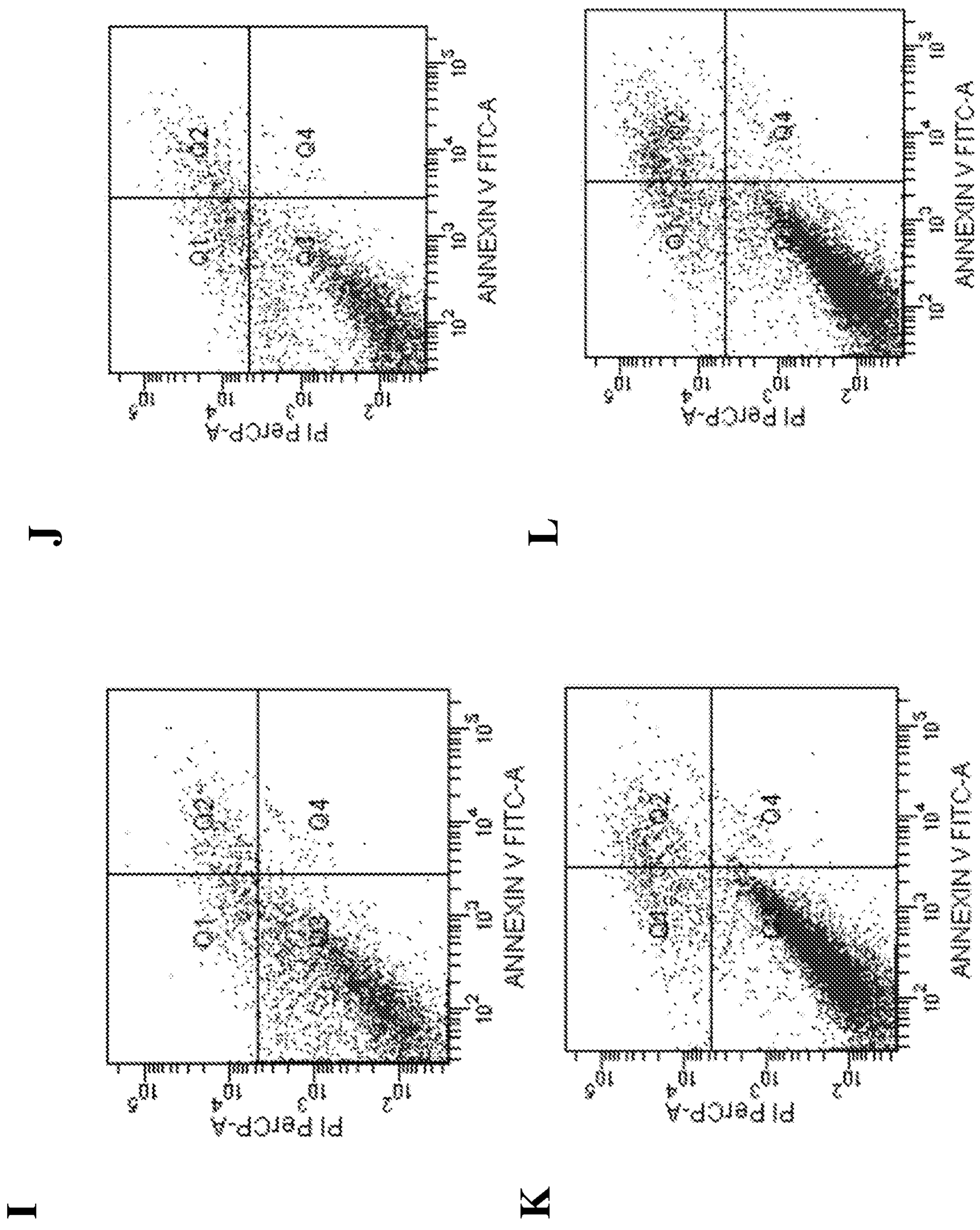


Figure 69 (Continued)

M

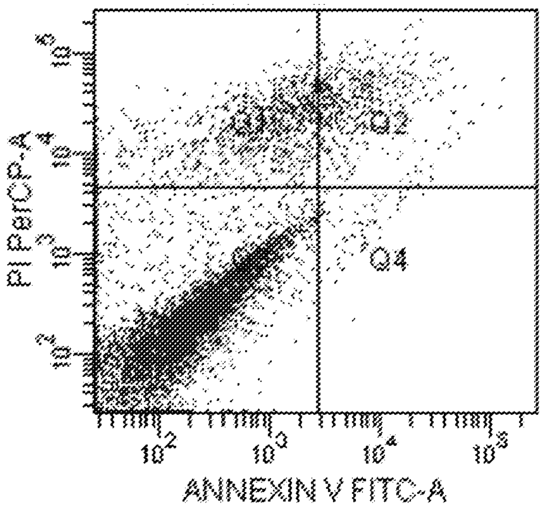


N

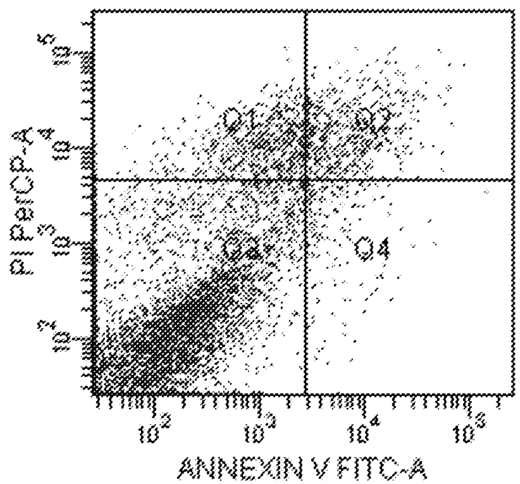


**Figure 69**
**(Continued)**

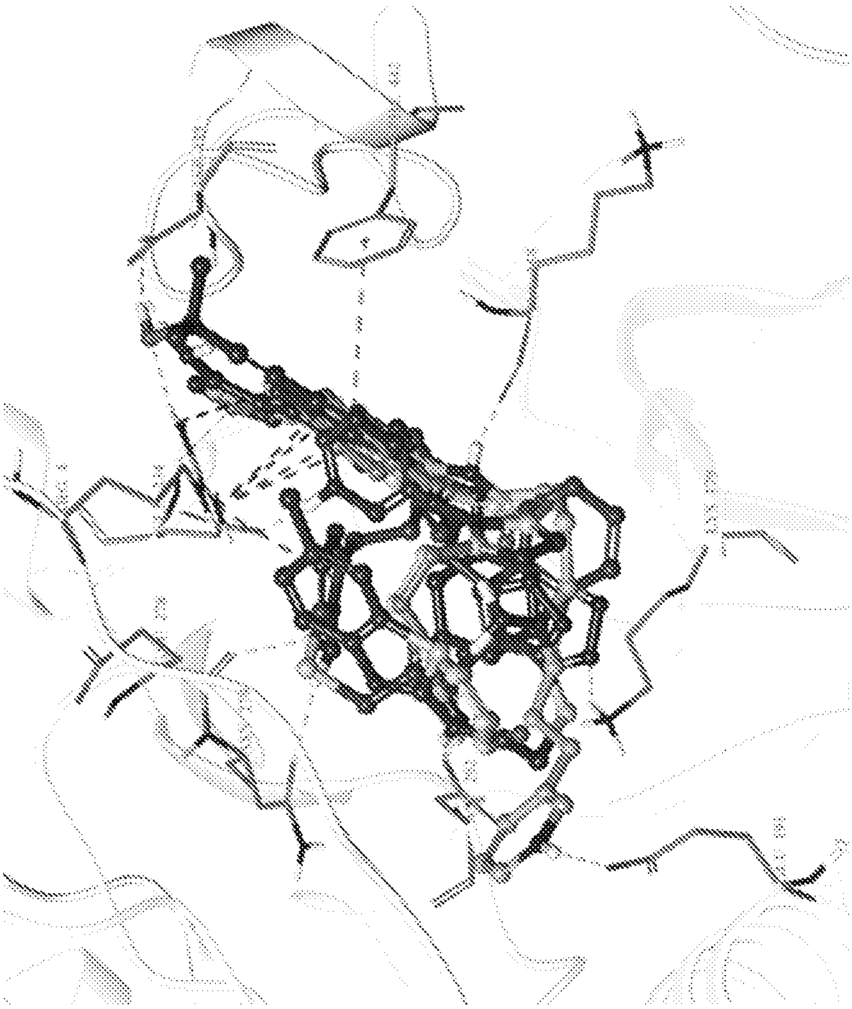

Figure 70

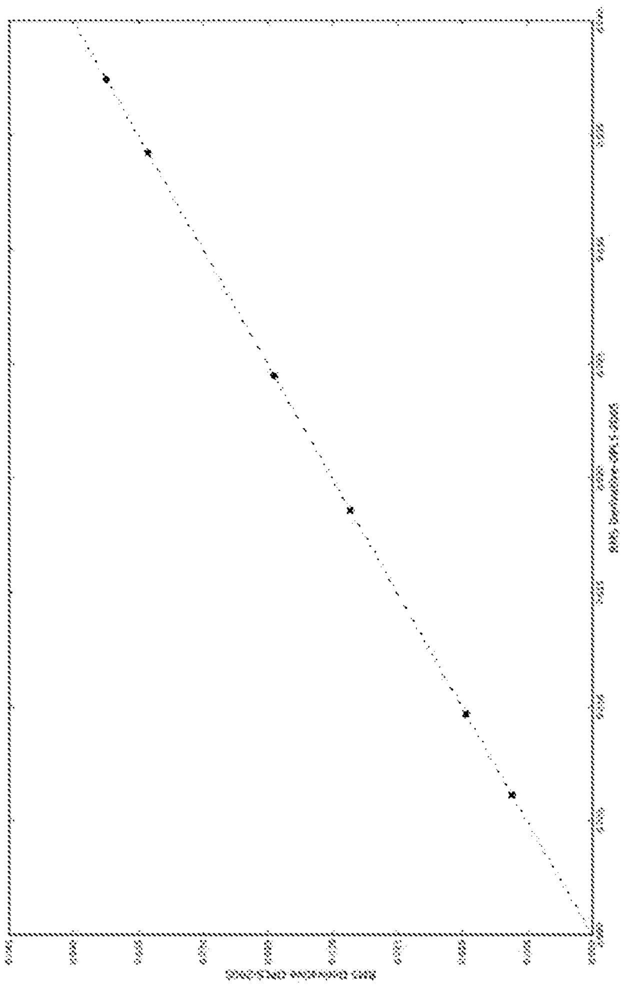

Figure 71

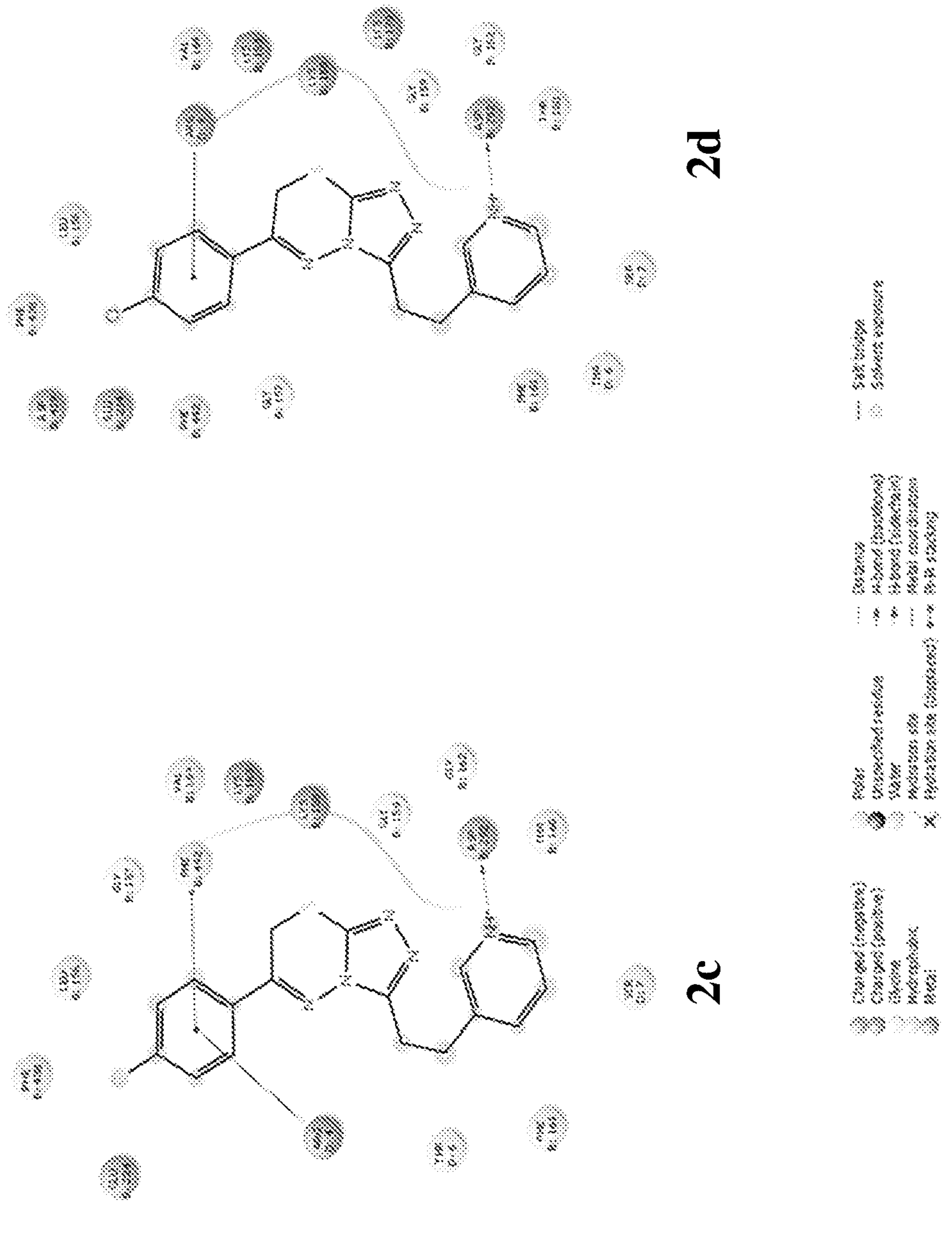

2c

2d

Figure 72

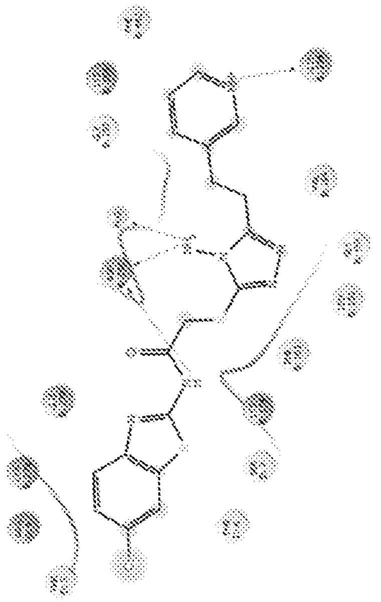

3d

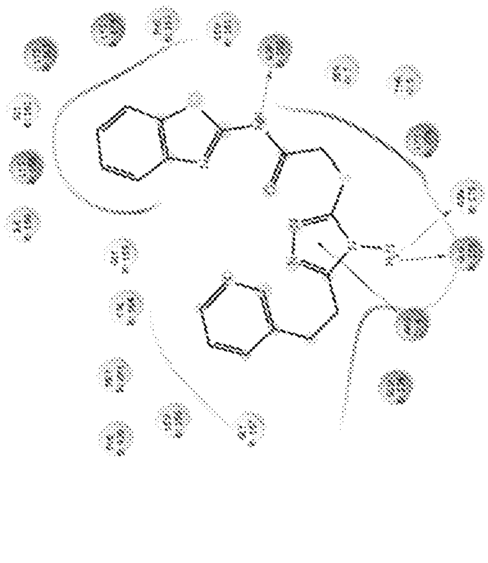

3b

Figure 73

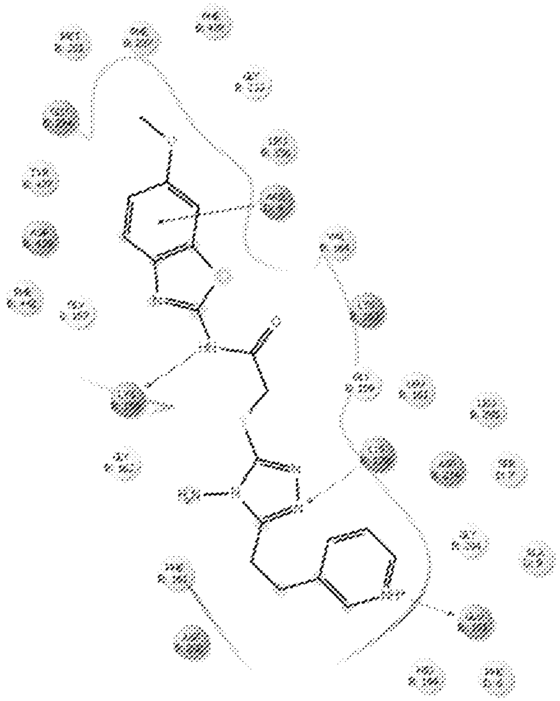

3g

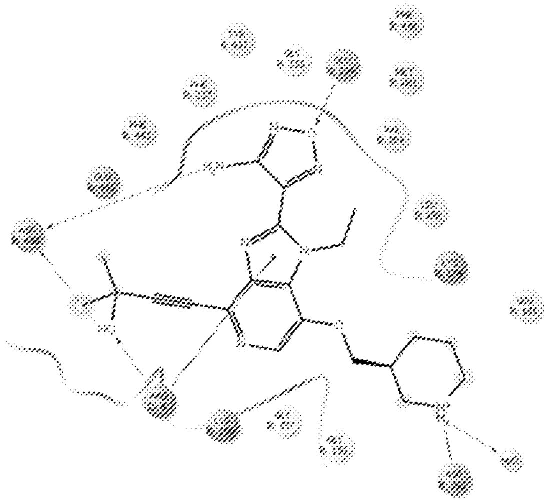

GSK690693

Charged (negative)
Charged (positive)
Glycine
Hydrophobic
Metal
Polar
Unspecified residue
Water
Hydration site
Hydration site (displaced)
Distance
H-bond (backbone)
H-bond (sidechain)
Metal coordination
Pi-Pi stacking
Salt bridge
Solvent exposure

**Figure 73**
**(Continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007064797 A


### Non-patent literature cited in the description


- **DURAN, A et al.** *Il Farmaco,* 2002, vol. 57, 559-564
- **SHIVARAMA HOLLA, B. et al.** *Eur. J. Med. Chem.,* 2003, vol. 38, 759-767
- **SUNIL, D. et al.** *Med. Chem. Res,* 2011, vol. 20, 1024-1032
- **EL-SAYED, W.A. et al.** *Der PharmaChemica,* 2012, vol. 4 (1), 23-32
- **SINGHA, T. et al.** *Ind. J. Pharm. Educ. Res,* 2012, vol. 46, 346-351
- **HASSAN, G.S. et al.** *Eur. J. Med. Chem.,* 2013, vol. 66, 135-145
- **SUMANGALA, V. et al.** *Med. Chem. Res,* 2013, vol. 22, 2921-2928
- **CIHAN-ÜSTÜNDA G.** *Lett. Drug Des. Discov,* 2014, vol. 11, 290-296
- **2015.** *J. Enzyme Inhib. Med. Chem,* vol. 30 (5), 778-785
- **ZHAO, P.L et al.** *Bioorg. Med. Chem. Lett.,* 2016, vol. 26, 3679-3683
- **KULABAS, N et al.** *Eur. J. Med. Chem.,* 2016, vol. 121, 58-70
- **MILOSEV, M.Z.** *Chem. Biol. Drug Des.,* 2017, vol. 89, 943-952
- **SHIVARAMA HOLLA, B. et al.** *Il Farmaco,* 2001, vol. 56, 565-570
- **SHIVARAMA HOLLA, B et al.** *Eur. J. Med. Chem.,* 2006, vol. 41 (5), 657-663
- **BHAT, K.S. et al.** *Eur. J. Med. Chem.,* 2009, vol. 44, 5066-5070
- **PUTHIYAPURAYIL, P et al.** *Eur. J. Med. Chem.,* 2012, vol. 57, 407-416
- **SUMANGALA, V et al.** *Eur. J. Med. Chem.,* 2012, vol. 54, 59-64
- **KAMEL, M.M ; MEGALLY ABDO, N.Y.** *Eur. J. Med. Chem.,* 2014, vol. 86, 75-80
- **KHAN, I. et al.** *Bioorg. Med. Chem,* 2014, vol. 22, 6163-6173
- **KHAN I. et al.** *Eur. J. Med. Chem.,* 2014, vol. 78, 167-177
- **SEVER, B et al.** *Eur. J. Med. Chem.,* 2016, vol. 113, 179-186
- **AYTAC, S.P et al.** *Bioorg. Med. Chem.,* 2016, vol. 24 (4), 858-872
- **IBRAR, A. et al.** *Arch. Pharm. Chem. Life Sci.,* 2016, vol. 349, 553-565
- **RADWAN, R.R. et al.** *Chem. Biol. Interact.,* 2017, vol. 274, 68-79
- **ANSARI, M. et al.** *Bioorg. Chem.,* 2019, vol. 93, 103300
- **ARANDKAR, V. ; VEDULA.** *Phosphorus Sulfur Silicon Relat. Elem,* 2019, vol. 194 (4-6), 533-539